# EUROPEAN PATENT APPLICATION

(11) **EP 3 037 547 A1**
(43) Date of publication of application: **29.06.2016**
(21) Application number: 14838178.3
(22) Date of filing: 20.08.2014
(51) Int. Cl.: C12Q 1/68, G01N 33/574, C12N 15/09

(54) **NEW FUSION GENE DETECTED IN LUNG CANCER**

(30) Priority: 20.08.2013 US 201361867921 P
(71) Applicant: National Cancer Center, Tokyo 104-0045 (JP); National Center for Global Health and Medicine, Tokyo 162-8655 (JP); LSIP, LLC, Chiyoda-ku Tokyo 100-0005 (JP)
(72) Inventor: KOHNO, Takashi, Tokyo 104-0045 (JP); TSUTA, Koji, Tokyo 104-0045 (JP); YASUDA, Kazuki, Tokyo 162-8655 (JP)
(74) Representative: Cabinet Plasseraud
(86) International application number: PCT/JP2014/071720
(87) International publication number: WO 2015/025866

(57) **Abstract**

A method for detecting a gene fusion serving as a responsible mutation (driver mutation) for cancer, the method comprising the step of detecting any one of an EZR-ERBB4 fusion polynucleotide, a KIAA1468-RET fusion polynucleotide, a TRIM24-BRAF fusion polynucleotide, a CD74-NRG1 fusion polynucleotide, and an SLC3A2-NRG1 fusion polynucleotide, or a polypeptide encoded thereby, in an isolated sample from a subject with cancer.

## Description

### TECHNICAL FIELD

The present invention relates mainly to a method for detecting gene fusions serving as responsible mutations for cancer, and a method for identifying patients with cancer or subjects with a risk of cancer, in which substances suppressing the expression and/or activity of polypeptides encoded by fusion polynucleotides produced by said gene fusions show a therapeutic effect.

### BACKGROUND ART

Cancer is the first-ranked disease among causes of death in Japan, and its therapies are in need of improvement. In particular, lung cancer is at the top of the causes of cancer death not only in Japan but also throughout the world, causing over a million deaths each year. Lung cancer is broadly divided into small-cell lung carcinoma and non-small-cell lung carcinoma, and the non-small-cell lung carcinoma is subdivided into three subgroups: lung adenocarcinoma (LADC), lung squamous cell carcinoma, and large-cell carcinoma. Among these subgroups, LADC accounts for about 50% of all cases of non-small-cell lung carcinoma, and besides its frequency is elevated (Non Patent Literature 1).

It has been found that a considerable proportion of LADCs develop through activation of oncogenes. It has also been revealed that when oncogenes are activated, somatic mutations in the EGFR gene (10-40%) or the KRAS gene (10-20%), fusion between the ALK gene and the EML4 (echinoderm microtubule-associated protein-like 4) gene, fusion between the ALK gene and the KIF5B gene (5%), or other alterations occur in a mutually exclusive way (Non Patent Literatures 2-6).

Advanced lung cancers are mainly treated with drugs, but individual patients exhibit greatly different responses to a drug, so there is needed a means for predicting what drug is therapeutically effective in each case. Thus, identification of molecules that can serve as indicators for such predictions, including mutant genes and fusion genes, is in progress as mentioned above; for example, it has been shown that tyrosine kinase inhibitors targeting EGFR and ALK proteins are particularly effective for the treatment of LADCs harboring EGFR mutations and/or ALK fusions. Further, a technique for detecting a fusion of the ALK tyrosine kinase gene as observed in 4-5% of lung cancer cases has been developed as a method to screen for cases indicated for an inhibitor against ALK protein tyrosine kinase, and a reagent for detecting a fusion of the ALK tyrosine kinase gene has been used as a diagnostic agent in clinical settings.

Meanwhile, the present inventors have identified in-frame fusion transcripts between the KIF5B (kinesin family 5B) gene and the RET gene, an oncogene encoding a receptor tyrosine kinase, by performing whole-transcriptome sequencing of LADCs (Patent Literature 1 and Non Patent Literature 7). The KIF5B-RET gene fusion occurred mutually exclusively with known oncogene-activating mutations such as EGFR or KRAS mutations or ALK fusions, and thus were found to be responsible mutations for oncogenesis. A fusion protein produced by said gene fusion may dimerize via the coiled-coil domain of the KIF5B portion without the need for a substrate, resulting in aberrant activation of RET kinase. Therefore, it is expected that RET tyrosine kinase inhibitors may be effective in patients with said gene fusion.

### CITATION LIST

### PATENT LITERATURE

[Patent Literature 1] International Patent Publication No. WO 2013/018882

### NON PATENT LITERATURE

[Non Patent Literature 1] Herbst, R. S., et al., The New England Journal of Medicine, 2008, 359, 1367-1380
[Non Patent Literature 2] Paez, J. G., et al., Science, 2004, 304, 1497-1500
[Non Patent Literature 3] Takeuchi, K., et al., Clin. Cancer Res., 2009, 15, 3143-3149
[Non Patent Literature 4] Soda, M., et al., Nature, 2007, 448, 561-566
[Non Patent Literature 5] Janku, F., et al., Nat. Rev. Clin. Oncol., 2010, 7, 401-414
[Non Patent Literature 6] Lovly, C. M., et al., Nat. Rev. Clin. Onco.l, 2011, 8, 68-70
[Non Patent Literature 7] Kohno, T., et al., Nat. Medicine, 2012, 18 (3), 375-377

### SUMMARY OF INVENTION

### TECHNICAL PROBLEM

Identification of mutations in various cancers including lung cancer has not yet been made thoroughly, and there is still a demand for further identification of mutations that can serve as indicators for predicting the effectiveness of drug treatments.

Accordingly, the objects of the present invention include but are not limited to the following: to identify mutations that can serve as indicators for predicting the effectiveness of drug treatments in various cancers including lung cancer; to provide a means for detecting said mutations; and to provide a means for identifying, based on said mutations, patients with cancer or subjects with a risk of cancer, in which drugs targeting genes having said mutations or proteins encoded by said genes show a therapeutic effect.

### SOLUTION TO PROBLEM

As the result of conducting intensive studies with a view to achieving the above-mentioned objects, the present inventors have found with the use of the whole-transcriptome sequencing method that the EZR-ERBB4, KIAA1468-RET, TRIM24-BRAF, CD74-NRG1, and SLC3A2-NRG1 gene fusions exist independently in lung cancers. The specimens positive for these gene fusions were negative for the following known responsible mutations (driver mutations) for lung cancer: EGFR point mutation/EGFR in-flame deletion mutation, KRAS point mutation, BRAF point mutation, HER2 in-flame insertion mutation, EML4-ALK fusion, KIF5B-RET fusion, CCDC6-RET fusion, CD74-ROS1 fusion, EZR-ROS1 fusion, and SLC34A2-ROS1 fusion; this fact showed that the above-mentioned five gene fusions are responsible mutations for oncogenesis.

It was estimated that the proteins produced by the EZR-ERBB4 and KIAA1468-RET gene fusions would dimerize via the coiled-coil domain without the need for a substrate, thereby becoming constitutively active, as in the case of known membrane tyrosine kinase fusion proteins (Kohno, T., et al., Nat. Medicine, 2012, 18 (3), 375-377). It was also presumed that the protein produced by the TRIM24-BRAF fusion would become constitutively active due to lack of the kinase inhibition domain located toward the N-terminus of the BRAF protein, as in the case of another already-identified BRAF fusion gene (Palanisamy N., et al., Nat. Medicine, 2010, 16 (7), 793-798). Therefore, it is considered that ERBB4, RET and BRAF kinase inhibitors, respectively, would produce a therapeutic effect on cancers positive for the above-mentioned three gene fusions.

Further, it was estimated that the proteins produced by the CD74-NRG1 and SLC3A2-NRG1 gene fusions would be highly expressed when gene fusion takes place, working positively for cell growth as well as survival by an autocrine mechanism, as in the case of already-identified NRG1 fusion proteins (Adélaïde J., et al., GENES, CHROMOSOMES & GANCER, 2003, 37, 333-345; and Wilson T. R., et al., Cancer Cell, 2011, 20, 158-172). Therefore, an antibody drug against the cell growth factor NRG1, or antibody drugs or kinase inhibitors against a group of HER proteins serving as NRG1 receptors could produce a therapeutic effect on cancers positive for these gene fusions.

Under these circumstances, the present inventors have made further studies and, as a result, have found that in the field of cancers such as lung cancer, patients with cancers or subjects with a risk of cancers, in which drugs targeting the above-mentioned genes or proteins encoded by said genes show a therapeutic effect, can be identified based on the above-mentioned gene fusions; and, thus, the inventors have completed the present invention.

More specifically, this invention is as follows.
[1] A method for detecting a gene fusion serving as a responsible mutation (driver mutation) for cancer, the method comprising the step of detecting a fusion polynucleotide of any one of (a) to (e) mentioned below, or a polypeptide encoded thereby, in an isolated sample from a subject with cancer:
   (a) an EZR-ERBB4 fusion polynucleotide which encodes a polypeptide comprising all or part of the coiled-coil domain of EZR, and the kinase domain of ERBB4, and having kinase activity;
   (b) a KIAA1468-RET fusion polynucleotide which encodes a polypeptide comprising all or part of the coiled-coil domain of KIAA1468, and the kinase domain of RET, and having kinase activity;
   (c) a TRIM24-BRAF fusion polynucleotide which encodes a polypeptide comprising the kinase domain of BRAF and having kinase activity;
   (d) a CD74-NRG1 fusion polynucleotide which encodes a polypeptide comprising the transmembrane domain of CD74 and the EGF domain of NRG1, and having intracellular signaling-enhancing activity; and
   (e) an SLC3A2-NRG1 fusion polynucleotide which encodes a polypeptide comprising the transmembrane domain of SLC3A2 and the EGF domain of NRG1, and having intracellular signaling-enhancing activity.
[2] The method according to [1], wherein the fusion polynucleotide is any one of (a) to (e) mentioned below:
   (a) an EZR-ERBB4 fusion polynucleotide encoding the polypeptide mentioned below:
      (i) a polypeptide consisting of the amino acid sequence of SEQ ID NO: 2,
      (ii) a polypeptide consisting of an amino acid sequence derived from the amino acid sequence of SEQ ID NO: 2 by deletion, substitution or addition of one or more amino acids, and the polypeptide having kinase activity, or
      (iii) a polypeptide consisting of an amino acid sequence having a sequence identity of at least 80% to the amino acid sequence of SEQ ID NO: 2, and the polypeptide having kinase activity;
   (b) a KIAA1468-RET fusion polynucleotide encoding the polypeptide mentioned below:
      (i) a polypeptide consisting of the amino acid sequence of SEQ ID NO: 4,
      (ii) a polypeptide consisting of an amino acid sequence derived from the amino acid sequence of SEQ ID NO: 4 by deletion, substitution or addition of one or more amino acids, and the polypeptide having kinase activity, or
      (iii) a polypeptide consisting of an amino acid sequence having a sequence identity of at least 80% to the amino acid sequence of SEQ ID NO: 4, and the polypeptide having kinase activity;
   (c) a TRIM24-BRAF fusion polynucleotide encoding the polypeptide mentioned below:
      (i) a polypeptide consisting of the amino acid sequence of SEQ ID NO: 6,
      (ii) a polypeptide consisting of an amino acid sequence derived from the amino acid sequence of SEQ ID NO: 6 by deletion, substitution or addition of one or more amino acids, and the polypeptide having kinase activity, or
      (iii) a polypeptide consisting of an amino acid sequence having a sequence identity of at least 80% to the amino acid sequence of SEQ ID NO: 6, and the polypeptide having kinase activity;
   (d) a CD74-NRG1 fusion polynucleotide encoding the polypeptide mentioned below:
      (i) a polypeptide consisting of the amino acid sequence of SEQ ID NO: 8 or 10,
      (ii) a polypeptide consisting of an amino acid sequence derived from the amino acid sequence of SEQ ID NO: 8 or 10 by deletion, substitution or addition of one or more amino acids, and the polypeptide having intracellular signaling-enhancing activity, or
      (iii) a polypeptide consisting of an amino acid sequence having a sequence identity of at least 80% to the amino acid sequence of SEQ ID NO: 8 or 10, and the polypeptide having intracellular signaling-enhancing activity; and
   (e) an SLC3A2-NRG1 fusion polynucleotide encoding the polypeptide mentioned below:
      (i) a polypeptide consisting of the amino acid sequence of SEQ ID NO: 36,
      (ii) a polypeptide consisting of an amino acid sequence derived from the amino acid sequence of SEQ ID NO: 36 by deletion, substitution or addition of one or more amino acids, and the polypeptide having intracellular signaling-enhancing activity, or
      (iii) a polypeptide consisting of an amino acid sequence having a sequence identity of at least 80% to the amino acid sequence of SEQ ID NO: 36, and the polypeptide having intracellular signaling-enhancing activity.
[3] The method according to [1] or [2], wherein the fusion polynucleotide is any one of (a) to (e) mentioned below:
   (a)
      (i) a polynucleotide consisting of the nucleotide sequence of SEQ ID NO: 1,
      (ii) a polynucleotide that hybridizes under stringent conditions with a polynucleotide consisting of a nucleotide sequence complementary to the polynucleotide consisting of the nucleotide sequence of SEQ ID NO: 1, and which encodes a polypeptide having kinase activity,
      (iii) a polynucleotide that consists of a nucleotide sequence derived from the nucleotide sequence of SEQ ID NO: 1 by deletion, substitution or addition of one or more nucleotides, and which encodes a polypeptide having kinase activity, or
      (iv) a polynucleotide that has a sequence identity of at least 80% to the polynucleotide consisting of the nucleotide sequence of SEQ ID NO: 1, and which encodes a polypeptide having kinase activity;
   (b)
      (i) a polynucleotide consisting of the nucleotide sequence of SEQ ID NO: 3,
      (ii) a polynucleotide that hybridizes under stringent conditions with a polynucleotide consisting of a nucleotide sequence complementary to the polynucleotide consisting of the nucleotide sequence of SEQ ID NO: 3, and which encodes a polypeptide having kinase activity,
      (iii) a polynucleotide that consists of a nucleotide sequence derived from the nucleotide sequence of SEQ ID NO: 3 by deletion, substitution or addition of one or more nucleotides, and which encodes a polypeptide having kinase activity, or
      (iv) a polynucleotide that has a sequence identity of at least 80% to the polynucleotide consisting of the nucleotide sequence of SEQ ID NO: 3, and which encodes a polypeptide having kinase activity;
   (c)
      (i) a polynucleotide consisting of the nucleotide sequence of SEQ ID NO: 5,
      (ii) a polynucleotide that hybridizes under stringent conditions with a polynucleotide consisting of a nucleotide sequence complementary to the polynucleotide consisting of the nucleotide sequence of SEQ ID NO: 5, and which encodes a polypeptide having kinase activity,
      (iii) a polynucleotide that consists of a nucleotide sequence derived from the nucleotide sequence of SEQ ID NO: 5 by deletion, substitution or addition of one or more nucleotides, and which encodes a polypeptide having kinase activity, or
      (iv) a polynucleotide that has a sequence identity of at least 80% to the polynucleotide consisting of the nucleotide sequence of SEQ ID NO: 5, and which encodes a polypeptide having kinase activity;
   (d)
      (i) a polynucleotide consisting of the nucleotide sequence of SEQ ID NO: 7 or 9,
      (ii) a polynucleotide that hybridizes under stringent conditions with a polynucleotide consisting of a nucleotide sequence complementary to the polynucleotide consisting of the nucleotide sequence of SEQ ID NO: 7 or 9, and which encodes a polypeptide having intracellular signaling-enhancing activity,
      (iii) a polynucleotide that consists of a nucleotide sequence derived from the nucleotide sequence of SEQ ID NO: 7 or 9 by deletion, substitution or addition of one or more nucleotides, and which encodes a polypeptide having intracellular signaling-enhancing activity, or
      (iv) a polynucleotide that has a sequence identity of at least 80% to the polynucleotide consisting of the nucleotide sequence of SEQ ID NO: 7 or 9, and which encodes a polypeptide having intracellular signaling-enhancing activity; and
   (e)
      (i) a polynucleotide consisting of the nucleotide sequence of SEQ ID NO: 35,
      (ii) a polynucleotide that hybridizes under stringent conditions with a polynucleotide consisting of a nucleotide sequence complementary to the polynucleotide consisting of the nucleotide sequence of SEQ ID NO: 35, and which encodes a polypeptide having intracellular signaling-enhancing activity,
      (iii) a polynucleotide that consists of a nucleotide sequence derived from the nucleotide sequence of SEQ ID NO: 35 by deletion, substitution or addition of one or more nucleotides, and which encodes a polypeptide having intracellular signaling-enhancing activity, or
      (iv) a polynucleotide that has a sequence identity of at least 80% to the polynucleotide consisting of the nucleotide sequence of SEQ ID NO: 35, and which encodes a polypeptide having intracellular signaling-enhancing activity.
[4] The method according to any one of [1] to [3], wherein the cancer is lung cancer.
[5] A method for identifying a patient with cancer or a subject with a risk of cancer, in which a substance suppressing the expression and/or activity of a polypeptide encoded by a fusion polynucleotide produced by a gene fusion serving as a responsible mutation (driver mutation) for cancer shows a therapeutic effect, the method comprising the steps of:
   (1) detecting a fusion polynucleotide of any one of (a) to (e) mentioned below, or a polypeptide encoded thereby, in an isolated sample from a subject:
      (a) an EZR-ERBB4 fusion polynucleotide which encodes a polypeptide comprising all or part of the coiled-coil domain of EZR, and the kinase domain of ERBB4, and having kinase activity,
      (b) a KIAA1468-RET fusion polynucleotide which encodes a polypeptide comprising all or part of the coiled-coil domain of KIAA1468, and the kinase domain of RET, and having kinase activity,
      (c) a TRIM24-BRAF fusion polynucleotide which encodes a polypeptide comprising the kinase domain of BRAF and having kinase activity,
      (d) a CD74-NRG1 fusion polynucleotide which encodes a polypeptide comprising the transmembrane domain of CD74 and the EGF domain of NRG1, and having intracellular signaling-enhancing activity, and
      (e) an SLC3A2-NRG1 fusion polynucleotide which encodes a polypeptide comprising the transmembrane domain of SLC3A2 and the EGF domain of NRG1, and having intracellular signaling-enhancing activity; and
   (2) determining that the substance suppressing the expression and/or activity of the polypeptide shows a therapeutic effect in the subject, in the case where the fusion polynucleotide of any one of (a) to (e) or the polypeptide encoded thereby is detected.
[6] A kit for detecting a gene fusion serving as a responsible mutation (driver mutation) for cancer, the kit comprising any one of (A) to (C) mentioned below, or a combination thereof:
   (A) a polynucleotide that serves as a probe designed to specifically recognize an EZR-ERBB4 fusion polynucleotide, a KIAA1468-RET fusion polynucleotide, a TRIM24-BRAF fusion polynucleotide, a CD74-NRG1 fusion polynucleotide, or an SLC3A2-NRG1 fusion polynucleotide;
   (B) polynucleotides that serve as a pair of primers designed to enable specific amplification of an EZR-ERBB4 fusion polynucleotide, a KIAA1468-RET fusion polynucleotide, a TRIM24-BRAF fusion polynucleotide, a CD74-NRG1 fusion polynucleotide, or an SLC3A2-NRG1 fusion polynucleotide; and
   (C) an antibody that specifically recognizes an EZR-ERBB4 fusion polypeptide, a KIAA1468-RET fusion polypeptide, a TRIM24-BRAF fusion polypeptide, a CD74-NRG1 fusion polypeptide, or an SLC3A2-NRG1 fusion polypeptide.
[7] An isolated EZR-ERBB4 fusion polypeptide or a fragment thereof, which comprises all or part of the coiled-coil domain of EZR protein, and the kinase domain of ERBB4 protein, and has kinase activity.
[8] An isolated KIAA1468-RET fusion polypeptide or a fragment thereof, which comprises all or part of the coiled-coil domain of KIAA1468 protein, and the kinase domain of RET protein, and has kinase activity.
[9] An isolated TRIM24-BRAF fusion polypeptide or a fragment thereof, which comprises the kinase domain ofBRAF protein and has kinase activity.
[10] An isolated CD74-NRG1 fusion polypeptide or a fragment thereof, which comprises the transmembrane domain of CD74 protein and the EGF domain of NRG1 protein, and has intracellular signaling-enhancing activity.
[11] An isolated SLC3A2-NRG1 fusion polypeptide or a fragment thereof, which comprises the transmembrane domain of SLC3A2 protein and the EGF domain of NRG1 protein, and has intracellular signaling-enhancing activity.
[12] A polynucleotide encoding the fusion polypeptide or the fragment thereof according to any one of [7] to [11].
[13] A method for treatment of cancer, comprising the step of administering a substance suppressing the expression and/or activity of a polypeptide encoded by a fusion polynucleotide produced by a gene fusion serving as a responsible mutation (driver mutation) for cancer, to a subject in which the substance is determined to show a therapeutic effect by the method according to [5].
[14] A method for screening a cancer therapeutic agent, the method comprising the steps of:
   (1) bringing a cell expressing the fusion polypeptide according to any one of [7] to [11] into contact with a test substance;
   (2) judging whether the substance suppresses the expression and/or activity of the fusion polypeptide or not; and
   (3) selecting the substance judged to suppress the expression and/or activity of the fusion polypeptide, as a cancer therapeutic agent.

### ADVANTAGEOUS EFFECTS OF INVENTION

The present invention makes it possible to detect unknown responsible mutations for particular cancers, which have been first identified according to the present invention; to identify, based on the presence of said responsible mutations, patients with said cancers or subjects with a risk of the cancers, in which cancer treatments take effect; and to treat said patients.

### BRIEF DESCRIPTION OF DRAWINGS

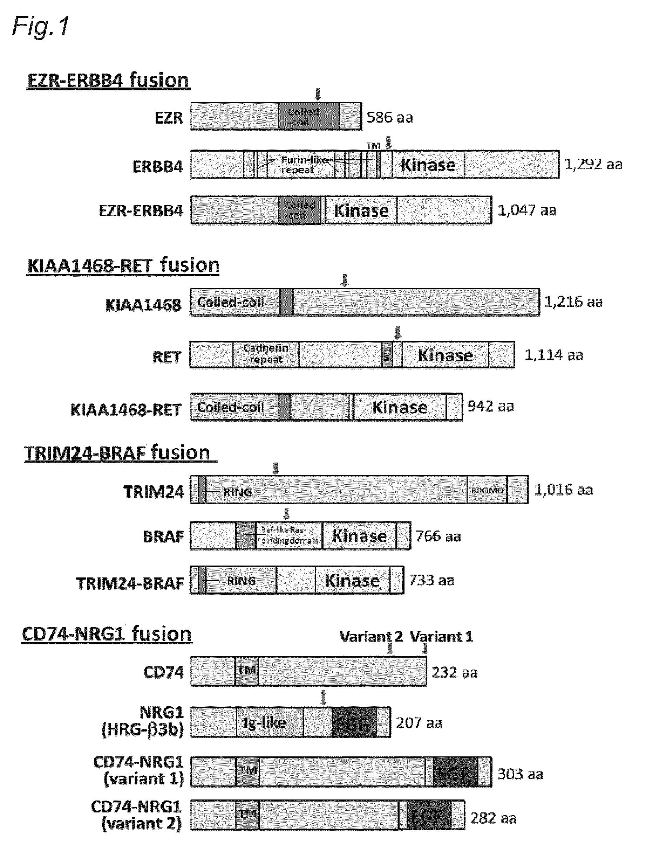

Fig. 1 depicts a schematic drawing showing examples of the domain structures of EZR-ERBB4, KIAA1468-RET, TRIM24-BRAF, and CD74-NRG1 fusion proteins. Down-pointing arrows indicate points of fusion.

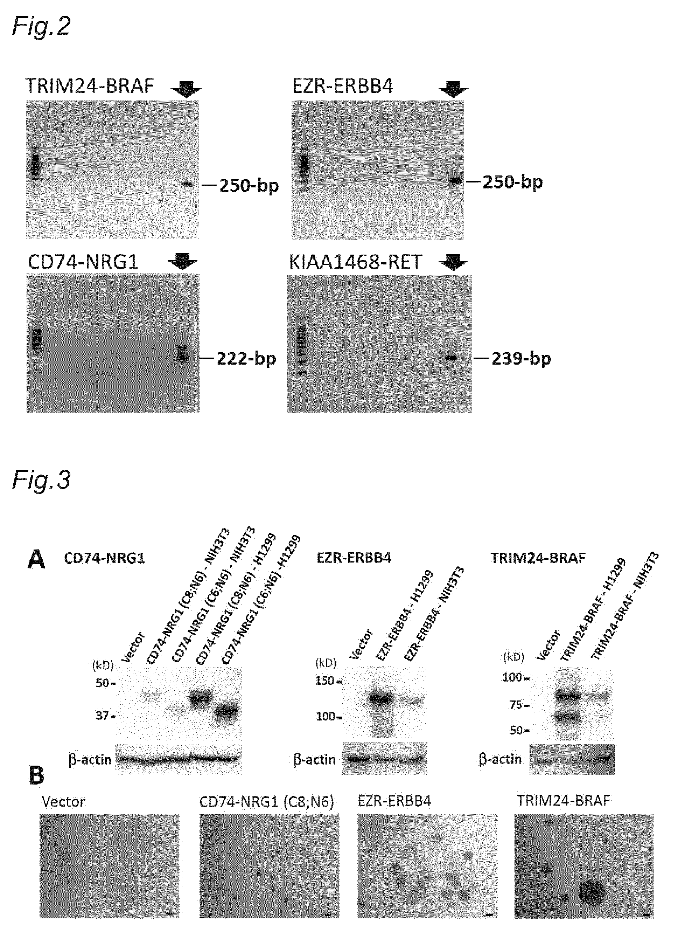

Fig. 2 depicts electrophoresis photos showing the results of detection by RT-PCR of EZR-ERBB4, KIAA1468-RET, TRIM24-BRAF, and CD74-NRG1 (variant 2) gene fusions, with cDNAs synthesized from cancer tissue-derived RNAs being used as templates. Down-pointing arrows indicate gene fusion-positive samples.
Fig. 3 shows the transformation of NIH3T3 cells expressing the cDNA of CD74-NRG1, EZR-ERBB4 or TRIM24-BRAF. (A) Expression of gene fusion products detected by Western blotting analysis in transiently transduced H1299 cells and virally infected NIH3T3 cells. There were used an antibody recognizing NRG1 peptides retained in a fusion protein (catalog No. RB-276, Thermo Scientific), an antibody recognizing ERBB4 peptides retained in a fusion protein (catalog No. 2218-1, Epitomics), and an antibody recognizing BRAF peptides retained in a fusion protein (catalog No. sc-166, Santa Cruz Biotechnology). (B) Photomicrographs showing anchorage-independent colony growth of NIH3T3 cells, which was induced by the expression of the cDNA of CD74-NRG1 (C8;N6), EZR-ERBB4 or TRIM24-BRAF (scale bar: 100 µm).

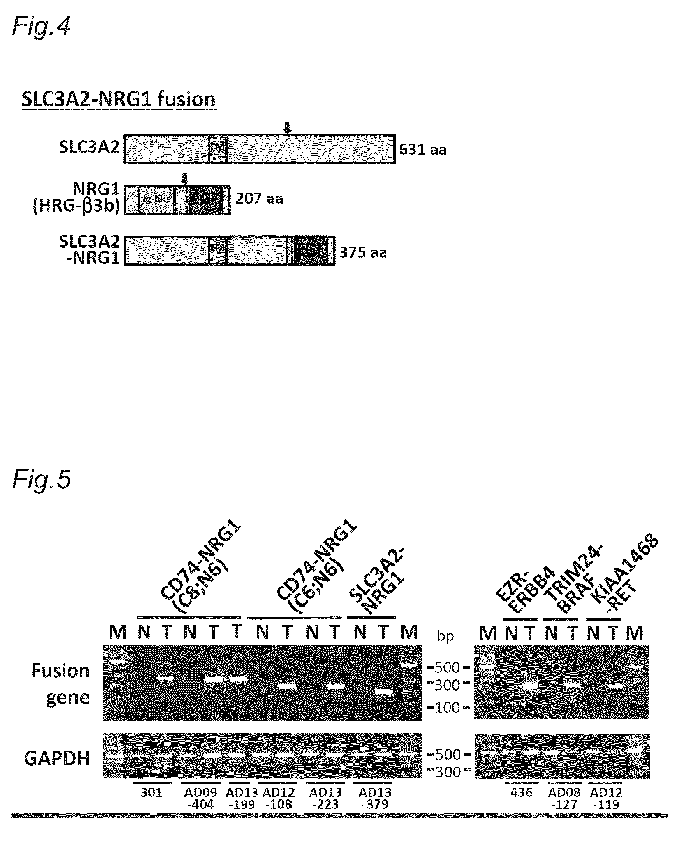

Fig. 4 shows a gene fusion causing oncogenesis in an invasive mucinous lung adenocarcinoma. This figure depicts a schematic drawing showing wild-type proteins and a newly identified fusion protein. Breakpoints are indicated by arrows. TM indicates a transmembrane domain. The location of a putative breakpoint in an NRG1 polypeptide is indicated by a broken line.
Fig. 5 shows the detection of gene fusion transcripts by RT-PCR. RT-PCR products of glyceraldehyde-3-phosphate dehydrogenase (GAPDH) are shown in the lower part of the figure. For each of six gene fusion-positive samples, the gel images of an IMA ("T") and its corresponding non-cancerous lung tissue ("N") are shown side by side. The labels found under the gel images indicate sample IDs (refer to Table 4).

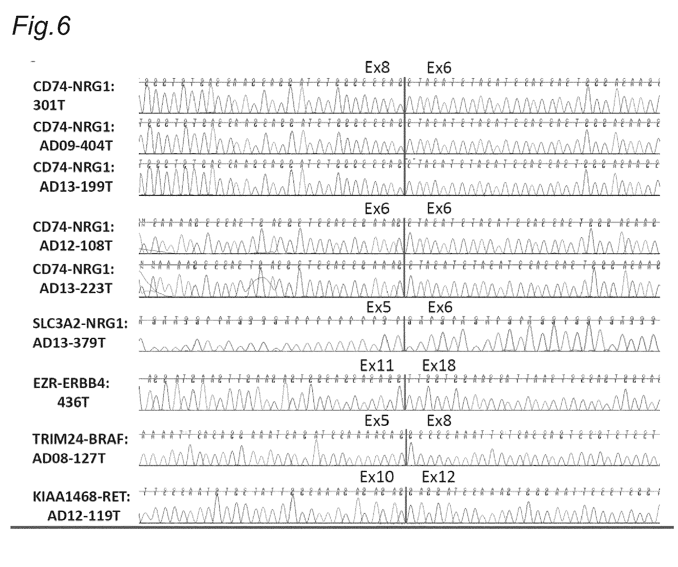

Fig. 6 depicts electropherograms from Sanger sequencing of the cDNAs of novel fusion transcripts, CD74-NRG1 (two variant forms), SLC3A2-NRG1, EZR-ERBB4, TRIM24-BRAF, and KIAA1468-RET fusions. The RT-PCR products were sequenced directly.

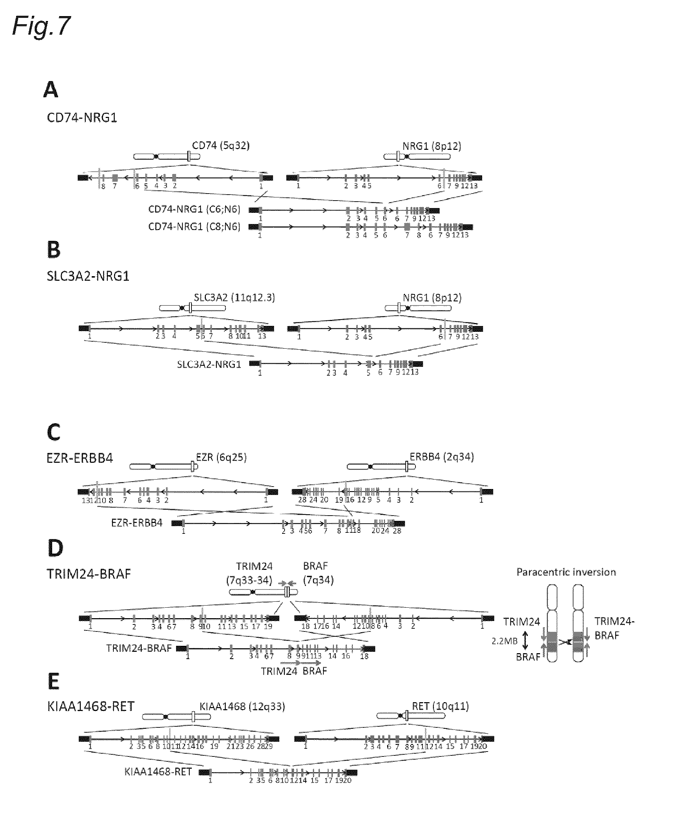

Fig. 7 depicts schematic drawings of the genomic organizations for NRG1, ERBB4, BRAF, and RET rearrangements. This figure shows the genome organizations of wild-type and fusion genes, including exons and untranslated regions, for the CD74-NRG1 (A), SLC3A2-NRG1 (B), EZR-ERBB4 (C), TRIM24-BRAF (D), and KIAA1468-RET (F) fusions. Numbers indicate exon numbers counted from the 5'-end; and arrows indicate transcription directions. The TRIM24 and BRAF genes are located within a region of 2.2 Mb on chromosome 7 in opposite directions. The TRIM24-BRAF fusion was deduced to have been generated through paracentric inversion in region 7q33-34 of the chromosome (B). The locations of breakpoints are indicated by longer vertical lines drawn on wild-type genes.

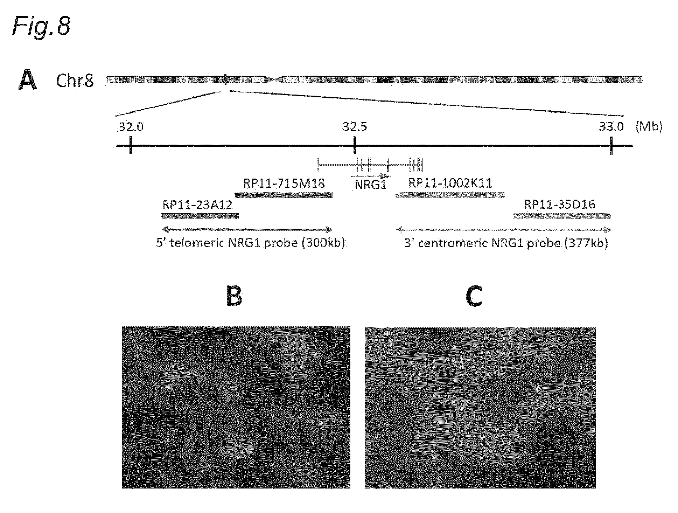

Fig. 8 shows the results of break-apart fluorescence *in situ* hybridization (FISH) of a NRG1 fusion. (A) Genomic organization of the NRG1 gene, and the locations of BAC clones used as probes. (B) Normal tissue. (C) An IMA with the CD74-NRG1 fusion. FISH revealed a separation between green (telomeric) and orange (centromeric) fluorescences derived from the two probes flanking the translocation site.

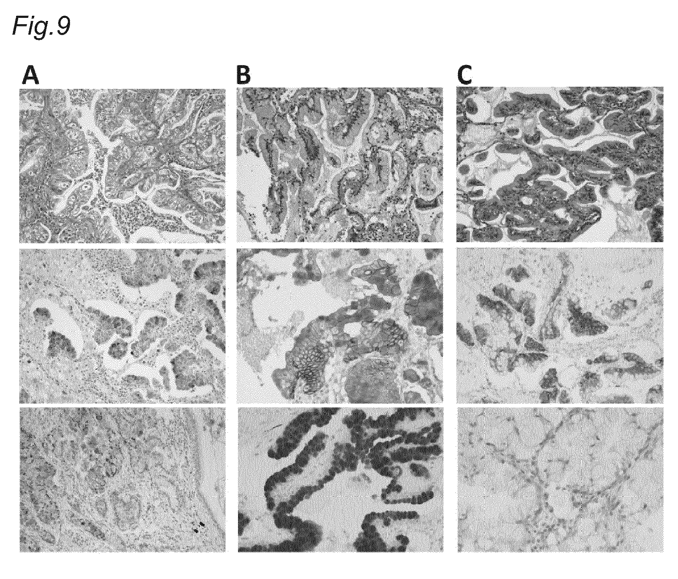

Fig. 9 depicts representative histological images obtained from fusion-positive IMAs. Immunostaining was performed using antibodies recognizing polypeptides retained in fusion proteins. (A) (Upper panel) An IMA with NRG1 rearrangement. The tumor was composed of tall columnar cells with fine eosinophilic cytoplasm and varying amounts of mucin. Nuclear enlargement with fine granular chromatins and dis-alignment of nuclei are visible (original magnification, x20). (Middle panel) NRG1 staining in a CD74-NRG1 fusion-positive IMA. Patchy granular cytoplasmic staining is visible in an adenocarcinoma component (original magnification, x20). (Lower panel) NRG1 staining in a fusion-negative IMA (original magnification, x20). Cytoplasmic granular staining as in fusion-positive tumors was observed in most (more than 80%) of the cases. (B) (Upper panel) An IMA with ERBB4 rearrangement. The tumor was composed of tall columnar cells with basally located small nuclei and mucin located in the upper portion of the cytoplasm (original magnification, x20). (Middle panel) ERBB4 staining in an EZR-ERBB4 fusion-positive IMA. Plasma membranous accentuation with cytoplasmic staining is visible (original magnification, x40). (Lower panel) ERBB4 staining in a fusion-negative IMA (original magnification, x40). Cytoplasmic staining without membranous accentuation was observed in more than 50% of the cases. (C) (Upper panel) An IMA with BRAF rearrangement. The tumor was composed of tall columnar cells with condensed eosinophilic mucin. Nuclear enlargement and overlapping of nuclei are visible (original magnification, x20). (Middle panel) BRAF staining in a TRIM24-BRAF fusion-positive IMA. Diffuse and strong granular cytoplasmic staining is visible in the adenocarcinoma component (original magnification, x20). (Lower panel) BRAF staining in a fusion-negative IMA (original magnification, x40). A subset (less than 10%) of the cases exhibited focal and weak cytoplasmic staining.

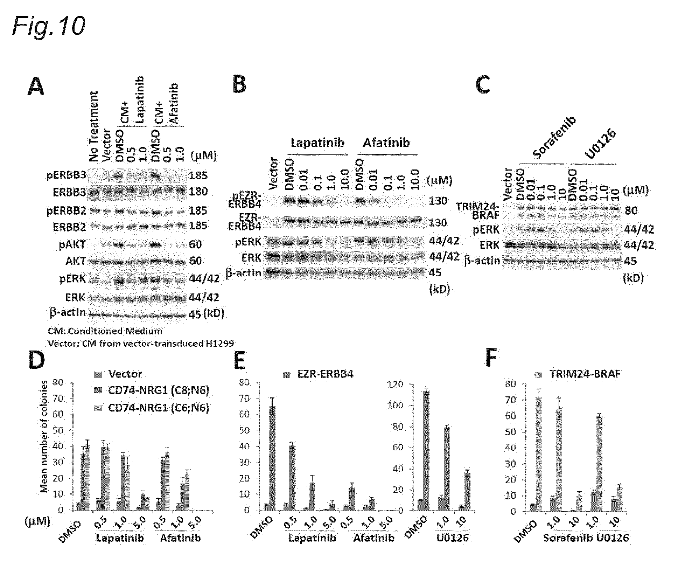

Fig. 10 shows the oncogenic properties of gene fusion products. A: ERBB3 activation by CD74-NRG1 fusion as demonstrated using an EFM-19 cell system. Phosphorylation of ERBB3, ERBB2, AKT, and ERK was examined in EFM-19 (reporter) cells treated for 30 minutes with a conditioned medium from H1299 cells exogenously expressing CD74-NRG1 cDNA. The phosphorylation was suppressed by HER-TKIs. B: ERBB4 activation by EZR-ERBB4 fusion. Stably transduced NIH3T3 cells were serum-starved for 24 hours and treated for 2 hours with DMSO (vehicle control) or TKI. Phosphorylation of ERBB4 and ERK was suppressed by ERBB4-TKI. EZR-ERBB4 protein was detected using an antibody recognizing an ERBB4 polypeptide retained in said fusion protein. C: BRAF activation by TRIM24-BRAF fusion. Stably transduced NIH3T3 cells were serum-starved for 24 hours and treated for 2 hours with DMSO or a kinase inhibitors. ERK phosphorylation (activation) was suppressed by sorafenib, a kinase inhibitor targeting BRAF, or by U0126, a MEK inhibitor. TRIM24-BRAF protein was detected using an antibody recognizing a BRAF polypeptide retained in said fusion protein. D to F: Anchorage-independent growth of NIH3T3 cells expressing the cDNA of CD74-NRG1 (D), EZR-ERBB4 (E), or TRIM24-BRAF (F), and suppression of this growth by a kinase inhibitor. Mock-, CD74-NRG1-, EZR-ERBB4-, and TRIM24-BRAF-transduced NIH3T3 cells were seeded in soft agar with DMSO alone or kinase inhibitors. After 14 days, colonies greater than 100 µm in diameter were counted. Graph bars show mean numbers of colonies ± S.E.M.

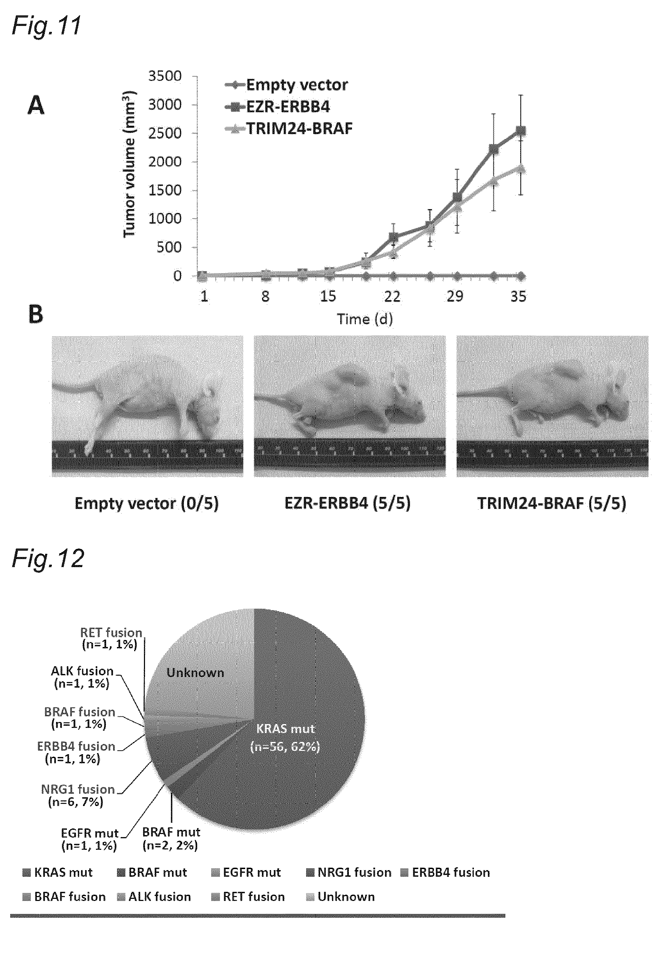

Fig. 11 shows the tumorigenicity of NIH3T3 cells expressing the cDNA of ERZ-ERBB4 or TRIM24-BRAF fusion. A: Tumor growth in nude mice injected with NIH3T3 cells expressing an empty vector, EZR-ERBB4 fusion or TRIM24-BRAF fusion. The cells were resuspended with 50% Matrigel and injected into the right flank of the nude mice. Tumor size measurements were done twice a week for 5 weeks. Data are shown as means ± S.E.M. B: Representative tumors were photographed on day 21. The values in parentheses indicate the ratios of the number of mice with tumors to the number of mice receiving cell injection.
Fig. 12 depicts the circle graph showing the proportions of IMAs with the driver mutations labeled in the graph.

### DESCRIPTION OF EMBODIMENTS

As disclosed below in Examples, the present inventors have first found, in cancer tissues, the following five types of gene fusions serving as responsible mutations (driver mutations) for cancer: EZR-ERBB4, KIAA1468-RET, TRIM24-BRAF, CD74-NRG1, and SLC3A2-NRG1 fusions. On the basis of this finding, the present invention mainly provides a method for detecting said gene fusions; a method for identifying, based on the presence of said responsible mutations, patients with said cancers or subjects with a risk of the cancers, in which cancer treatments take effect; a method for treatment of cancer; a cancer therapeutic agent; and a method for screening a cancer therapeutic agent.

For the purpose of the present invention, the "responsible mutations for cancer" is a term used interchangeably with the "driver mutations", and refers to mutations that are present in cancer tissues and which are capable of inducing oncogenesis of cells. Typically speaking, if a mutation is found in a cancer tissue in which none of known oncogene mutations (at least, EGFR point mutation/EGFR in-flame deletion mutation, KRAS point mutation, BRAF point mutation, HER2 in-flame insertion mutation, ALK-EML4 fusion, KIF5B-RET fusion, CCDC6-RET fusion, CD74-ROS1 fusion, EZR-ROS1 fusion, and SLC34A2-ROS1 fusion) exists (in other words, if a mutation exists mutually exclusively with the known oncogene mutations), then the mutation can be said as a responsible mutation for cancer.

### <Specific responsible mutations for cancer>

Hereafter, the respective gene fusions are explained. For the purpose of the present specification, the "point of fusion" in a fusion polynucleotide refers to a boundary that connects gene segments extending toward the 5'- and 3'-ends, in other words, a boundary between two nucleotide residues. The "point of fusion" in a fusion polypeptide refers to a boundary that connects polypeptides extending toward the N- and the C-terminus, in other words, a boundary between two amino acid residues, or, if a gene fusion occurs in one codon, one amino acid residue *per se* encoded by the codon.

### (1) EZR-ERBB4 fusion

This gene fusion is a mutation that causes expression of a fusion protein between EZR protein and ERBB4 protein (hereinafter also referred to as the "EZR-ERBB4 fusion polypeptide") and which is caused by a translocation (t(2;6)) having breakpoints in regions 6q25 and 2q34 of a human chromosome.

EZR protein is a protein encoded by the gene located on chromosome 6q25 in a human, and is typically a protein consisting of the amino acid sequence of SEQ ID NO: 20 (NCBI accession No. NP_001104547.1 (09-JUN-2013)). EZR protein is characterized by having a coiled-coil domain (Fig. 1), which corresponds to the amino acid sequence at positions 300 to 550 of the amino acid sequence of SEQ ID NO: 20 in a human.

ERBB4 protein is a protein encoded by the gene located on chromosome 2q34 in a human, and is typically a protein consisting of the amino acid sequence of SEQ ID NO: 22 (NCBI accession No. NP_001036064.1 (15-JUN-2013)). ERBB4 protein is characterized by having a kinase domain (Fig. 1), which corresponds to the amino acid sequence at positions 708 to 964 of the amino acid sequence of SEQ ID NO: 22 in a human.

In ERBB4 protein, furin-like repeats and a transmembrane domain (e.g., positions 183 to 665 of the amino acid sequence of SEQ ID NO: 22) are present in a region toward the N-terminus relative to the kinase domain.

The EZR-ERBB4 fusion polypeptide is a polypeptide comprising all or part of the coiled-coil domain of EZR protein, and the kinase domain of ERBB4 protein, and having kinase activity.

The EZR-ERBB4 fusion polypeptide may comprise all of the coiled-coil domain of EZR protein, or may comprise part of the coiled-coil domain as long as the EZR-ERBB4 fusion polypeptide can dimerize. Whether the EZR-ERBB4 fusion polypeptide dimerizes or not can be confirmed by a known method such as gel filtration chromatography or a combination of treatment with a crosslinking agent and SDS-polyacrylamide gel electrophoresis.

The EZR-ERBB4 fusion polypeptide may comprise all of the kinase domain of ERBB4 protein, or may comprise part of the kinase domain as long as the EZR-ERBB4 fusion polypeptide has kinase activity.

The expression that the EZR-ERBB4 fusion polypeptide "has kinase activity" means that said fusion polypeptide is active as an enzyme phosphorylating tyrosine due to the kinase domain derived from ERBB4 protein. The kinase activity of the EZR-ERBB4 fusion polypeptide is determined by a conventional method, and is commonly determined by incubating the fusion polypeptide with a substrate (e.g., synthetic peptide substrate) and ATP under appropriate conditions and then detecting phosphorylated tyrosine in the substrate. The kinase activity can also be measured using a commercially available measurement kit.

The EZR-ERBB4 fusion polypeptide may comprise all or part of the furin-like repeats and the transmembrane domain of ERBB4 protein, but preferably does not comprise any of them.

Although the present invention is not intended to be bound by any particular theory, it is believed that the EZR-ERBB4 fusion polypeptide would dimerize via the coiled-coil domain present in a region toward the N-terminus to undergo autophosphorylation and become constitutively active, thereby contributing to oncogenesis.

In the present invention, the polynucleotide encoding the EZR-ERBB4 fusion polypeptide (hereinafter also referred to as the "EZR-ERBB4 fusion polynucleotide") is a polynucleotide that encodes the polypeptide comprising all or part of the coiled-coil domain of EZR protein, and the kinase domain of ERBB4 protein, and having kinase activity. The EZR-ERBB4 fusion polynucleotide can be any of mRNA, cDNA and genomic DNA.

The EZR-ERBB4 fusion polynucleotide according to the present invention can be, for example, an EZR-ERBB4 fusion polynucleotide encoding the polypeptide mentioned below:
(i) a polypeptide consisting of the amino acid sequence of SEQ ID NO: 2;
(ii) a polypeptide consisting of an amino acid sequence derived from the amino acid sequence of SEQ ID NO: 2 by deletion, substitution or addition of one or more amino acids, and the polypeptide having kinase activity; or
(iii) a polypeptide consisting of an amino acid sequence having a sequence identity of at least 80% to the amino acid sequence of SEQ ID NO: 2, and the polypeptide having kinase activity.

As disclosed below in Examples, the amino acid sequence of SEQ ID NO: 2 is an amino acid sequence encoded by an EZR-ERBB4 fusion polynucleotide found in samples from human cancer tissues. In the amino acid sequence of SEQ ID NO: 2, the point of fusion is located in Arg at position 448.

As used above in (ii), the phrase "one or more amino acids" refers to generally 1 to 50 amino acids, preferably 1 to 30 amino acids, more preferably 1 to 10 amino acids, still more preferably one to several amino acids (for example, 1 to 5 amino acids, 1 to 4 amino acids, 1 to 3 amino acids, 1 or 2 amino acids, or one amino acid).

As used above in (iii), the phrase "sequence identity of at least 80%" refers to a sequence identity of preferably at least 85%, more preferably at least 90% or at least 95%, still more preferably at least 97%, at least 98% or at least 99%. Amino acid sequence identity can be determined using the BLASTX or BLASTP program (Altschul S. F., et al., J. Mol. Biol., 1990, 215: 403) which is based on the BLAST algorithm developed by Karlin and Altschul (Proc. Natl. Acad. Sci. USA, 1990, 87: 2264-2268; *and* Proc. Natl. Acad. Sci. USA, 1993, 90: 5873). In the process of making amino acid sequence analysis using BLASTX, the parameter setting is typically made as follows: score = 50 and wordlength = 3. In the process of making amino acid sequence analysis using the BLAST and Gapped BLAST programs, the default parameters of these programs are used. The specific procedures for conducting these analyses are known to those skilled in the art (e.g.,
http://www.ncbi.nlm.nih.gov/).

Also, the EZR-ERBB4 fusion polynucleotide according to the present invention can be, for example, any one of the polynucleotides mentioned below:
(i) a polynucleotide consisting of the nucleotide sequence of SEQ ID NO: 1;
(ii) a polynucleotide that hybridizes under stringent conditions with a polynucleotide consisting of a nucleotide sequence complementary to the polynucleotide consisting of the nucleotide sequence of SEQ ID NO: 1, and which encodes a polypeptide having kinase activity;
(iii) a polynucleotide that consists of a nucleotide sequence derived from the nucleotide sequence of SEQ ID NO: 1 by deletion, substitution or addition of one or more nucleotides, and which encodes a polypeptide having kinase activity; and
(iv) a polynucleotide that has a sequence identity of at least 80% to the polynucleotide consisting of the nucleotide sequence of SEQ ID NO: 1, and which encodes a polypeptide having kinase activity.

As disclosed below in Examples, the nucleotide sequence of SEQ ID NO: 1 is a nucleotide sequence of an EZR-ERBB4 fusion polynucleotide found in samples from human cancer tissues. In the nucleotide sequence of SEQ ID NO: 1, the point of fusion is located between the guanines at positions 1524 and 1525.

As used above in (ii), the phrase "under stringent conditions" refers to moderately or highly stringent conditions, unless particularly specified.

The moderately stringent conditions can be easily designed by those skilled in the art on the basis of, for example, the length of the polynucleotide of interest. Basic conditions are described in Sambrook, et al., Molecular Cloning: A Laboratory Manual, 3rd ed., ch. 6-7, Cold Spring Harbor Laboratory Press, 2001. Typically, the moderately stringent conditions comprise: prewashing of a nitrocellulose filter in 5×SSC, 0.5% SDS, 1.0 mM EDTA (pH 8.0); hybridization in ca. 50% formamide, 2-6×SSC at about 40-50°C (or any other similar hybridization solution like a Stark's solution in ca. 50% formamide at about 42°C); and washing of the filter in 0.5-6×SSC, 0.1% SDS at about 40°C-60°C. The moderately stringent conditions preferably comprises hybridization in 6×SSC at about 50°C, and may comprise the prewashing and/or washing under the above-mentioned conditions.

The highly stringent conditions can also be easily designed by those skilled in the art on the basis of, for example, the length of the polynucleotide of interest. The highly stringent conditions involve a higher temperature and/or a lower salt concentration than the moderately stringent conditions. Typically, the highly stringent conditions comprise hybridization in 0.2-6×SSC, preferably 6×SSC, more preferably 2×SSC, still more preferably 0.2×SSC, at about 65°C. In any case, the highly stringent conditions preferably comprise washing in 0.2×SSC, 0.1% SDS at about 65-68°C.

In any case, as a buffer for use in hybridization, prewashing and washing, SSPE (1×SSPE: 0.15 M NaCl, 10 mM NaH₂PO₄, and 1.25 mM EDTA, pH 7.4) can be used in place of SSC (1×SSC: 0.15 M NaCl and 15 mM sodium citrate). In any case, washing can be done for about 15 minutes after the completion of hybridization.

As used above in (iii), the phrase "one or more nucleotides" refers to generally 1 to 50 nucleotides, preferably 1 to 30 nucleotides, more preferably 1 to 10 nucleotides, still more preferably one to several nucleotides (for example, 1 to 5 nucleotides, 1 to 4 nucleotides, 1 to 3 nucleotides, 1 or 2 nucleotides, or one nucleotide).

As used above in (iv), the phrase "sequence identity of at least 80%" refers to a sequence identity of preferably at least 85%, more preferably at least 90% or at least 95%, still more preferably at least 97%, at least 98% or at least 99%. Nucleotide sequence identity can be determined using the BLASTN program (Altschul S. F., *et al., J. Mol. Biol,* 1990, 215: 403) which is based on the above-mentioned BLAST algorithm. In the process of making nucleotide sequence analysis using BLASTN, the parameter setting is typically made as follows: score = 100 and wordlength = 12.

### (2) KIAA1468-RET fusion

This gene fusion is a mutation that causes expression of a fusion protein between KIAA1468 protein and RET protein (hereinafter also referred to as the "KIAA1468-RET fusion polypeptide") and which is caused by a translocation (t(10;18)) having breakpoints in regions 18q21 and 10q11 of a human chromosome.

KIAA1468 protein is a protein encoded by the gene located on chromosome 18q21 in a human, and is typically a protein consisting of the amino acid sequence of SEQ ID NO: 24 (NCBI accession No. NP_065905.2 (17-APR-2013)). KIAA1468 protein is characterized by having a coiled-coil domain (Fig. 1), which corresponds to the amino acid sequence at positions 360 to 396 of the amino acid sequence of SEQ ID NO: 24 in a human.

RET protein is a protein encoded by the gene located on chromosome 10q11 in a human, and is typically a protein consisting of the amino acid sequence of SEQ ID NO: 26 (NCBI accession No. NP_066124.1 (07-JULY-2013)). RET protein is characterized by having a kinase domain (Fig. 1), which corresponds to the amino acid sequence at positions 723 to 1012 of the amino acid sequence of SEQ ID NO: 26 in a human.

In RET protein, a cadherin repeat and a transmembrane domain are present in a region toward the N-terminus relative to the kinase domain.

The KIAA1468-RET fusion polypeptide is a polypeptide comprising all or part of the coiled-coil domain of KIAA1468 protein, and the kinase domain of RET protein, and having kinase activity.

The KIAA1468-RET fusion polypeptide may comprise all of the coiled-coil domain of KIAA1468 protein, or may comprise part of the coiled-coil domain as long as the KIAA1468-RET fusion polypeptide can dimerize. Whether the KIAA1468-RET fusion polypeptide dimerizes or not can be confirmed by a known method such as gel filtration chromatography or a combination of treatment with a crosslinking agent and SDS-polyacrylamide gel electrophoresis.

The KIAA1468-RET fusion polypeptide may comprise all of the kinase domain of RET protein, or may comprise part of the kinase domain as long as the KIAA1468-RET fusion polypeptide has kinase activity.

The expression that the KIAA1468-RET fusion polypeptide "has kinase activity" means that said fusion polypeptide is active an enzyme phosphorylating tyrosine due to the kinase domain derived from RET protein. The kinase activity of the KIAA1468-RET fusion polypeptide is determined by a conventional method, and is commonly determined by incubating the fusion polypeptide with a substrate (e.g., synthetic peptide substrate) and ATP under appropriate conditions and then detecting phosphorylated tyrosine in the substrate. The kinase activity can also be measured using a commercially available measurement kit.

The KIAA1468-RET fusion polypeptide may comprise all or part of the cadherin repeat and the transmembrane domain of RET protein, but preferably does not comprise any of them.

Although the present invention is not intended to be bound by any particular theory, it is believed that the KIAA1468-RET fusion polynucleotide would dimerize via the coiled-coil domain present in a region toward the N-terminus to undergo autophosphorylation and become constitutively active, thereby contributing to oncogenesis.

In the present invention, the polynucleotide encoding the KIAA1468-RET fusion polypeptide (hereinafter also referred to as the "KIAA1468-RET fusion polynucleotide") is a polynucleotide that encodes the polypeptide comprising all or part of the coiled-coil domain of KIAA1468 protein, and the kinase domain of RET protein, and having kinase activity. The KIAA1468-RET fusion polynucleotide can be any of mRNA, cDNA and genomic DNA.

The KIAA1468-RET fusion polynucleotide according to the present invention can be, for example, a KIAA1468-RET fusion polynucleotide encoding the polypeptide mentioned below:
(i) a polypeptide consisting of the amino acid sequence of SEQ ID NO: 4;
(ii) a polypeptide consisting of an amino acid sequence derived from the amino acid sequence of SEQ ID NO: 4 by deletion, substitution or addition of one or more amino acids, and the polypeptide having kinase activity; or
(iii) a polypeptide consisting of an amino acid sequence having a sequence identity of at least 80% to the amino acid sequence of SEQ ID NO: 4, and the polypeptide having kinase activity.

As disclosed below in Examples, the amino acid sequence of SEQ ID NO: 4 is an amino acid sequence encoded by a KIAA1468-RET fusion polynucleotide found in samples from human cancer tissues. In the amino acid sequence of SEQ ID NO: 4, the point of fusion is located between Glu at position 540 and Glu at position 541.

The phrases "one or more amino acids" and "sequence identity of at least 80%" as used above in (ii) and (iii), respectively, have the same meanings as described above in "(1) EZR-ERBB4 fusion".

Also, the KIAA1468-RET fusion polynucleotide according to the present invention can be, for example, any one of the polynucleotides mentioned below:
(i) a polynucleotide consisting of the nucleotide sequence of SEQ ID NO: 3;
(ii) a polynucleotide that hybridizes under stringent conditions with a polynucleotide consisting of a nucleotide sequence complementary to the polynucleotide consisting of the nucleotide sequence of SEQ ID NO: 3, and which encodes a polypeptide having kinase activity;
(iii) a polynucleotide that consists of a nucleotide sequence derived from the nucleotide sequence of SEQ ID NO: 3 by deletion, substitution or addition of one or more nucleotides, and which encodes a polypeptide having kinase activity; and
(iv) a polynucleotide that has a sequence identity of at least 80% to the polynucleotide consisting of the nucleotide sequence of SEQ ID NO: 3, and which encodes a polypeptide having kinase activity.

As disclosed below in Examples, the nucleotide sequence of SEQ ID NO: 3 is a nucleotide sequence of a KIAA1468-RET fusion polynucleotide found in samples from human cancer tissues. In the nucleotide sequence of SEQ ID NO: 3, the point of fusion is located between the guanines at positions 1835 and 1836.

The phrases "under stringent conditions", "one or more nucleotides", and "sequence identity of at least 80%" as used above in (ii), (iii) and (iv), respectively, have the same meanings as described above in "(1) EZR-ERBB4 fusion".

### (3) TRIM24-BRAF fusion

This gene fusion is a mutation that causes expression of a fusion protein between TRIM24 protein and BRAF protein (hereinafter also referred to as the "TRIM24-BRAF fusion polypeptide") and which is caused by an inversion (inv7) having breakpoints in regions 7q33 and 7q34 of a human chromosome.

TRIM24 protein is a protein encoded by the gene located on chromosome 7q33 in a human, and is typically a protein consisting of the amino acid sequence of SEQ ID NO: 28 (NCBI accession No. NP_003843.3 (17-APR-2013)). TRIM24 protein is characterized by having a RING finger domain (Fig. 1), which corresponds to the amino acid sequence at positions 56 to 82 of the amino acid sequence of SEQ ID NO: 28 in a human.

BRAF protein is a protein encoded by the gene located on chromosome 7q34 in a human, and is typically a protein consisting of the amino acid sequence of SEQ ID NO: 30 (NCBI accession No. NP_004324.2 (16-JUN-2013)). BRAF protein is characterized by having a kinase domain (Fig. 1), which corresponds to the amino acid sequence at positions 457 to 717 of the amino acid sequence of SEQ ID NO: 30 in a human.

In BRAF protein, a Raf-like Ras-binding domain (at positions 155 to 227 of the amino acid sequence of SEQ ID NO: 22) serving as a kinase inhibition domain is present in a region toward the N-terminus relative to the kinase domain.

The TRIM24-BRAF fusion polypeptide is a polypeptide comprising the kinase domain of BRAF protein and having kinase activity.

The TRIM24-BRAF fusion polypeptide may or may not comprise all or part of the RING finger domain of TRIM24 protein.

The TRIM24-BRAF fusion polypeptide may comprise all of the kinase domain of BRAF protein, or may comprise part of the kinase domain as long as the TRIM24-BRAF fusion polypeptide has kinase activity.

The expression that the TRIM24-BRAF fusion polypeptide "has kinase activity" means that said fusion polypeptide is active as an enzyme phosphorylating serine or threonine due to the kinase domain derived from BRAF protein. The kinase activity of the TRIM24-BRAF fusion polypeptide is determined by a conventional method, and is commonly determined by incubating the fusion polypeptide with a substrate (e.g., synthetic peptide substrate) and ATP under appropriate conditions and then detecting phosphorylated serine or threonine in the substrate. The kinase activity can also be measured using a commercially available measurement kit.

The EZR-ERBB4 fusion polypeptide preferably does not comprise a Raf-like Ras-binding domain.

Although the present invention is not intended to be bound by any particular theory, it is believed that the TRIM24-BRAF fusion polypeptide would lack a kinase inhibition domain present in a region of wild-type BRAF protein extending toward the N-terminus to become constitutively active, thereby contributing to oncogenesis.

In the present invention, the polynucleotide encoding the TRIM24-BRAF fusion polypeptide (hereinafter also referred to as the "TRIM24-BRAF fusion polynucleotide") is a polynucleotide that encodes the polypeptide comprising the kinase domain of BRAF protein and having kinase activity. The TRIM24-BRAF fusion polynucleotide can be any of mRNA, cDNA and genomic DNA.

The TRIM24-BRAF fusion polynucleotide according to the present invention can be, for example, a TRIM24-BRAF fusion polynucleotide encoding the polypeptide mentioned below:
(i) a polypeptide consisting of the amino acid sequence of SEQ ID NO: 6;
(ii) a polypeptide consisting of an amino acid sequence derived from the amino acid sequence of SEQ ID NO: 6 by deletion, substitution or addition of one or more amino acids, and the polypeptide having kinase activity; or
(iii) a polypeptide consisting of an amino acid sequence having a sequence identity of at least 80% to the amino acid sequence of SEQ ID NO: 6, and the polypeptide having kinase activity.

As disclosed below in Examples, the amino acid sequence of SEQ ID NO: 6 is an amino acid sequence encoded by a TRIM24-BRAF fusion polynucleotide found in samples from human cancer tissues. In the amino acid sequence of SEQ ID NO: 6, the point of fusion is located in Arg at position 294.

The phrases "one or more amino acids" and "sequence identity of at least 80%" as used above in (ii) and (iii), respectively, have the same meanings as described above in "(1) EZR-ERBB4 fusion".

Also, the TRIM24-BRAF fusion polynucleotide according to the present invention can be, for example, any one of the polynucleotides mentioned below:
(i) a polynucleotide consisting of the nucleotide sequence of SEQ ID NO: 5;
(ii) a polynucleotide that hybridizes under stringent conditions with a polynucleotide consisting of a nucleotide sequence complementary to the polynucleotide consisting of the nucleotide sequence of SEQ ID NO: 5, and which encodes a polypeptide having kinase activity;
(iii) a polynucleotide that consists of a nucleotide sequence derived from the nucleotide sequence of SEQ ID NO: 5 by deletion, substitution or addition of one or more nucleotides, and which encodes a polypeptide having kinase activity; and
(iv) a polynucleotide that has a sequence identity of at least 80% to the polynucleotide consisting of the nucleotide sequence of SEQ ID NO: 5, and which encodes a polypeptide having kinase activity.

As disclosed below in Examples, the nucleotide sequence of SEQ ID NO: 5 is a nucleotide sequence of a TRIM24-BRAF fusion polynucleotide found in samples from human cancer tissues. In the nucleotide sequence of SEQ ID NO: 5, the point of fusion is located between the guanines at positions 1096 and 1097.

The phrases "under stringent conditions", "one or more nucleotides", and "sequence identity of at least 80%" as used above in (ii), (iii) and (iv), respectively, have the same meanings as described above in "(1) EZR-ERBB4 fusion".

### (4) CD74-NRG1 fusion

This gene fusion is a mutation that causes expression of a fusion protein between CD74 protein and NRG1 protein (hereinafter also referred to as the "CD74-NRG1 fusion polypeptide") and which is caused by a translocation (t(5;8)) having breakpoints in regions 5q32 and 8p12 of human chromosome.

CD74 protein is a protein encoded by the gene located on chromosome 5q32 in a human, and is typically a protein consisting of the amino acid sequence of SEQ ID NO: 32 (NCBI accession No. NP_004346.1 (29-APR-2013)). CD74 protein is characterized by having a transmembrane domain (Fig. 1), which corresponds to the amino acid sequence at positions 47 to 72 of the amino acid sequence of SEQ ID NO: 32 in a human.

NRG1 protein is a protein encoded by the gene located on chromosome 8p12 in a human, and is typically a protein consisting of the amino acid sequence of SEQ ID NO: 34 (NCBI accession No. NP_001153477.1 (07-JUL-2013)). NRG1 protein is characterized by having an EGF domain (Fig. 1), which corresponds to the amino acid sequence at positions 143 to 187 of the amino acid sequence of SEQ ID NO: 34 in a human.

The CD74-NRG1 fusion polypeptide is a polypeptide comprising the transmembrane domain of CD74 protein and the EGF domain of NRG1 protein, and having intracellular signaling-enhancing activity.

The CD74-NRG1 fusion polypeptide may comprise all or part of the transmembrane domain of CD74 protein.

The CD74-NRG1 fusion polypeptide may comprise all of the EGF domain of NRG1 protein, or may comprise part of the EGF domain as long as the CD74-NRG1 fusion polypeptide has intracellular signaling-enhancing activity.

The expression that the CD74-NRG1 fusion polypeptide "has intracellular signaling-enhancing activity" means that said fusion polypeptide is active in enhancing intracellular signaling due to the EGF domain derived from NRG1 protein. This activity is determined by the following method described in Wilson, T. R., et al., Cancer Cell, 2011, 20, 158-172.

A test substance is added to EFM-19 cells (DSMZ, No. ACC-231) cultured in a serum-starved condition, the cells are treated for 30 minutes and then lysed to extract protein. The phosphorylation of EGFR, ERBB2, ERBB3 or ERBB4 is analyzed by Western blotting. If phosphorylation is higher than in the case where no test substance is added, it is determined that the test substance has intracellular signaling-enhancing activity. As the test substance as referred to herein, the entire CD74-NRG1 fusion polypeptide may be used, or a fragment thereof which lacks a transmembrane domain but contains an EGF domain may be used in consideration of solubility or other factors.

Although the present invention is not intended to be bound by any particular theory, it is believed that the CD74-NRG1 fusion polynucleotide is more highly expressed than wild-type NRG1 protein and works positively for enhancement of intracellular signaling as well as survival by an autocrine mechanism, thereby contributing to oncogenesis.

In the present invention, the polynucleotide encoding the CD74-NRG1 fusion polypeptide (hereinafter also referred to as the "CD74-NRG1 fusion polynucleotide") is a polynucleotide that encodes the polypeptide comprising the transmembrane domain of CD74 protein and the EGF domain of NRG1 protein, and having intracellular signaling-enhancing activity. The CD74-NRG1 fusion polynucleotide can be any of mRNA, cDNA and genomic DNA.

The CD74-NRG1 fusion polynucleotide according to the present invention can be, for example, a CD74-NRG1 fusion polynucleotide encoding the polypeptide mentioned below:
(i) a polypeptide consisting of the amino acid sequence of SEQ ID NO: 8 or 10;
(ii) a polypeptide consisting of an amino acid sequence derived from the amino acid sequence of SEQ ID NO: 8 or 10 by deletion, substitution or addition of one or more amino acids, and the polypeptide having intracellular signaling-enhancing activity; or
(iii) a polypeptide consisting of an amino acid sequence having a sequence identity of at least 80% to the amino acid sequence of SEQ ID NO: 8 or 10, and the polypeptide having intracellular signaling-enhancing activity.

As disclosed below in Examples, the amino acid sequence of SEQ ID NO: 8 or 10 is an amino acid sequence encoded by a CD74-NRG1 fusion polynucleotide found in samples from human cancer tissues. In the amino acid sequence of SEQ ID NO: 8, the point of fusion is located in Ala at position 230. In the amino acid sequence of SEQ ID NO: 10, the point of fusion is located in Ala at position 209.

The phrases "one or more amino acids" and "sequence identity of at least 80%" as used above in (ii) and (iii), respectively, have the same meanings as described above in "(1) EZR-ERBB4 fusion".

Also, the CD74-NRG1 fusion polynucleotide according to the present invention can be, for example, any one of the polynucleotides mentioned below:
(i) a polynucleotide consisting of the nucleotide sequence of SEQ ID NO: 7 or 9;
(ii) a polynucleotide that hybridizes under stringent conditions with a polynucleotide consisting of a nucleotide sequence complementary to the polynucleotide consisting of the nucleotide sequence of SEQ ID NO: 7 or 9, and which encodes a polypeptide having intracellular signaling-enhancing activity;
(iii) a polynucleotide that consists of a nucleotide sequence derived from the nucleotide sequence of SEQ ID NO: 7 or 9 by deletion, substitution or addition of one or more nucleotides, and which encodes a polypeptide having intracellular signaling-enhancing activity; or
(iv) a polynucleotide that has a sequence identity of at least 80% to the polynucleotide consisting of the nucleotide sequence of SEQ ID NO: 7 or 9, and which encodes a polypeptide having intracellular signaling-enhancing activity.

As disclosed below in Examples, the nucleotide sequence of SEQ ID NO: 7 or 9 is a nucleotide sequence of a CD74-NRG1 fusion polynucleotide found in samples from human cancer tissues. In the nucleotide sequence of SEQ ID NO: 7, the point of fusion is located between the guanine at position 875 and the cytosine at position 876. In the nucleotide sequence of SEQ ID NO: 9, the point of fusion is located between the guanine at position 812 and the cytosine at position 813.

The phrases "under stringent conditions", "one or more nucleotides", and "sequence identity of at least 80%" as used above in (ii), (iii) and (iv), respectively, have the same meanings as described above in "(1) EZR-ERBB4 fusion".

### (5) SLC3A2-NRG1 fusion

This gene fusion is a mutation that causes expression of a fusion protein between SLC3A2 protein and NRG1 protein (hereinafter also referred to as the "SLC3A2-NRG1 fusion polypeptide") and which is caused by a translocation (t(8;11)) having breakpoints in regions 11q12.3 and 8p12 of a human chromosome.

SLC3A2 protein is a protein encoded by the gene located on chromosome 11q12.3 in a human, and is typically a protein consisting of the amino acid sequence of SEQ ID NO: 39 (NCBI accession No. NP_001012680.1 (27-APR-2014)). SLC3A2 protein is characterized by having a transmembrane domain (Fig. 4), which corresponds to the amino acid sequence at positions 184 to 207
(http://www.hprd.org/sequence?hprd_id=01148&isoform_id=01148_3&isoform_name=Isofo rm_2) or the amino acid sequence at positions 184 to 206
(http://asia.ensembl.org/Homo_sapiens/Transcript/ProteinSummary?g=ENSG00000168003;r =11:62623583-62656332;t=ENST00000377891#), of the amino acid sequence of SEQ ID NO: 39 in a human.

NRG1 protein is as described above in (4) CD74-NRG1 fusion".

The SLC3A2-NRG1 fusion polypeptide is a polypeptide comprising the transmembrane domain of SLC3A2 protein and the EGF domain of NRG1 protein, and having intracellular signaling-enhancing activity.

The SLC3A2-NRG1 fusion polypeptide may comprise all or part of the transmembrane domain of SLC3A2 protein.

The SLC3A2-NRG1 fusion polypeptide may comprise all of the EGF domain of NRG1 protein, or may comprise part of the EGF domain as long as the SLC3A2-NRG1 fusion polypeptide has intracellular signaling-enhancing activity.

The expression that the SLC3A2-NRG1 fusion polypeptide "has intracellular signaling-enhancing activity" means that said fusion polypeptide is active in enhancing intracellular signaling due to the EGF domain derived from NRG1 protein. This activity is determined by the following method described in Wilson, T. R., et al., Cancer Cell, 2011, 20, 158-172.

A test substance is added to EFM-19 cells (DSMZ, No. ACC-231) cultured in a serum-starved condition, the cells are treated for 30 minutes and then lysed to extract protein. The phosphorylation of EGFR, ERBB2, ERBB3 or ERBB4 is analyzed by Western blotting. If phosphorylation is higher than in the case where no test substance is added, it is determined that the test substance has intracellular signaling-enhancing activity. As the test substance as referred to herein, the entire SLC3A2-NRG1 fusion polypeptide may be used, or a fragment thereof which lacks a transmembrane domain but contains an EGF domain may be used in consideration of solubility or other factors.

Although the present invention is not intended to be bound by any particular theory, it is believed that the SLC3A2-NRG1 fusion polynucleotide is more highly expressed than wild-type NRG1 protein and works positively for enhancement of intracellular signaling as well as survival by an autocrine mechanism, thereby contributing to oncogenesis.

In the present invention, the polynucleotide encoding the SLC3A2-NRG1 fusion polypeptide (hereinafter also referred to as the "SLC3A2-NRG1 fusion polynucleotide") is a polynucleotide that encodes the polypeptide comprising the transmembrane domain of SLC3A2 protein and the EGF domain of NRG1 protein, and having intracellular signaling-enhancing activity. The SLC3A2-NRG1 fusion polynucleotide can be any of mRNA, cDNA and genomic DNA.

The SLC3A2-NRG1 fusion polynucleotide according to the present invention can be, for example, an SLC3A2-NRG1 fusion polynucleotide encoding the polypeptide mentioned below:
(i) a polypeptide consisting of the amino acid sequence of SEQ ID NO: 36;
(ii) a polypeptide consisting of an amino acid sequence derived from the amino acid sequence of SEQ ID NO: 36 by deletion, substitution or addition of one or more amino acids, and the polypeptide having intracellular signaling-enhancing activity; or
(iii) a polypeptide consisting of an amino acid sequence having a sequence identity of at least 80% to the amino acid sequence of SEQ ID NO: 36, and the polypeptide having intracellular signaling-enhancing activity.

As disclosed below in Examples, the amino acid sequence of SEQ ID NO: 36 is an amino acid sequence encoded by an SLC3A2-NRG1 fusion polynucleotide found in samples from human cancer tissues. In the amino acid sequence of SEQ ID NO: 36, the point of fusion is located in the threonine at position 302.

The phrases "one or more amino acids" and "sequence identity of at least 80%" as used above in (ii) and (iii), respectively, have the same meanings as described above in "(1) EZR-ERBB4 fusion".

Also, the SLC3A2-NRG1 fusion polynucleotide according to the present invention can be, for example, any one of the polynucleotides mentioned below:
(i) a polynucleotide consisting of the nucleotide sequence of SEQ ID NO: 35;
(ii) a polynucleotide that hybridizes under stringent conditions with a polynucleotide consisting of a nucleotide sequence complementary to the polynucleotide consisting of the nucleotide sequence of SEQ ID NO: 35, and which encodes a polypeptide having intracellular signaling-enhancing activity;
(iii) a polynucleotide that consists of a nucleotide sequence derived from the nucleotide sequence of SEQ ID NO: 35 by deletion, substitution or addition of one or more nucleotides, and which encodes a polypeptide having intracellular signaling-enhancing activity; or
(iv) a polynucleotide that has a sequence identity of at least 80% to the polynucleotide consisting of the nucleotide sequence of SEQ ID NO: 35, and which encodes a polypeptide having intracellular signaling-enhancing activity.

As disclosed below in Examples, the nucleotide sequence of SEQ ID NO: 35 is a nucleotide sequence of an SLC3A2-NRG1 fusion polynucleotide found in samples from human cancer tissues. In the nucleotide sequence of SEQ ID NO: 35, the point of fusion is located between the adenine at position 904 and the cytosine at position 905.

The phrases "under stringent conditions", "one or more nucleotides", and "sequence identity of at least 80%" as used above in (ii), (iii) and (iv), respectively, have the same meanings as described above in "(1) EZR-ERBB4 fusion".

### <Method for detecting gene fusions serving as responsible mutations (driver mutations) for cancer>

The present invention provides a method for detecting the above-mentioned five types of gene fusions (hereinafter also referred to as the "inventive detection method"). The inventive detection method comprises the step of detecting any one of the above-mentioned fusion polynucleotides, or a polypeptide encoded thereby, in an isolated sample from a subject with cancer.

In the inventive detection method, the subject is not particularly limited as long as it is a mammal. Examples of the mammal include: rodents such as mouse, rat, hamster, chipmunk and guinea pig; rabbit, pig, cow, goat, horse, sheep, mink, dog, cat; and primates such as human, monkey, cynomolgus monkey, rhesus monkey, marmoset, orangutan, and chimpanzee, with human being preferred.

The subject with cancer may be not only a subject affected with cancer, but also a subject suspected of having cancer or a subject with a future risk of cancer. The "cancer" to which the inventive detection method is to be applied is not particularly limited as long as it is a cancer in which any of the above-mentioned five types of gene fusions can be detected, with lung cancer being preferred, non-small-cell lung carcinoma being more preferred, and lung adenocarcinoma being particularly preferred.

The "isolated sample" from the subject encompasses not only biological samples (for example, cells, tissues, organs, body fluids (e.g., blood, lymphs), digestive juices, sputum, bronchoalveolar/bronchial lavage fluids, urine, and feces), but also nucleic acid extracts from these biological samples (e.g., genomic DNA extracts, mRNA extracts, and cDNA and cRNA preparations from mRNA extracts) and protein extracts. The genomic DNA, mRNA, cDNA or protein can be prepared by those skilled in the art through considering various factors including the type and state of the sample and selecting a known technique suitable therefor. The sample may also be the one that is fixed with formalin or alcohol, frozen, or embedded in paraffin.

Further, the "isolated sample" is preferably the one derived from an organ having or suspected of having the above-mentioned cancer, can be exemplified by those derived from the small intestine, spleen, kidney, liver, stomach, lung, adrenal gland, heart, brain, pancreas, aorta, and other organs, with an isolated sample from the lung being more preferred.

In the inventive detection method, the detection of a fusion polynucleotide or a polypeptide encoded thereby can be made using a *per se* known technique.

If the object to be detected is a transcript from a genomic DNA (mRNA, or cDNA prepared from mRNA), a fusion polynucleotide in the form of mRNA or cDNA can be detected using, for example, RT-PCR, sequencing, TaqMan probe method, Northern blotting, dot blotting, or cDNA microarray analysis.

If the object to be detected is a genomic DNA, a fusion polynucleotide in the form of genomic DNA can be detected using, for example, *in situ* hybridization (ISH), genomic PCR, sequencing, TaqMan probe method, Southern blotting, or genome microarray analysis.

The above-mentioned detection techniques can be used alone or in combination. For example, since the above-mentioned five types of gene fusions are believed to contribute to oncogenesis by expressing fusion polypeptides, it is also preferred that if a fusion polynucleotide in the form of genomic DNA is detected (e.g., by *in situ* hybridization or the like), production of a transcript or a protein should be further confirmed (e.g., by RT-PCR, immunostaining or the like).

If a fusion polynucleotide is detected by a hybridization technique (e.g., TaqMan probe method, Northern blotting, Southern blotting, dot blotting, microarray analysis, *in situ* hybridization (ISH)), there can be used a polynucleotide that serves as a probe designed to specifically recognize the fusion polynucleotide. As used herein, the phrase "specifically recognize the fusion polynucleotide" means that under stringent conditions, the probe distinguishes and recognizes the fusion polynucleotide from other polynucleotides, including wild-type genes from which to derive both segments of the fusion polynucleotide each extending from the point of fusion toward the 5'- or 3'-end.

Since biological samples (e.g., biopsy samples) obtained in the process of treatment or diagnosis are often fixed in formalin, it is preferred to use *in situ* hybridization in the inventive detection method, because the genomic DNA to be detected is stable even when fixed in formalin and the detection sensitivity is high.

According to *in situ* hybridization, the genomic DNA (fusion polynucleotide) encoding a fusion polypeptide can be detected by hybridizing, to such a biological sample, the following polynucleotide (a) or (b) which has a chain length of at least 15 nucleotides and serves as a probe(s) designed to specifically recognize said fusion polynucleotide:
(a) a polynucleotide for each of the above-mentioned gene fusions, which serves as at least one probe selected from the group consisting of a probe that hybridizes to the nucleotide sequence of a fusion partner gene toward the 5'-end (e.g., EZR, KIAA1468, TRIM24, CD74 or SLC3A2 gene) and a probe that hybridizes to the nucleotide sequence of a fusion partner gene toward the 3'-end (e.g., ERBB4, RET, BRAF or NRG1 gene); or
(b) a polynucleotide for each of the above-mentioned gene fusions, which serves as a probe that hybridizes to a nucleotide sequence containing the point of fusion between a fusion partner gene toward the 5'-end and a fusion partner gene toward the 3'-end.

The EZR gene according to the present invention, as far as it is derived from humans, is typically a gene consisting of the DNA sequence from position 159186773 to position 159240456 in the genome sequence identified in Genbank accession No. NC_000006.11.

The KIAA1468 gene according to the present invention, as far as it is derived from humans, is typically a gene consisting of the DNA sequence from position 59854524 to position 59974355 in the genome sequence identified in Genbank accession No.NC_000018.9.

The TRIM24 gene according to the present invention, as far as it is derived from humans, is typically a gene consisting of the DNA sequence from position 138145079 to position 138270333 in the genome sequence identified in Genbank accession No.NC_000007.13.

The CD74 gene according to the present invention, as far as it is derived from humans, is typically a gene consisting of the DNA sequence from position 149781200 to position 149792499 in the genome sequence identified in Genbank accession No.NC_000005.9.

The SLC3A2 gene according to the present invention, as far as it is derived from humans, is typically a gene consisting of the DNA sequence from position 62856012 to position 62888883 in the genome sequence identified in Genbank accession No.NC_000011.10.

The ERBB4 gene according to the present invention, as far as it is derived from humans, is typically a gene consisting of the DNA sequence from position 212240442 to position 213403352 in the genome sequence identified in Genbank accession No.NC_000002.11.

The RET gene according to the present invention, as far as it is derived from humans, is typically a gene consisting of the DNA sequence from position 43572517 to position 43625799 in the genome sequence identified in Genbank accession No. NC_000010.10.

The BRAF gene according to the present invention, as far as it is derived from humans, is typically a gene consisting of the DNA sequence from position 140433812 to position 140624564 in the genome sequence identified in Genbank accession No.NC_000007.13.

The NRG1 gene according to the present invention, as far as it is derived from humans, is typically a gene consisting of the DNA sequence from position 31496820 to position 32622558 in the genome sequence identified in Genbank accession No.NC_000008.10.

However, the DNA sequences of genes can change in nature (i.e., in a non-artificial way) due to their mutations and the like. Thus, such native mutants can also be encompassed by the present invention (the same applies hereinafter).

The polynucleotide mentioned in (a) according to the present invention can be of any type as far as it is capable of detecting the presence of the genomic DNA encoding a fusion polypeptide in the above-mentioned biological sample by hybridizing to a nucleotide sequence(s) targeted by said polynucleotide, i.e., the nucleotide sequence of a fusion partner gene toward the 5'-end (e.g., EZR, KIAA1468, TRIM24, CD74 or SLC3A2 gene) and/or the nucleotide sequence of a fusion partner gene toward the 3'-end (e.g., ERBB4, RET, BRAF or NRG1 gene); preferably, the polynucleotide (a) is any of the polynucleotides mentioned below in (a1) to (a3):
(a1) a combination of a polynucleotide that hybridizes to the nucleotide sequence of that region of the fusion partner gene toward the 5'-end which extends upstream from its breakpoint toward the 5'-end (this polynucleotide is hereinafter also referred to as the "5' fusion partner gene probe 1"), and a polynucleotide that hybridizes to the nucleotide sequence of that region of the fusion partner gene toward the 3'-end which extends downstream from its breakpoint toward the 3'-end (this polynucleotide is hereinafter also referred to as the "3' fusion partner gene probe 1 ");
(a2) a combination of a polynucleotide that hybridizes to the nucleotide sequence of that region of the fusion partner gene toward the 5'-end which extends upstream from its breakpoint toward the 5'-end (this polynucleotide is hereinafter also referred to as the "5' fusion partner gene probe 1"), and a polynucleotide that hybridizes to the nucleotide sequence of that region of the fusion partner gene toward the 5'-end which extends downstream from its breakpoint toward the 3'-end (this polynucleotide is hereinafter also referred to as the "5' fusion partner gene probe 2"); and
(a3) a combination of a polynucleotide that hybridizes to the nucleotide sequence of that region of the fusion partner gene toward the 3'-end which extends upstream from its breakpoint toward the 5'-end (this polynucleotide is hereinafter also referred to as the "3' fusion partner gene probe 2"), and a polynucleotide that hybridizes to the nucleotide sequence of that region of the fusion partner gene toward the 3'-end which extends downstream from its breakpoint toward the 3'-end (this polynucleotide is hereinafter also referred to as the "3' fusion partner gene probe 1").

As for (a1) to (a3) mentioned above, if the fusion partner gene toward the 5'-end is the EZR gene, the nucleotide sequence of that region of said gene which extends upstream from its breakpoint toward the 5'-end contains a cording region for all or part of the coiled-coil domain of EZR protein.

As for (a1) to (a3) mentioned above, if the fusion partner gene toward the 3'-end is the ERBB4 gene, the nucleotide sequence of that region of said gene which extends downstream from its breakpoint toward the 3'-end contains a cording region for all or part of the kinase domain of ERBB4 protein.

As for (a1) to (a3) mentioned above, if the fusion partner gene toward the 5'-end is the KIAA1468 gene, the nucleotide sequence of that region of said gene which extends upstream from its breakpoint toward the 5'-end contains a cording region for all or part of the coiled-coil domain of KIAA1468 protein.

As for (a1) to (a3) mentioned above, if the fusion partner gene toward the 3'-end is the RET gene, the nucleotide sequence of that region of said gene which extends downstream from its breakpoint toward the 3'-end contains a cording region for all or part of the kinase domain of RET protein.

As for (a1) to (a3) mentioned above, if the fusion partner gene toward the 5'-end is the TRIM24 gene, the nucleotide sequence of that region of said gene which extends upstream from its breakpoint toward the 5'-end contains a cording region for all or part of the RING finger domain of TRIM24 protein.

As for (a1) to (a3) mentioned above, if the fusion partner gene toward the 3'-end is the BRAF gene, the nucleotide sequence of that region of said gene which extends downstream from its breakpoint toward the 3'-end contains a cording region for all or part of the kinase domain of BRAF protein, and also the nucleotide sequence of that region of said gene which extends upstream from its breakpoint toward the 5'-end contains the cording region for the Raf-like Ras-binding domain of BRAF protein.

As for (a1) to (a3) mentioned above, if the fusion partner gene toward the 5'-end is the CD74 gene, the nucleotide sequence of that region of said gene which extends upstream from its breakpoint toward the 5'-end contains the cording region for the transmembrane domain of CD74 protein.

As for (a1) to (a3) mentioned above, if the fusion partner gene toward the 3'-end is the NRG1 gene, the nucleotide sequence of that region of said gene which extends downstream from its breakpoint toward the 3'-end contains a cording region for all or part of the EGF domain of NRG1 protein.

As for (a1) to (a3) mentioned above, if the fusion partner gene toward the 5'-end is the SLC3A2 gene, the nucleotide sequence of that region of said gene which extends upstream from its breakpoint toward the 5'-end contains the cording region for the transmembrane domain of SLC3A2 protein.

The polynucleotides mentioned above in (a1) can be exemplified by the polynucleotide combinations mentioned below in (a1-1) to (a1-5):
(a1-1) a combination of a polynucleotide that hybridizes to a coding region for all or part of the coiled-coil domain of EZR protein, and a polynucleotide that hybridizes to a cording region for all or part of the kinase domain of ERBB4 protein;
(a1-2) a combination of a polynucleotide that hybridizes to a coding region for all or part of the coiled-coil domain of KIAA1468 protein, and a polynucleotide that hybridizes to a cording region for all or part of the kinase domain of RET protein;
(a1-3) a combination of a polynucleotide that hybridizes to a coding region for all or part of the RING finger domain of TRIM24 protein, and a polynucleotide that hybridizes to a cording region for all or part of the kinase domain of BRAF protein;
(a1-4) a combination of a polynucleotide that hybridizes to the coding region for the transmembrane domain of CD74 protein, and a polynucleotide that hybridizes to a cording region for all or part of the EGF domain of NRG1 protein; and
(a1-5) a combination of a polynucleotide that hybridizes to the coding region for the transmembrane domain of SLC3A2 protein, and a polynucleotide that hybridizes to a cording region for all or part of the EGF domain of NRG1 protein.

In the present invention, it is preferred from the viewpoint of specificity for the target nucleotide sequence and detection sensitivity that the region to which the polynucleotide for use for *in situ* hybridization as mentioned above in (a1) is to hybridize (such a region is hereinafter referred to as the "target nucleotide sequence") should be located not more than 1000000 nucleotides away from the point of fusion between the fusion partner gene toward the 5'-end (e.g., EZR, KIAA1468, TRIM24, CD74 or SLC3A2 gene) and the fusion partner gene toward the 3'-end (e.g., ERBB4, RET, BRAF or NRG1 gene).

In the present invention, the polynucleotide for use for *in situ* hybridization as mentioned above in (b) can be of any type as far as it is capable of detecting the presence of the genomic DNA encoding a fusion polypeptide in the above-mentioned biological sample by hybridizing to a nucleotide sequence targeted by said polynucleotide, i.e., a nucleotide sequence containing the point of fusion between a fusion partner gene toward the 5'-end and a fusion partner gene toward the 3'-end; and typical examples of the polynucleotide (b) are those which each hybridize to a nucleotide sequence containing a point of fusion in the nucleotide sequence of SEQ ID NO: 1, 3, 5, 7, 9 or 35.

Further, in the present invention, it is preferred from the viewpoint of specificity for the target nucleotide sequence and detection sensitivity that the polynucleotide for use for *in situ* hybridization as mentioned above in (a) or (b) should be a group consisting of multiple types of polynucleotides which can cover the entire target nucleotide sequence. In such a case, each of the polynucleotides constituting the group has a length of at least 15 nucleotides, and preferably 100 to 1000 nucleotides.

The polynucleotide for use for *in situ* hybridization as mentioned above in (a) or (b) is preferably labeled for detection with a fluorescent dye or the like. Examples of such a fluorescent dye include, but are not limited to, DEAC, FITC, R6G, TexRed, and Cy5. Aside from the fluorescent dye, the polynucleotide may also be labeled with a radioactive isotope (e.g., ¹²⁵I, ¹³¹I, ³H, ¹⁴C, ³³P, ³²P), an enzyme (e.g., β-galactosidase, β-glucosidase, alkaline phosphatase, peroxidase, malate dehydrogenase), or a luminescent substance (e.g., luminol, luminol derivative, luciferin, lucigenin, 3,3'-diaminobenzidine (DAB)).

When *in situ* hybridization is performed using a combination of 5' fusion partner gene probe 1 and 3' fusion partner gene probe 1, a combination of 5' fusion partner gene probe 1 and 5' fusion partner gene probe 2, or a combination of 3' fusion partner gene probe 2 and 3' fusion partner gene probe 1, the probes of each combination are preferably labeled with different dyes from each other. If, as the result of *in situ* hybridization using such a combination of probes labeled with different dyes, an overlap is observed between the signal (e.g., fluorescence) emitted from the label on 5' fusion partner gene probe 1 and the signal emitted from the label on 3' fusion partner gene probe 1, then it can be determined that a genomic DNA encoding a fusion polypeptide of interest has been detected successfully. Also, if a split is observed between the signal emitted from the label on 5' fusion partner gene probe 1 and the signal emitted from the label on 5' fusion partner gene probe 2, or between the signal emitted from the label on 3' fusion partner gene probe 2 and the signal emitted from the label on 3' fusion partner gene probe 1, then it can be determined that a genomic DNA encoding a fusion polypeptide of interest has been detected successfully.

Polynucleotide labeling can be effected by a known method. For example, polynucleotides can be labeled by nick translation or random priming, in which the polynucleotides are caused to incorporate substrate nucleotides labeled with a fluorescent dye or the like.

The conditions for hybridizing the polynucleotide mentioned above in (a) or (b) to the above-mentioned biological sample by *in situ* hybridization can vary with various factors including the length of said polynucleotide; and exemplary highly stringent hybridization conditions are 0.2×SSC at 65°C, and exemplary low stringent hybridization conditions are 2.0×SSC at 50°C. Those skilled in the art could realize comparable stringent hybridization conditions to those mentioned above, by appropriately selecting salt concentration (e.g., SSC dilution rate), temperature, and various other conditions including concentrations of surfactant (e.g., NP-40) and formamide, and pH.

In addition to the *in situ* hybridization, other examples of the method for detecting a genomic DNA encoding a fusion polypeptide of interest using the polynucleotide mentioned above in (a) or (b) include Southern blotting, Northern blotting and dot blotting. According to these methods, the fusion gene of interest is detected by hybridizing said polynucleotide (a) or (b) to a membrane in which a nucleic acid extract from the above-mentioned biological sample is transcribed. In the case of using said polynucleotide (a), if a polynucleotide that hybridizes to the nucleotide sequence of a fusion partner gene toward the 5'-end and a polynucleotide that hybridizes to the nucleotide sequence of a fusion partner gene toward the 3'-end both recognize the same band developed in the membrane, then it can be determined that a genomic DNA encoding a fusion polypeptide of interest has been detected successfully.

Additional examples of the method for detecting a genomic DNA encoding a fusion polypeptide of interest using said polynucleotide (b) include genome microarray analysis and DNA microarray analysis. According to these methods, the genomic DNA is detected by preparing an array in which said polynucleotide (b) is immobilized on a substrate and bringing the above-mentioned biological sample into contact with the polynucleotide immobilized on the array. The substrate is not particularly limited as long as it allows conversion of an oligo- or polynucleotide into a solid phase, and examples include glass plate, nylon membrane, microbeads, silicon chip, and capillary.

In the inventive detection method, it is also preferred to detect a fusion polynucleotide of interest using PCR.

In the process of PCR, there can be used polynucleotides serving as a pair of primers designed to specifically amplify a fusion polynucleotide using DNA (e.g., genomic DNA, cDNA) or RNA prepared from the above-mentioned biological sample as a template. As used herein, the phrase "specifically amplify a fusion polynucleotide" means that the primers do not amplify wild-type genes from which to derive both segments of a fusion polynucleotide of interest each extending from a point of fusion toward the 5'- or 3'-end, but can amplify said fusion polynucleotide alone. It is acceptable to amplify all of the fusion polynucleotide or to amplify that part of the fusion polynucleotide which contains a point of fusion.

The "polynucleotides serving as a pair of primers" to be used for PCR or the like consist of a sense primer (forward primer) and an anti-sense primer (reverse primer) that specifically amplify a target fusion polynucleotide. The sense primer is designed from the nucleotide sequence of that region of said fusion polynucleotide which extends from the point of fusion toward the 5'-end. The anti-sense primer is designed from the nucleotide sequence of that region of said fusion polynucleotide which extends from the point of fusion toward the 3'-end. From the viewpoint of the accuracy and sensitivity of PCR detection, these primers are commonly designed such that a PCR product of not more than 5 kb in size can be amplified. The primers can be designed as appropriate by a known method, for example, using the Primer Express® software (Applied Biosystems). The length of these polynucleotides are generally not less than 15 nucleotides (preferably not less than 16, 17, 18, 19 or 20 nucleotides, more preferably not less than 21 nucleotides) and not more than 100 nucleotides (preferably not more than 90, 80, 70, 60, 50 or 40 nucleotides, more preferably not more than 30 nucleotides).

Preferred examples of the "polynucleotides serving as a pair of primers" include: a primer set against the EZR-ERBB4 fusion polynucleotide, which consists of a polynucleotide consisting of the nucleotide sequence of SEQ ID NO: 11 and a polynucleotide consisting of the nucleotide sequence of SEQ ID NO: 12; a primer set against the KIAA1468-RET fusion polynucleotide, which consists of a polynucleotide consisting of the nucleotide sequence of SEQ ID NO: 13 and a polynucleotide consisting of the nucleotide sequence of SEQ ID NO: 14; a primer set against the TRIM24-BRAF fusion polynucleotide, which consists of a polynucleotide consisting of the nucleotide sequence of SEQ ID NO: 15 and a polynucleotide consisting of the nucleotide sequence of SEQ ID NO: 16; a primer set against the CD74-NRG1 fusion polynucleotide, which consists of a polynucleotide consisting of the nucleotide sequence of SEQ ID NO: 17 and a polynucleotide consisting of the nucleotide sequence of SEQ ID NO: 18; and a primer set against the SLC3A2-NRG1 fusion polynucleotide, which consists of a polynucleotide consisting of the nucleotide sequence of SEQ ID NO: 37 and a polynucleotide consisting of the nucleotide sequence of SEQ ID NO: 18 (refer to Table 1 given below).

In the process of detecting a fusion polynucleotide by PCR, direct sequencing is performed on PCR products to sequence a nucleotide sequence containing a point of fusion, whereby it can be confirmed that a gene segment toward the 5'-end and a gene segment toward the 3'-end are joined in-frame and/or that a specified domain is contained in the fusion polynucleotide. Sequencing can be done by a known method -- it can be easily done by using a sequencer (e.g., ABI-PRISM 310 Genetic Analyzer (Applied Biosystems Inc.)) in accordance with its operating instructions.

Also, in the process of detecting a fusion polynucleotide by PCR, it can be confirmed by the TaqMan probe method that a gene segment toward the 5'-end and a gene segment toward the 3'-end are joined in-frame and/or that a specified domain is contained in the fusion polynucleotide. The probe to be used in the TaqMan probe method can be exemplified by the polynucleotide mentioned above in (a) or (b). The probe is labeled with a reporter dye (e.g., FAM, FITC, VIC) and a quencher (e.g., TAMRA, Eclipse, DABCYL, MGB).

The above-mentioned primers and probes may be DNA, RNA, or DNA/RNA chimera, and preferably is DNA. Alternatively, the primers and probes may be such that part or all of the nucleotides are substituted by an artificial nucleic acid such as PNA (polyamide nucleic acid: a peptide nucleic acid), LNA® (Locked Nucleic Acid; a bridged nucleic acid), ENA® (2'-0,4'-C-Ethylene-bridged Nucleic Acid), GNA (glycerol nucleic acid) or TNA (threose nucleic acid). Further, the primers and probes may be double- or single-stranded, and preferably are single-stranded.

As far as the primers and probes are capable of specifically hybridizing to a target sequence, they may contain one or more nucleotide mismatches, generally have at least 80% identity, preferably at least 90%, at least 91%, at least 92%, at least 93%, or at least 94% identity, more preferably at least 95%, at least 96%, at least 97%, at least 98%, or at least 99% identity, and most preferably 100% identity, to a sequence complementary to the target sequence.

The primers and probes can be synthesized, for example, according to a conventional method using an automatic DNA/RNA synthesizer on the basis of the information on the nucleotide sequences disclosed in the present specification.

In the inventive detection method, it is also acceptable to detect a fusion polynucleotide of interest by whole-transcriptome sequencing (RNA sequencing) or genome sequencing. These techniques can be carried out, for example, using a next-generation sequencer (e.g., Genome Analyzer IIx (Illumina), HiSeq sequencer (HiSeq 2000, Illumina), Genome Sequencer FLX System (Roche)), or the like according to the manufacturer's instructions. RNA sequencing can be done by, for example, preparing a cDNA library from a total RNA using a commercially available kit (e.g., mRNA-Seq sample preparation kit (Illumina)) according to the manufacturer's instructions and sequencing the prepared library using a next-generation sequencer.

In the inventive detection method, if the object to be detected is a translation product of a fusion polynucleotide (i.e., fusion polynucleotide), the translation product can be detected using, for example, immunostaining, Western blotting, RIA, ELISA, flow cytometry, immunoprecipitation, or antibody array analysis. These techniques use an antibody that specifically recognizes a fusion polypeptide. As used herein, the phrase "specifically recognizes a fusion polypeptide" means that the antibody does not recognize other proteins than said fusion polynucleotide, including wild-type proteins from which to derive both segments of said fusion polynucleotide each extending from a point of fusion toward the Nor C-terminus, but recognizes said fusion polynucleotide alone. The antibody that "specifically recognizes a fusion polypeptide", which is to be used in the inventive detection method, can be one antibody or a combination of two or more antibodies.

The "antibody that specifically recognizes a fusion polypeptide" can be exemplified by an antibody specific to a polypeptide containing a point of fusion in said fusion polypeptide (hereinafter referred to as the "fusion point-specific antibody"). As referred to herein, the "fusion point-specific antibody" means an antibody that specifically binds to the polypeptide containing said fusion point but does not bind to wild-type proteins from which to derive the segments of the fusion polypeptide each extending toward the N- or C-terminus.

Also, the "antibody that specifically recognizes a fusion polypeptide" can be exemplified by a combination of an antibody binding to a polypeptide consisting of that region of the fusion polypeptide which extends from a point of fusion toward the N-terminus and an antibody binding to a polypeptide consisting of that region of the fusion polypeptide which extends from a point of fusion toward the C-terminus. The fusion polypeptide can be detected by performing sandwich ELISA, immunostaining, immunoprecipitation, Western blotting or the like using these two antibodies.

In the present invention, examples of the antibodies include, but are not limited to, natural antibodies such as polyclonal antibodies and monoclonal antibodies (mAb), and chimeric, humanized and single-stranded antibodies which can be prepared using genetic recombination techniques, and binding fragments thereof. The "binding fragments" refers to partial regions of the above-mentioned antibodies which have specific binding activity, and specific examples include Fab, Fab', F(ab')₂, Fv, and single-chain antibodies. The class of antibody is not particularly limited, and any antibody having any isotype, such as IgG, IgM, IgA, IgD or IgE, is acceptable, with IgG being preferred in consideration of ease of purification or other factors.

The "antibody that specifically recognizes a fusion polypeptide" can be prepared by those skilled in the art through selection of a known technique as appropriate. Examples of such a known technique include: a method in which a polypeptide containing a point of fusion in the fusion polypeptide, a polypeptide consisting of that region of the fusion polypeptide which extends from a point of fusion toward the N-terminus, or a polypeptide consisting of that region of the fusion polypeptide which extends from a point of fusion toward the C-terminus is inoculated into an immune animal, the immune system of the animal is activated, and then the serum (polyclonal antibody) of the animal is collected; as well as monoclonal antibody preparation methods such as hybridoma method, recombinant DNA method, and phage display method. Commercially available antibodies may also be used. If an antibody having a labeling agent attached thereto is used, the target protein can be detected directly by detecting this label. The labeling agent is not particularly limited as long as it is capable of binding to an antibody and is detectable, and examples include peroxidase, β-D-galactosidase, microperoxidase, horseradish peroxidase (HRP), fluorescein isothiocyanate (FITC), rhodamine isothiocyanate (RITC), alkaline phosphatase, biotin, and radioactive materials. In addition to the direct detection of the target protein using the antibody having a labeling agent attached thereto, the target protein can also be detected indirectly using a secondary antibody having a labeling agent attached thereto, Protein G or A, or the like.

### <Kit for detecting gene fusions serving as responsible mutations (driver mutations) for cancer>

As described above, fusion polynucleotides produced by gene fusions serving as responsible mutations for cancer, or polypeptides encoded thereby, can be detected using such a primer, probe, or antibody as mentioned above, or a combination thereof, whereby the gene fusions can be detected. Thus, the present invention provides a kit for detecting a gene fusion serving as a responsible mutation for cancer, the kit comprising any one of (A) to (C) mentioned below, or a combination thereof (hereinafter also referred to as the "inventive kit"):
(A) a polynucleotide that serves as a probe designed to specifically recognize an EZR-ERBB4 fusion polynucleotide, a KIAA1468-RET fusion polynucleotide, a TRIM24-BRAF fusion polynucleotide, a CD74-NRG1 fusion polynucleotide, or an SLC3A2-NRG1 fusion polynucleotide;
(B) polynucleotides that serve as a pair of primers designed to enable specific amplification of an EZR-ERBB4 fusion polynucleotide, a KIAA1468-RET fusion polynucleotide, a TRIM24-BRAF fusion polynucleotide, a CD74-NRG1 fusion polynucleotide, or an SLC3A2-NRG1 fusion polynucleotide; and
(C) an antibody that specifically recognizes an EZR-ERBB4 fusion polypeptide, a KIAA1468-RET fusion polypeptide, a TRIM24-BRAF fusion polypeptide, a CD74-NRG1 fusion polypeptide, or an SLC3A2-NRG1 fusion polypeptide.

In addition to the above-mentioned polynucleotide(s) or antibody, the inventive kit can also contain an appropriate combination of other components, including: a substrate required for detecting a label attached to the polynucleotide(s) or the antibody; a positive control (e.g., EZR-ERBB4 fusion polynucleotide, KIAA1468-RET fusion polynucleotide, TRIM24-BRAF fusion polynucleotide, CD74-NRG1 fusion polynucleotide, or SLC3A2-NRG1 fusion polynucleotide; or EZR-ERBB4 fusion polypeptide, KIAA1468-RET fusion polypeptide, TRIM24-BRAF fusion polypeptide, CD74-NRG1 fusion polypeptide, or SLC3A2-NRG1 fusion polypeptide; or cells bearing the same); a negative control; a PCR reagent; a counterstaining reagent for use for *in situ* hybridization or the like (e.g., DAPI); a molecule required for antibody detection (e.g., secondary antibody, Protein G, Protein A); and a buffer solution for use in sample dilution or washing. The inventive kit can contain instructions for use thereof. The inventive detection method can be easily carried out by using the inventive kit.

The inventive detection method and kit, which enable detection of gene fusions newly discovered as responsible mutations for cancer, are very useful in identifying subjects positive for said gene fusions and applying personalized medicine to each of the subjects, as described below.

### <Method for identifying patients with cancer or subjects with a risk of cancer>

The above-mentioned five types of gene fusions, serving as responsible mutations for cancer, are each believed to lead to constitutive activation of ERBB4 kinase activity, constitutive activation of RET kinase activity, constitutive activation of BRAF kinase activity, and enhancement of the function of NRG1 as a cell growth factor, thereby contributing to malignant transformation of cancers. Thus, it is highly probable that cancer patients with detection of such a gene fusion are responsive to the treatment with substances suppressing the expression and/or activity of polypeptides encoded by fusion polynucleotides produced by said gene fusions.

Thus, the present invention provides a method for identifying patients with cancer or subjects with a risk of cancer, in which substances suppressing the expression and/or activity of polypeptides encoded by fusion polynucleotides produced by gene fusions serving as responsible mutations (driver mutations) for cancer show a therapeutic effect (hereinafter also referred to as the "inventive identification method").

The inventive identification method comprises the steps of:
(1) detecting a fusion polynucleotide of any one of (a) to (e) mentioned below, or a polypeptide encoded thereby, in an isolated sample from a subject:
   (a) an EZR-ERBB4 fusion polynucleotide which encodes a polypeptide comprising all or part of the coiled-coil domain of EZR, and the kinase domain of ERBB4, and having kinase activity,
   (b) a KIAA1468-RET fusion polynucleotide which encodes a polypeptide comprising all or part of the coiled-coil domain of KIAA1468, and the kinase domain of RET, and having kinase activity,
   (c) a TRIM24-BRAF fusion polynucleotide which encodes a polypeptide comprising the kinase domain of BRAF and having kinase activity,
   (d) a CD74-NRG1 fusion polynucleotide which encodes a polypeptide comprising the transmembrane domain of CD74 and the EGF domain of NRG1, and having intracellular signaling-enhancing activity, and
   (e) an SLC3A2-NRG1 fusion polynucleotide which encodes a polypeptide comprising the transmembrane domain of SLC3A2 and the EGF domain of NRG1, and having intracellular signaling-enhancing activity; and
(2) determining that a substance suppressing the expression and/or activity of the polypeptide shows a therapeutic effect in the subject, in the case where the fusion polynucleotide of any one of (a) to (e) or the polypeptide encoded thereby is detected.

In the inventive identification method, the "patients with cancer or subjects with a risk of cancer" refers to mammals, preferably humans, which are affected with or suspected of having cancer. The "cancer" to which the inventive identification method is to be applied is not particularly limited as long as it is a cancer in which any of the above-mentioned five types of gene fusions can be detected, with lung cancer being preferred, non-small-cell lung carcinoma being more preferred, and lung adenocarcinoma being particularly preferred.

In the inventive identification method, the "therapeutic effect" is not particularly limited as long as it is a cancer treatment effect of benefit to a patient, and examples include a tumor shrinkage effect, a progression-free survival prolongation effect, and a life lengthening effect.

In the inventive identification method, the "substance suppressing the expression and/or activity of a polypeptide encoded by a fusion polynucleotide produced by a gene fusion serving as a responsible mutation (driver mutation) for cancer", which is to be evaluated for effectiveness in cancer treatment (this substance is hereinafter also referred to as the "substance to be evaluated in the inventive identification method"), with regard to EZR-ERBB4 fusion, is not particularly limited as long as it is a substance that directly or indirectly inhibits the expression and/or function of an EZR-ERBB4 fusion polypeptide.

Examples of the substance inhibiting the expression of an EZR-ERBB4 fusion polypeptide include: siRNAs (small interfering RNAs), shRNAs (short hairpin RNA), miRNAs (micro RNAs), and antisense nucleic acids which suppress the expression of an EZR-ERBB4 fusion polypeptide; expression vectors capable of expressing these polynucleotides; and low-molecular-weight compounds.

Examples of the substance inhibiting the function of an EZR-ERBB4 fusion polypeptide include substances inhibiting the kinase domain of ERBB4 (e.g., low-molecular-weight compounds), and antibodies binding to an EZR-ERBB4 fusion polypeptide.

These substances may be substances that specifically suppress the expression and/or activity of an EZR-ERBB4 fusion polypeptide, or may be substances that suppress even the expression and/or activity of wild-type ERBB4 protein. Specific examples of such substances include afatinib and dacomitinib.

These substances can be prepared by a *per se* known technique on the basis of the sequence information of an EZR-ERBB4 fusion polynucleotide and/or an EZR-ERBB4 fusion polypeptide which are disclosed in the present specification, or other data. Commercially available substances may also be used.

These substances are effective as cancer therapeutic agents for subjects with cancer, in the case where an EZR-ERBB4 fusion polynucleotide or a polypeptide encoded thereby is detected in isolated samples from said subjects.

The substance to be evaluated in the inventive identification method, with regard to KIAA1468-RET fusion, is not particularly limited as long as it is a substance that directly or indirectly inhibits the expression and/or function of a KIAA1468-RET fusion polypeptide.

Examples of the substance inhibiting the expression of a KIAA1468-RET fusion polypeptide include: siRNAs (small interfering RNAs), shRNAs (short hairpin RNA), miRNAs (micro RNAs), and antisense nucleic acids which suppress the expression of a KIAA1468-RET fusion polypeptide; expression vectors capable of expressing these polynucleotides; and low-molecular-weight compounds.

Examples of the substance inhibiting the function of a KIAA1468-RET fusion polypeptide include substances inhibiting the kinase activity of RET (e.g., low-molecular-weight compounds), and antibodies binding to a KIAA1468-RET fusion polypeptide.

These substances may be substances that specifically suppress the expression and/or activity of a KIAA1468-RET fusion polypeptide, or may be substances that suppress even the expression and/or activity of wild-type RET protein. Specific examples of such substances include vandetanib, cabozantinib, sorafenib, sunitinib, lenvatinib, and ponatinib.

These substances can be prepared by a *per se* known technique on the basis of the sequence information of a KIAA1468-RET fusion polynucleotide and/or a KIAA1468-RET fusion polypeptide which are disclosed in the present specification, or other data. Commercially available substances may also be used.

These substances are effective as cancer therapeutic agents for subjects with cancer, in the case where a KIAA1468-RET fusion polynucleotide or a polypeptide encoded thereby is detected in isolated samples from said subjects.

The substance to be evaluated in the inventive identification method, with regard to TRIM24-BRAF fusion, is not particularly limited as long as it is a substance that directly or indirectly inhibits the expression and/or function of a TRIM24-BRAF fusion polypeptide.

Examples of the substance inhibiting the expression of a TRIM24-BRAF fusion polypeptide include: siRNAs (small interfering RNAs), shRNAs (short hairpin RNA), miRNAs (micro RNAs), and antisense nucleic acids which suppress the expression of a TRIM24-BRAF fusion polypeptide; expression vectors capable of expressing these polynucleotides; and low-molecular-weight compounds.

Examples of the substance inhibiting the function of a TRIM24-BRAF fusion polypeptide include substances inhibiting the kinase activity of BRAF (e.g., low-molecular-weight compounds), and antibodies binding to a TRIM24-BRAF fusion polypeptide.

These substances may be substances that specifically suppress the expression and/or activity of a TRIM24-BRAF fusion polypeptide, or may be substances that suppress even the expression and/or activity of wild-type BRAF protein. Specific examples of such substances include vemurafenib and dabrafenib.

These substances can be prepared by a *per se* known technique on the basis of the sequence information of a TRIM24-BRAF fusion polynucleotide and/or a TRIM24-BRAF fusion polypeptide which are disclosed in the present specification, or other data. Commercially available substances may also be used.

These substances are effective as cancer therapeutic agents for subjects with cancer, in the case where a TRIM24-BRAF fusion polynucleotide or a polypeptide encoded thereby is detected in isolated samples from said subjects.

The substance to be evaluated in the inventive identification method, with regard to CD74-NRG1 fusion, is not particularly limited as long as it is a substance that directly or indirectly inhibits the expression and/or function of a CD74-NRG1 fusion polypeptide.

Examples of the substance inhibiting the expression of a CD74-NRG1 fusion polypeptide include: siRNAs (small interfering RNAs), shRNAs (short hairpin RNA), miRNAs (micro RNAs), and antisense nucleic acids which suppress the expression of a CD74-NRG1 fusion polypeptide; expression vectors capable of expressing these polynucleotides; and low-molecular-weight compounds.

Examples of the substance inhibiting the function of a CD74-NRG1 fusion polypeptide include substances inhibiting the intracellular signaling-enhancing activity of NRG1 (e.g., low-molecular-weight compounds), and antibodies binding to a CD74-NRG1 fusion polypeptide.

These substances may be substances that specifically suppress the expression and/or activity of a CD74-NRG1 fusion polypeptide, or may be substances that suppress even the expression and/or activity of wild-type NRG1 protein. Specific examples of such substances include the BACE protein inhibitors MK-8931 and E2609 which are involved in the cleavage of NRG1 protein.

These substances can be prepared by a *per se* known technique on the basis of the sequence information of a CD74-NRG1 fusion polynucleotide and/or a CD74-NRG1 fusion polypeptide which are disclosed in the present specification, or other data. Commercially available substances may also be used.

Further, since wild-type NRG1 protein is believed to enhance intracellular signaling via a protein belonging to a group of HER proteins serving as receptors for the wild-type NRG1 protein, said substance to be evaluated in the inventive identification method can also be exemplified by substances that directly or indirectly suppress the expression and/or function of HER proteins. As referred to herein, the group of HER proteins is a group of tyrosine kinase receptors which consists of the following four proteins: HER1 (ErbB1), HER2 (ErbB2), HER3 (ErbB3), and HER4 (ErbB4).

Examples of the substances inhibiting the expression of HER proteins include: siRNAs (small interfering RNAs), shRNAs (short hairpin RNA), miRNAs (micro RNAs), and antisense nucleic acids which suppress the expression of HER proteins; expression vectors capable of expressing these polynucleotides; and low-molecular-weight compounds.

Examples of the substances inhibiting the function of HER proteins include substances inhibiting the kinase activity of HER proteins (e.g., low-molecular-weight compounds), and antibodies binding to HER proteins. Specific examples of such substances include lapatinib, afatinib, dacomitinib, and trastuzumab.

These substances can be prepared by a *per se* known technique on the basis of known sequence information or other data. Commercially available substances may also be used.

These substances are effective as cancer therapeutic agents for subjects with cancer, in the case where a CD74-NRG1 fusion polynucleotide or a polypeptide encoded thereby is detected in isolated samples from said subjects.

The substance to be evaluated in the inventive identification method, with regard to SLC3A2-NRG1 fusion, is not particularly limited as long as it is a substance that directly or indirectly inhibits the expression and/or function of an SLC3A2-NRG1 fusion polypeptide.

Examples of the substance inhibiting the expression of an SLC3A2-NRG1 fusion polypeptide include: siRNAs (small interfering RNAs), shRNAs (short hairpin RNA), miRNAs (micro RNAs), and antisense nucleic acids which suppress the expression of an SLC3A2-NRG1 fusion polypeptide; expression vectors capable of expressing these polynucleotides; and low-molecular-weight compounds.

Examples of the substance inhibiting the function of an SLC3A2-NRG1 fusion polypeptide include substances inhibiting the intracellular signaling-enhancing activity of NRG1 (e.g., low-molecular-weight compounds), and antibodies binding to an SLC3A2-NRG1 fusion polypeptide.

These substances may be substances that specifically suppress the expression and/or activity of an SLC3A2-NRG1 fusion polypeptide, or may be substances that suppress even the expression and/or activity of wild-type NRG1 protein. Specific examples of such substances include the BACE protein inhibitors MK-8931 and E2609 which are involved in the cleavage ofNRG1 protein.

These substances can be prepared by a *per se* known technique on the basis of the sequence information of an SLC3A2-NRG1 fusion polynucleotide and/or an SLC3A2-NRG1 fusion polypeptide which are disclosed in the present specification, or other data. Commercially available substances may also be used.

Further, since wild-type NRG1 protein is believed to enhance intracellular signaling via a protein belonging to a group of HER proteins serving as receptors for the wild-type NRG1 protein, said substance to be evaluated in the inventive identification method can also be exemplified by substances that directly or indirectly suppress the expression and/or function of HER proteins. Examples of these substances include the substances mentioned above in relation to the CD74-NRG1 fusion.

These substances can be prepared by a *per se* known technique on the basis of known sequence information or other data. Commercially available substances may also be used.

These substances are effective as cancer therapeutic agents for subjects with cancer, in the case where an SLC3A2-NRG1 fusion polynucleotide or a polypeptide encoded thereby is detected in isolated samples from said subjects.

Step (1) in the inventive identification method can be carried out in the same way as the step included in the inventive detection method mentioned above.

At step (2) in the inventive identification method, the substance to be evaluated in the inventive identification method is determined to show a therapeutic effect in a subject with cancer (i.e., a patient with cancer or a subject with a risk of cancer), in the case where a fusion polynucleotide of interest or a polypeptide encoded thereby is detected in an isolated sample from the subject at step (1); however, the substance to be evaluated in the inventive identification method is determined to be unlikely to show a therapeutic effect in the subject, in the case where none of the fusion polynucleotide of interest or the polypeptide encoded thereby is detected.

According to the inventive identification method, it is possible to detect subjects positive for the gene fusions newly discovered as responsible mutations for cancer from among patients with cancer or subjects with a risk of cancer, and to identify patients with cancer or subjects with a risk of cancer, in which substances suppressing the expression and/or activity of polypeptides encoded by fusion polynucleotides produced by said gene fusions show a therapeutic effect; thus, the present invention is useful in that it enables provision of suitable treatment for such subjects.

### <Method for treatment of cancer and cancer therapeutic agent>

As described above, the inventive identification method identifies patients with cancer, in which substances suppressing the expression and/or activity of polypeptides encoded by fusion polynucleotides produced by any of the above-mentioned five types of gene fusions show a therapeutic effect. Thus, efficient cancer treatments can be performed by administering said substances selectively to those cancer patients who carry said fusion genes. Therefore, the present invention provides a method for treating cancer, comprising the step of administering said substances to subjects in which said substances are determined to show a therapeutic effect by the inventive identification method mentioned above (hereinafter also referred to as the "inventive treatment method").

Also, since the substances to be administered in the inventive treatment method function as cancer therapeutic agents, the present invention further provides a cancer therapeutic agent comprising, as an active ingredient, a substance suppressing the expression and/or activity of a polypeptide encoded by a fusion polynucleotide produced by any of the above-mentioned five types of gene fusions (hereinafter also referred to as the "inventive cancer therapeutic agent").

The inventive cancer therapeutic agent can be exemplified by the substances that are mentioned, in relation to the inventive identification method, as substances suppressing the expression and/or activity of polypeptides encoded by fusion polynucleotides produced by any of the above-mentioned five types of gene fusions.

The inventive cancer therapeutic agent can be prepared as a pharmaceutical composition using a pharmaceutically acceptable carrier, an excipient and/or other additives which are commonly used in pharmaceutical manufacturing.

The method for administering the inventive cancer therapeutic agent is selected as appropriate depending on the type of the inhibitor and the type of cancer, and exemplary modes of administration that can be adopted include oral, intravenous, intraperitoneal, transdermal, intramuscular, intratracheal (aerosol), rectal and intravaginal administrations.

The dose of the inventive cancer therapeutic agent can be determined as appropriate in consideration of the activity and type of an active ingredient, the mode of administration (e.g., oral or parenteral administration), the severity of a disease, the animal species, drug receptivity, body weight, and age of the subject to be administered the inventive agent, and other factors.

The treatment method and cancer therapeutic agent of the present invention are useful in that they allow treatment of patients with the particular responsible mutations for cancer which have been conventionally unknown and were first discovered according to this invention.

### <Method for screening cancer therapeutic agents>

The present invention provides a method for screening cancer therapeutic agents which show a therapeutic effect in cancer patients having any of the above-mentioned five types of gene fusions (hereinafter also referred to as the "inventive screening method"). According to the inventive screening method, substances suppressing the expression and/or activity of any of the above-mentioned five types of fusion polypeptides (i.e., EZR-ERBB4 fusion polypeptide, KIAA1468-RET fusion polypeptide, TRIM24-BRAF fusion polypeptide, CD74-NRG1 fusion polypeptide, and SLC3A2-NRG1 fusion polypeptide) can be obtained as cancer therapeutic agents.

The test substance to be subjected to the inventive screening method can be any compound or composition and can be exemplified by nucleic acids (e.g., nucleoside, oligonucleoside, polynucleoside), saccharides (e.g., monosaccharide, disaccharide, oligosaccharide, polysaccharide), fats (e.g., saturated or unsaturated, straight-chain, branched-chain and/or cyclic fatty acids), amino acids, proteins (e.g., oligopeptide, polypeptide), low-molecular-weight compounds, compound libraries, random peptide libraries, natural ingredients (e.g., ingredients derived from microbes, animals and plants, marine organisms, and others), foods, and the like.

The inventive screening method can be of any type as long as it enables evaluation of whether a test substance suppresses the expression and/or activity of any of the above-mentioned five types of fusion polypeptides. Typically, the inventive screening method comprises the following steps:
(1) bringing a cell expressing an EZR-ERBB4 fusion polypeptide, a KIAA1468-RET fusion polypeptide, a TRIM24-BRAF fusion polypeptide, a CD74-NRG1 fusion polypeptide, or an SLC3A2-NRG1 fusion polypeptide into contact with a test substance;
(2) judging whether the substance suppresses the expression and/or activity of the fusion polypeptide or not; and
(3) selecting the substance judged to suppress the expression and/or activity of the fusion polypeptide, as a cancer therapeutic agent.

At step (1), the cell expressing any of the above-mentioned five types of fusion polypeptides is brought into contact with the test substance. A test substance-free solvent (e.g., DMSO) can be used as a control. The contact can be effected in a medium. The medium is selected as appropriate depending on various factors including the type of the cell to be used, and examples include a minimum essential medium (MEM) supplemented with about 5-20% fetal bovine serum, a Dulbecco's modified eagle's medium (DMEM), RPMI1640 medium, and 199 medium. The culture conditions are also selected as appropriate depending on various factors including the type of the cell to be used, and for example, the pH of the medium is in the range of about 6 to about 8, the culture temperature is in the range of about 30°C to about 40°C, and the culture time is in the range of about 12 hours to about 72 hours.

Examples of the cell expressing any of the above-mentioned five types of fusion polypeptides include, but are not limited to, cancer tissue-derived cells intrinsically expressing said fusion polypeptides, cell lines induced from said cells, and cell lines made by genetic engineering. Whether a cell expresses any of the above-mentioned five types of fusion polypeptides can also be confirmed using the inventive detection method described above. The cell is generally a mammalian cell, preferably a human cell.

At step (2), it is judged whether the test substance suppresses the expression and/or activity of said fusion polypeptide or not. The expression of fusion polypeptides can be measured by determining the mRNA or protein level in a cell using a known analysis technique such as Northern blotting, quantitative PCR, immunoblotting, or ELISA. Also, the activity of fusion polypeptides can be measured by a known analysis technique (e.g., kinase activity assay). The resulting measured value is compared with the value measured in a control cell not contacted with the test substance. The comparison of the measured values is made preferably based on the presence or absence of a significant difference. If the value measured in the cell contacted with the test substance is significantly lower than that measured in the control cell, it can be judged that the test substance suppresses the expression and/or activity of said fusion polypeptide.

Alternatively, since the cells expressing these types of fusion polypeptides show enhanced growth, the growth of said cells can be used as an indicator for the judgment at this step. In this case, the growth of such a cell contacted with the test substance is measured as a first step. The cell growth measurement can be made by a *per se* known technique such as cell count, ³H-thymidine incorporation, or BRDU. Next, the growth of the cell contacted with the test substance is compared with that of a control cell not contacted with the test substance. The growth level comparison is made preferably based on the presence or absence of a significant difference. The value for the growth of the control cell not contacted with the test substance can be a value measured prior to, or at the same time as, the measurement of the growth of the cell contacted with the test substance, and the value measured at the same time is preferred from the viewpoint of the accuracy and reproducibility of the test. If the results of the comparison show that the growth of the cell contacted with the test substance is suppressed, it can be judged that the test substance suppresses the expression and/or activity of said fusion polypeptide.

At step (3), the test substance judged to suppress the expression and/or activity of said fusion polypeptide at step (2) is selected as a cancer therapeutic agent.

Thus, the inventive screening method makes it possible to obtain cancer therapeutic agents applicable to the treatment of patients with responsible mutations for cancer which have been conventionally unknown.

### <Isolated fusion polypeptides or fragments thereof, and polynucleotides encoding the same>

The present invention provides the isolated fusion polypeptide (hereinafter also referred to as the "inventive fusion polypeptides") mentioned below, or fragments thereof:
(1) an isolated EZR-ERBB4 fusion polypeptide which comprises all or part of the coiled-coil domain of EZR protein, and the kinase domain of ERBB4 protein, and has kinase activity;
(2) an isolated KIAA1468-RET fusion polypeptide which comprises all or part of the coiled-coil domain of KIAA1468 protein, and the kinase domain of RET protein, and has kinase activity;
(3) an isolated TRIM24-BRAF fusion polypeptide which comprises the kinase domain of BRAF protein and has kinase activity;
(4) an isolated CD74-NRG1 fusion polypeptide which comprises the transmembrane domain of CD74 protein and the EGF domain ofNRG1 protein, and has intracellular signaling-enhancing activity; and
(5) an isolated SLC3A2-NRG1 fusion polypeptide which comprises the transmembrane domain of SLC3A2 protein and the EGF domain of NRG1 protein, and has intracellular signaling-enhancing activity.

For the purpose of the present specification, the "isolated" substance refers to a substance substantially separated or purified from other substances (preferably, biological factors) found in an environment in which the substance naturally occurs (e.g., in a cell of an organism) (for example, if the substance of interest is a nucleic acid, the "other substances" corresponds to other factors than nucleic acids as well as nucleic acids containing other nucleic acid sequences than that of the nucleic acid of interest; and if the substance of interest is a protein, the "other substances" corresponds to other factors than proteins as well as amino acids containing other amino acid sequences than that of the protein of interest). For the purpose of the specification, the term "isolated" means that a substance has a purity of preferably at least 75% by weight, more preferably at least 85% by weight, still more preferably at least 95% by weight, and most preferably at least 96% by weight, at least 97% by weight, at least 98% by weight, at least 99% by weight, or 100%. The "isolated" polynucleotides and polypeptides include not only polynucleotides and polypeptides purified by standard purification techniques but also chemically synthesized polynucleotides and polypeptides.

The meanings of other terms used above in (1) to (5) are as defined above in <Specific responsible mutations for cancer>.

The "fragments" refers to fragments of the inventive fusion polypeptides, which each consist of a consecutive partial sequence comprising sequences upstream and downstream from the point of fusion. The sequence upstream from the point of fusion, as contained in said partial sequence, can comprise at least one amino acid residue (for example, at least 2, 3, 4, 5, 6, 7, 8, 9, 10, 20, 30, 40, 50 or 100 amino acid residues) from the point of fusion to the N-terminus of any of the inventive fusion polypeptides. The sequence downstream from the point of fusion, as contained in said partial sequence, can comprise at least one amino acid residue (for example, at least 2, 3, 4, 5, 6, 7, 8, 9, 10, 20, 30, 40, 50 or 100 amino acid residues) from the point of fusion to the C-terminus of any of the inventive fusion polypeptides. The length of the fragments is not particularly limited, and is generally at least 8 amino acid residues (for example, at least 9, 10, 11, 12, 13, 14, 15, 20, 25, 50 or 100 amino acid residues).

Also, the inventive fusion polypeptides can be, for example, the isolated fusion polypeptides mentioned below:
(1) an EZR-ERBB4 fusion polypeptide which is any one of (i) to (iii) mentioned below:
   (i) a polypeptide consisting of the amino acid sequence of SEQ ID NO: 2,
   (ii) a polypeptide consisting of an amino acid sequence derived from the amino acid sequence of SEQ ID NO: 2 by deletion, substitution or addition of one or more amino acids, and the polypeptide having kinase activity, and
   (iii) a polypeptide consisting of an amino acid sequence having a sequence identity of at least 80% to the amino acid sequence of SEQ ID NO: 2, and the polypeptide having kinase activity;
(2) a KIAA1468-RET fusion polypeptide which is any one of (i) to (iii) mentioned below:
   (i) a polypeptide consisting of the amino acid sequence of SEQ ID NO: 4,
   (ii) a polypeptide consisting of an amino acid sequence derived from the amino acid sequence of SEQ ID NO: 4 by deletion, substitution or addition of one or more amino acids, and the polypeptide having kinase activity, and
   (iii) a polypeptide consisting of an amino acid sequence having a sequence identity of at least 80% to the amino acid sequence of SEQ ID NO: 4, and the polypeptide having kinase activity;
(3) a TRIM24-BRAF fusion polypeptide which is any one of (i) to (iii) mentioned below:
   (i) a polypeptide consisting of the amino acid sequence of SEQ ID NO: 6,
   (ii) a polypeptide consisting of an amino acid sequence derived from the amino acid sequence of SEQ ID NO: 6 by deletion, substitution or addition of one or more amino acids, and the polypeptide having kinase activity, and
   (iii) a polypeptide consisting of an amino acid sequence having a sequence identity of at least 80% to the amino acid sequence of SEQ ID NO: 6, and the polypeptide having kinase activity;
(4) a CD74-NRG1 fusion polypeptide which is any one of (i) to (iii) mentioned below:
   (i) a polypeptide consisting of the amino acid sequence of SEQ ID NO: 8 or 10,
   (ii) a polypeptide consisting of an amino acid sequence derived from the amino acid sequence of SEQ ID NO: 8 or 10 by deletion, substitution or addition of one or more amino acids, and the polypeptide having intracellular signaling-enhancing activity, and
   (iii) a polypeptide consisting of an amino acid sequence having a sequence identity of at least 80% to the amino acid sequence of SEQ ID NO: 8 or 10, and the polypeptide having intracellular signaling-enhancing activity; and
(5) an SLC3A2-NRG1 fusion polypeptide which is any one of (i) to (iii) mentioned below:
   (i) a polypeptide consisting of the amino acid sequence of SEQ ID NO: 36,
   (ii) a polypeptide consisting of an amino acid sequence derived from the amino acid sequence of SEQ ID NO: 36 by deletion, substitution or addition of one or more amino acids, and the polypeptide having intracellular signaling-enhancing activity, or
   (iii) a polypeptide consisting of an amino acid sequence having a sequence identity of at least 80% to the amino acid sequence of SEQ ID NO: 36, and the polypeptide having intracellular signaling-enhancing activity.

The meanings of the terms used above in (i) to (iii) are as defined above in <Specific responsible mutations for cancer>.

Further, the present invention provides isolated polynucleotides encoding the inventive fusion polypeptides or the fragments thereof as described above (hereinafter also referred to as the "inventive polynucleotides"). The inventive polynucleotides can be any of mRNA, cDNA and genomic DNA. Also, the polynucleotides may be double- or single-stranded.

A typical example of the cDNA encoding the EZR-ERBB4 fusion polypeptide is a polynucleotide consisting of the nucleotide sequence of SEQ ID NO: 1.

A typical example of the cDNA encoding the KIAA1468-RET fusion polypeptide is a polynucleotide consisting of the nucleotide sequence of SEQ ID NO: 3.

A typical example of the cDNA encoding the TRIM24-BRAF fusion polypeptide is a polynucleotide consisting of the nucleotide sequence of SEQ ID NO: 5.

A typical example of the cDNA encoding the CD74-NRG1 fusion polypeptide is a polynucleotide consisting of the nucleotide sequence of SEQ ID NO: 7 or 9.

A typical example of the cDNA encoding the SLC3A2-NRG1 fusion polypeptide is a polynucleotide consisting of the nucleotide sequence of SEQ ID NO: 35.

The inventive polynucleotides can be made by a *per se* known technique. For example, the inventive polynucleotides can be extracted using a known hybridization technique from a cDNA library or genomic library prepared from cancer tissues or the like harboring an EZR-ERBB4 fusion polynucleotide, a KIAA1468-RET fusion polynucleotide, a TRIM24-BRAF fusion polynucleotide, a CD74-NRG1 fusion polynucleotide, or an SLC3A2-NRG1 fusion polynucleotide. The inventive polynucleotides can also be prepared by amplification utilizing a known gene amplification technique (PCR), with mRNA, cDNA or genomic DNA prepared from the cancer tissues or the like being used as a template. Alternatively, the polynucleotides can be prepared utilizing a known gene amplification or genetic recombination technique such as PCR, restriction enzyme treatment, or site-directed mutagenesis (Kramer, W. & Fritz, H. J., *Methods Enzymol.,* 1987, 154, 350), using, as starting materials, the cDNAs of wild-type genes from which to derive those segments of each of the fusion polynucleotides which extend toward the 5'- or 3'-end.

The inventive fusion polypeptides or fragments thereof can also be made by a *per se* known technique. For example, after such a polynucleotide prepared as mentioned above is inserted into an appropriate expression vector, the vector is introduced into a cell-free protein synthesis system (e.g., reticulocyte extract, wheat germ extract) and the system is incubated, or alternatively the vector is introduced into appropriate cells (e.g., *E coli.,* yeast, insect cells, animal cells) and the resulting transformant is cultured; in either way, the inventive polypeptides can be prepared.

The inventive fusion polypeptides or fragments thereof can be used as a marker in the inventive detection method or the like, or can be used in other applications including preparation of antibodies against the inventive fusion polypeptides.

### EXAMPLES

On the pages that follow, the present invention will be more specifically described based on Examples, but this invention is not limited to the examples given below.

### <Samples>

Total RNAs were prepared from lung tissues taken from cancer patients.

Total RNAs were extracted from grossly dissected, snap-frozen tissue samples using a TRIzol reagent according to the manufacturer's instructions, and were examined for quality using the model 2100 bioanalyzer (Agilent Technologies). As a result, all samples showed RIN (RNA integrity number) values greater than 6. Genomic DNAs were also extracted from the tissue samples using the QIAamp® DNA Mini kit (Qiagen). The present study was conducted with the approval by the institutional review boards of the institutions involved in the study.

### <RNA sequencing>

cDNA libraries for RNA sequencing were prepared using the mRNA-Seq sample preparation kit (Illumina) according to the manufacturer's standard protocol. Briefly, poly-A(+)RNA was purified from 2 µg of total RNA and fragmented by heating at 94°C for 5 minutes in a fragmentation buffer, before being used for double-stranded cDNA synthesis. After the resulting double-stranded cDNA was ligated to the PE adapter DNA and then amplified by PCR. The thus-created libraries were subjected to paired-end sequencing of 50- or 75-bp reads using the Genome Analyzer IIx (GAIIx) sequencer (Illumina) or the HiSeq sequencer (HiSeq 2000, Illumina).

### <Detection of fusion transcripts>

Detection of fusion transcripts was performed using the deFuse program described in McPherson, A., *et al., PLoS Comput. Biol.,* May 2011; 7 (5): e1001138. To be specific, paired-end reads were aligned with a reference sequence consisting of spliced and unspliced gene sequences. Next, for ambiguous discordant alignments which did not agree with the reference sequence, possible gene fusions of two genes were assumed and aligned. Then, such split reads across two genes that support gene fusions at a nucleotide level were detected and taken as candidates for gene fusions in consideration of the degree of corroboration with the split reads and the paired-end reads (spanning reads) consisting of two reads respectively mapped to two genes, as well as the consistency in nucleotide length between the spanning reads. Next, from these candidates, there were extracted gene fusions whose putatively encoded amino acid structures would cause activation of protein kinases and intracellular signaling pathways governed by said kinases.

### <RT-PCR, genomic PCR, Sanger sequencing>

Total RNAs (500 ng) were reverse-transcribed using Superscript® III Reverse Transcriptase (Invitrogen). The resulting cDNAs (corresponding to 10 ng total RNAs) or 10 ng genomic DNAs were subjected to PCR amplification using KAPA Taq DNA Polymerase (KAPA Biosystems). The reactions were effected in a thermal cycler under the following conditions: 40 cycles of reactions at 95°C for 30 seconds, at 60°C for 30 seconds, and at 72°C for 2 minutes, followed by a final extension reaction at 72°C for 10 minutes. The gene encoding glyceraldehyde-3-phosphate dehydrogenase (GAPDH) was amplified for estimating the efficiency of cDNA synthesis. Further, the PCR products were directly nucleotide-sequenced in both directions using the BigDye Terminator kit and the ABI 3130x1 DNA Sequencer (Applied Biosystems). The primers used in the present study are shown in Table 1.

**[Table 1]**

| Fusion gene | Forward primer | Reverse primer | RT-PCR product size (bp) |
|---|---|---|---|
| EZR-ERBB4 | AAGGAGGAGCTGGAGAGACA (SEQ ID NO: 11) | CACCTGAGCCAAGGACTTTT (SEQ ID NO: 12) | 250 |
| KIAA1468-RET | TGTCTCCTGCATTCCATCAA (SEQ ID NO: 13) | TCCAAATTCGCCTTCTCCTA (SEQ ID NO: 14) | 239 |
| TRIM24-BRAF | TGTCGAGACTGTCAGTTGTTAGAA (SEQ ID NO: 15) | GCCCAAATTGATTTCGATGA (SEQ ID NO: 16) | 250 |
| CD74-NRG1 variant 1 | CGGAGAACCTGAGACACCTT (SEQ ID NO: 17) | ACTCCCCTCCATTCACACAG (SEQ ID NO: 18) | 285 |
| CD74-NRG1 variant 2 | CGGAGAACCTGAGACACCTT (SEQ ID NO: 17) | ACTCCCCTCCATTCACACAG (SEQ ID NO: 18) | 222 |
| SLC3A2-NRG1 | CAGAAGGATGATGTCGCTCA (SEQ ID NO: 37) | ACTCCCCTCCATTCACACAG (SEQ ID NO: 18) | 184 |

### (Example 1)

This example describes the identification of novel fusion transcripts in lung cancer tissues.

In order to identify novel fusion transcripts as potential targets for therapy, 114 LADC samples and 3 non-cancerous tissues were subjected to whole-transcriptome sequencing (RNA sequencing; refer to Meyerson, M., et al., Nat. Rev. Genet., 2010, vol. 11, p. 685-696).

Paired-end reads obtained by the RNA sequencing were analyzed to perform Sanger sequencing of the reverse transcription (RT)-PCR products. As a result, there were identified four novel fusion gene products as shown in Table 2 and Fig. 1.

**[Table 2]**

| Fusion gene | Location of gene toward the 5'-end | Location of gene toward the 3'-end | Causative chromosomal aberration |
|---|---|---|---|
| EZR-ERBB4 | 6q25 | 2q34 | Translocation, t(2;6) |
| KIAA1468-RET | 18q21 | 10q11 | Translocation, t(10;18) |
| TRIM24-BRAF | 7q33 | 7q34 | Inversion, inv7 |
| CD74-NRG1 | 5q32 | 8p12 | Translocation, t(5;8) |

EZR-ERBB4 is a fusion gene created by a chromosomal translocation t(2;6) between the EZR gene on chromosome 6q25 and the ERBB4 gene on chromosome 2q34.

KIAA1468-RET is a fusion gene created by a chromosomal translocation t(10;18) between the KIAA1468 gene on chromosome 18q21 and the RET gene on chromosome 10q11.

TRIM24-BRAF is a fusion gene created by a chromosomal inversion inv7 between the TRIM24 gene on chromosome 7q33 and the BRAF gene on chromosome 7q34.

CD74-NRG1 is a fusion gene created by a chromosomal translocation t(5;8) between the CD74 gene on chromosome 5q32 and the NRG1 gene on chromosome 8p12.

Among these genes, EZR-ERBB4, KIAA1468-RET and TRIM24-BRAF were each detected in one LADC sample. For the CD74-NRG1 gene, variants (variants 1 and 2) with different breakpoints were detected from two different LADC samples.

### (Example 2)

This example describes the detection of the gene fusions found in Example 1 by RT-PCR.

For each of the fusion genes, there were prepared PCR primers (forward and reverse primers) each derived from the cDNA sequence of the gene fragment toward the 5'- or 3'-end (Table 1). PCR amplification was performed with these primers using as a template a cDNA synthesized from a cancer tissue-derived RNA.

FIG. 2 depicts the electropherograms of PCR products. Amplification of specific bands was observed in some samples, and sequencing of the nucleotide sequences of the PCR products confirmed that the fusion genes were partially amplified.

These results demonstrated that the gene fusions found in Example 1 can be detected by testing for the presence or absence of the amplification of specific bands through RT-PCR and by sequencing of the nucleotide sequences of the PCR products.

### (Example 3)

This example demonstrates that the gene fusions found in Example 1 are highly likely to be responsible mutations for lung cancer.

The five lung cancer samples in which the novel gene fusions were found in Example 1 were investigated for the presence or absence of other known responsible mutations for cancer -- i.e., EGFR point mutation/EGFR in-flame deletion mutation, KRAS point mutation, BRAF point mutation, HER2 in-flame insertion mutation, EML4-ALK fusion, KIF5B-RET fusion, CCDC6-RET fusion, CD74-ROS1 fusion, EZR-ROS1 fusion, and SLC34A2-ROS1 fusion.

As a result, these five lung cancer samples were all negative for the other known mutations, and the four types of novel gene fusions had a mutually exclusive relationship with the other known responsible mutations for cancer.

These results showed that the four types of novel gene fusions are responsible mutations for cancer.

### (Example 4)

Examples 4 and 5 show the results of further analysis of 90 of 114 cases analyzed in Example 1.

### Materials and methods

### <Samples>

The 90 cases of invasive mucinous adenocarcinoma (IMA) were identified from a consecutive series of patients with primary lung adenocarcinoma who were treated surgically at the National Cancer Center Hospital (Tokyo, Japan) between 1998 and 2013. Histological diagnosis was made based on the latest classifications of LADC provided by the World Health Organization and the International Association for the Study of Lung Cancer/American Thoracic Society/European Respiratory Society (IASLC/ATS/ERS) (Travis W. D., et al., J. Thorac. Oncol., 2011, 6, 244-85; and Travis W. D., Brambilla, E., Muller-Hermelink, H. K. and Harris, C. C., editor. World Health Organization Classification of Tumors; Pathology and Genetics, Tumours of Lung, Pleura, Thymus and Heart, Lyon: IARC Press; 2004). Total RNAs were extracted from grossly dissected, snap-frozen tissue samples using TRIzol (Invitrogen, Carlsbad, CA, USA). The present study was conducted with the approval by the institutional review boards of the participating institutions.

### <RNA sequencing>

RNA sequencing libraries were prepared from 1 µg or 2 µg of total RNA using the mRNA-Seq sample preparation kit or the TruSeq RNA sample preparation kit (Illumina, San Diego, CA, USA). The obtained libraries were subjected to paired-end sequencing of 50- or 75-bp reads on the Genome Analyzer IIx (GAIIx) sequencer or the HiSeq 2000 sequencer (Illumina). Fusion transcripts were detected using the TopHat-Fusion algorithm (Kim D., Salzberg S. L., TopHat-Fusion: an algorithm for discovery of novel fusion transcripts, Genome Biol., 2011, 12, R72).

### <Analysis of fusion products for oncogenic properties>

For the purpose of constructing lentiviral vectors for expression of CD74-NRG1, EZR-ERBB4 and TRIM24-BRAF fusion proteins, full-length cDNAs were amplified by PCR from tumor cDNAs and inserted into the pLenti-6/V5-DEST plasmids (Invitrogen). The integrity of the respective inserted cDNAs was verified by Sanger sequencing. The expression of fusion products of predicted size was verified by Western blotting analysis in transiently transfected cells and virally infected cells (Fig. 3).

### <Samples>

IMA patients constituted approximately 2% of all LADC cases who were treated surgically at the National Cancer Center Hospital (Tokyo, Japan) between 1998 and 2013. The resected tissues had been fixed in 10% formalin and embedded in paraffin. Serial 4 µm sections were stained with hematoxylin and eosin using the Alcian blue/periodic acid Schiff method to visualize cytoplasmic mucin production. Total RNAs were extracted from grossly dissected, snap-frozen tissue samples using TRIzol (Invitrogen, Carlsbad, CA, USA), and was examined for quality using the 2100 Bioanalyzer (Agilent Technologies, Santa Clara, CA, USA). All samples had RNA Integrity Numbers (RINs) greater than 6.0. Genomic DNAs were also extracted from the tissue samples using the QIAamp DNA Mini kit (Qiagen, Valencia, CA, USA). Hotspot mutations in the EGFR, KRAS, BRAF, and HER2 genes were examined by the high-resolution melting (HRM) method, and the EML4- or KIF5B-ALK, KIF5B- or CCDC6-RET, and CD74-, EZR-, or SLC34A2-ROS1 fusions were examined by RT-PCR. Detailed methods were described previously (Kohno T., et al., Nat. Med., 2012, 18, 375-7; Yoshida A., et al., Am. J. Surg. Pathol., 2013, 37, 554-62; and Kinno T., et al., Ann. Oncol., 2014, 25, 138-42).

### <RT-PCR and Sanger sequencing>

Total RNAs (500 ng) were reverse-transcribed into cDNA using Superscript III Reverse Transcriptase (Invitrogen). cDNAs (corresponding to 10 ng total RNA) or 10 ng genomic DNAs were subjected to PCR amplification using KAPA Taq DNA Polymerase (KAPA Biosystems, Woburn, MA, USA). The reactions were effected in a thermal cycler under the following conditions: 40 cycles of reactions at 95°C for 30 seconds, at 60°C for 30 seconds, and at 72°C for 2 minutes, followed by a final extension reaction at 72°C for 10 minutes. The gene encoding glyceraldehyde-3-phosphate dehydrogenase (GAPDH) was amplified for estimating the efficiency of cDNA synthesis. The PCR products were directly nucleotide-sequenced in both directions on the ABI 3130x1 DNA Sequencer (Applied Biosystems, Foster City, CA, USA) using the BigDye Terminator kit. The primers used in this study are shown in Table 1.

### <Cell lines and reagents>

NIH3T3 cells were provided by Dr. T. Yamamoto of the Okinawa Institute of Science and Technology Graduate University, Okinawa, Japan. NCI-H1299 cells were provided by Dr. J. D. Minna of the UT Southwestern Medical Center. EFM19 and 293FT cells were obtained from DSMZ (Braunschweig, Germany) and Invitrogen, respectively. H1299 and EFM-19 were cultured in an RPMI medium supplemented with 10% FBS, and NIH3T3 and 293FT were cultured in a DMEM medium supplemented with 10% FBS.

Lapatinib, afatinib, and sorafenib were purchased from Selleck (Houston, TX, USA). U0126 was purchased from Calbiochem (San Diego, CA, USA).

Primary antibodies against ERBB4 (catalog No. 2218-1) and ERBB2 (catalog No. 2064-1) were purchased from Epitomics (Burlingame, CA, USA). Antibodies against BRAF (catalog No. sc-166) and ERBB3 (catalog No. sc-285) were purchased from Santa Cruz Biotechnology (Dallas, TX, USA). An antibody against the NRG1 EGF-like domain (Wilson T. R., et al., Cancer Cell, 2011, 20, 158-72) (catalog No. RB-276) was purchased from Thermo Scientific (Fremont, CA, USA). Antibodies against phospho-ERBB4 pTyr1284 (catalog No. 4757), phospho-ERBB3 pTyr1289 (catalog No. 4791), phospho-ERBB2 pTyr1248 (catalog No. 2247), AKT (catalog No. 4691), phospho-AKT pSer473 (catalog No. 4060), total ERK1/2 (catalog No. 4695), phospho-ERK1/2 pThr202/Tyr204 (catalog No. 4370), and β-actin (catalog No. 3700) were purchased from Cell Signaling Technology (Danvers, MA, USA).

### <Immunohistochemistry>

Immunohistochemistry was performed on tissue microarray sections. Four-micrometer-thick sections were deparaffinized, and heat-induced epitope retrieval was performed using targeted retrieval solution 9 (Dako, Carpinteria, CA, USA) for BRAF and NRG, and using a citrate buffer for ERBB4. The slides were treated with 3% hydrogen peroxide for 20 minutes to block endogenous peroxidase activity, and then were washed with deionized water for 2 or 3 minutes. The slides were then incubated with the primary antibodies against BRAF (1:800, polyclonal, Sigma, St. Louis, Mo, USA), NRG1 (1:500, polyclonal, Thermo Scientific), or ERBB4 (1:100, clone E200; Abcam, Cambridge, UK) at room temperature for one hour. Immunoreactions were detected using the EnVision-FLEX and LINKER systems (Dako). The reactions were visualized with 3,3'-diaminobenzidine, followed by counterstaining with hematoxylin. Cytoplasmic staining of more than 10% of tumor cells was considered positive for BRAF and NRG1, and membrane staining was considered positive for ERBB4.

### <Fluorescence in situ hybridization>

To identify NRG1 rearrangements, fluorescence *in situ* hybridization (FISH) was performed on formalin-fixed, paraffin-embedded tumors using a break-apart probe for NRG1 (Chromosome Science Labo, Sapporo, Japan; Spectrum Orange-labeled RP11-1002K11 + RP11-35D16 as a 3' centromeric probe, and Spectrum Green-labeled RP11-23A12 + RP11-715M18 as a 5' telomeric probe).

### <Construction of lentiviral vectors for expression of CD74-NRG1, EZR-ERBB4 and TRIM24-BRAF fusion proteins>

Full-length CD74-NRG1, EZR-ERBB4, and TRIM24-BRAF cDNAs were obtained by PCR amplification of cDNAs from each index tumor sample using KOD-PLUS Taq polymerase (Toyobo, Osaka, Japan). The PCR products were digested with restriction endonucleases and ligated into pLenti-6/V5-DEST plasmids (Invitrogen). The integrity of the respective inserted cDNAs was verified by Sanger sequencing. Lentiviruses expressing CD74-NRG1, EZR-ERBB4, or TRIM24-BRAF were produced by transfecting each of the expression plasmids together with the ViraPower packaging mix (Invitrogen) into 293FT cells using the Lipofectamine 2000 reagent (Invitrogen).

For transient expression, empty plasmids or plasmids expressing a CD74-NRG1, TRIM24-BRAF or EZR-ERBB4 cDNA were transfected into NCI-H1299 lung cancer cells at 80% confluence using the Lipofectamine 2000 reagent. After incubation in a supplemented RPMI medium for 24 hours, the cells were used for assays.

For stable expression, NIH3T3 fibroblasts at 60-70% confluence were infected with empty, CD74-NRG1-, EZR-ERBB4-, or TRIM24-BRAF-expressing lentiviruses, and then treated with blasticidin (4 µg/mL) for 2 weeks. Mass-cultured blasticidin-resistant cells were used for assays.

### <HER2:HER3 signaling activation by CD74-NRG1>

To determine whether cells expressing CD74-NRG1 cDNA secreted NRG1 ligands that activate HER2:HER3 intracellular signaling, EFM-19 breast cancer cells which express both the ERRB2/HER2 and ERBB3/HER3 proteins were used as reporter cells, as previously described (Wilson T. R., et al., Cancer Cell., 2011, 20, 158-72). NCI-H1299 cells transiently transfected with CD74-NRG1-expressing plasmids or empty (control) plasmids were washed twice with PBS and serum-starved overnight by incubation in a serum-free medium. After harvesting of the medium, centrifugation was performed at 4°C for 5 minutes to remove cell debris. Sub-confluent EMF-19 cells were incubated for 30 minutes in the conditioned media supplemented with DMSO (vehicle control) or HER-TKI. Then, whole-cell lysates were subjected to SDS-PAGE and immunoblotting.

### <Constitutive activation of ERBB4 and BRAF kinases and its inhibition by a tyrosine kinase inhibitor>

NIH3T3 cells stably transduced with plasmids expressing EZR-ERBB4 or TRIM24-BRAF cDNA or with empty plasmids were maintained in a serum-free medium overnight, and then treated with DMSO (Sigma) or the indicated inhibitor (dissolved in DMSO) for 2 hours. Whole-cell lysates were subjected to immunoblotting.

### <Immunoblotting>

Cells were lysed in a RIPA buffer supplemented with Complete Protease and PhosSTOP Phosphatase Inhibitor Cocktail (Roche, Mannheim, Germany). Proteins were subjected to SDS-PAGE, followed by immunoblotting onto polyvinylidene difluoride membranes. The membranes were blocked for one hour with TBS supplemented with 0.1% Tween 20 and 1.0% BSA, and then probed with primary antibodies. After washing with TBS supplemented with 0.1% Tween 20, the membranes were incubated with horseradish peroxidase-conjugated anti-mouse or anti-rabbit secondary antibodies, and then visualized with an enhanced chemiluminescence reagent (Perkin Elmer, Waltham, MA, USA). Signal intensity was calculated using the LAS3000 imaging system (Quansys Biosciences, West Logan, UT, USA).

### <Soft-agar assay>

NIH3T3 cells infected with empty, CD74-NRG1, EZR-ERBB4, or TRIM24-BRAF lentiviruses were seeded in triplicate in a top layer of 0.3% SeaPlaque agarose (Lonza, Rockland, ME, USA) on a base layer of 0.6% agarose, at a density of 4,000 cells per well in 24-well plates. A medium supplemented with DMSO or a tyrosine kinase inhibitor was added to top agar, as well as on top of the 0.3% agarose layer. A cover medium was replaced twice a week. After 14 days, colonies larger than 100 µm in diameter were counted.

### <Tumorigenicity assay in nude mice>

Stable NIH3T3 cells (5×10⁶) harboring an empty vector or a vector expressing EZR-ERBB4 or TRIM24-BRAF fusion protein were resuspended in PBS supplemented with 50% Matrigel (BD Biosciences, Bedford, MA, USA). The cells were injected subcutaneously into the right flank of 6-week-old female nu/nu mice. Tumor size measurement was taken twice a week until tumor size reached approximately 2 cm × 2 cm. Photographs were taken on day 21. All studies involving mice were approved by the institutional review board on animal experiments at the National Cancer Center.

### Results and discussion

We established an invasive mucinous adenocarcinoma (IMA) cohort of 90 cases which consisted of 56 (62%) cases with KRAS mutation and 34 (38%) cases without KRAS mutation. The 34 KRAS-negative cases included two cases with BRAF mutation, one case with EGFR mutation, and one case with EML4-ALK fusion; and the remaining 30 cases were "pan-negative" for representative driver mutations in LADCs.

Thirty-two IMAs consisting of 27 pan-negative and 5 KRAS mutation-positive ones were subjected to RNA sequencing (Table 3). Analysis of more than 2×10⁷ paired-end reads obtained by RNA sequencing and subsequent validation by Sanger sequencing of RT-PCR products revealed one other novel gene fusion transcript (SLC3A2-NRG1), in addition to the above-mentioned four types of novel gene fusion transcripts (CD74-NRG1, EZR-ERBB4, TRIM24-BRAF, and KIAA1468-RET). These transcripts were detected only in the pan-negative IMAs (Figs. 1 and 4-6, and Tables 1 and 4).

[Table 3]

**Table 3. DNA sequencing of 32 IMAs**

| No. | Sample ID | Driver oncogene aberration | Library preparation kit | Quantity of RNA used for RNA sequencing (µg) | New generation sequencer | Size of pair-end reads | Novel gene fusions detected RNA sequencing |
|---|---|---|---|---|---|---|---|
| 1 | 258T | Pan-negarive | mRNA-Seq Kit | 2.0 | GAllx | 50 base PE | |
| 2 | AD09-031T | Pan-negarive | TruSeq RNA Kit | 2.0 | HiSeq2000 | 50 base PE | |
| 3 | AD08_220T | KRAS | TruSeq RNA Kit | 1.0 | GAllx | 75 base PE | |
| 4 | 301T | Pan-negarive | TruSeq RNA Kit | 1.0 | GAllx | 75 base PE | CD74-NRG1 |
| 5 | AD08_127T | Pan-negarive | TruSeq RNA Kit | 1.0 | GAllx | 75 base PE | TRIM24-BRAF |
| 6 | AD09-398T | Pan-negarive | TruSeq RNA Kit | 1.0 | GAllx | 75 base PE | |
| 7 | AD12-113T | Pan-negarive | TruSeq RNA Kit | 1.0 | GAllx | 75 base PE | |
| 8 | AD12-119T | Pan-negarive | TruSeq RNA Kit | 1.0 | GAllx | 75 base PE | KIAA1468-RET |
| 9 | AD12-121T | KRAS | TruSeq RNA Kit | 1.0 | GAllx | 75 base PE | |
| 10 | AD12-127T | KRAS | TruSeq RNA Kit | 1.0 | GAllx | 75 base PE | |
| 11 | AD12-129T | Pan-negarive | TruSeq RNA Kit | 1.0 | GAllx | 75 base PE | |
| 12 | 310T | Pan-negarive | TruSeq RNA Kit | 1.0 | GAllx | 75 base PE | |
| 13 | 436T | Pan-negarive | TruSeq RNA Kit | 1.0 | GAllx | 75 base PE | EZR-ERBB4 |
| 14 | AD09-231T | KRAS | TruSeq RNA Kit | 1.0 | GAllx | 75 base PE | |
| 15 | AD09-303T | Pan-negarive | TruSeq RNA Kit | 1.0 | GAllx | 75 base PE | |
| 16 | AD09-317T | KRAS | TruSeq RNA Kit | 1.0 | GAllx | 75 base PE | |
| 17 | AD12-108T | Pan-negarive | TruSeq RNA Kit | 1.0 | GAllx | 75 base PE | CD74-NRG1 |
| 18 | AD12-111T | Pan-negarive | TruSeq RNA Kit | 1.0 | GAllx | 75 base PE | |
| 19 | AD12-112T | Pan-negarive | TruSeq RNA Kit | 1.0 | GAllx | 75 base PE | |
| 20 | AD12-114T | Pan-negarive | TruSeq RNA Kit | 1.0 | GAllx | 75 base PE | |
| 21 | AD12-120T | Pan-negarive | TruSeq RNA Kit | 1.0 | GAllx | 75 base PE | |
| 22 | AD12-126T | Pan-negarive | TruSeq RNA Kit | 1.0 | GAllx | 75 base PE | |
| 23 | AD13-121T | Pan-negarive | TruSeq RNA Kit | 1.0 | GAllx | 75 base PE | |
| 24 | AD13-199T | Pan-negarive | TruSeq RNA Kit | 1.0 | GAllx | 75 base PE | CD74-NRG1 |
| 25 | AD13-223T | Pan-negarive | TruSeq RNA Kit | 1.0 | GAllx | 75 base PE | CD74-NRG1 |
| 26 | AD13-227T | Pan-negarive | TruSeq RNA Kit | 1.0 | GAllx | 75 base PE | |
| 27 | AD13-257T | Pan-negarive | TruSeq RNA Kit | 1.0 | GAllx | 75 base PE | |
| 28 | AD13-362T | Pan-negarive | TruSeq RNA Kit | 1.0 | GAllx | 75 base PE | |
| 29 | AD13-364T | Pan-negarive | TruSeq RNA Kit | 1.0 | GAllx | 75 base PE | |
| 30 | AD13-373T | Pan-negarive | TruSeq RNA Kit | 1.0 | GAllx | 75 base PE | |
| 31 | AD13-377T | Pan-negarive | TruSeq RNA Kit | 1.0 | GAllx | 75 base PE | |
| 32 | AD13-379T | Pan-negarive | TruSeq RNA Kit | 1.0 | GAllx | 75 base PE | SLC3A2-NRG1 |

[Table 4]

**Table 4. Characteristics of invasive mucinous lung adenocarcinomas with novel gene fusion**

| No. | Sample | Sex | Age | Smoking (Pack-years) | Gene fusion (fused exons) | Chromosome aberration | Oncogene mutation* | Pathological stage | TTF1 | HNF4A |
|---|---|---|---|---|---|---|---|---|---|---|
| 1 | 301T | M | 55 | Ever (47) | CD74-NRG1 (C8;N6) | | None | 1a | - | + |
| 2 | AD12-108T | F | 68 | Never | CD74-NRG1 (C6;N6) | | None | 2b | - | + |
| 3 | AD09-404T | F | 78 | Never | CD74-NRG1 (C8;N6) | t(5;8)(q32;p12) | None | 1a | - | + |
| 4 | AD13-199T | F | 47 | Never | CD74-NRG1 (C8;N6) | | None | 1b | - | + |
| 5 | AD13-223T | F | 53 | Never | CD74-NRG1 (C6;N6) | | None | 1a | - | + |
| 6 | AD13-379T | F | 66 | Never | SLC3A2-NRG1 (S5;N6) | t(8;11)(p12;q13) | None | 1b | Not tested | Not tested |
| 7 | 436T | M | 61 | Ever (41) | EZR-ERBB4 (E11;E18) | t(2;6)(q25;q34) | None | 1b | - | + |
| 8 | AD08_127T | F | 66 | Never | TRIM24-BRAF (T5;B8) | Inv7(q33;q34) | None | 1a | + | + |
| 9 | AD12-119T | M | 62 | Current (63) | KIAA1468-RET (K10;R12) | t(10;18)(q21;q11) | None | 1a | + | - |

| | | | | | | | | | | |
|---|---|---|---|---|---|---|---|---|---|---|
| **EGFR, KRAS, BRAF* and *HER2* mutations and *ALK, RET* and *ROSI* fusions. | | | | | | | | | | |

RT-PCR screening of these fusions in remaining 58 IMAs that had not been subjected to RNA sequencing revealed one additional pan-negative case with the CD74-NRG1 fusion. Thus, the CD74-NRG1 fusion, detected in 5 of 34 cases (14.7%) negative for KRAS mutations, was the most frequent fusion among KRAS mutation-negative IMAs. The fusion ofNRG1 with CD74 or SLC3A2 was present in 6 of 34 cases (17.6%). The five novel fusions occurred mutually exclusively and were not observed in any of the KRAS mutation-positive cases (Table 5).

[Table 5]

**Table 5. Characteristics of 90 invasive mucinous lung adenocarcinomas**

| | All | Mutation | | | Fusion | | | | | None (%) |
|---|---|---|---|---|---|---|---|---|---|---|
| | | *KRAS* | *BRAF* | *EGFR* | *CD74-NRG1* or SLC3A2-NRG1 | EZR-ERBB4 | *TRIM24-BRAF* | *EML4-ALK* | *KIAA1468*-*RET* | |
| Total | 90 (100 ) | 56 (62.2 ) | 2 (2.2 ) | 1 (1.1 ) | 6 (6.7 ) | 1 (1.1 ) | 1 (1.1 ) | 1 (1.1 ) | 1 (1.1 ) | 21 (23.3 ) |
| Age (mean ± SD; years) | 67.2 ± 9.7 | 68.1 ± 9.7 | 66-5 ± 3.5 | 50 | 61.2 ± 11.5 | 61 | 66 | 64 | 62 | 68.1 ± 9.6 |
| Sex | | | | | | | | | | |
| Male (%) | 39 (43.3 ) | 28 (50.0 ) | 0 (0 ) | 0 (0 ) | 1 (16.7 ) | 1 (100 ) | 0 (0 ) | 0 (0 ) | 1 (100 ) | 8 (38.1 ) |
| Female (%) | 51 (56.7 ) | 28 (50.0 ) | 2 (100 ) | 1 (100 ) | 5 (83.3 ) | 0 (0 ) | 1 (100 ) | 1 (00 ) | 0(0 ) | 13 (61.9) |
| Smocking habit | | | | | | | | | | |
| Never-smoker (%) | 51 (56.7 ) | 29 (51.8 ) | 2 (100 ) | 1 (100 ) | 4 (66.7 ) | 0 (0 ) | 1 (100 ) | 1 (100 ) | 0 (0 ) | 13 (61.9 ) |
| Ever-smoker (%) | 39 (43.3 ) | 27 (48.2 ) | 0 (0 ) | 0 (0 ) | 2 (33.3 ) | 1 (100 ) | 0 (0 ) | 0 (0 ) | 1 (100 ) | 8 (38.1 ) |

### (Example 5)

The four novel fusion genes, CD74-NRG1, SLC3A2-NRG1, EZR-ERBB4, and TRIM24-BRAF, involved rearrangement of genes encoding protein kinases or ligands for receptor protein kinases (NRG1/neuregulin/heregulin) -- no rearrangement inducing oncogenesis in lung cancer had been reported in these genes (Fig. 7). The remaining fusion gene was a novel type involving the RET oncogene. RET fusions have been observed in 1 to 2% of LADCs (Drilon A., et al., Cancer Discov., 2013, 3, 630-5; Takeuchi K., et al., Nat. Med., 2012, 18, 378-81; Lipson D., et al., Nat. Med., 2012, 18, 382-4; Kohno T., et al., Nat. Med., 2012, 18, 375-7; and Kohno T., et al., Cancer Sci., 2013, 104, 1396-400). As the result of screening of 315 LADCs without IMA features from Japanese patients and 144 consecutive LADCs from U.S. patients, all tumors were negative for all of the NRG1, BRAF, and ERBB4 fusions, and the novel RET fusion. Therefore, these fusions are believed to be driver mutations specific to LADCs with IMA features. It is highly likely that the four gene fusions, CD74-NRG1, SLC3A2-NRG1, EZR-ERBB4, and KIAA1468-RET, were caused by inter-chromosomal translocations, and that the TRIM24-BRAF fusion was caused by paracentric inversion (Table 4 and Fig. 7). In consistence with this, separation of the signals generated by the probes flanking the translocation sites of NRG1 in fusion-positive tumors was observed by fluorescence *in situ* hybridization (FISH) analysis of CD74-NRG1 fusion-positive tumors (Fig. 8). Immunohistochemical analysis using antibodies recognizing polypeptides retained in the fusion proteins confirmed over-expression of NRG1, ERBB4, and BRAF proteins in tumor cells carrying the corresponding fusions. The expression of NRG1, ERBB4, and BRAF proteins was also observed in some fusion-negative cases (Fig. 9). Although IMAs harboring gene fusions were obtained from both male and female patients, NRG1 fusion-positive cases were preferentially from female never smokers (Table 4).

The CD74-NRG1 and SLC3A2-NRG1 fusion proteins, whose sequences were deduced from RNA sequencing data, contained the CD74 or SLC3A2 transmembrane domain and retained the epidermal growth factor (EGF)-like domain of the NRG1 protein (NRG1 III-β3 form) (Figs. 1 and 4). The NRG1 III-β3 protein has a cytosolic N-terminus and a membrane-tethered EGF-lilce domain, and mediates juxtacrine signals signaling through HER2:HER3 receptors (Falls D. L., Exp. Cell Res., 2003, 284, 14-30). Because parts of CD74 or SLC3A2 replaced the transmembrane domain of wild-type NRG1 III-β3, it was speculated that the membrane-tethered EGF-like domain might activate juxtacrine signaling through HER2:HER3 receptors. In addition, it is possible that expression of these fusion proteins resulted in the production of soluble NRG1 protein due to proteolytic cleavage at NRG1-derived sites (located toward the N-terminus of the EGF domain), as recently suggested for NRG1 type III proteins (Fleck D., et al., J. Neurosci., 2013, 33, 7856-69; and Dislich B. and Lichtenthaler S. F., Front Physiol., 2012, 3, 8). Exposing EFM-19 cells to a conditioned medium from H1299 human lung cancer cells expressing exogenous CD74-NRG1 fusion protein resulted in phosphorylation of endogenous ERBB2/HER2 and ERBB3/HER3 proteins, suggesting that autocrine HER2:HER3 signaling was activated by secreted NRG1 ligands generated from CD74-NRG1 polypeptides (Fig. 10A). Phosphorylation of ERK and AKT, downstream mediators of HER2:HER3, was also elevated. Phosphorylation of HER2, HER3 and ERK was suppressed by lapatinib and afatinib, FDA-approved TKIs that target HER kinases (Majem M. and Pallares C., Clin. Transl. Oncol., 2013, 15, 343-57; Perez E. A. and Spano J. P., Cancer, 2012, 118, 3014-25; and Nelson V., et al., Onco. Targets Ther., 2013, 6, 135-43). To put the above together, these observations indicate that the NRG1 fusions activated HER2:HER3 signaling by juxtacrine and/or autocrine mechanisms.

The EZR-ERBB4 fusion protein contained the EZR coiled-coil domain which functions in protein dimerization, and also retained the full-length ERBB4 kinase domain (Fig. 1). These features indicated that the EZR-ERBB4 protein is likely to form a homodimer via the coiled-coil domain of EZR, causing aberrant activation of the kinase function of ERBB4, as in the case of the EZR-ROS1 fusion (Takeuchi K., et al., Nat. Med., 2012, 18, 378-81). Indeed, when the EZR-ERBB4 cDNA was exogenously expressed in NIH3T3 fibroblasts, tyrosine 1258 located in the activation loop of the ERBB4 kinase site was phosphorylated in the absence of serum stimulation, which indicates that the fusion with EZR aberrantly activated the ERBB4 kinase (Fig. 10B). In consistence with this, phosphorylation of ERK, a downstream mediator, was also elevated. Phosphorylation of ERBB4 and ERK was suppressed by lapatinib and afatinib which inhibit ERBB4 protein (Majem M. and Pallares C., Clin. Transl. Oncol., 2013, 15, 343-57; Perez E. A. and Spano J. P., Cancer, 2012, 118, 3014-25; and Nelson V., et al., Onco. Targets Ther., 2013, 6, 135-43).

The TRIM24-BRAF fusion protein retained the BRAF kinase domain but lacked an N-terminal RAS-binding domain responsible for negatively regulating BRAF kinase. These features suggested that this fusion protein was constitutively active, as in the cases of the ESRP1-BRAF and AGTRAP-BRAF fusions in other cancers (Palanisamy N., et al., Nat. Med., 2010, 16, 793-8). When the TRIM24-BRAF cDNA was exogenously expressed in NIH3T3 cells, ERK, a downstream mediator of BRAF, was phosphorylated in the absence of serum stimulation, which indicates that the fusion with TRIM24 aberrantly activated the BRAF kinase (Fig. 10C). ERK phosphorylation was suppressed by sorafenib, an FDA-approved drug originally identified as a RAF kinase inhibitor (Wilhelm S. M., et al., Mol. Cancer Ther., 2008, 7, 3129-40), and also by the MEK inhibitor U0126 (Fig. 10C).

Exogenous expression of fusion gene cDNAs induced anchorage-independent growth of NIH3T3 fibroblasts, which indicates that the fusion genes have transforming activity (Figs. 10D to 10F). This growth was suppressed by the kinase inhibitors that suppressed fusion-induced activation of signal transduction, as described above. NIH3T3 cells expressing the cDNA of the EZR-ERBB4 or TRIM24-BRAF fusion formed tumors in nude mice (Fig. 11). Therefore, it was concluded that these three fusion genes function as driver mutations in IMA development. As the result of screening of 200 commonly used human lung cancer cell lines, all were negative for these three fusions (data not shown).

The results given here suggest that the NRG1, ERBB4 and BRAF fusions are novel driver mutations involved in the development of IMA in the lung (Fig. 12) and potential targets for existing TKIs. The recurrent NRG1 fusions are especially notable because NRG1 has been identified as a regulator of goblet-cell formation in primary culture of human bronchial epithelial cells (Kettle R., et al., Am. J. Respir. Cell Mol. Biol., 2010, 42, 472-81); therefore, it is likely that the NRG1-mediated signaling pathway(s) might play a part in IMA development by contributing to both cell transformation and acquisition of goblet-cell morphology. In addition to a small fraction of known druggable aberrations (ALK fusion and EGFR mutation), more than 10% (11/90; 12.2%) of IMAs harbored other druggable aberrations targeted by existing kinase inhibitors. These aberrations were represented by fusions involved by NRG1, ERBB4, BRAF or RET, or BRAF mutations (Table 5 and Fig. 12). Accordingly, the gene fusions identified here are useful not only as promising targets for the treatment of IMAs but also as markers for the diagnosis of IMAs.

### INDUSTRIAL APPLICABILITY

The present invention makes it possible to detect gene fusions newly discovered as responsible mutations for cancer; to identify patients with cancer or subjects with a risk of cancer, in which substances suppressing the expression and/or activity of polypeptides encoded by fusion polynucleotides produced by said gene fusions show a therapeutic effect; and to provide suitable treatment for such cancer patients.

### SEQUENCE LISTING FREE TEXT

SEQ ID NO: 1: EZR-ERBB4 fusion polynucleotide
SEQ ID NO: 2: EZR-ERBB4 fusion polypeptide
SEQ ID NO: 3: KIAA1468-RET fusion polynucleotide
SEQ ID NO: 4: KIAA1468-RET fusion polypeptide
SEQ ID NO: 5: TRIM24-BRAF fusion polynucleotide
SEQ ID NO: 6: TRIM24-BRAF fusion polypeptide
SEQ ID NO: 7: CD74-NRG1 fusion polynucleotide (variant 1)
SEQ ID NO: 8: CD74-NRG1 fusion polypeptide (variant 1)
SEQ ID NO: 9: CD74-NRG1 fusion polynucleotide (variant 2)
SEQ ID NO: 10: CD74-NRG1 fusion polypeptide (variant 2)
SEQ ID NOs: 11-18: Primer sequences
SEQ ID NO: 19: EZR cDNA
SEQ ID NO: 20: EZR polypeptide
SEQ ID NO: 21: ERBB4 cDNA
SEQ ID NO: 22: ERBB4 polypeptide
SEQ ID NO: 23: KIAA1468 cDNA
SEQ ID NO: 24: KIAA1468 polypeptide
SEQ ID NO: 25: RET cDNA
SEQ ID NO: 26: RET polypeptide
SEQ ID NO: 27: TRIM24 cDNA
SEQ ID NO: 28: TRIM24 polypeptide
SEQ ID NO: 29: BRAF cDNA
SEQ ID NO: 30: BRAF polypeptide
SEQ ID NO: 31: CD74 cDNA
SEQ ID NO: 32: CD74 polypeptide
SEQ ID NO: 33: NRG1 cDNA
SEQ ID NO: 34: NRG1 polypeptide
SEQ ID NO: 35: SLC3A2-NRG1 fusion polynucleotide
SEQ ID NO: 36: SLC3A2-NRG1 fusion polypeptide
SEQ ID NO: 37: Primer sequence
SEQ ID NO: 38: SLC3A2 cDNA
SEQ ID NO: 39: SLC3A2 polypeptide

## Claims

1. A method for detecting a gene fusion serving as a responsible mutation (driver mutation) for cancer, the method comprising the step of detecting a fusion polynucleotide of any one of (a) to (e) mentioned below, or a polypeptide encoded thereby, in an isolated sample from a subject with cancer:
(a) an EZR-ERBB4 fusion polynucleotide which encodes a polypeptide comprising all or part of the coiled-coil domain of EZR, and the kinase domain of ERBB4, and having kinase activity;
(b) a KIAA1468-RET fusion polynucleotide which encodes a polypeptide comprising all or part of the coiled-coil domain of KIAA1468, and the kinase domain of RET, and having kinase activity;
(c) a TRIM24-BRAF fusion polynucleotide which encodes a polypeptide comprising the kinase domain of BRAF and having kinase activity;
(d) a CD74-NRG1 fusion polynucleotide which encodes a polypeptide comprising the transmembrane domain of CD74 and the EGF domain of NRG1, and having intracellular signaling-enhancing activity; and
(e) an SLC3A2-NRG1 fusion polynucleotide which encodes a polypeptide comprising the transmembrane domain of SLC3A2 and the EGF domain ofNRG1, and having intracellular signaling-enhancing activity.

2. The method according to Claim 1, wherein the fusion polynucleotide is any one of (a) to (e) mentioned below:
(a) an EZR-ERBB4 fusion polynucleotide encoding the polypeptide mentioned below:
(i) a polypeptide consisting of the amino acid sequence of SEQ ID NO: 2,
(ii) a polypeptide consisting of an amino acid sequence derived from the amino acid sequence of SEQ ID NO: 2 by deletion, substitution or addition of one or more amino acids, and the polypeptide having kinase activity, or
(iii) a polypeptide consisting of an amino acid sequence having a sequence identity of at least 80% to the amino acid sequence of SEQ ID NO: 2, and the polypeptide having kinase activity;
(b) a KIAA1468-RET fusion polynucleotide encoding the polypeptide mentioned below:
(i) a polypeptide consisting of the amino acid sequence of SEQ ID NO: 4,
(ii) a polypeptide consisting of an amino acid sequence derived from the amino acid sequence of SEQ ID NO: 4 by deletion, substitution or addition of one or more amino acids, and the polypeptide having kinase activity, or
(iii) a polypeptide consisting of an amino acid sequence having a sequence identity of at least 80% to the amino acid sequence of SEQ ID NO: 4, and the polypeptide having kinase activity;
(c) a TRIM24-BRAF fusion polynucleotide encoding the polypeptide mentioned below:
(i) a polypeptide consisting of the amino acid sequence of SEQ ID NO: 6,
(ii) a polypeptide consisting of an amino acid sequence derived from the amino acid sequence of SEQ ID NO: 6 by deletion, substitution or addition of one or more amino acids, and the polypeptide having kinase activity, or
(iii) a polypeptide consisting of an amino acid sequence having a sequence identity of at least 80% to the amino acid sequence of SEQ ID NO: 6, and the polypeptide having kinase activity;
(d) a CD74-NRG1 fusion polynucleotide encoding the polypeptide mentioned below:
(i) a polypeptide consisting of the amino acid sequence of SEQ ID NO: 8 or 10,
(ii) a polypeptide consisting of an amino acid sequence derived from the amino acid sequence of SEQ ID NO: 8 or 10 by deletion, substitution or addition of one or more amino acids, and the polypeptide having intracellular signaling-enhancing activity, or
(iii) a polypeptide consisting of an amino acid sequence having a sequence identity of at least 80% to the amino acid sequence of SEQ ID NO: 8 or 10, and the polypeptide having intracellular signaling-enhancing activity; and
(e) an SLC3A2-NRG1 fusion polynucleotide encoding the polypeptide mentioned below:
(i) a polypeptide consisting of the amino acid sequence of SEQ ID NO: 36,
(ii) a polypeptide consisting of an amino acid sequence derived from the amino acid sequence of SEQ ID NO: 36 by deletion, substitution or addition of one or more amino acids, and the polypeptide having intracellular signaling-enhancing activity, or
(iii) a polypeptide consisting of an amino acid sequence having a sequence identity of at least 80% to the amino acid sequence of SEQ ID NO: 36, and the polypeptide having intracellular signaling-enhancing activity.

3. The method according to Claim 1 or 2, wherein the fusion polynucleotide is any one of (a) to (e) mentioned below:
(a)
(i) a polynucleotide consisting of the nucleotide sequence of SEQ ID NO: 1,
(ii) a polynucleotide that hybridizes under stringent conditions with a polynucleotide consisting of a nucleotide sequence complementary to the polynucleotide consisting of the nucleotide sequence of SEQ ID NO: 1, and which encodes a polypeptide having kinase activity,
(iii) a polynucleotide that consists of a nucleotide sequence derived from the nucleotide sequence of SEQ ID NO: 1 by deletion, substitution or addition of one or more nucleotides, and which encodes a polypeptide having kinase activity, or
(iv) a polynucleotide that has a sequence identity of at least 80% to the polynucleotide consisting of the nucleotide sequence of SEQ ID NO: 1, and which encodes a polypeptide having kinase activity;
(b)
(i) a polynucleotide consisting of the nucleotide sequence of SEQ ID NO: 3,
(ii) a polynucleotide that hybridizes under stringent conditions with a polynucleotide consisting of a nucleotide sequence complementary to the polynucleotide consisting of the nucleotide sequence of SEQ ID NO: 3, and which encodes a polypeptide having kinase activity,
(iii) a polynucleotide that consists of a nucleotide sequence derived from the nucleotide sequence of SEQ ID NO: 3 by deletion, substitution or addition of one or more nucleotides, and which encodes a polypeptide having kinase activity, or
(iv) a polynucleotide that has a sequence identity of at least 80% to the polynucleotide consisting of the nucleotide sequence of SEQ ID NO: 3, and which encodes a polypeptide having kinase activity;
(c)
(i) a polynucleotide consisting of the nucleotide sequence of SEQ ID NO: 5,
(ii) a polynucleotide that hybridizes under stringent conditions with a polynucleotide consisting of a nucleotide sequence complementary to the polynucleotide consisting of the nucleotide sequence of SEQ ID NO: 5, and which encodes a polypeptide having kinase activity,
(iii) a polynucleotide that consists of a nucleotide sequence derived from the nucleotide sequence of SEQ ID NO: 5 by deletion, substitution or addition of one or more nucleotides, and which encodes a polypeptide having kinase activity, or
(iv) a polynucleotide that has a sequence identity of at least 80% to the polynucleotide consisting of the nucleotide sequence of SEQ ID NO: 5, and which encodes a polypeptide having kinase activity;
(d)
(i) a polynucleotide consisting of the nucleotide sequence of SEQ ID NO: 7 or 9,
(ii) a polynucleotide that hybridizes under stringent conditions with a polynucleotide consisting of a nucleotide sequence complementary to the polynucleotide consisting of the nucleotide sequence of SEQ ID NO: 7 or 9, and which encodes a polypeptide having intracellular signaling-enhancing activity,
(iii) a polynucleotide that consists of a nucleotide sequence derived from the nucleotide sequence of SEQ ID NO: 7 or 9 by deletion, substitution or addition of one or more nucleotides, and which encodes a polypeptide having intracellular signaling-enhancing activity, or
(iv) a polynucleotide that has a sequence identity of at least 80% to the polynucleotide consisting of the nucleotide sequence of SEQ ID NO: 7 or 9, and which encodes a polypeptide having intracellular signaling-enhancing activity; and
(e)
(i) a polynucleotide consisting of the nucleotide sequence of SEQ ID NO: 35,
(ii) a polynucleotide that hybridizes under stringent conditions with a polynucleotide consisting of a nucleotide sequence complementary to the polynucleotide consisting of the nucleotide sequence of SEQ ID NO: 35, and which encodes a polypeptide having intracellular signaling-enhancing activity,
(iii) a polynucleotide that consists of a nucleotide sequence derived from the nucleotide sequence of SEQ ID NO: 35 by deletion, substitution or addition of one or more nucleotides, and which encodes a polypeptide having intracellular signaling-enhancing activity, or
(iv) a polynucleotide that has a sequence identity of at least 80% to the polynucleotide consisting of the nucleotide sequence of SEQ ID NO: 35, and which encodes a polypeptide having intracellular signaling-enhancing activity.

4. The method according to any one of Claims 1 to 3, wherein the cancer is lung cancer.

5. A method for identifying a patient with cancer or a subject with a risk of cancer, in which a substance suppressing the expression and/or activity of a polypeptide encoded by a fusion polynucleotide produced by a gene fusion serving as a responsible mutation (driver mutation) for cancer shows a therapeutic effect, the method comprising the steps of:
(1) detecting a fusion polynucleotide of any one of (a) to (e) mentioned below, or a polypeptide encoded thereby, in an isolated sample from a subject:
(a) an EZR-ERBB4 fusion polynucleotide which encodes a polypeptide comprising all or part of the coiled-coil domain of EZR, and the kinase domain of ERBB4, and having kinase activity,
(b) a KIAA1468-RET fusion polynucleotide which encodes a polypeptide comprising all or part of the coiled-coil domain of KIAA1468, and the kinase domain of RET, and having kinase activity,
(c) a TRIM24-BRAF fusion polynucleotide which encodes a polypeptide comprising the kinase domain of BRAF and having kinase activity,
(d) a CD74-NRG1 fusion polynucleotide which encodes a polypeptide comprising the transmembrane domain of CD74 and the EGF domain of NRG1, and having intracellular signaling-enhancing activity, and
(e) an SLC3A2-NRG1 fusion polynucleotide which encodes a polypeptide comprising the transmembrane domain of SLC3A2 and the EGF domain of NRG1, and having intracellular signaling-enhancing activity; and
(2) determining that the substance suppressing the expression and/or activity of the polypeptide shows a therapeutic effect in the subject, in the case where the fusion polynucleotide of any one of (a) to (e) or the polypeptide encoded thereby is detected.

6. A kit for detecting a gene fusion serving as a responsible mutation (driver mutation) for cancer, the kit comprising any one of (A) to (C) mentioned below, or a combination thereof:
(A) a polynucleotide that serves as a probe designed to specifically recognize an EZR-ERBB4 fusion polynucleotide, a KIAA1468-RET fusion polynucleotide, a TRIM24-BRAF fusion polynucleotide, a CD74-NRG1 fusion polynucleotide, or an SLC3A2-NRG1 fusion polynucleotide;
(B) polynucleotides that serve as a pair of primers designed to enable specific amplification of an EZR-ERBB4 fusion polynucleotide, a KIAA1468-RET fusion polynucleotide, a TRIM24-BRAF fusion polynucleotide, a CD74-NRG1 fusion polynucleotide, or an SLC3A2-NRG1 fusion polynucleotide; and
(C) an antibody that specifically recognizes an EZR-ERBB4 fusion polypeptide, a KIAA1468-RET fusion polypeptide, a TRIM24-BRAF fusion polypeptide, a CD74-NRG1 fusion polypeptide, or an SLC3A2-NRG1 fusion polypeptide.

7. An isolated EZR-ERBB4 fusion polypeptide or a fragment thereof, which comprises all or part of the coiled-coil domain of EZR protein, and the kinase domain of ERBB4 protein, and has kinase activity.

8. An isolated KIAA1468-RET fusion polypeptide or a fragment thereof, which comprises all or part of the coiled-coil domain of KIAA1468 protein, and the kinase domain of RET protein, and has kinase activity.

9. An isolated TRIM24-BRAF fusion polypeptide or a fragment thereof, which comprises the kinase domain of BRAF protein and has kinase activity.

10. An isolated CD74-NRG1 fusion polypeptide or a fragment thereof, which comprises the transmembrane domain of CD74 protein and the EGF domain ofNRG1 protein, and has intracellular signaling-enhancing activity.

11. An isolated SLC3A2-NRG1 fusion polypeptide or a fragment thereof, which comprises the transmembrane domain of SLC3A2 protein and the EGF domain of NRG1 protein, and has intracellular signaling-enhancing activity.

12. A polynucleotide encoding the fusion polypeptide or the fragment thereof according to any one of Claims 7 to 11.

13. A method for treatment of cancer, comprising the step of administering a substance suppressing the expression and/or activity of a polypeptide encoded by a fusion polynucleotide produced by a gene fusion serving as a responsible mutation (driver mutation) for cancer, to a subject in which the substance is determined to show a therapeutic effect by the method according to Claim 5.

14. A method for screening a cancer therapeutic agent, the method comprising the steps of:
(1) bringing a cell expressing the fusion polypeptide according to any one of Claims 7 to 11 into contact with a test substance;
(2) judging whether the substance suppresses the expression and/or activity of the fusion polypeptide or not; and
(3) selecting the substance judged to suppress the expression and/or activity of the fusion polypeptide, as a cancer therapeutic agent.


SEQUENCE LISTING

<110>  National Cancer Center
       National Center for Global Health and Medicine
       LSIP, LLC

<120>  Novel fusion genes identified in lung cancer

<130>  FA4009-14103

<150>  US 61/867921
<151>  2013-08-20

<160>  39

<170>  PatentIn version 3.5

<210>  1
<211>  11241
<212>  DNA
<213>  Homo sapiens



<220>
<221>  CDS
<222>  (182)..(3325)

<400>  1
   ggcgtggtcc cgggacccgc cccgccgggg cttttgggag cgcgggcagc gagcgcactc      60
   ggcggacgca agggcggcgg ggagcacacg gagcactgca ggcgccgggt tgggacagcg     120
   tcttcgctgc tgctggatag tcgtgttttc ggggatcgag gatactcacc agaaaccgaa     180
   a atg ccg aaa cca atc aat gtc cga gtt acc acc atg gat gca gag ctg     229
     Met Pro Lys Pro Ile Asn Val Arg Val Thr Thr Met Asp Ala Glu Leu
     1               5                   10                  15
   gag ttt gca atc cag cca aat aca act gga aaa cag ctt ttt gat cag       277
   Glu Phe Ala Ile Gln Pro Asn Thr Thr Gly Lys Gln Leu Phe Asp Gln
               20                  25                  30
   gtg gta aag act atc ggc ctc cgg gaa gtg tgg tac ttt ggc ctc cac       325
   Val Val Lys Thr Ile Gly Leu Arg Glu Val Trp Tyr Phe Gly Leu His
           35                  40                  45
   tat gtg gat aat aaa gga ttt cct acc tgg ctg aag ctg gat aag aag       373
   Tyr Val Asp Asn Lys Gly Phe Pro Thr Trp Leu Lys Leu Asp Lys Lys
       50                  55                  60
   gtg tct gcc cag gag gtc agg aag gag aat ccc ctc cag ttc aag ttc       421
   Val Ser Ala Gln Glu Val Arg Lys Glu Asn Pro Leu Gln Phe Lys Phe
   65                  70                  75                  80
   cgg gcc aag ttc tac cct gaa gat gtg gct gag gag ctc atc cag gac       469
   Arg Ala Lys Phe Tyr Pro Glu Asp Val Ala Glu Glu Leu Ile Gln Asp
                   85                  90                  95
   atc acc cag aaa ctt ttc ttc ctc caa gtg aag gaa gga atc ctt agc       517
   Ile Thr Gln Lys Leu Phe Phe Leu Gln Val Lys Glu Gly Ile Leu Ser
               100                 105                 110
   gat gag atc tac tgc ccc cct gag act gcc gtg ctc ttg ggg tcc tac       565
   Asp Glu Ile Tyr Cys Pro Pro Glu Thr Ala Val Leu Leu Gly Ser Tyr
           115                 120                 125
   gct gtg cag gcc aag ttt ggg gac tac aac aaa gaa gtg cac aag tct        613
   Ala Val Gln Ala Lys Phe Gly Asp Tyr Asn Lys Glu Val His Lys Ser
       130                 135                 140
   ggg tac ctc agc tct gag cgg ctg atc cct caa aga gtg atg gac cag        661
   Gly Tyr Leu Ser Ser Glu Arg Leu Ile Pro Gln Arg Val Met Asp Gln
   145                 150                 155                 160
   cac aaa ctt acc agg gac cag tgg gag gac cgg atc cag gtg tgg cat        709
   His Lys Leu Thr Arg Asp Gln Trp Glu Asp Arg Ile Gln Val Trp His
                   165                 170                 175
   gcg gaa cac cgt ggg atg ctc aaa gat aat gct atg ttg gaa tac ctg        757
   Ala Glu His Arg Gly Met Leu Lys Asp Asn Ala Met Leu Glu Tyr Leu
               180                 185                 190
   aag att gct cag gac ctg gaa atg tat gga atc aac tat ttc gag ata        805
   Lys Ile Ala Gln Asp Leu Glu Met Tyr Gly Ile Asn Tyr Phe Glu Ile
           195                 200                 205
   aaa aac aag aaa gga aca gac ctt tgg ctt gga gtt gat gcc ctt gga        853
   Lys Asn Lys Lys Gly Thr Asp Leu Trp Leu Gly Val Asp Ala Leu Gly
       210                 215                 220
   ctg aat att tat gag aaa gat gat aag tta acc cca aag att ggc ttt        901
   Leu Asn Ile Tyr Glu Lys Asp Asp Lys Leu Thr Pro Lys Ile Gly Phe
   225                 230                 235                 240
   cct tgg agt gaa atc agg aac atc tct ttc aat gac aaa aag ttt gtc        949
   Pro Trp Ser Glu Ile Arg Asn Ile Ser Phe Asn Asp Lys Lys Phe Val
                   245                 250                 255
   att aaa ccc atc gac aag aag gca cct gac ttt gtg ttt tat gcc cca        997
   Ile Lys Pro Ile Asp Lys Lys Ala Pro Asp Phe Val Phe Tyr Ala Pro
               260                 265                 270
   cgt ctg aga atc aac aag cgg atc ctg cag ctc tgc atg ggc aac cat       1045
   Arg Leu Arg Ile Asn Lys Arg Ile Leu Gln Leu Cys Met Gly Asn His
           275                 280                 285
   gag ttg tat atg cgc cgc agg aag cct gac acc atc gag gtg cag cag       1093
   Glu Leu Tyr Met Arg Arg Arg Lys Pro Asp Thr Ile Glu Val Gln Gln
       290                 295                 300
   atg aag gcc cag gcc cgg gag gag aag cat cag aag cag ctg gag cgg       1141
   Met Lys Ala Gln Ala Arg Glu Glu Lys His Gln Lys Gln Leu Glu Arg
   305                 310                 315                 320
   caa cag ctg gaa aca gag aag aaa agg aga gaa acc gtg gag aga gag       1189
   Gln Gln Leu Glu Thr Glu Lys Lys Arg Arg Glu Thr Val Glu Arg Glu
                   325                 330                 335
   aaa gag cag atg atg cgc gag aag gag gag ttg atg ctg cgg ctg cag       1237
   Lys Glu Gln Met Met Arg Glu Lys Glu Glu Leu Met Leu Arg Leu Gln
               340                 345                 350
   gac tat gag gag aag aca aag aag gca gag aga gag ctc tcg gag cag       1285
   Asp Tyr Glu Glu Lys Thr Lys Lys Ala Glu Arg Glu Leu Ser Glu Gln
           355                 360                 365
   att cag agg gcc ctg cag ctg gag gag gag agg aag cgg gca cag gag 1333
   Ile Gln Arg Ala Leu Gln Leu Glu Glu Glu Arg Lys Arg Ala Gln Glu
   370 375 380
   gag gcc gag cgc cta gag gct gac cgt atg gct gca ctg cgg gct aag 1381
   Glu Ala Glu Arg Leu Glu Ala Asp Arg Met Ala Ala Leu Arg Ala Lys
   385 390 395 400
   gag gag ctg gag aga cag gcg gtg gat cag ata aag agc cag gag cag 1429
   Glu Glu Leu Glu Arg Gln Ala Val Asp Gln Ile Lys Ser Gln Glu Gln
   405 410 415
   ctg gct gcg gag ctt gca gaa tac act gcc aag att gcc ctc ctg gaa 1477
   Leu Ala Ala Glu Leu Ala Glu Tyr Thr Ala Lys Ile Ala Leu Leu Glu
   420 425 430
   gag gcg cgg agg cgc aag gag gat gaa gtt gaa gag tgg cag cac agg 1525
   Glu Ala Arg Arg Arg Lys Glu Asp Glu Val Glu Glu Trp Gln His Arg
   435 440 445
   ttg gtg gaa cca tta act ccc agt ggc aca gca ccc aat caa gct caa 1573
   Leu Val Glu Pro Leu Thr Pro Ser Gly Thr Ala Pro Asn Gln Ala Gln
   450 455 460
   ctt cgt att ttg aaa gaa act gag ctg aag agg gta aaa gtc ctt ggc 1621
   Leu Arg Ile Leu Lys Glu Thr Glu Leu Lys Arg Val Lys Val Leu Gly
   465 470 475 480
   tca ggt gct ttt gga acg gtt tat aaa ggt att tgg gta cct gaa gga 1669
   Ser Gly Ala Phe Gly Thr Val Tyr Lys Gly Ile Trp Val Pro Glu Gly
   485 490 495
   gaa act gtg aag att cct gtg gct att aag att ctt aat gag aca act 1717
   Glu Thr Val Lys Ile Pro Val Ala Ile Lys Ile Leu Asn Glu Thr Thr
   500 505 510
   ggt ccc aag gca aat gtg gag ttc atg gat gaa gct ctg atc atg gca 1765
   Gly Pro Lys Ala Asn Val Glu Phe Met Asp Glu Ala Leu Ile Met Ala
   515 520 525
   agt atg gat cat cca cac cta gtc cgg ttg ctg ggt gtg tgt ctg agc 1813
   Ser Met Asp His Pro His Leu Val Arg Leu Leu Gly Val Cys Leu Ser
   530 535 540
   cca acc atc cag ctg gtt act caa ctt atg ccc cat ggc tgc ctg ttg 1861
   Pro Thr Ile Gln Leu Val Thr Gln Leu Met Pro His Gly Cys Leu Leu
   545 550 555 560
   gag tat gtc cac gag cac aag gat aac att gga tca caa ctg ctg ctt 1909
   Glu Tyr Val His Glu His Lys Asp Asn Ile Gly Ser Gln Leu Leu Leu
   565 570 575
   aac tgg tgt gtc cag ata gct aag gga atg atg tac ctg gaa gaa aga 1957
   Asn Trp Cys Val Gln Ile Ala Lys Gly Met Met Tyr Leu Glu Glu Arg
   580 585 590
   cga ctc gtt cat cgg gat ttg gca gcc cgt aat gtc tta gtg aaa tct 2005
   Arg Leu Val His Arg Asp Leu Ala Ala Arg Asn Val Leu Val Lys Ser
   595 600 605
   cca aac cat gtg aaa atc aca gat ttt ggg cta gcc aga ctc ttg gaa 2053
   Pro Asn His Val Lys Ile Thr Asp Phe Gly Leu Ala Arg Leu Leu Glu
   610 615 620
   gga gat gaa aaa gag tac aat gct gat gga gga aag atg cca att aaa          2101
   Gly Asp Glu Lys Glu Tyr Asn Ala Asp Gly Gly Lys Met Pro Ile Lys
   625                 630                 635                 640
   tgg atg gct ctg gag tgt ata cat tac agg aaa ttc acc cat cag agt          2149
   Trp Met Ala Leu Glu Cys Ile His Tyr Arg Lys Phe Thr His Gln Ser
                   645                 650                 655
   gac gtt tgg agc tat gga gtt act ata tgg gaa ctg atg acc ttt gga          2197
   Asp Val Trp Ser Tyr Gly Val Thr Ile Trp Glu Leu Met Thr Phe Gly
               660                 665                 670
   gga aaa ccc tat gat gga att cca acg cga gaa atc cct gat tta tta          2245
   Gly Lys Pro Tyr Asp Gly Ile Pro Thr Arg Glu Ile Pro Asp Leu Leu
           675                 680                 685
   gag aaa gga gaa cgt ttg cct cag cct ccc atc tgc act att gac gtt          2293
   Glu Lys Gly Glu Arg Leu Pro Gln Pro Pro Ile Cys Thr Ile Asp Val
       690                 695                 700
   tac atg gtc atg gtc aaa tgt tgg atg att gat gct gac agt aga cct          2341
   Tyr Met Val Met Val Lys Cys Trp Met Ile Asp Ala Asp Ser Arg Pro
   705                 710                 715                 720
   aaa ttt aag gaa ctg gct gct gag ttt tca agg atg gct cga gac cct          2389
   Lys Phe Lys Glu Leu Ala Ala Glu Phe Ser Arg Met Ala Arg Asp Pro
                   725                 730                 735
   caa aga tac cta gtt att cag ggt gat gat cgt atg aag ctt ccc agt          2437
   Gln Arg Tyr Leu Val Ile Gln Gly Asp Asp Arg Met Lys Leu Pro Ser
               740                 745                 750
   cca aat gac agc aag ttc ttt cag aat ctc ttg gat gaa gag gat ttg          2485
   Pro Asn Asp Ser Lys Phe Phe Gln Asn Leu Leu Asp Glu Glu Asp Leu
           755                 760                 765
   gaa gat atg atg gat gct gag gag tac ttg gtc cct cag gct ttc aac          2533
   Glu Asp Met Met Asp Ala Glu Glu Tyr Leu Val Pro Gln Ala Phe Asn
       770                 775                 780
   atc cca cct ccc atc tat act tcc aga gca aga att gac tcg aat agg          2581
   Ile Pro Pro Pro Ile Tyr Thr Ser Arg Ala Arg Ile Asp Ser Asn Arg
   785                 790                 795                 800
   aac cag ttt gta tac cga gat gga ggt ttt gct gct gaa caa gga gtg          2629
   Asn Gln Phe Val Tyr Arg Asp Gly Gly Phe Ala Ala Glu Gln Gly Val
                   805                 810                 815
   tct gtg ccc tac aga gcc cca act agc aca att cca gaa gct cct gtg          2677
   Ser Val Pro Tyr Arg Ala Pro Thr Ser Thr Ile Pro Glu Ala Pro Val
               820                 825                 830
   gca cag ggt gct act gct gag att ttt gat gac tcc tgc tgt aat ggc          2725
   Ala Gln Gly Ala Thr Ala Glu Ile Phe Asp Asp Ser Cys Cys Asn Gly
           835                 840                 845
   acc cta cgc aag cca gtg gca ccc cat gtc caa gag gac agt agc acc          2773
   Thr Leu Arg Lys Pro Val Ala Pro His Val Gln Glu Asp Ser Ser Thr
       850                 855                 860
   cag agg tac agt gct gac ccc acc gtg ttt gcc cca gaa cgg agc cca          2821
   Gln Arg Tyr Ser Ala Asp Pro Thr Val Phe Ala Pro Glu Arg Ser Pro
   865                 870                 875                 880
   cga gga gag ctg gat gag gaa ggt tac atg act cct atg cga gac aaa      2869
   Arg Gly Glu Leu Asp Glu Glu Gly Tyr Met Thr Pro Met Arg Asp Lys
                   885                 890                 895
   ccc aaa caa gaa tac ctg aat cca gtg gag gag aac cct ttt gtt tct      2917
   Pro Lys Gln Glu Tyr Leu Asn Pro Val Glu Glu Asn Pro Phe Val Ser
               900                 905                 910
   cgg aga aaa aat gga gac ctt caa gca ttg gat aat ccc gaa tat cac      2965
   Arg Arg Lys Asn Gly Asp Leu Gln Ala Leu Asp Asn Pro Glu Tyr His
           915                 920                 925
   aat gca tcc aat ggt cca ccc aag gcc gag gat gag tat gtg aat gag      3013
   Asn Ala Ser Asn Gly Pro Pro Lys Ala Glu Asp Glu Tyr Val Asn Glu
       930                 935                 940
   cca ctg tac ctc aac acc ttt gcc aac acc ttg gga aaa gct gag tac      3061
   Pro Leu Tyr Leu Asn Thr Phe Ala Asn Thr Leu Gly Lys Ala Glu Tyr
   945                 950                 955                 960
   ctg aag aac aac ata ctg tca atg cca gag aag gcc aag aaa gcg ttt      3109
   Leu Lys Asn Asn Ile Leu Ser Met Pro Glu Lys Ala Lys Lys Ala Phe
                   965                 970                 975
   gac aac cct gac tac tgg aac cac agc ctg cca cct cgg agc acc ctt      3157
   Asp Asn Pro Asp Tyr Trp Asn His Ser Leu Pro Pro Arg Ser Thr Leu
               980                 985                 990
   cag cac cca gac tac ctg cag gag  tac agc aca aaa tat  ttt tat aaa      3205
   Gln His Pro Asp Tyr Leu Gln Glu  Tyr Ser Thr Lys Tyr  Phe Tyr Lys
           995                 1000                1005
   cag aat  ggg cgg atc cgg cct  att gtg gca gag aat  cct gaa tac      3250
   Gln Asn  Gly Arg Ile Arg Pro  Ile Val Ala Glu Asn  Pro Glu Tyr
       1010                1015                1020
   ctc tct  gag ttc tcc ctg aag  cca ggc act gtg ctg  ccg cct cca      3295
   Leu Ser  Glu Phe Ser Leu Lys  Pro Gly Thr Val Leu  Pro Pro Pro
       1025                1030                1035
   cct tac  aga cac cgg aat act  gtg gtg taa gctcagttgt ggttttttag      3345
   Pro Tyr  Arg His Arg Asn Thr  Val Val
       1040                 1045
   gtggagagac acacctgctc caatttcccc acccccctct ctttctctgg tggtcttcct      3405
   tctaccccaa ggccagtagt tttgacactt cccagtggaa gatacagaga tgcaatgata      3465
   gttatgtgct tacctaactt gaacattaga gggaaagact gaaagagaaa gataggagga      3525
   accacaatgt ttcttcattt ctctgcatgg gttggtcagg agaatgaaac agctagagaa      3585
   ggaccagaaa atgtaaggca atgctgccta ctatcaaact agctgtcact ttttttcttt      3645
   ttctttttct ttctttgttt ctttcttcct cttctttttt tttttttttt ttaaagcaga      3705
   tggttgaaac acccatgcta tctgttccta tctgcaggaa ctgatgtgtg catatttagc      3765
   atccctggaa atcataataa agtttccatt agaacaaaag aataacattt tctataacat      3825
   atgatggtgt ctgaaattga gaatccagtt tctttcccca gcagtttctg tcctagcaag      3885
   taagaatggc caactcaact ttcataattt aaaaatctcc attaaagtta taactagtaa 3945
   ttatgttttc aacacttttt ggtttttttc attttgtttt gctctgaccg attcctttat 4005
   atttgctccc ctatttttgg ctttaatttc taattgcaaa gatgtttaca tcaaagcttc 4065
   ttcacagaat ttaagcaaga aatattttaa tatagtgaaa tggccactac tttaagtata 4125
   caatctttaa aataagaaag ggaggctaat atttttcatg ctatcaaatt atcttcaccc 4185
   tcatccttta catttttcaa catttttttt tctccataaa tgacactact tgataggccg 4245
   ttggttgtct gaagagtaga agggaaacta agagacagtt ctctgtggtt caggaaaact 4305
   actgatactt tcaggggtgg cccaatgagg gaatccattg aactggaaga aacacactgg 4365
   attgggtatg tctacctggc agatactcag aaatgtagtt tgcacttaag ctgtaatttt 4425
   atttgttctt tttctgaact ccattttgga ttttgaatca agcaatatgg aagcaaccag 4485
   caaattaact aatttaagta catttttaaa aaaagagcta agataaagac tgtggaaatg 4545
   ccaaaccaag caaattagga accttgcaac ggtatccagg gactatgatg agaggccagc 4605
   acattatctt catatgtcac ctttgctacg caaggaaatt tgttcagttc gtatacttcg 4665
   taagaaggaa tgcgagtaag gattggcttg aattccatgg aatttctagt atgagactat 4725
   ttatatgaag tagaaggtaa ctctttgcac ataaattggt ataataaaaa gaaaaacaca 4785
   aacattcaaa gcttagggat aggtccttgg gtcaaaagtt gtaaataaat gtgaaacatc 4845
   ttctcatgca attattttat tatccaacac actaatcttt tgatacttta tataattccc 4905
   tttcttcata tactgcatcc agtactagaa ccatcattat tatgtatcat tttgaaagaa 4965
   tacctgatga gatgaaggat gagaacaaat gacagagatg agtctccaag taaagggggc 5025
   ctcacatcaa taattaggaa acttagatat aagtcgccct tttctgaaaa ttctacccca 5085
   agtcatttag atttttaaaa aatatttcta atgttaaaat attgggacca aattagaatc 5145
   aatagtataa gattaattaa ttagagtaaa aatatctatt aaggcagaga aagtttagag 5205
   aaaaaaatcc aaagaaattt gtgtttcttc ctattctgaa caagtaaatc catccatcca 5265
   tccatccaaa cctcctttat ctaactgtgt ctactaaaag caccatgttt tgtggggaac 5325
   actcagataa atggaatatc atcctcaact tcaaaattct atgatctagg agatttaatt 5385
   aaaatgacat tttaattttt ctatgcgttc caacaatcag attgcatagt ctcttttgtg 5445
   aatagctgtc atataatcag ttgtactgta agatatctcc tttaaactca tttgggatat 5505
   aagttaaaca tccttcaaat tgttgatgtt gacaaacagg ataatttcaa taatattatt 5565
   caaacataaa ctggtctagg agaatattgc atcactgact aattagccta tctagagtct 5625
   aacttcacca ttaaaccaaa agcagatggt ggtccttggc caagaatatt ggagacattg 5685
   gagttggttt ttttctaagc tataagaagt gaggcgagct gaaaaagtat ggtagagcag 5745
   gagaagggtt tgtgagattc cttctagtga agttcaccct caaacttttc aggggtaaag 5805
   acacagagtg attcaggggc cacaatctaa tagctcaggg ctctcctatc cattcagaga 5865
   agtctctagg aaaagggatc tcatatcagt acttatgaaa aattgaatat aagcctccct 5925
   ttctaaataa atctgcatcg agtcatcaca gccctctttt tggatactat accttgattt 5985
   tttttttctg atttacaata tgcatatggt ttctactggg ctatagaaag cagaatcact 6045
   cattttggag aaggaaaaaa tgaatagtta aaacaaactt ttaactgtta aggtaacaga 6105
   aatgtattta gtgaatgtct ctttcctcct aagaacacaa gacttctaca tgttgggtaa 6165
   tacctagaga tgcatgtagg aataatccaa aatgacccaa atgctttata atagcaccac 6225
   tttataattc ttttgaatga tttctgtagt atataattga cttcagttgt ttgagtgttt 6285
   tttgttttat ttttgtcccc cctgggaaaa catatttcag catgtataag agggagaaaa 6345
   aaagtttcat tccttccaga gaataactta tttagtccag tagggtagaa ttttaaaatg 6405
   tcagttaaag tcttcaaagt gcttgggggg atatcagatt ccagaggcca attgtagcaa 6465
   ttgaaatttg cagaatcaat tatgtaaatc tgagacaaat tagtattaaa attacacgga 6525
   gtatattttt taaatcaccc aactttgtag attataccta ttttgggcag gtatggaaaa 6585
   attttgcagt taaatgattg cctaaagaaa gtggtaaaca ggtgaggaaa gatggcctct 6645
   gatctaggat agatccagaa ccacaaagca tctgcaccac aaaaggtgtt agactaccaa 6705
   gcagctcctg gttttctgca tagtattagt agcacagctt aggatgagaa tcctttctcc 6765
   agtaacattc ttaaaatagc atgaaaaaca acgcaaaact caaatttcta ttaaaacaca 6825
   caaactaaaa tcaagtgatt cttttttgta gattagggag aaggactgaa tatctaattt 6885
   aagagaagga atagtgttta agtgttatag tgtgtgagct aataccttct aaaggaaaga 6945
   catggcatga agattgtgca tacttacaat gctaaggaaa aatcaagaaa aggactgtgt 7005
   gaggctctgc tactagatga agttggaagg actattaatg tgcttcttga agtatcaaaa 7065
   atgaaaagaa aattaaaatt gtttaagcct gacagggaag gatgtaaata caagtttttc 7125
   tagagctctc taacctttat ttcaaaactg gaattattca tccatctgta attgttgata 7185
   atttaactag tatatgtagt tcataaggta atagaaaagg tgatcatgaa agcatgtata 7245
   taactggaca gaaccacgat aatgctataa gatgtagatt tagttaggtt atcagatgtt 7305
   aaatgatttt aatattatta aataaatcaa actagaaaac taaccacaag tataatgtaa 7365
   caaagttaaa tgcaggatat aaaaatgtag gatggatttt gcatagtaaa aagataagtt 7425
   tgccatttaa aattgttgtt tgttgggttt agctgaaagt aggcatatat ggttccactt 7485
   gggaaaactt gctttaaagc attacaatga acaatttttt ctcattctct tattccttta 7545
   tcacttttta aatgtaaaga aaattgtatt tatttatttt tttaaataaa caccaccttg 7605
   cagaatttaa taggcaaaca tgttacatat gactaagtaa gggtcttcaa gatgaagtaa 7665
   agaaaatgta aatgttctat taccttatgc agagacaaaa aaaaaaagga gtggtgtcat 7725
   ttagctagca aacaaacaaa atacagttaa ttggtgatat gtcctttctt ttctcactat 7785
   gccctcttgc ctccaaaaat gacaacaaag aatcacaatt tttctgataa ataaatgcta 7845
   aaccaagcgt ttcaaactat tgcattgcca ttcttttgga ctttagttat tagaatgatg 7905
   attgttatag ggcaaatgag aaatccatgt gcatcagctt ctagttgtta aaaaaaccag 7965
   ataaattaac ttctactgta tactgtgggc agaggatcct agagctgatc ctacaacatc 8025
   agcttctagt tgttaaaaaa aaaaaaagaa acagataaat taacttctac tgtatatact 8085
   gtgggcagag gatcttactg tgcctctgtt tgtgtacatg gacttcggtg tgtatcagtt 8145
   tgaaggacag ccttgcccca tgtaaacata taaatgcaga ttggtatcgc ctggttgcta 8205
   tttgcttaag aacaaatatt atacagatga gatcaggcat aattttaaaa gatcattatc 8265
   agtggagacc tcattattac tgatattaca atggggccag tttttatact tctgggtaga 8325
   attaataaaa tttttctgat cccagagatc tgagttctct ctgcagttgg aaacaagaag 8385
   ctgttgtggg cattgtgtcg ggccaggggc ccttgtgttt gtgtgggcaa atatctttta 8445
   gcagtgtgag ctgctttttt cttttcatta aaagtctctc taaaataata gaaatttcag 8505
   atactcggtt caagtctcac tgattttgta gaggtccaaa aatgtaggat ctgtcacttt 8565
   tgcaggcccc tgcctcacct aattcctggc caggtgacat tttgggcaga agtaaatgct 8625
   tctatagtca caagctaaaa tgactctaag ccccaatttc acggggggta ttcacatgct 8685
   tcctctggaa aatactcttt gacagtcagc tttgcaagta agtgattacc ttgttaggaa 8745
   tcaaagaaaa atgtatttct ctctgacctt tagaggaaaa tagaatcctt cccttttttg 8805
   cccattgaca caactggcac tgctctcttc cctttctacc accctggttc aaagtagtcc 8865
   cccgatgctg tcctgttcct ttcttaagcc atagtggatc tctgagatcc tacaccccac 8925
   tttgtgaaac actgacttca tctttgccct cgaatgcctg attttttcat aagagattct 8985
   agcaatttgg acactgttta agtgaactat caaactaccg catagagaat atttaagcta 9045
   ttaaaattat ggtttcccat gaagatcaat tctctgtgtc cttccctata ggaatttgag 9105
   acgagttagc cctgtgatga atcttgaaac tcacatatgt ccacatacac ttggtagaac 9165
   ttcgatttaa tctttacata aaagctgtac atataaccaa gaagttattt ttgccagtaa 9225
   attaacttat ttgctttatt catcttattt ggttcctaat cgtaaatatt ttgtagctgc 9285
   tgtaaatttt tttctcccaa atgaggagtc ttattatcat aaaggtaaag gctattcagc 9345
   tttgataacc acctgcaatt cttttttgga tcattcatcc atctaacaaa tacataatga 9405
   ggacagttca tgttaatgaa aatccatgtt gtttaataga atgccatcct ttacctactt 9465
   ttgctcttta tggacgtttt tcttttcatg ctctagtgag ctttccctat atcatgagaa 9525
   gtggttatat ttgtgcaaat atacaaatat aggaaaacaa agattcatac ctgtaggcaa 9585
   tagtctaact tgtccaaacc actttgcctt tactgctatt tttatcccca atgcgtagat 9645
   atttccccca ggcctatagc ctttgtgaag gaaagcaaat catacctcct gtatattgac 9705
   acgaatctgg ttttcaaatg tcatttccag attttttagt taattggggg ttgtcctttt 9765
   cccttaatgt gagagtcatt ttcctgtata tttctggatc tctcaggggc tgggaggggg 9825
   gagtgagggg actacaacca tagcactcca agaacccttt tgggattact ccagtaatca 9885
   actacgaaag ttattttcta aatgtagata tgtaaggtgt tcttttaaag taaggtactt 9945
   tgaaatatgt agcataaact ggtactgctg ttaaatgggt cgattattaa acggagcagc 10005
   tgtgtgaggg cagctaactt tgaatgcctg tctccctggc tggtgtgtct ccttctcatg 10065
   ttgagagcac cagggattgc gtggctgcat gctgaaaccg cattttccca tggtgtatga 10125
   ctagttcatc tctttcttga gcaccattac aagaagatca aatgaaaatg agatcaatgt 10185
   ggaagacaat tcatagcaca aaaaaagtca tcttaaatct actctcaaac attcatctta 10245
   tacatgcatc aaagtaattt actgacatca gtttgggtga gagagggagt cactttactg 10305
   aaaaggcaga ggcttaaggt gtatacattt gtactcactt ccttattttc ttaacttgta 10365
   agcagaaaac aagccctctc tcttgtgaag tatcttcaaa ggattggggt gcaaaaatac 10425
   cttgctggta agccatcaat gttttattta aatccctgca ttcaaagtta gctgcctttt 10485
   tgaaataaac aaacaaaaaa tactactgta tgtttgaaaa tgtgaatagt atttttatag 10545
   cttgttaaag acatggctag ttgcatttgt aaataagtat aatgttgctt tgattttctt 10605
   ttgtggacat ctttatttgg aacataattg tctttagggt tgatttgtat ataagtaatt 10665
   ggcctgtgat tgtttctttt ttggttggaa gttatcattt tgacattact tgtgattctg 10725
   tgttcagcac tattgtgatg tgttcaacct ctgcactcgc ttacacaata ggatatgcca 10785
   attgtgtgtg gtgtaatgtt attttgattt ttttccatgt tattgatgaa ggatcatgca 10845
   cctaacacat actaactttt ttaatgttag gcatattttt agtatacttt ctcttattct 10905
   ttcttctcct ccaacctttt acccatcctc cttcctttcc ctcattcctg ttgttatttg 10965
   agaatgaggg agaaacagta ttttacattt atgtaattag gcttttccgt tagttctcaa 11025
   ggatcctctt ttggctcttg ggaaagaatt gtacctgtac aaggcaatta tagaatgcga 11085
   actgctttgc ctcattccat actgatcatc ccagctgaac aatttgaaaa ctgttctgcc 11145
   tttttgttac atgaatctgt cagaaatata tttttaattt aatataaatg aaattcaata 11205
   aaatatgaaa caaacgttaa aaaaaaaaaa aaaaaa 11241

<210> 2
<211> 1047
<212> PRT
<213> Homo sapiens

<400>  2

Met Pro Lys Pro Ile Asn Val Arg Val Thr Thr Met Asp Ala Glu Leu
1               5                   10                  15

Glu Phe Ala Ile Gln Pro Asn Thr Thr Gly Lys Gln Leu Phe Asp Gln
            20                  25                  30

Val Val Lys Thr Ile Gly Leu Arg Glu Val Trp Tyr Phe Gly Leu His
        35                  40                  45

Tyr Val Asp Asn Lys Gly Phe Pro Thr Trp Leu Lys Leu Asp Lys Lys
    50                  55                  60

Val Ser Ala Gln Glu Val Arg Lys Glu Asn Pro Leu Gln Phe Lys Phe
65                  70                  75                  80

Arg Ala Lys Phe Tyr Pro Glu Asp Val Ala Glu Glu Leu Ile Gln Asp
                85                  90                  95

Ile Thr Gln Lys Leu Phe Phe Leu Gln Val Lys Glu Gly Ile Leu Ser
            100                 105                 110

Asp Glu Ile Tyr Cys Pro Pro Glu Thr Ala Val Leu Leu Gly Ser Tyr
        115                 120                 125

Ala Val Gln Ala Lys Phe Gly Asp Tyr Asn Lys Glu Val His Lys Ser
    130                 135                 140

Gly Tyr Leu Ser Ser Glu Arg Leu Ile Pro Gln Arg Val Met Asp Gln
145                 150                 155                 160

His Lys Leu Thr Arg Asp Gln Trp Glu Asp Arg Ile Gln Val Trp His
                165                 170                 175

Ala Glu His Arg Gly Met Leu Lys Asp Asn Ala Met Leu Glu Tyr Leu
            180                 185                 190

Lys Ile Ala Gln Asp Leu Glu Met Tyr Gly Ile Asn Tyr Phe Glu Ile
        195                 200                 205

Lys Asn Lys Lys Gly Thr Asp Leu Trp Leu Gly Val Asp Ala Leu Gly
    210                 215                 220

Leu Asn Ile Tyr Glu Lys Asp Asp Lys Leu Thr Pro Lys Ile Gly Phe
225                 230                 235                 240

Pro Trp Ser Glu Ile Arg Asn Ile Ser Phe Asn Asp Lys Lys Phe Val
                245                 250                 255

Ile Lys Pro Ile Asp Lys Lys Ala Pro Asp Phe Val Phe Tyr Ala Pro
            260                 265                 270

Arg Leu Arg Ile Asn Lys Arg Ile Leu Gln Leu Cys Met Gly Asn His
        275                 280                 285

Glu Leu Tyr Met Arg Arg Arg Lys Pro Asp Thr Ile Glu Val Gln Gln
    290                 295                 300

Met Lys Ala Gln Ala Arg Glu Glu Lys His Gln Lys Gln Leu Glu Arg
305                 310                 315                 320

Gln Gln Leu Glu Thr Glu Lys Lys Arg Arg Glu Thr Val Glu Arg Glu
                325                 330                 335

Lys Glu Gln Met Met Arg Glu Lys Glu Glu Leu Met Leu Arg Leu Gln
            340                 345                 350

Asp Tyr Glu Glu Lys Thr Lys Lys Ala Glu Arg Glu Leu Ser Glu Gln
        355                 360                 365

Ile Gln Arg Ala Leu Gln Leu Glu Glu Glu Arg Lys Arg Ala Gln Glu
    370                 375                 380

Glu Ala Glu Arg Leu Glu Ala Asp Arg Met Ala Ala Leu Arg Ala Lys
385                 390                 395                 400

Glu Glu Leu Glu Arg Gln Ala Val Asp Gln Ile Lys Ser Gln Glu Gln
                405                 410                 415

Leu Ala Ala Glu Leu Ala Glu Tyr Thr Ala Lys Ile Ala Leu Leu Glu
            420                 425                 430

Glu Ala Arg Arg Arg Lys Glu Asp Glu Val Glu Glu Trp Gln His Arg
        435                 440                 445

Leu Val Glu Pro Leu Thr Pro Ser Gly Thr Ala Pro Asn Gln Ala Gln
    450                 455                 460

Leu Arg Ile Leu Lys Glu Thr Glu Leu Lys Arg Val Lys Val Leu Gly
465                 470                 475                 480

Ser Gly Ala Phe Gly Thr Val Tyr Lys Gly Ile Trp Val Pro Glu Gly
                485                 490                 495

Glu Thr Val Lys Ile Pro Val Ala Ile Lys Ile Leu Asn Glu Thr Thr
        500                 505                 510

Gly Pro Lys Ala Asn Val Glu Phe Met Asp Glu Ala Leu Ile Met Ala
    515                 520                 525

Ser Met Asp His Pro His Leu Val Arg Leu Leu Gly Val Cys Leu Ser
    530                 535                 540

Pro Thr Ile Gln Leu Val Thr Gln Leu Met Pro His Gly Cys Leu Leu
545                 550                 555                 560

Glu Tyr Val His Glu His Lys Asp Asn Ile Gly Ser Gln Leu Leu Leu
                565                 570                 575

Asn Trp Cys Val Gln Ile Ala Lys Gly Met Met Tyr Leu Glu Glu Arg
        580                 585                 590

Arg Leu Val His Arg Asp Leu Ala Ala Arg Asn Val Leu Val Lys Ser
    595                 600                 605

Pro Asn His Val Lys Ile Thr Asp Phe Gly Leu Ala Arg Leu Leu Glu
    610                 615                 620

Gly Asp Glu Lys Glu Tyr Asn Ala Asp Gly Gly Lys Met Pro Ile Lys
625                 630                 635                 640

Trp Met Ala Leu Glu Cys Ile His Tyr Arg Lys Phe Thr His Gln Ser
                645                 650                 655

Asp Val Trp Ser Tyr Gly Val Thr Ile Trp Glu Leu Met Thr Phe Gly
        660                 665                 670

Gly Lys Pro Tyr Asp Gly Ile Pro Thr Arg Glu Ile Pro Asp Leu Leu
    675                 680                 685

Glu Lys Gly Glu Arg Leu Pro Gln Pro Pro Ile Cys Thr Ile Asp Val
    690                 695                 700

Tyr Met Val Met Val Lys Cys Trp Met Ile Asp Ala Asp Ser Arg Pro
705                 710                 715                 720

Lys Phe Lys Glu Leu Ala Ala Glu Phe Ser Arg Met Ala Arg Asp Pro
                725                 730                 735

Gln Arg Tyr Leu Val Ile Gln Gly Asp Asp Arg Met Lys Leu Pro Ser
        740                 745                 750

Pro Asn Asp Ser Lys Phe Phe Gln Asn Leu Leu Asp Glu Glu Asp Leu
        755                 760                 765

Glu Asp Met Met Asp Ala Glu Glu Tyr Leu Val Pro Gln Ala Phe Asn
    770                 775                 780

Ile Pro Pro Pro Ile Tyr Thr Ser Arg Ala Arg Ile Asp Ser Asn Arg
785                 790                 795                 800

Asn Gln Phe Val Tyr Arg Asp Gly Gly Phe Ala Ala Glu Gln Gly Val
                805                 810                 815

Ser Val Pro Tyr Arg Ala Pro Thr Ser Thr Ile Pro Glu Ala Pro Val
            820                 825                 830

Ala Gln Gly Ala Thr Ala Glu Ile Phe Asp Asp Ser Cys Cys Asn Gly
        835                 840                 845

Thr Leu Arg Lys Pro Val Ala Pro His Val Gln Glu Asp Ser Ser Thr
    850                 855                 860

Gln Arg Tyr Ser Ala Asp Pro Thr Val Phe Ala Pro Glu Arg Ser Pro
865                 870                 875                 880

Arg Gly Glu Leu Asp Glu Glu Gly Tyr Met Thr Pro Met Arg Asp Lys
                885                 890                 895

Pro Lys Gln Glu Tyr Leu Asn Pro Val Glu Glu Asn Pro Phe Val Ser
            900                 905                 910

Arg Arg Lys Asn Gly Asp Leu Gln Ala Leu Asp Asn Pro Glu Tyr His
        915                 920                 925

Asn Ala Ser Asn Gly Pro Pro Lys Ala Glu Asp Glu Tyr Val Asn Glu
    930                 935                 940

Pro Leu Tyr Leu Asn Thr Phe Ala Asn Thr Leu Gly Lys Ala Glu Tyr
945                 950                 955                 960

Leu Lys Asn Asn Ile Leu Ser Met Pro Glu Lys Ala Lys Lys Ala Phe
                965                 970                 975

Asp Asn Pro Asp Tyr Trp Asn His Ser Leu Pro Pro Arg Ser Thr Leu
            980                 985                 990

Gln His Pro Asp Tyr Leu Gln Glu  Tyr Ser Thr Lys Tyr  Phe Tyr Lys

995                 1000                1005

Gln Asn Gly Arg Ile Arg Pro Ile Val Ala Glu Asn Pro Glu Tyr
    1010                1015                1020

Leu Ser Glu Phe Ser Leu Lys Pro Gly Thr Val Leu Pro Pro Pro
    1025                1030                1035

Pro Tyr Arg His Arg Asn Thr Val Val
    1040                1045

<210>  3
<211>  5138
<212>  DNA
<213>  Homo sapiens

<220>
<221>  CDS
<222>  (216)..(3044)

<400>  3
agagccgggc tgctggtgca gcagaggctg aggcatcagg tgcagctgca tccggatctc      60

ctgccttgga gcgtactcct tgtctctaag tcgggaggca ggacgtggtc aggccggggc     120

tgtggaggtg cgctgtgtcc cctgaggcct agaggattcg ggctgcggcc cgtcggaacc     180

agtcagggag gcgcccacac tcctgacagg ataag atg gcg gcg atg gcg cct        233
                                       Met Ala Ala Met Ala Pro
                                       1               5

gga ggt agt ggc agt ggt ggc ggc gtg aat cca ttt ctc agt gat tcg       281
Gly Gly Ser Gly Ser Gly Gly Gly Val Asn Pro Phe Leu Ser Asp Ser
            10                  15                  20

gat gag gac gat gac gag gta gct gca aca gag gaa cgg cgg gca gta       329
Asp Glu Asp Asp Asp Glu Val Ala Ala Thr Glu Glu Arg Arg Ala Val
        25                  30                  35

ctt cgg ctg ggc gcc gga agt ggc cta gat cct ggc tct gcg ggc tcg       377
Leu Arg Leu Gly Ala Gly Ser Gly Leu Asp Pro Gly Ser Ala Gly Ser
    40                  45                  50

ctg tcg cca cag gat ccc gtg gcc tta gga agc agt gcg cgg cca ggg       425
Leu Ser Pro Gln Asp Pro Val Ala Leu Gly Ser Ser Ala Arg Pro Gly
55                  60                  65                  70

ctc cct ggg gag gcg tcg gcg gct gca gtg gcc ctg ggg ggc acc ggg       473
Leu Pro Gly Glu Ala Ser Ala Ala Ala Val Ala Leu Gly Gly Thr Gly
                75                  80                  85

gag acc ccg gcc cga tta tca att gat gcg atc gct gct cag ctg ttg       521
Glu Thr Pro Ala Arg Leu Ser Ile Asp Ala Ile Ala Ala Gln Leu Leu
            90                  95                  100

cgc gat caa tac ttg ctg acc gcc ctg gag ctg cat acc gag ctg tta       569
Arg Asp Gln Tyr Leu Leu Thr Ala Leu Glu Leu His Thr Glu Leu Leu
        105                 110                 115

gag agt ggc cgg gag ctg cct cgg ctg cgc gac tac ttc tcc aat cca 617
Glu Ser Gly Arg Glu Leu Pro Arg Leu Arg Asp Tyr Phe Ser Asn Pro
    120                 125                 130

ggc aac ttc gag agg caa agt gga acc ccg ccg ggg atg ggg gcg cca 665
Gly Asn Phe Glu Arg Gln Ser Gly Thr Pro Pro Gly Met Gly Ala Pro
135                 140                 145                 150

ggg gtc cct gga gca gcc ggc gtt ggg ggc gct gga ggt cgg gaa ccg 713
Gly Val Pro Gly Ala Ala Gly Val Gly Gly Ala Gly Gly Arg Glu Pro
                155                 160                 165

agt aca gcg tcg ggc ggg gga cag ctc aat cga gct ggg agc att agt 761
Ser Thr Ala Ser Gly Gly Gly Gln Leu Asn Arg Ala Gly Ser Ile Ser
            170                 175                 180

acc ctt gat tct tta gac ttt gca aga tat tca gat gat ggt aac agg 809
Thr Leu Asp Ser Leu Asp Phe Ala Arg Tyr Ser Asp Asp Gly Asn Arg
        185                 190                 195

gaa aca gat gaa aaa gtg gca gtc ctg gag ttt gaa cta cgg aaa gcc 857
Glu Thr Asp Glu Lys Val Ala Val Leu Glu Phe Glu Leu Arg Lys Ala
    200                 205                 210

aag gag acc att cag gcc ctc cga gcc aac ctg aca aag gcc gca gaa 905
Lys Glu Thr Ile Gln Ala Leu Arg Ala Asn Leu Thr Lys Ala Ala Glu
215                 220                 225                 230

cat gaa gtt cct tta cag gaa cga aaa aat tac aaa tca agt cct gaa 953
His Glu Val Pro Leu Gln Glu Arg Lys Asn Tyr Lys Ser Ser Pro Glu
                235                 240                 245

att cag gag cca atc aaa cct ctt gaa aag aga gct cta aac ttc tta 1001
Ile Gln Glu Pro Ile Lys Pro Leu Glu Lys Arg Ala Leu Asn Phe Leu
            250                 255                 260

gtc aat gaa ttt tta ttg aag aat aac tat aag ctt aca tca ata acc 1049
Val Asn Glu Phe Leu Leu Lys Asn Asn Tyr Lys Leu Thr Ser Ile Thr
        265                 270                 275

ttt tca gat gaa aac gat gat cag gat ttt gaa tta tgg gat gat gta 1097
Phe Ser Asp Glu Asn Asp Asp Gln Asp Phe Glu Leu Trp Asp Asp Val
    280                 285                 290

gga tta aac att cca aaa cct cca gac tta ttg caa ctc tac cgg gat 1145
Gly Leu Asn Ile Pro Lys Pro Pro Asp Leu Leu Gln Leu Tyr Arg Asp
295                 300                 305                 310

ttt gga aat cat caa gta act gga aaa gat ctt gta gat gtg gcc agt 1193
Phe Gly Asn His Gln Val Thr Gly Lys Asp Leu Val Asp Val Ala Ser
                315                 320                 325

gga gta gaa gaa gat gaa tta gag gcc ctt aca cca att ata agc aac 1241
Gly Val Glu Glu Asp Glu Leu Glu Ala Leu Thr Pro Ile Ile Ser Asn
            330                 335                 340

ctt cct cca act ctt gaa act ccc cag cct gca gag aac tcc atg tta 1289
Leu Pro Pro Thr Leu Glu Thr Pro Gln Pro Ala Glu Asn Ser Met Leu
        345                 350                 355

gta cag aaa tta gaa gat aaa att agt ttg tta aat agt gag aaa tgg 1337
Val Gln Lys Leu Glu Asp Lys Ile Ser Leu Leu Asn Ser Glu Lys Trp

360                 365                 370

tca ttg atg gag caa atc aga aga ctt aaa agt gaa atg gac ttc ctc      1385
Ser Leu Met Glu Gln Ile Arg Arg Leu Lys Ser Glu Met Asp Phe Leu
375                 380                 385                 390

aaa aat gaa cac ttt gcc atc cca gca gtt tgt gac tct gtt cag cct      1433
Lys Asn Glu His Phe Ala Ile Pro Ala Val Cys Asp Ser Val Gln Pro
                395                 400                 405

cct ttg gat cag ttg ccc cac aaa gac tct gag gac agt gga cag cat      1481
Pro Leu Asp Gln Leu Pro His Lys Asp Ser Glu Asp Ser Gly Gln His
            410                 415                 420

cca gat gta aat agt tca gac aag gga aaa aac aca gac atc cat ctt      1529
Pro Asp Val Asn Ser Ser Asp Lys Gly Lys Asn Thr Asp Ile His Leu
        425                 430                 435

tca ata tca gat gaa gct gat tcc act att cct aaa gag aat tcc cca      1577
Ser Ile Ser Asp Glu Ala Asp Ser Thr Ile Pro Lys Glu Asn Ser Pro
    440                 445                 450

aat tca ttc ccc agg aga gaa aga gaa gga atg cca cct tct tct cta      1625
Asn Ser Phe Pro Arg Arg Glu Arg Glu Gly Met Pro Pro Ser Ser Leu
455                 460                 465                 470

tca agt aaa aag aca gtt cat ttt gat aaa cct aat agg aaa ttg tct      1673
Ser Ser Lys Lys Thr Val His Phe Asp Lys Pro Asn Arg Lys Leu Ser
                475                 480                 485

cct gca ttc cat caa gca cta ctc tct ttt tgt cga atg tca gca gat      1721
Pro Ala Phe His Gln Ala Leu Leu Ser Phe Cys Arg Met Ser Ala Asp
            490                 495                 500

agt cgt tta gga tac gag gtg tct cgt att gca gac agt gaa aaa agc      1769
Ser Arg Leu Gly Tyr Glu Val Ser Arg Ile Ala Asp Ser Glu Lys Ser
        505                 510                 515

gtt atg tta atg ctg gga cgc tgc ctg cca cac att gtt ccc aat gtg      1817
Val Met Leu Met Leu Gly Arg Cys Leu Pro His Ile Val Pro Asn Val
    520                 525                 530

cta ttg gca aag aga gag gag gat cca aag tgg gaa ttc cct cgg aag      1865
Leu Leu Ala Lys Arg Glu Glu Asp Pro Lys Trp Glu Phe Pro Arg Lys
535                 540                 545                 550

aac ttg gtt ctt gga aaa act cta gga gaa ggc gaa ttt gga aaa gtg      1913
Asn Leu Val Leu Gly Lys Thr Leu Gly Glu Gly Glu Phe Gly Lys Val
                555                 560                 565

gtc aag gca acg gcc ttc cat ctg aaa ggc aga gca ggg tac acc acg      1961
Val Lys Ala Thr Ala Phe His Leu Lys Gly Arg Ala Gly Tyr Thr Thr
            570                 575                 580

gtg gcc gtg aag atg ctg aaa gag aac gcc tcc ccg agt gag ctt cga      2009
Val Ala Val Lys Met Leu Lys Glu Asn Ala Ser Pro Ser Glu Leu Arg
        585                 590                 595

gac ctg ctg tca gag ttc aac gtc ctg aag cag gtc aac cac cca cat      2057
Asp Leu Leu Ser Glu Phe Asn Val Leu Lys Gln Val Asn His Pro His
    600                 605                 610

gtc atc aaa ttg tat ggg gcc tgc agc cag gat ggc ccg ctc ctc ctc      2105

Val Ile Lys Leu Tyr Gly Ala Cys Ser Gln Asp Gly Pro Leu Leu Leu
615                 620                 625                 630

atc gtg gag tac gcc aaa tac ggc tcc ctg cgg ggc ttc ctc cgc gag      2153
Ile Val Glu Tyr Ala Lys Tyr Gly Ser Leu Arg Gly Phe Leu Arg Glu
                635                 640                 645

agc cgc aaa gtg ggg cct ggc tac ctg ggc agt gga ggc agc cgc aac      2201
Ser Arg Lys Val Gly Pro Gly Tyr Leu Gly Ser Gly Gly Ser Arg Asn
            650                 655                 660

tcc agc tcc ctg gac cac ccg gat gag cgg gcc ctc acc atg ggc gac      2249
Ser Ser Ser Leu Asp His Pro Asp Glu Arg Ala Leu Thr Met Gly Asp
        665                 670                 675

ctc atc tca ttt gcc tgg cag atc tca cag ggg atg cag tat ctg gcc      2297
Leu Ile Ser Phe Ala Trp Gln Ile Ser Gln Gly Met Gln Tyr Leu Ala
    680                 685                 690

gag atg aag ctc gtt cat cgg gac ttg gca gcc aga aac atc ctg gta      2345
Glu Met Lys Leu Val His Arg Asp Leu Ala Ala Arg Asn Ile Leu Val
695                 700                 705                 710

gct gag ggg cgg aag atg aag att tcg gat ttc ggc ttg tcc cga gat      2393
Ala Glu Gly Arg Lys Met Lys Ile Ser Asp Phe Gly Leu Ser Arg Asp
                715                 720                 725

gtt tat gaa gag gat tcc tac gtg aag agg agc cag ggt cgg att cca      2441
Val Tyr Glu Glu Asp Ser Tyr Val Lys Arg Ser Gln Gly Arg Ile Pro
            730                 735                 740

gtt aaa tgg atg gca att gaa tcc ctt ttt gat cat atc tac acc acg      2489
Val Lys Trp Met Ala Ile Glu Ser Leu Phe Asp His Ile Tyr Thr Thr
        745                 750                 755

caa agt gat gta tgg tct ttt ggt gtc ctg ctg tgg gag atc gtg acc      2537
Gln Ser Asp Val Trp Ser Phe Gly Val Leu Leu Trp Glu Ile Val Thr
    760                 765                 770

cta ggg gga aac ccc tat cct ggg att cct cct gag cgg ctc ttc aac      2585
Leu Gly Gly Asn Pro Tyr Pro Gly Ile Pro Pro Glu Arg Leu Phe Asn
775                 780                 785                 790

ctt ctg aag acc ggc cac cgg atg gag agg cca gac aac tgc agc gag      2633
Leu Leu Lys Thr Gly His Arg Met Glu Arg Pro Asp Asn Cys Ser Glu
                795                 800                 805

gag atg tac cgc ctg atg ctg caa tgc tgg aag cag gag ccg gac aaa      2681
Glu Met Tyr Arg Leu Met Leu Gln Cys Trp Lys Gln Glu Pro Asp Lys
            810                 815                 820

agg ccg gtg ttt gcg gac atc agc aaa gac ctg gag aag atg atg gtt      2729
Arg Pro Val Phe Ala Asp Ile Ser Lys Asp Leu Glu Lys Met Met Val
        825                 830                 835

aag agg aga gac tac ttg gac ctt gcg gcg tcc act cca tct gac tcc      2777
Lys Arg Arg Asp Tyr Leu Asp Leu Ala Ala Ser Thr Pro Ser Asp Ser
    840                 845                 850

ctg att tat gac gac ggc ctc tca gag gag gag aca ccg ctg gtg gac      2825
Leu Ile Tyr Asp Asp Gly Leu Ser Glu Glu Glu Thr Pro Leu Val Asp
855                 860                 865                 870

tgt aat aat gcc ccc ctc cct cga gcc ctc cct tcc aca tgg att gaa     2873
Cys Asn Asn Ala Pro Leu Pro Arg Ala Leu Pro Ser Thr Trp Ile Glu
                875                 880                 885

aac aaa ctc tat ggc atg tca gac ccg aac tgg cct gga gag agt cct     2921
Asn Lys Leu Tyr Gly Met Ser Asp Pro Asn Trp Pro Gly Glu Ser Pro
            890                 895                 900

gta cca ctc acg aga gct gat ggc act aac act ggg ttt cca aga tat     2969
Val Pro Leu Thr Arg Ala Asp Gly Thr Asn Thr Gly Phe Pro Arg Tyr
        905                 910                 915

cca aat gat agt gta tat gct aac tgg atg ctt tca ccc tca gcg gca     3017
Pro Asn Asp Ser Val Tyr Ala Asn Trp Met Leu Ser Pro Ser Ala Ala
    920                 925                 930

aaa tta atg gac acg ttt gat agt taa catttctttg tgaaaggtaa           3064
Lys Leu Met Asp Thr Phe Asp Ser
935                 940

tggactcaca aggggaagaa acatgctgag aatggaaagt ctaccggccc tttctttgtg    3124

aacgtcacat tggccgagcc gtgttcagtt cccaggtggc agactcgttt ttggtagttt    3184

gttttaactt ccaaggtggt tttacttctg atagccggtg attttccctc ctagcagaca    3244

tgccacaccg ggtaagagct ctgagtctta gtggttaagc attcctttct cttcagtgcc    3304

cagcagcacc cagtgttggt ctgtgtccat cagtgaccac caacattctg tgttcacatg    3364

tgtgggtcca acacttacta cctggtgtat gaaattggac ctgaactgtt ggatttttct    3424

agttgccgcc aaacaaggca aaaaaattta aacatgaagc acacacacaa aaaaggcagt    3484

aggaaaaatg ctggccctga tgacctgtcc ttattcagaa tgagagactg cggggggggc    3544

ctgggggtag tgtcaatgcc cctccagggc tggaggggaa gaggggcccc gaggatgggc    3604

ctgggctcag cattcgagat cttgagaatg attttttttt aatcatgcaa cctttcctta    3664

ggaagacatt tggttttcat catgattaag atgattccta gatttagcac aatggagaga    3724

ttccatgcca tctttactat gtggatggtg gtatcaggga agagggctca caagacacat    3784

ttgtcccccg ggcccaccac atcatcctca cgtgttcggt actgagcagc cactacccct    3844

gatgagaaca gtatgaagaa agggggctgt tggagtccca gaattgctga cagcagaggc    3904

tttgctgctg tgaatcccac ctgccaccag cctgcagcac accccacagc caagtagagg    3964

cgaaagcagt ggctcatcct acctgttagg agcaggtagg gcttgtactc actttaattt    4024

gaatcttatc aacttactca taaagggaca ggctagctag ctgtgttaga agtagcaatg    4084

acaatgacca aggactgcta cacctctgat tacaattctg atgtgaaaaa gatggtgttt    4144

ggctcttata gagcctgtgt gaaaggccca tggatcagct cttcctgtgt ttgtaattta    4204

atgctgctac aagatgtttc tgtttcttag attctgacca tgactcataa gcttcttgtc    4264

attcttcatt gcttgtttgt ggtcacagat gcacaacact cctccagtct tgtgggggca    4324

gcttttggga agtctcagca gctcttctgg ctgtgttgtc agcactgtaa cttcgcagaa    4384

aagagtcgga ttaccaaaac actgcctgct cttcagactt aaagcactga taggacttaa 4444

aatagtctca ttcaaatact gtattttata taggcatttc acaaaaacag caaaattgtg 4504

gcattttgtg aggccaaggc ttggatgcgt gtgtaataga gccttgtggt gtgtgcgcac 4564

acacccagag ggagagtttg aaaaatgctt attggacacg taacctggct ctaatttggg 4624

ctgtttttca gatacactgt gataagttct tttacaaata tctatagaca tggtaaactt 4684

ttggttttca gatatgctta atgatagtct tactaaatgc agaaataaga ataaactttc 4744

tcaaattatt aaaaatgcct acacagtaag tgtgaattgc tgcaacaggt ttgttctcag 4804

gagggtaaga actccaggtc taaacagctg acccagtgat ggggaattta tccttgacca 4864

atttatcctt gaccaataac ctaattgtct attcctgagt tataaaagtc cccatcctta 4924

ttagctctac tggaattttc atacacgtaa atgcagaagt tactaagtat taagtattac 4984

tgagtattaa gtagtaatct gtcagttatt aaaatttgta aaatctattt atgaaaggtc 5044

attaaaccag atcatgttcc tttttttgta atcaaggtga ctaagaaaat cagttgtgta 5104

aataaaatca tgtatcataa aaaaaaaaaa aaaa 5138

<210> 4
<211> 942
<212> PRT
<213> Homo sapiens

<400> 4

Met Ala Ala Met Ala Pro Gly Gly Ser Gly Ser Gly Gly Gly Val Asn
1               5                   10                  15

Pro Phe Leu Ser Asp Ser Asp Glu Asp Asp Asp Glu Val Ala Ala Thr
            20                  25                  30

Glu Glu Arg Arg Ala Val Leu Arg Leu Gly Ala Gly Ser Gly Leu Asp
        35                  40                  45

Pro Gly Ser Ala Gly Ser Leu Ser Pro Gln Asp Pro Val Ala Leu Gly
    50                  55                  60

Ser Ser Ala Arg Pro Gly Leu Pro Gly Glu Ala Ser Ala Ala Ala Val
65                  70                  75                  80

Ala Leu Gly Gly Thr Gly Glu Thr Pro Ala Arg Leu Ser Ile Asp Ala
                85                  90                  95

Ile Ala Ala Gln Leu Leu Arg Asp Gln Tyr Leu Leu Thr Ala Leu Glu
            100                 105                 110

Leu His Thr Glu Leu Leu Glu Ser Gly Arg Glu Leu Pro Arg Leu Arg
        115                 120                 125

Asp Tyr Phe Ser Asn Pro Gly Asn Phe Glu Arg Gln Ser Gly Thr Pro
    130                 135                 140

Pro Gly Met Gly Ala Pro Gly Val Pro Gly Ala Ala Gly Val Gly Gly
145                 150                 155                 160

Ala Gly Gly Arg Glu Pro Ser Thr Ala Ser Gly Gly Gly Gln Leu Asn
                165                 170                 175

Arg Ala Gly Ser Ile Ser Thr Leu Asp Ser Leu Asp Phe Ala Arg Tyr
            180                 185                 190

Ser Asp Asp Gly Asn Arg Glu Thr Asp Glu Lys Val Ala Val Leu Glu
        195                 200                 205

Phe Glu Leu Arg Lys Ala Lys Glu Thr Ile Gln Ala Leu Arg Ala Asn
    210                 215                 220

Leu Thr Lys Ala Ala Glu His Glu Val Pro Leu Gln Glu Arg Lys Asn
225                 230                 235                 240

Tyr Lys Ser Ser Pro Glu Ile Gln Glu Pro Ile Lys Pro Leu Glu Lys
                245                 250                 255

Arg Ala Leu Asn Phe Leu Val Asn Glu Phe Leu Leu Lys Asn Asn Tyr
            260                 265                 270

Lys Leu Thr Ser Ile Thr Phe Ser Asp Glu Asn Asp Asp Gln Asp Phe
        275                 280                 285

Glu Leu Trp Asp Asp Val Gly Leu Asn Ile Pro Lys Pro Pro Asp Leu
    290                 295                 300

Leu Gln Leu Tyr Arg Asp Phe Gly Asn His Gln Val Thr Gly Lys Asp
305                 310                 315                 320

Leu Val Asp Val Ala Ser Gly Val Glu Glu Asp Glu Leu Glu Ala Leu
                325                 330                 335

Thr Pro Ile Ile Ser Asn Leu Pro Pro Thr Leu Glu Thr Pro Gln Pro
            340                 345                 350

Ala Glu Asn Ser Met Leu Val Gln Lys Leu Glu Asp Lys Ile Ser Leu
        355                 360                 365

Leu Asn Ser Glu Lys Trp Ser Leu Met Glu Gln Ile Arg Arg Leu Lys
    370                 375                 380

Ser Glu Met Asp Phe Leu Lys Asn Glu His Phe Ala Ile Pro Ala Val
385                 390                 395                 400

Cys Asp Ser Val Gln Pro Pro Leu Asp Gln Leu Pro His Lys Asp Ser
                405                 410                 415

Glu Asp Ser Gly Gln His Pro Asp Val Asn Ser Ser Asp Lys Gly Lys
            420                 425                 430

Asn Thr Asp Ile His Leu Ser Ile Ser Asp Glu Ala Asp Ser Thr Ile
        435                 440                 445

Pro Lys Glu Asn Ser Pro Asn Ser Phe Pro Arg Arg Glu Arg Glu Gly
    450                 455                 460

Met Pro Pro Ser Ser Leu Ser Ser Lys Lys Thr Val His Phe Asp Lys
465                 470                 475                 480

Pro Asn Arg Lys Leu Ser Pro Ala Phe His Gln Ala Leu Leu Ser Phe
                485                 490                 495

Cys Arg Met Ser Ala Asp Ser Arg Leu Gly Tyr Glu Val Ser Arg Ile
            500                 505                 510

Ala Asp Ser Glu Lys Ser Val Met Leu Met Leu Gly Arg Cys Leu Pro
        515                 520                 525

His Ile Val Pro Asn Val Leu Leu Ala Lys Arg Glu Glu Asp Pro Lys
    530                 535                 540

Trp Glu Phe Pro Arg Lys Asn Leu Val Leu Gly Lys Thr Leu Gly Glu
545                 550                 555                 560

Gly Glu Phe Gly Lys Val Val Lys Ala Thr Ala Phe His Leu Lys Gly
                565                 570                 575

Arg Ala Gly Tyr Thr Thr Val Ala Val Lys Met Leu Lys Glu Asn Ala
            580                 585                 590

Ser Pro Ser Glu Leu Arg Asp Leu Leu Ser Glu Phe Asn Val Leu Lys
        595                 600                 605

Gln Val Asn His Pro His Val Ile Lys Leu Tyr Gly Ala Cys Ser Gln
    610                 615                 620

Asp Gly Pro Leu Leu Leu Ile Val Glu Tyr Ala Lys Tyr Gly Ser Leu
625                 630                 635                 640

Arg Gly Phe Leu Arg Glu Ser Arg Lys Val Gly Pro Gly Tyr Leu Gly
                645                 650                 655

Ser Gly Gly Ser Arg Asn Ser Ser Ser Leu Asp His Pro Asp Glu Arg
            660                 665                 670

Ala Leu Thr Met Gly Asp Leu Ile Ser Phe Ala Trp Gln Ile Ser Gln
        675                 680                 685

Gly Met Gln Tyr Leu Ala Glu Met Lys Leu Val His Arg Asp Leu Ala
    690                 695                 700

Ala Arg Asn Ile Leu Val Ala Glu Gly Arg Lys Met Lys Ile Ser Asp
705                 710                 715                 720

Phe Gly Leu Ser Arg Asp Val Tyr Glu Glu Asp Ser Tyr Val Lys Arg
                725                 730                 735

Ser Gln Gly Arg Ile Pro Val Lys Trp Met Ala Ile Glu Ser Leu Phe
            740                 745                 750

Asp His Ile Tyr Thr Thr Gln Ser Asp Val Trp Ser Phe Gly Val Leu
        755                 760                 765

Leu Trp Glu Ile Val Thr Leu Gly Gly Asn Pro Tyr Pro Gly Ile Pro
    770                 775                 780

Pro Glu Arg Leu Phe Asn Leu Leu Lys Thr Gly His Arg Met Glu Arg
785                 790                 795                 800

Pro Asp Asn Cys Ser Glu Glu Met Tyr Arg Leu Met Leu Gln Cys Trp
                805                 810                 815

Lys Gln Glu Pro Asp Lys Arg Pro Val Phe Ala Asp Ile Ser Lys Asp
            820                 825                 830

Leu Glu Lys Met Met Val Lys Arg Arg Asp Tyr Leu Asp Leu Ala Ala
        835                 840                 845

Ser Thr Pro Ser Asp Ser Leu Ile Tyr Asp Asp Gly Leu Ser Glu Glu
    850                 855                 860

Glu Thr Pro Leu Val Asp Cys Asn Asn Ala Pro Leu Pro Arg Ala Leu

865                 870                 875                 880

Pro Ser Thr Trp Ile Glu Asn Lys Leu Tyr Gly Met Ser Asp Pro Asn
                885                 890                 895

Trp Pro Gly Glu Ser Pro Val Pro Leu Thr Arg Ala Asp Gly Thr Asn
            900                 905                 910

Thr Gly Phe Pro Arg Tyr Pro Asn Asp Ser Val Tyr Ala Asn Trp Met
        915                 920                 925

Leu Ser Pro Ser Ala Ala Lys Leu Met Asp Thr Phe Asp Ser
    930                 935                 940

<210>  5
<211>  3004
<212>  DNA
<213>  Homo sapiens

<220>
<221>  CDS
<222>  (216)..(2417)

<400>  5
gacagatacc ctccttccgg ccgcgccact cgggaggcgg atcccgtggg cctgaggagg      60

cttcccccgc ccggtttgct ttccctccct cgctggcgct gccgcgagtc caccgagcgg     120

cctctgagga gcagccgcag gaggaggagg aggtcgtcgg gggcggcggg cggagaccgc     180

gctctcgctt ccccggcggc ggcaagggca ggaca atg gag gtg gcg gtg gag        233
                                       Met Glu Val Ala Val Glu
                                       1               5

aag gcg gtg gcg gcg gcg gca gcg gcc tcg gct gcg gcc tcc ggg ggg       281
Lys Ala Val Ala Ala Ala Ala Ala Ala Ser Ala Ala Ala Ser Gly Gly
            10                  15                  20

ccc tcg gcg gcg ccg agc ggg gag aac gag gcc gag agt cgg cag ggc       329
Pro Ser Ala Ala Pro Ser Gly Glu Asn Glu Ala Glu Ser Arg Gln Gly
        25                  30                  35

ccg gac tcg gag cgc ggc ggc gag gcg gcc cgg ctc aac ctg ttg gac       377
Pro Asp Ser Glu Arg Gly Gly Glu Ala Ala Arg Leu Asn Leu Leu Asp
    40                  45                  50

act tgc gcc gtg tgc cac cag aac atc cag agc cgg gcg ccc aag ctg       425
Thr Cys Ala Val Cys His Gln Asn Ile Gln Ser Arg Ala Pro Lys Leu
55                  60                  65                  70

ctg ccc tgc ctg cac tct ttc tgc cag cgc tgc ctg ccc gcg ccc cag       473
Leu Pro Cys Leu His Ser Phe Cys Gln Arg Cys Leu Pro Ala Pro Gln
                75                  80                  85

cgc tac ctc atg ctg ccc gcg ccc atg ctg ggc tcg gcc gag acc ccg       521
Arg Tyr Leu Met Leu Pro Ala Pro Met Leu Gly Ser Ala Glu Thr Pro
            90                  95                  100

cca ccc gtc cct gcc ccc ggc tcg ccg gtc agc ggc tcg tcg ccg ttc 569
Pro Pro Val Pro Ala Pro Gly Ser Pro Val Ser Gly Ser Ser Pro Phe
        105                 110                 115

gcc acc caa gtt gga gtc att cgt tgc cca gtt tgc agc caa gaa tgt 617
Ala Thr Gln Val Gly Val Ile Arg Cys Pro Val Cys Ser Gln Glu Cys
    120                 125                 130

gca gag aga cac atc ata gat aac ttt ttt gtg aag gac act act gag 665
Ala Glu Arg His Ile Ile Asp Asn Phe Phe Val Lys Asp Thr Thr Glu
135                 140                 145                 150

gtt ccc agc agt aca gta gaa aag tca aat cag gta tgt aca agc tgt 713
Val Pro Ser Ser Thr Val Glu Lys Ser Asn Gln Val Cys Thr Ser Cys
                155                 160                 165

gag gac aac gca gaa gcc aat ggg ttt tgt gta gag tgt gtt gaa tgg 761
Glu Asp Asn Ala Glu Ala Asn Gly Phe Cys Val Glu Cys Val Glu Trp
            170                 175                 180

ctc tgc aag acg tgt atc aga gct cat cag agg gta aag ttc aca aaa 809
Leu Cys Lys Thr Cys Ile Arg Ala His Gln Arg Val Lys Phe Thr Lys
        185                 190                 195

gac cac act gtc aga cag aaa gag gaa gta tct cca gag gca gtt ggt 857
Asp His Thr Val Arg Gln Lys Glu Glu Val Ser Pro Glu Ala Val Gly
    200                 205                 210

gtc acc agc cag cga cca gtg ttt tgt cct ttt cat aaa aag gag cag 905
Val Thr Ser Gln Arg Pro Val Phe Cys Pro Phe His Lys Lys Glu Gln
215                 220                 225                 230

ctg aag ctg tac tgt gag aca tgt gac aaa ctg aca tgt cga gac tgt 953
Leu Lys Leu Tyr Cys Glu Thr Cys Asp Lys Leu Thr Cys Arg Asp Cys
                235                 240                 245

cag ttg tta gaa cat aaa gag cat aga tac caa ttt ata gaa gaa gct 1001
Gln Leu Leu Glu His Lys Glu His Arg Tyr Gln Phe Ile Glu Glu Ala
            250                 255                 260

ttt cag aat cag aaa gtg atc ata gat aca cta atc acc aaa ctg atg 1049
Phe Gln Asn Gln Lys Val Ile Ile Asp Thr Leu Ile Thr Lys Leu Met
        265                 270                 275

gaa aaa aca aaa tac ata aaa ttc aca gga aat cag atc caa aac agg 1097
Glu Lys Thr Lys Tyr Ile Lys Phe Thr Gly Asn Gln Ile Gln Asn Arg
    280                 285                 290

ccc caa att ctc acc agt ccg tct cct tca aaa tcc att cca att cca 1145
Pro Gln Ile Leu Thr Ser Pro Ser Pro Ser Lys Ser Ile Pro Ile Pro
295                 300                 305                 310

cag ccc ttc cga cca gca gat gaa gat cat cga aat caa ttt ggg caa 1193
Gln Pro Phe Arg Pro Ala Asp Glu Asp His Arg Asn Gln Phe Gly Gln
                315                 320                 325

cga gac cga tcc tca tca gct ccc aat gtg cat ata aac aca ata gaa 1241
Arg Asp Arg Ser Ser Ser Ala Pro Asn Val His Ile Asn Thr Ile Glu
            330                 335                 340

cct gtc aat att gat gac ttg att aga gac caa gga ttt cgt ggt gat 1289
Pro Val Asn Ile Asp Asp Leu Ile Arg Asp Gln Gly Phe Arg Gly Asp

345                 350                 355

gga gga tca acc aca ggt ttg tct gct acc ccc cct gcc tca tta cct      1337
Gly Gly Ser Thr Thr Gly Leu Ser Ala Thr Pro Pro Ala Ser Leu Pro
    360                 365                 370

ggc tca cta act aac gtg aaa gcc tta cag aaa tct cca gga cct cag      1385
Gly Ser Leu Thr Asn Val Lys Ala Leu Gln Lys Ser Pro Gly Pro Gln
375                 380                 385                 390

cga gaa agg aag tca tct tca tcc tca gaa gac agg aat cga atg aaa      1433
Arg Glu Arg Lys Ser Ser Ser Ser Ser Glu Asp Arg Asn Arg Met Lys
                395                 400                 405

aca ctt ggt aga cgg gac tcg agt gat gat tgg gag att cct gat ggg      1481
Thr Leu Gly Arg Arg Asp Ser Ser Asp Asp Trp Glu Ile Pro Asp Gly
            410                 415                 420

cag att aca gtg gga caa aga att gga tct gga tca ttt gga aca gtc      1529
Gln Ile Thr Val Gly Gln Arg Ile Gly Ser Gly Ser Phe Gly Thr Val
        425                 430                 435

tac aag gga aag tgg cat ggt gat gtg gca gtg aaa atg ttg aat gtg      1577
Tyr Lys Gly Lys Trp His Gly Asp Val Ala Val Lys Met Leu Asn Val
    440                 445                 450

aca gca cct aca cct cag cag tta caa gcc ttc aaa aat gaa gta gga      1625
Thr Ala Pro Thr Pro Gln Gln Leu Gln Ala Phe Lys Asn Glu Val Gly
455                 460                 465                 470

gta ctc agg aaa aca cga cat gtg aat atc cta ctc ttc atg ggc tat      1673
Val Leu Arg Lys Thr Arg His Val Asn Ile Leu Leu Phe Met Gly Tyr
                475                 480                 485

tcc aca aag cca caa ctg gct att gtt acc cag tgg tgt gag ggc tcc      1721
Ser Thr Lys Pro Gln Leu Ala Ile Val Thr Gln Trp Cys Glu Gly Ser
            490                 495                 500

agc ttg tat cac cat ctc cat atc att gag acc aaa ttt gag atg atc      1769
Ser Leu Tyr His His Leu His Ile Ile Glu Thr Lys Phe Glu Met Ile
        505                 510                 515

aaa ctt ata gat att gca cga cag act gca cag ggc atg gat tac tta      1817
Lys Leu Ile Asp Ile Ala Arg Gln Thr Ala Gln Gly Met Asp Tyr Leu
    520                 525                 530

cac gcc aag tca atc atc cac aga gac ctc aag agt aat aat ata ttt      1865
His Ala Lys Ser Ile Ile His Arg Asp Leu Lys Ser Asn Asn Ile Phe
535                 540                 545                 550

ctt cat gaa gac ctc aca gta aaa ata ggt gat ttt ggt cta gct aca      1913
Leu His Glu Asp Leu Thr Val Lys Ile Gly Asp Phe Gly Leu Ala Thr
                555                 560                 565

gtg aaa tct cga tgg agt ggg tcc cat cag ttt gaa cag ttg tct gga      1961
Val Lys Ser Arg Trp Ser Gly Ser His Gln Phe Glu Gln Leu Ser Gly
            570                 575                 580

tcc att ttg tgg atg gca cca gaa gtc atc aga atg caa gat aaa aat      2009
Ser Ile Leu Trp Met Ala Pro Glu Val Ile Arg Met Gln Asp Lys Asn
        585                 590                 595

cca tac agc ttt cag tca gat gta tat gca ttt gga att gtt ctg tat      2057

Pro Tyr Ser Phe Gln Ser Asp Val Tyr Ala Phe Gly Ile Val Leu Tyr
    600                 605                 610

gaa ttg atg act gga cag tta cct tat tca aac atc aac aac agg gac      2105
Glu Leu Met Thr Gly Gln Leu Pro Tyr Ser Asn Ile Asn Asn Arg Asp
615                 620                 625                 630

cag ata att ttt atg gtg gga cga gga tac ctg tct cca gat ctc agt      2153
Gln Ile Ile Phe Met Val Gly Arg Gly Tyr Leu Ser Pro Asp Leu Ser
                635                 640                 645

aag gta cgg agt aac tgt cca aaa gcc atg aag aga tta atg gca gag      2201
Lys Val Arg Ser Asn Cys Pro Lys Ala Met Lys Arg Leu Met Ala Glu
            650                 655                 660

tgc ctc aaa aag aaa aga gat gag aga cca ctc ttt ccc caa att ctc      2249
Cys Leu Lys Lys Lys Arg Asp Glu Arg Pro Leu Phe Pro Gln Ile Leu
        665                 670                 675

gcc tct att gag ctg ctg gcc cgc tca ttg cca aaa att cac cgc agt      2297
Ala Ser Ile Glu Leu Leu Ala Arg Ser Leu Pro Lys Ile His Arg Ser
    680                 685                 690

gca tca gaa ccc tcc ttg aat cgg gct ggt ttc caa aca gag gat ttt      2345
Ala Ser Glu Pro Ser Leu Asn Arg Ala Gly Phe Gln Thr Glu Asp Phe
695                 700                 705                 710

agt cta tat gct tgt gct tct cca aaa aca ccc atc cag gca ggg gga      2393
Ser Leu Tyr Ala Cys Ala Ser Pro Lys Thr Pro Ile Gln Ala Gly Gly
                715                 720                 725

tat ggt gcg ttt cct gtc cac tga aacaaatgag tgagagagtt caggagagta      2447
Tyr Gly Ala Phe Pro Val His
            730

gcaacaaaag gaaaataaat gaacatatgt ttgcttatat gttaaattga ataaaatact      2507

ctcttttttt ttaaggtgaa ccaaagaaca cttgtgtggt taaagactag atataatttt      2567

tccccaaact aaaatttata cttaacattg gatttttaac atccaagggt taaaatacat      2627

agacattgct aaaaattggc agagcctctt ctagaggctt tactttctgt tccgggtttg      2687

tatcattcac ttggttattt taagtagtaa acttcagttt ctcatgcaac ttttgttgcc      2747

agctatcaca tgtccactag ggactccaga agaagaccct acctatgcct gtgtttgcag      2807

gtgagaagtt ggcagtcggt tagcctgggt tagataaggc aaactgaaca gatctaattt      2867

aggaagtcag tagaatttaa taattctatt attattctta ataatttttc tataactatt      2927

tctttttata acaatttgga aaatgtggat gtcttttatt tccttgaagc aataaactaa      2987

gtttcttttt ataaaaa                                                    3004

<210>  6
<211>  733
<212>  PRT
<213>  Homo sapiens

<400>  6
   Met Glu Val Ala Val Glu Lys Ala Val Ala Ala Ala Ala Ala Ala Ser
   1               5                   10                  15
   Ala Ala Ala Ser Gly Gly Pro Ser Ala Ala Pro Ser Gly Glu Asn Glu
               20                  25                  30
   Ala Glu Ser Arg Gln Gly Pro Asp Ser Glu Arg Gly Gly Glu Ala Ala
           35                  40                  45
   Arg Leu Asn Leu Leu Asp Thr Cys Ala Val Cys His Gln Asn Ile Gln
       50                  55                  60
   Ser Arg Ala Pro Lys Leu Leu Pro Cys Leu His Ser Phe Cys Gln Arg
   65                  70                  75                  80
   Cys Leu Pro Ala Pro Gln Arg Tyr Leu Met Leu Pro Ala Pro Met Leu
                   85                  90                  95
   Gly Ser Ala Glu Thr Pro Pro Pro Val Pro Ala Pro Gly Ser Pro Val
               100                 105                 110
   Ser Gly Ser Ser Pro Phe Ala Thr Gln Val Gly Val Ile Arg Cys Pro
           115                 120                 125
   Val Cys Ser Gln Glu Cys Ala Glu Arg His Ile Ile Asp Asn Phe Phe
       130                 135                 140
   Val Lys Asp Thr Thr Glu Val Pro Ser Ser Thr Val Glu Lys Ser Asn
   145                 150                 155                 160
   Gln Val Cys Thr Ser Cys Glu Asp Asn Ala Glu Ala Asn Gly Phe Cys
                   165                 170                 175
   Val Glu Cys Val Glu Trp Leu Cys Lys Thr Cys Ile Arg Ala His Gln
               180                 185                 190
   Arg Val Lys Phe Thr Lys Asp His Thr Val Arg Gln Lys Glu Glu Val
           195                 200                 205
   Ser Pro Glu Ala Val Gly Val Thr Ser Gln Arg Pro Val Phe Cys Pro
       210                 215                 220
   Phe His Lys Lys Glu Gln Leu Lys Leu Tyr Cys Glu Thr Cys Asp Lys
   225                 230                 235                 240
   Leu Thr Cys Arg Asp Cys Gln Leu Leu Glu His Lys Glu His Arg Tyr
                   245                 250                 255
   Gln Phe Ile Glu Glu Ala Phe Gln Asn Gln Lys Val Ile Ile Asp Thr
                   260                 265                 270
   Leu Ile Thr Lys Leu Met Glu Lys Thr Lys Tyr Ile Lys Phe Thr Gly
               275                 280                 285
   Asn Gln Ile Gln Asn Arg Pro Gln Ile Leu Thr Ser Pro Ser Pro Ser
       290                 295                 300
   Lys Ser Ile Pro Ile Pro Gln Pro Phe Arg Pro Ala Asp Glu Asp His
   305                 310                 315                 320
   Arg Asn Gln Phe Gly Gln Arg Asp Arg Ser Ser Ser Ala Pro Asn Val
                   325                 330                 335
   His Ile Asn Thr Ile Glu Pro Val Asn Ile Asp Asp Leu Ile Arg Asp
                   340                 345                 350
   Gln Gly Phe Arg Gly Asp Gly Gly Ser Thr Thr Gly Leu Ser Ala Thr
               355                 360                 365
   Pro Pro Ala Ser Leu Pro Gly Ser Leu Thr Asn Val Lys Ala Leu Gln
       370                 375                 380
   Lys Ser Pro Gly Pro Gln Arg Glu Arg Lys Ser Ser Ser Ser Ser Glu
   385                 390                 395                 400
   Asp Arg Asn Arg Met Lys Thr Leu Gly Arg Arg Asp Ser Ser Asp Asp
                   405                 410                 415
   Trp Glu Ile Pro Asp Gly Gln Ile Thr Val Gly Gln Arg Ile Gly Ser
                   420                 425                 430
   Gly Ser Phe Gly Thr Val Tyr Lys Gly Lys Trp His Gly Asp Val Ala
               435                 440                 445
   Val Lys Met Leu Asn Val Thr Ala Pro Thr Pro Gln Gln Leu Gln Ala
       450                 455                 460
   Phe Lys Asn Glu Val Gly Val Leu Arg Lys Thr Arg His Val Asn Ile
   465                 470                 475                 480
   Leu Leu Phe Met Gly Tyr Ser Thr Lys Pro Gln Leu Ala Ile Val Thr
                   485                 490                 495
   Gln Trp Cys Glu Gly Ser Ser Leu Tyr His His Leu His Ile Ile Glu
                   500                 505                 510
   Thr Lys Phe Glu Met Ile Lys Leu Ile Asp Ile Ala Arg Gln Thr Ala
           515                 520                 525
   Gln Gly Met Asp Tyr Leu His Ala Lys Ser Ile Ile His Arg Asp Leu
       530                 535                 540
   Lys Ser Asn Asn Ile Phe Leu His Glu Asp Leu Thr Val Lys Ile Gly
   545                 550                 555                 560
   Asp Phe Gly Leu Ala Thr Val Lys Ser Arg Trp Ser Gly Ser His Gln
                   565                 570                 575
   Phe Glu Gln Leu Ser Gly Ser Ile Leu Trp Met Ala Pro Glu Val Ile
               580                 585                 590
   Arg Met Gln Asp Lys Asn Pro Tyr Ser Phe Gln Ser Asp Val Tyr Ala
           595                 600                 605
   Phe Gly Ile Val Leu Tyr Glu Leu Met Thr Gly Gln Leu Pro Tyr Ser
       610                 615                 620
   Asn Ile Asn Asn Arg Asp Gln Ile Ile Phe Met Val Gly Arg Gly Tyr
   625                 630                 635                 640
   Leu Ser Pro Asp Leu Ser Lys Val Arg Ser Asn Cys Pro Lys Ala Met
                   645                 650                 655
   Lys Arg Leu Met Ala Glu Cys Leu Lys Lys Lys Arg Asp Glu Arg Pro
               660                 665                 670
   Leu Phe Pro Gln Ile Leu Ala Ser Ile Glu Leu Leu Ala Arg Ser Leu
           675                 680                 685
   Pro Lys Ile His Arg Ser Ala Ser Glu Pro Ser Leu Asn Arg Ala Gly
       690                 695                 700
   Phe Gln Thr Glu Asp Phe Ser Leu Tyr Ala Cys Ala Ser Pro Lys Thr
   705                 710                 715                 720
   Pro Ile Gln Ala Gly Gly Tyr Gly Ala Phe Pro Val His
                   725                 730

<210>  7
<211>  1596
<212>  DNA
<213>  Homo sapiens

<220>
<221>  CDS
<222>  (188)..(1099)

<400>  7
ctgcctgggg agcccccccg ccccacatcc tgccccgcaa aaggcagctt caccaaagtg      60

gggtatttcc agcctttgta gctttcactt ccacatctac caagtgggcg gagtggcctt     120

ctgtggacga atcagattcc tctccagcac cgactttaag aggcgagccg gggggtcagg     180

gtcccag atg cac agg agg aga agc agg agc tgt cgg gaa gat cag aag       229
        Met His Arg Arg Arg Ser Arg Ser Cys Arg Glu Asp Gln Lys
        1               5                   10

cca gtc atg gat gac cag cgc gac ctt atc tcc aac aat gag caa ctg       277
Pro Val Met Asp Asp Gln Arg Asp Leu Ile Ser Asn Asn Glu Gln Leu
15                  20                  25                  30

ccc atg ctg ggc cgg cgc cct ggg gcc ccg gag agc aag tgc agc cgc       325
Pro Met Leu Gly Arg Arg Pro Gly Ala Pro Glu Ser Lys Cys Ser Arg
                35                  40                  45

gga gcc ctg tac aca ggc ttt tcc atc ctg gtg act ctg ctc ctc gct       373
Gly Ala Leu Tyr Thr Gly Phe Ser Ile Leu Val Thr Leu Leu Leu Ala
            50                  55                  60

ggc cag gcc acc acc gcc tac ttc ctg tac cag cag cag ggc cgg ctg       421
Gly Gln Ala Thr Thr Ala Tyr Phe Leu Tyr Gln Gln Gln Gly Arg Leu
        65                  70                  75

gac aaa ctg aca gtc acc tcc cag aac ctg cag ctg gag aac ctg cgc       469
Asp Lys Leu Thr Val Thr Ser Gln Asn Leu Gln Leu Glu Asn Leu Arg
    80                  85                  90

atg aag ctt ccc aag cct ccc aag cct gtg agc aag atg cgc atg gcc       517
Met Lys Leu Pro Lys Pro Pro Lys Pro Val Ser Lys Met Arg Met Ala
95                  100                 105                 110

acc ccg ctg ctg atg cag gcg ctg ccc atg gga gcc ctg ccc cag ggg       565
Thr Pro Leu Leu Met Gln Ala Leu Pro Met Gly Ala Leu Pro Gln Gly
                115                 120                 125

ccc atg cag aat gcc acc aag tat ggc aac atg aca gag gac cat gtg       613
Pro Met Gln Asn Ala Thr Lys Tyr Gly Asn Met Thr Glu Asp His Val
            130                 135                 140

atg cac ctg ctc cag aat gct gac ccc ctg aag gtg tac ccg cca ctg       661
Met His Leu Leu Gln Asn Ala Asp Pro Leu Lys Val Tyr Pro Pro Leu
        145                 150                 155

aag ggg agc ttc ccg gag aac ctg aga cac ctt aag aac acc atg gag       709
Lys Gly Ser Phe Pro Glu Asn Leu Arg His Leu Lys Asn Thr Met Glu
    160                 165                 170

acc ata gac tgg aag gtc ttt gag agc tgg atg cac cat tgg ctc ctg       757
Thr Ile Asp Trp Lys Val Phe Glu Ser Trp Met His His Trp Leu Leu
175                 180                 185                 190

ttt gaa atg agc agg cac tcc ttg gag caa aag ccc act gac gct cca       805
Phe Glu Met Ser Arg His Ser Leu Glu Gln Lys Pro Thr Asp Ala Pro
                195                 200                 205

ccg aaa gag tca ctg gaa ctg gag gac ccg tct tct ggg ctg ggt gtg     853
Pro Lys Glu Ser Leu Glu Leu Glu Asp Pro Ser Ser Gly Leu Gly Val
            210                 215                 220

acc aag cag gat ctg ggc cca gct aca tct aca tcc acc act ggg aca     901
Thr Lys Gln Asp Leu Gly Pro Ala Thr Ser Thr Ser Thr Thr Gly Thr
        225                 230                 235

agc cat ctt gta aaa tgt gcg gag aag gag aaa act ttc tgt gtg aat     949
Ser His Leu Val Lys Cys Ala Glu Lys Glu Lys Thr Phe Cys Val Asn
    240                 245                 250

gga ggg gag tgc ttc atg gtg aaa gac ctt tca aac ccc tcg aga tac     997
Gly Gly Glu Cys Phe Met Val Lys Asp Leu Ser Asn Pro Ser Arg Tyr
255                 260                 265                 270

ttg tgc aag tgc cca aat gag ttt act ggt gat cgc tgc caa aac tac     1045
Leu Cys Lys Cys Pro Asn Glu Phe Thr Gly Asp Arg Cys Gln Asn Tyr
                275                 280                 285

gta atg gcc agc ttc tac agt acg tcc act ccc ttt ctg tct ctg cct     1093
Val Met Ala Ser Phe Tyr Ser Thr Ser Thr Pro Phe Leu Ser Leu Pro
            290                 295                 300

gaa tag gagcatgctc agttggtgct gctttcttgt tgctgcatct cccctcagat     1149
Glu

tccacctaga gctagatgtg tcttaccaga tctaatattg actgcctctg cctgtcgcat     1209

gagaacatta acaaaagcaa ttgtattact tcctctgttc gcgactagtt ggctctgaga     1269

tactaatagg tgtgtgaggc tccggatgtt tctggaattg atattgaatg atgtgataca     1329

aattgatagt caatatcaag cagtgaaata tgataataaa ggcatttcaa agtctcactt     1389

ttattgataa aataaaaatc attctactga acagtccatc ttctttatac aatgaccaca     1449

tcctgaaaag ggtgttgcta agctgtaacc gatatgcact tgaaatgatg gtaagttaat     1509

tttgattcag aatgtgttat ttgtcacaaa taaacataat aaaaggagtt cagatgtttt     1569

tcttcattaa ccaaaaaaaa aaaaaaa     1596

<210>  8
<211>  303
<212>  PRT
<213>  Homo sapiens

<400>  8

Met His Arg Arg Arg Ser Arg Ser Cys Arg Glu Asp Gln Lys Pro Val
1               5                   10                  15

Met Asp Asp Gln Arg Asp Leu Ile Ser Asn Asn Glu Gln Leu Pro Met
            20                  25                  30

Leu Gly Arg Arg Pro Gly Ala Pro Glu Ser Lys Cys Ser Arg Gly Ala
        35                  40                  45

Leu Tyr Thr Gly Phe Ser Ile Leu Val Thr Leu Leu Leu Ala Gly Gln
    50                  55                  60

Ala Thr Thr Ala Tyr Phe Leu Tyr Gln Gln Gln Gly Arg Leu Asp Lys
65                  70                  75                  80

Leu Thr Val Thr Ser Gln Asn Leu Gln Leu Glu Asn Leu Arg Met Lys
                85                  90                  95

Leu Pro Lys Pro Pro Lys Pro Val Ser Lys Met Arg Met Ala Thr Pro
            100                 105                 110

Leu Leu Met Gln Ala Leu Pro Met Gly Ala Leu Pro Gln Gly Pro Met
        115                 120                 125

Gln Asn Ala Thr Lys Tyr Gly Asn Met Thr Glu Asp His Val Met His
    130                 135                 140

Leu Leu Gln Asn Ala Asp Pro Leu Lys Val Tyr Pro Pro Leu Lys Gly
145                 150                 155                 160

Ser Phe Pro Glu Asn Leu Arg His Leu Lys Asn Thr Met Glu Thr Ile
                165                 170                 175

Asp Trp Lys Val Phe Glu Ser Trp Met His His Trp Leu Leu Phe Glu
            180                 185                 190

Met Ser Arg His Ser Leu Glu Gln Lys Pro Thr Asp Ala Pro Pro Lys
        195                 200                 205

Glu Ser Leu Glu Leu Glu Asp Pro Ser Ser Gly Leu Gly Val Thr Lys
    210                 215                 220

Gln Asp Leu Gly Pro Ala Thr Ser Thr Ser Thr Thr Gly Thr Ser His
225                 230                 235                 240

Leu Val Lys Cys Ala Glu Lys Glu Lys Thr Phe Cys Val Asn Gly Gly
                245                 250                 255

Glu Cys Phe Met Val Lys Asp Leu Ser Asn Pro Ser Arg Tyr Leu Cys
            260                 265                 270

Lys Cys Pro Asn Glu Phe Thr Gly Asp Arg Cys Gln Asn Tyr Val Met
        275                 280                 285

Ala Ser Phe Tyr Ser Thr Ser Thr Pro Phe Leu Ser Leu Pro Glu

290                 295                 300

<210>  9
<211>  1533
<212>  DNA
<213>  Homo sapiens

<220>
<221>  CDS
<222>  (188)..(1036)

<400>  9
ctgcctgggg agcccccccg ccccacatcc tgccccgcaa aaggcagctt caccaaagtg      60

gggtatttcc agcctttgta gctttcactt ccacatctac caagtgggcg gagtggcctt     120

ctgtggacga atcagattcc tctccagcac cgactttaag aggcgagccg gggggtcagg     180

gtcccag atg cac agg agg aga agc agg agc tgt cgg gaa gat cag aag       229
        Met His Arg Arg Arg Ser Arg Ser Cys Arg Glu Asp Gln Lys
        1               5                   10

cca gtc atg gat gac cag cgc gac ctt atc tcc aac aat gag caa ctg       277
Pro Val Met Asp Asp Gln Arg Asp Leu Ile Ser Asn Asn Glu Gln Leu
15                  20                  25                  30

ccc atg ctg ggc cgg cgc cct ggg gcc ccg gag agc aag tgc agc cgc       325
Pro Met Leu Gly Arg Arg Pro Gly Ala Pro Glu Ser Lys Cys Ser Arg
                35                  40                  45

gga gcc ctg tac aca ggc ttt tcc atc ctg gtg act ctg ctc ctc gct       373
Gly Ala Leu Tyr Thr Gly Phe Ser Ile Leu Val Thr Leu Leu Leu Ala
            50                  55                  60

ggc cag gcc acc acc gcc tac ttc ctg tac cag cag cag ggc cgg ctg       421
Gly Gln Ala Thr Thr Ala Tyr Phe Leu Tyr Gln Gln Gln Gly Arg Leu
        65                  70                  75

gac aaa ctg aca gtc acc tcc cag aac ctg cag ctg gag aac ctg cgc       469
Asp Lys Leu Thr Val Thr Ser Gln Asn Leu Gln Leu Glu Asn Leu Arg
    80                  85                  90

atg aag ctt ccc aag cct ccc aag cct gtg agc aag atg cgc atg gcc       517
Met Lys Leu Pro Lys Pro Pro Lys Pro Val Ser Lys Met Arg Met Ala
95                  100                 105                 110

acc ccg ctg ctg atg cag gcg ctg ccc atg gga gcc ctg ccc cag ggg       565
Thr Pro Leu Leu Met Gln Ala Leu Pro Met Gly Ala Leu Pro Gln Gly
                115                 120                 125

ccc atg cag aat gcc acc aag tat ggc aac atg aca gag gac cat gtg       613
Pro Met Gln Asn Ala Thr Lys Tyr Gly Asn Met Thr Glu Asp His Val
            130                 135                 140

atg cac ctg ctc cag aat gct gac ccc ctg aag gtg tac ccg cca ctg       661
Met His Leu Leu Gln Asn Ala Asp Pro Leu Lys Val Tyr Pro Pro Leu
        145                 150                 155

aag ggg agc ttc ccg gag aac ctg aga cac ctt aag aac acc atg gag       709
Lys Gly Ser Phe Pro Glu Asn Leu Arg His Leu Lys Asn Thr Met Glu
    160                 165                 170

acc ata gac tgg aag gtc ttt gag agc tgg atg cac cat tgg ctc ctg     757
Thr Ile Asp Trp Lys Val Phe Glu Ser Trp Met His His Trp Leu Leu
175                 180                 185                 190

ttt gaa atg agc agg cac tcc ttg gag caa aag ccc act gac gct cca     805
Phe Glu Met Ser Arg His Ser Leu Glu Gln Lys Pro Thr Asp Ala Pro
                195                 200                 205

ccg aaa gct aca tct aca tcc acc act ggg aca agc cat ctt gta aaa     853
Pro Lys Ala Thr Ser Thr Ser Thr Thr Gly Thr Ser His Leu Val Lys
            210                 215                 220

tgt gcg gag aag gag aaa act ttc tgt gtg aat gga ggg gag tgc ttc     901
Cys Ala Glu Lys Glu Lys Thr Phe Cys Val Asn Gly Gly Glu Cys Phe
        225                 230                 235

atg gtg aaa gac ctt tca aac ccc tcg aga tac ttg tgc aag tgc cca     949
Met Val Lys Asp Leu Ser Asn Pro Ser Arg Tyr Leu Cys Lys Cys Pro
    240                 245                 250

aat gag ttt act ggt gat cgc tgc caa aac tac gta atg gcc agc ttc     997
Asn Glu Phe Thr Gly Asp Arg Cys Gln Asn Tyr Val Met Ala Ser Phe
255                 260                 265                 270

tac agt acg tcc act ccc ttt ctg tct ctg cct gaa tag gagcatgctc     1046
Tyr Ser Thr Ser Thr Pro Phe Leu Ser Leu Pro Glu
                275                 280

agttggtgct gctttcttgt tgctgcatct cccctcagat tccacctaga gctagatgtg     1106

tcttaccaga tctaatattg actgcctctg cctgtcgcat gagaacatta acaaaagcaa     1166

ttgtattact tcctctgttc gcgactagtt ggctctgaga tactaatagg tgtgtgaggc     1226

tccggatgtt tctggaattg atattgaatg atgtgataca aattgatagt caatatcaag     1286

cagtgaaata tgataataaa ggcatttcaa agtctcactt ttattgataa aataaaaatc     1346

attctactga acagtccatc ttctttatac aatgaccaca tcctgaaaag ggtgttgcta     1406

agctgtaacc gatatgcact tgaaatgatg gtaagttaat tttgattcag aatgtgttat     1466

ttgtcacaaa taaacataat aaaaggagtt cagatgtttt tcttcattaa ccaaaaaaaa     1526

aaaaaaa                                                               1533

<210>  10
<211>  282
<212>  PRT
<213>  Homo sapiens

<400>  10

Met His Arg Arg Arg Ser Arg Ser Cys Arg Glu Asp Gln Lys Pro Val
1               5                   10                  15

Met Asp Asp Gln Arg Asp Leu Ile Ser Asn Asn Glu Gln Leu Pro Met
            20                  25                  30

Leu Gly Arg Arg Pro Gly Ala Pro Glu Ser Lys Cys Ser Arg Gly Ala
        35                  40                  45

Leu Tyr Thr Gly Phe Ser Ile Leu Val Thr Leu Leu Leu Ala Gly Gln
    50                  55                  60

Ala Thr Thr Ala Tyr Phe Leu Tyr Gln Gln Gln Gly Arg Leu Asp Lys
65                  70                  75                  80

Leu Thr Val Thr Ser Gln Asn Leu Gln Leu Glu Asn Leu Arg Met Lys
                85                  90                  95

Leu Pro Lys Pro Pro Lys Pro Val Ser Lys Met Arg Met Ala Thr Pro
            100                 105                 110

Leu Leu Met Gln Ala Leu Pro Met Gly Ala Leu Pro Gln Gly Pro Met
        115                 120                 125

Gln Asn Ala Thr Lys Tyr Gly Asn Met Thr Glu Asp His Val Met His
    130                 135                 140

Leu Leu Gln Asn Ala Asp Pro Leu Lys Val Tyr Pro Pro Leu Lys Gly
145                 150                 155                 160

Ser Phe Pro Glu Asn Leu Arg His Leu Lys Asn Thr Met Glu Thr Ile
                165                 170                 175

Asp Trp Lys Val Phe Glu Ser Trp Met His His Trp Leu Leu Phe Glu
            180                 185                 190

Met Ser Arg His Ser Leu Glu Gln Lys Pro Thr Asp Ala Pro Pro Lys
        195                 200                 205

Ala Thr Ser Thr Ser Thr Thr Gly Thr Ser His Leu Val Lys Cys Ala
    210                 215                 220

Glu Lys Glu Lys Thr Phe Cys Val Asn Gly Gly Glu Cys Phe Met Val
225                 230                 235                 240

Lys Asp Leu Ser Asn Pro Ser Arg Tyr Leu Cys Lys Cys Pro Asn Glu
                245                 250                 255

Phe Thr Gly Asp Arg Cys Gln Asn Tyr Val Met Ala Ser Phe Tyr Ser
            260                 265                 270

Thr Ser Thr Pro Phe Leu Ser Leu Pro Glu
        275                 280

<210>  11
<211>  20
<212>  DNA
<213>  Artificial Sequence

<220>
<223>  Primer sequence

<400>  11
aaggaggagc tggagagaca                                                    20


<210>  12
<211>  20
<212>  DNA
<213>  Artificial Sequence

<220>
<223>  Primer sequence

<400>  12
cacctgagcc aaggactttt                                                    20


<210>  13
<211>  20
<212>  DNA
<213>  Artificial Sequence

<220>
<223>  Primer sequence

<400>  13
tgtctcctgc attccatcaa                                                    20


<210>  14
<211>  20
<212>  DNA
<213>  Artificial Sequence

<220>
<223>  Primer sequence

<400>  14
tccaaattcg ccttctccta                                                    20


<210>  15
<211>  24
<212>  DNA
<213>  Artificial Sequence

<220>
<223>  Primer sequence

<400>  15
tgtcgagact gtcagttgtt agaa                                               24


<210>  16
<211>  20

<212>  DNA
<213>  Artificial Sequence

<220>
<223>  Primer sequence

<400>  16
gcccaaattg atttcgatga                                                   20


<210>  17
<211>  20
<212>  DNA
<213>  Artificial Sequence

<220>
<223>  Primer sequence

<400>  17
cggagaacct gagacacctt                                                   20


<210>  18
<211>  20
<212>  DNA
<213>  Artificial Sequence

<220>
<223>  Primer sequence

<400>  18
actcccctcc attcacacag                                                   20


<210>  19
<211>  3138
<212>  DNA
<213>  Homo sapiens


<220>
<221>  CDS
<222>  (182)..(1942)

<400>  19
ggcgtggtcc cgggacccgc cccgccgggg cttttgggag cgcgggcagc gagcgcactc      60

ggcggacgca agggcggcgg ggagcacacg gagcactgca ggcgccgggt tgggacagcg     120

tcttcgctgc tgctggatag tcgtgttttc ggggatcgag gatactcacc agaaaccgaa     180

a atg ccg aaa cca atc aat gtc cga gtt acc acc atg gat gca gag ctg     229
  Met Pro Lys Pro Ile Asn Val Arg Val Thr Thr Met Asp Ala Glu Leu
  1               5                   10                  15

gag ttt gca atc cag cca aat aca act gga aaa cag ctt ttt gat cag       277
Glu Phe Ala Ile Gln Pro Asn Thr Thr Gly Lys Gln Leu Phe Asp Gln
            20                  25                  30

gtg gta aag act atc ggc ctc cgg gaa gtg tgg tac ttt ggc ctc cac       325
Val Val Lys Thr Ile Gly Leu Arg Glu Val Trp Tyr Phe Gly Leu His
        35                  40                  45

tat gtg gat aat aaa gga ttt cct acc tgg ctg aag ctg gat aag aag 373
Tyr Val Asp Asn Lys Gly Phe Pro Thr Trp Leu Lys Leu Asp Lys Lys
    50                  55                  60

gtg tct gcc cag gag gtc agg aag gag aat ccc ctc cag ttc aag ttc 421
Val Ser Ala Gln Glu Val Arg Lys Glu Asn Pro Leu Gln Phe Lys Phe
65                  70                  75                  80

cgg gcc aag ttc tac cct gaa gat gtg gct gag gag ctc atc cag gac 469
Arg Ala Lys Phe Tyr Pro Glu Asp Val Ala Glu Glu Leu Ile Gln Asp
                85                  90                  95

atc acc cag aaa ctt ttc ttc ctc caa gtg aag gaa gga atc ctt agc 517
Ile Thr Gln Lys Leu Phe Phe Leu Gln Val Lys Glu Gly Ile Leu Ser
            100                 105                 110

gat gag atc tac tgc ccc cct gag act gcc gtg ctc ttg ggg tcc tac 565
Asp Glu Ile Tyr Cys Pro Pro Glu Thr Ala Val Leu Leu Gly Ser Tyr
        115                 120                 125

gct gtg cag gcc aag ttt ggg gac tac aac aaa gaa gtg cac aag tct 613
Ala Val Gln Ala Lys Phe Gly Asp Tyr Asn Lys Glu Val His Lys Ser
    130                 135                 140

ggg tac ctc agc tct gag cgg ctg atc cct caa aga gtg atg gac cag 661
Gly Tyr Leu Ser Ser Glu Arg Leu Ile Pro Gln Arg Val Met Asp Gln
145                 150                 155                 160

cac aaa ctt acc agg gac cag tgg gag gac cgg atc cag gtg tgg cat 709
His Lys Leu Thr Arg Asp Gln Trp Glu Asp Arg Ile Gln Val Trp His
                165                 170                 175

gcg gaa cac cgt ggg atg ctc aaa gat aat gct atg ttg gaa tac ctg 757
Ala Glu His Arg Gly Met Leu Lys Asp Asn Ala Met Leu Glu Tyr Leu
            180                 185                 190

aag att gct cag gac ctg gaa atg tat gga atc aac tat ttc gag ata 805
Lys Ile Ala Gln Asp Leu Glu Met Tyr Gly Ile Asn Tyr Phe Glu Ile
        195                 200                 205

aaa aac aag aaa gga aca gac ctt tgg ctt gga gtt gat gcc ctt gga 853
Lys Asn Lys Lys Gly Thr Asp Leu Trp Leu Gly Val Asp Ala Leu Gly
    210                 215                 220

ctg aat att tat gag aaa gat gat aag tta acc cca aag att ggc ttt 901
Leu Asn Ile Tyr Glu Lys Asp Asp Lys Leu Thr Pro Lys Ile Gly Phe
225                 230                 235                 240

cct tgg agt gaa atc agg aac atc tct ttc aat gac aaa aag ttt gtc 949
Pro Trp Ser Glu Ile Arg Asn Ile Ser Phe Asn Asp Lys Lys Phe Val
                245                 250                 255

att aaa ccc atc gac aag aag gca cct gac ttt gtg ttt tat gcc cca 997
Ile Lys Pro Ile Asp Lys Lys Ala Pro Asp Phe Val Phe Tyr Ala Pro
            260                 265                 270

cgt ctg aga atc aac aag cgg atc ctg cag ctc tgc atg ggc aac cat 1045
Arg Leu Arg Ile Asn Lys Arg Ile Leu Gln Leu Cys Met Gly Asn His
        275                 280                 285

gag ttg tat atg cgc cgc agg aag cct gac acc atc gag gtg cag cag 1093
Glu Leu Tyr Met Arg Arg Arg Lys Pro Asp Thr Ile Glu Val Gln Gln
    290                 295                 300

atg aag gcc cag gcc cgg gag gag aag cat cag aag cag ctg gag cgg 1141
Met Lys Ala Gln Ala Arg Glu Glu Lys His Gln Lys Gln Leu Glu Arg
305                 310                 315                 320

caa cag ctg gaa aca gag aag aaa agg aga gaa acc gtg gag aga gag 1189
Gln Gln Leu Glu Thr Glu Lys Lys Arg Arg Glu Thr Val Glu Arg Glu
                325                 330                 335

aaa gag cag atg atg cgc gag aag gag gag ttg atg ctg cgg ctg cag 1237
Lys Glu Gln Met Met Arg Glu Lys Glu Glu Leu Met Leu Arg Leu Gln
            340                 345                 350

gac tat gag gag aag aca aag aag gca gag aga gag ctc tcg gag cag 1285
Asp Tyr Glu Glu Lys Thr Lys Lys Ala Glu Arg Glu Leu Ser Glu Gln
        355                 360                 365

att cag agg gcc ctg cag ctg gag gag gag agg aag cgg gca cag gag 1333
Ile Gln Arg Ala Leu Gln Leu Glu Glu Glu Arg Lys Arg Ala Gln Glu
    370                 375                 380

gag gcc gag cgc cta gag gct gac cgt atg gct gca ctg cgg gct aag 1381
Glu Ala Glu Arg Leu Glu Ala Asp Arg Met Ala Ala Leu Arg Ala Lys
385                 390                 395                 400

gag gag ctg gag aga cag gcg gtg gat cag ata aag agc cag gag cag 1429
Glu Glu Leu Glu Arg Gln Ala Val Asp Gln Ile Lys Ser Gln Glu Gln
                405                 410                 415

ctg gct gcg gag ctt gca gaa tac act gcc aag att gcc ctc ctg gaa 1477
Leu Ala Ala Glu Leu Ala Glu Tyr Thr Ala Lys Ile Ala Leu Leu Glu
            420                 425                 430

gag gcg cgg agg cgc aag gag gat gaa gtt gaa gag tgg cag cac agg 1525
Glu Ala Arg Arg Arg Lys Glu Asp Glu Val Glu Glu Trp Gln His Arg
        435                 440                 445

gcc aaa gaa gcc cag gat gac ctg gtg aag acc aag gag gag ctg cac 1573
Ala Lys Glu Ala Gln Asp Asp Leu Val Lys Thr Lys Glu Glu Leu His
    450                 455                 460

ctg gtg atg aca gca ccc ccg ccc cca cca ccc ccc gtg tac gag ccg 1621
Leu Val Met Thr Ala Pro Pro Pro Pro Pro Pro Pro Val Tyr Glu Pro
465                 470                 475                 480

gtg agc tac cat gtc cag gag agc ttg cag gat gag ggc gca gag ccc 1669
Val Ser Tyr His Val Gln Glu Ser Leu Gln Asp Glu Gly Ala Glu Pro
                485                 490                 495

acg ggc tac agc gcg gag ctg tct agt gag ggc atc cgg gat gac cgc 1717
Thr Gly Tyr Ser Ala Glu Leu Ser Ser Glu Gly Ile Arg Asp Asp Arg
            500                 505                 510

aat gag gag aag cgc atc act gag gca gag aag aac gag cgt gtg cag 1765
Asn Glu Glu Lys Arg Ile Thr Glu Ala Glu Lys Asn Glu Arg Val Gln
        515                 520                 525

cgg cag ctg ctg acg ctg agc agc gag ctg tcc cag gcc cga gat gag 1813
Arg Gln Leu Leu Thr Leu Ser Ser Glu Leu Ser Gln Ala Arg Asp Glu
    530                 535                 540

aat aag agg acc cac aat gac atc atc cac aac gag aac atg agg caa 1861
Asn Lys Arg Thr His Asn Asp Ile Ile His Asn Glu Asn Met Arg Gln

545                 550                 555                 560

ggc cgg gac aag tac aag acg ctg cgg cag atc cgg cag ggc aac acc      1909
Gly Arg Asp Lys Tyr Lys Thr Leu Arg Gln Ile Arg Gln Gly Asn Thr
                565                 570                 575

aag cag cgc atc gac gag ttc gag gcc ctg taa cagccaggcc aggaccaagg    1962
Lys Gln Arg Ile Asp Glu Phe Glu Ala Leu
            580                 585

gcagaggggt gctcatagcg ggcgctgcca gccccgccac gcttgtgtct ttagtgctcc    2022

aagtctagga actccctcag atcccagttc ctttagaaag cagttaccca acagaaacat    2082

tctgggctgg gaaccaggga ggcgccctgg tttgttttcc ccagttgtaa tagtgccaag    2142

caggcctgat tctcgcgatt attctcgaat cacctcctgt gttgtgctgg gagcaggact    2202

gattgaatta cggaaaatgc ctgtaaagtc tgagtaagaa acttcatgct ggcctgtgtg    2262

atacaagagt cagcatcatt aaaggaaacg tggcaggact tccatctgtg ccatacttgt    2322

tctgtattcg aaatgagctc aaattgattt tttaatttct atgaaggatc catctttgta    2382

tatttacatg cttagagggg tgaaaattat tttggaaatt gagtctgaag cactctcgca    2442

cacacagtga ttccctcctc ccgtcactcc acgcagctgg cagagagcac agtgatcacc    2502

agcgtgagtg gtggaggagg acacttggat tttttttttt gttttttttt tttttgctta    2562

acagttttag aatacattgt acttatacac cttattaatg atcagctata tactatttat    2622

atacaagtga taatacagat ttgtaacatt agttttaaaa agggaaagtt ttgttctgta    2682

tattttgtta ccttttacag aataaaagaa ttacatatga aaaaccctct aaaccatggc    2742

acttgatgtg atgtggcagg agggcagtgg tggagctgga cctgcctgct gcagtcacgt    2802

gtaaacagga ttattattag tgttttatgc atgtaatgga ctatgcacac ttttaatttt    2862

gtcagattca cacatgccac tatgagcttt cagactccag ctgtgaagag actctgtttg    2922

cttgtgtttg tttgtttgca gtctctctct gccatggcct tggcaggctg ctggaaggca    2982

gcttgtggag gccgttggtt ccgcccactc attccttctc gtgcactgct ttctccttca    3042

cagctaagat gccatgtgca ggtggattcc atgccgcaga catgaaataa aagctttgca    3102

aaggcacgaa gcaaaaaaaa aaaaaaaaaa aaaaaa                              3138

<210>  20
<211>  586
<212>  PRT
<213>  Homo sapiens

<400>  20

Met Pro Lys Pro Ile Asn Val Arg Val Thr Thr Met Asp Ala Glu Leu
1               5                   10                  15

Glu Phe Ala Ile Gln Pro Asn Thr Thr Gly Lys Gln Leu Phe Asp Gln

20                  25                  30

Val Val Lys Thr Ile Gly Leu Arg Glu Val Trp Tyr Phe Gly Leu His
        35                  40                  45

Tyr Val Asp Asn Lys Gly Phe Pro Thr Trp Leu Lys Leu Asp Lys Lys
    50                  55                  60

Val Ser Ala Gln Glu Val Arg Lys Glu Asn Pro Leu Gln Phe Lys Phe
65                  70                  75                  80

Arg Ala Lys Phe Tyr Pro Glu Asp Val Ala Glu Glu Leu Ile Gln Asp
                85                  90                  95

Ile Thr Gln Lys Leu Phe Phe Leu Gln Val Lys Glu Gly Ile Leu Ser
            100                 105                 110

Asp Glu Ile Tyr Cys Pro Pro Glu Thr Ala Val Leu Leu Gly Ser Tyr
        115                 120                 125

Ala Val Gln Ala Lys Phe Gly Asp Tyr Asn Lys Glu Val His Lys Ser
    130                 135                 140

Gly Tyr Leu Ser Ser Glu Arg Leu Ile Pro Gln Arg Val Met Asp Gln
145                 150                 155                 160

His Lys Leu Thr Arg Asp Gln Trp Glu Asp Arg Ile Gln Val Trp His
                165                 170                 175

Ala Glu His Arg Gly Met Leu Lys Asp Asn Ala Met Leu Glu Tyr Leu
            180                 185                 190

Lys Ile Ala Gln Asp Leu Glu Met Tyr Gly Ile Asn Tyr Phe Glu Ile
        195                 200                 205

Lys Asn Lys Lys Gly Thr Asp Leu Trp Leu Gly Val Asp Ala Leu Gly
    210                 215                 220

Leu Asn Ile Tyr Glu Lys Asp Asp Lys Leu Thr Pro Lys Ile Gly Phe
225                 230                 235                 240

Pro Trp Ser Glu Ile Arg Asn Ile Ser Phe Asn Asp Lys Lys Phe Val
                245                 250                 255

Ile Lys Pro Ile Asp Lys Lys Ala Pro Asp Phe Val Phe Tyr Ala Pro
            260                 265                 270

Arg Leu Arg Ile Asn Lys Arg Ile Leu Gln Leu Cys Met Gly Asn His
        275                 280                 285

Glu Leu Tyr Met Arg Arg Arg Lys Pro Asp Thr Ile Glu Val Gln Gln
    290                 295                 300

Met Lys Ala Gln Ala Arg Glu Glu Lys His Gln Lys Gln Leu Glu Arg
305                 310                 315                 320

Gln Gln Leu Glu Thr Glu Lys Lys Arg Arg Glu Thr Val Glu Arg Glu
                325                 330                 335

Lys Glu Gln Met Met Arg Glu Lys Glu Glu Leu Met Leu Arg Leu Gln
            340                 345                 350

Asp Tyr Glu Glu Lys Thr Lys Lys Ala Glu Arg Glu Leu Ser Glu Gln
        355                 360                 365

Ile Gln Arg Ala Leu Gln Leu Glu Glu Glu Arg Lys Arg Ala Gln Glu
    370                 375                 380

Glu Ala Glu Arg Leu Glu Ala Asp Arg Met Ala Ala Leu Arg Ala Lys
385                 390                 395                 400

Glu Glu Leu Glu Arg Gln Ala Val Asp Gln Ile Lys Ser Gln Glu Gln
                405                 410                 415

Leu Ala Ala Glu Leu Ala Glu Tyr Thr Ala Lys Ile Ala Leu Leu Glu
            420                 425                 430

Glu Ala Arg Arg Arg Lys Glu Asp Glu Val Glu Glu Trp Gln His Arg
        435                 440                 445

Ala Lys Glu Ala Gln Asp Asp Leu Val Lys Thr Lys Glu Glu Leu His
    450                 455                 460

Leu Val Met Thr Ala Pro Pro Pro Pro Pro Pro Pro Val Tyr Glu Pro
465                 470                 475                 480

Val Ser Tyr His Val Gln Glu Ser Leu Gln Asp Glu Gly Ala Glu Pro
                485                 490                 495

Thr Gly Tyr Ser Ala Glu Leu Ser Ser Glu Gly Ile Arg Asp Asp Arg
            500                 505                 510

Asn Glu Glu Lys Arg Ile Thr Glu Ala Glu Lys Asn Glu Arg Val Gln
        515                 520                 525

Arg Gln Leu Leu Thr Leu Ser Ser Glu Leu Ser Gln Ala Arg Asp Glu
    530                 535                 540

Asn Lys Arg Thr His Asn Asp Ile Ile His Asn Glu Asn Met Arg Gln
545                 550                 555                 560

Gly Arg Asp Lys Tyr Lys Thr Leu Arg Gln Ile Arg Gln Gly Asn Thr
                565                 570                 575

Lys Gln Arg Ile Asp Glu Phe Glu Ala Leu
            580                 585

<210>  21
<211>  11893
<212>  DNA
<213>  Homo sapiens

<220>
<221>  CDS
<222>  (99)..(3977)

<400>  21
cacgcgcgcc cggctggggg atctcctccg cgtgcccgaa agggggatat gccatttgga      60

catgtaattg tcagcacggg atctgagact tccaaaaa atg aag ccg gcg aca gga     116
                                          Met Lys Pro Ala Thr Gly
                                          1               5

ctt tgg gtc tgg gtg agc ctt ctc gtg gcg gcg ggg acc gtc cag ccc     164
Leu Trp Val Trp Val Ser Leu Leu Val Ala Ala Gly Thr Val Gln Pro
            10                  15                  20

agc gat tct cag tca gtg tgt gca gga acg gag aat aaa ctg agc tct     212
Ser Asp Ser Gln Ser Val Cys Ala Gly Thr Glu Asn Lys Leu Ser Ser
        25                  30                  35

ctc tct gac ctg gaa cag cag tac cga gcc ttg cgc aag tac tat gaa     260
Leu Ser Asp Leu Glu Gln Gln Tyr Arg Ala Leu Arg Lys Tyr Tyr Glu
    40                  45                  50

aac tgt gag gtt gtc atg ggc aac ctg gag ata acc agc att gag cac     308
Asn Cys Glu Val Val Met Gly Asn Leu Glu Ile Thr Ser Ile Glu His
55                  60                  65                  70

aac cgg gac ctc tcc ttc ctg cgg tct gtt cga gaa gtc aca ggc tac     356
Asn Arg Asp Leu Ser Phe Leu Arg Ser Val Arg Glu Val Thr Gly Tyr
                75                  80                  85

gtg tta gtg gct ctt aat cag ttt cgt tac ctg cct ctg gag aat tta     404
Val Leu Val Ala Leu Asn Gln Phe Arg Tyr Leu Pro Leu Glu Asn Leu
            90                  95                  100

cgc att att cgt ggg aca aaa ctt tat gag gat cga tat gcc ttg gca     452
Arg Ile Ile Arg Gly Thr Lys Leu Tyr Glu Asp Arg Tyr Ala Leu Ala
        105                 110                 115

ata ttt tta aac tac aga aaa gat gga aac ttt gga ctt caa gaa ctt     500

Ile Phe Leu Asn Tyr Arg Lys Asp Gly Asn Phe Gly Leu Gln Glu Leu
    120                 125                 130

gga tta aag aac ttg aca gaa atc cta aat ggt gga gtc tat gta gac      548
Gly Leu Lys Asn Leu Thr Glu Ile Leu Asn Gly Gly Val Tyr Val Asp
135                 140                 145                 150

cag aac aaa ttc ctt tgt tat gca gac acc att cat tgg caa gat att      596
Gln Asn Lys Phe Leu Cys Tyr Ala Asp Thr Ile His Trp Gln Asp Ile
                155                 160                 165

gtt cgg aac cca tgg cct tcc aac ttg act ctt gtg tca aca aat ggt      644
Val Arg Asn Pro Trp Pro Ser Asn Leu Thr Leu Val Ser Thr Asn Gly
            170                 175                 180

agt tca gga tgt gga cgt tgc cat aag tcc tgt act ggc cgt tgc tgg      692
Ser Ser Gly Cys Gly Arg Cys His Lys Ser Cys Thr Gly Arg Cys Trp
        185                 190                 195

gga ccc aca gaa aat cat tgc cag act ttg aca agg acg gtg tgt gca      740
Gly Pro Thr Glu Asn His Cys Gln Thr Leu Thr Arg Thr Val Cys Ala
    200                 205                 210

gaa caa tgt gac ggc aga tgc tac gga cct tac gtc agt gac tgc tgc      788
Glu Gln Cys Asp Gly Arg Cys Tyr Gly Pro Tyr Val Ser Asp Cys Cys
215                 220                 225                 230

cat cga gaa tgt gct gga ggc tgc tca gga cct aag gac aca gac tgc      836
His Arg Glu Cys Ala Gly Gly Cys Ser Gly Pro Lys Asp Thr Asp Cys
                235                 240                 245

ttt gcc tgc atg aat ttc aat gac agt gga gca tgt gtt act cag tgt      884
Phe Ala Cys Met Asn Phe Asn Asp Ser Gly Ala Cys Val Thr Gln Cys
            250                 255                 260

ccc caa acc ttt gtc tac aat cca acc acc ttt caa ctg gag cac aat      932
Pro Gln Thr Phe Val Tyr Asn Pro Thr Thr Phe Gln Leu Glu His Asn
        265                 270                 275

ttc aat gca aag tac aca tat gga gca ttc tgt gtc aag aaa tgt cca      980
Phe Asn Ala Lys Tyr Thr Tyr Gly Ala Phe Cys Val Lys Lys Cys Pro
    280                 285                 290

cat aac ttt gtg gta gat tcc agt tct tgt gtg cgt gcc tgc cct agt     1028
His Asn Phe Val Val Asp Ser Ser Ser Cys Val Arg Ala Cys Pro Ser
295                 300                 305                 310

tcc aag atg gaa gta gaa gaa aat ggg att aaa atg tgt aaa cct tgc     1076
Ser Lys Met Glu Val Glu Glu Asn Gly Ile Lys Met Cys Lys Pro Cys
                315                 320                 325

act gac att tgc cca aaa gct tgt gat ggc att ggc aca gga tca ttg     1124
Thr Asp Ile Cys Pro Lys Ala Cys Asp Gly Ile Gly Thr Gly Ser Leu
            330                 335                 340

atg tca gct cag act gtg gat tcc agt aac att gac aaa ttc ata aac     1172
Met Ser Ala Gln Thr Val Asp Ser Ser Asn Ile Asp Lys Phe Ile Asn
        345                 350                 355

tgt acc aag atc aat ggg aat ttg atc ttt cta gtc act ggt att cat     1220
Cys Thr Lys Ile Asn Gly Asn Leu Ile Phe Leu Val Thr Gly Ile His
    360                 365                 370

ggg gac cct tac aat gca att gaa gcc ata gac cca gag aaa ctg aac 1268
Gly Asp Pro Tyr Asn Ala Ile Glu Ala Ile Asp Pro Glu Lys Leu Asn
375                 380                 385                 390

gtc ttt cgg aca gtc aga gag ata aca ggt ttc ctg aac ata cag tca 1316
Val Phe Arg Thr Val Arg Glu Ile Thr Gly Phe Leu Asn Ile Gln Ser
                395                 400                 405

tgg cca cca aac atg act gac ttc agt gtt ttt tct aac ctg gtg acc 1364
Trp Pro Pro Asn Met Thr Asp Phe Ser Val Phe Ser Asn Leu Val Thr
            410                 415                 420

att ggt gga aga gta ctc tat agt ggc ctg tcc ttg ctt atc ctc aag 1412
Ile Gly Gly Arg Val Leu Tyr Ser Gly Leu Ser Leu Leu Ile Leu Lys
        425                 430                 435

caa cag ggc atc acc tct cta cag ttc cag tcc ctg aag gaa atc agc 1460
Gln Gln Gly Ile Thr Ser Leu Gln Phe Gln Ser Leu Lys Glu Ile Ser
    440                 445                 450

gca gga aac atc tat att act gac aac agc aac ctg tgt tat tat cat 1508
Ala Gly Asn Ile Tyr Ile Thr Asp Asn Ser Asn Leu Cys Tyr Tyr His
455                 460                 465                 470

acc att aac tgg aca aca ctc ttc agc aca atc aac cag aga ata gta 1556
Thr Ile Asn Trp Thr Thr Leu Phe Ser Thr Ile Asn Gln Arg Ile Val
                475                 480                 485

atc cgg gac aac aga aaa gct gaa aat tgt act gct gaa gga atg gtg 1604
Ile Arg Asp Asn Arg Lys Ala Glu Asn Cys Thr Ala Glu Gly Met Val
            490                 495                 500

tgc aac cat ctg tgt tcc agt gat ggc tgt tgg gga cct ggg cca gac 1652
Cys Asn His Leu Cys Ser Ser Asp Gly Cys Trp Gly Pro Gly Pro Asp
        505                 510                 515

caa tgt ctg tcg tgt cgc cgc ttc agt aga gga agg atc tgc ata gag 1700
Gln Cys Leu Ser Cys Arg Arg Phe Ser Arg Gly Arg Ile Cys Ile Glu
    520                 525                 530

tct tgt aac ctc tat gat ggt gaa ttt cgg gag ttt gag aat ggc tcc 1748
Ser Cys Asn Leu Tyr Asp Gly Glu Phe Arg Glu Phe Glu Asn Gly Ser
535                 540                 545                 550

atc tgt gtg gag tgt gac ccc cag tgt gag aag atg gaa gat ggc ctc 1796
Ile Cys Val Glu Cys Asp Pro Gln Cys Glu Lys Met Glu Asp Gly Leu
                555                 560                 565

ctc aca tgc cat gga ccg ggt cct gac aac tgt aca aag tgc tct cat 1844
Leu Thr Cys His Gly Pro Gly Pro Asp Asn Cys Thr Lys Cys Ser His
            570                 575                 580

ttt aaa gat ggc cca aac tgt gtg gaa aaa tgt cca gat ggc tta cag 1892
Phe Lys Asp Gly Pro Asn Cys Val Glu Lys Cys Pro Asp Gly Leu Gln
        585                 590                 595

ggg gca aac agt ttc att ttc aag tat gct gat cca gat cgg gag tgc 1940
Gly Ala Asn Ser Phe Ile Phe Lys Tyr Ala Asp Pro Asp Arg Glu Cys
    600                 605                 610

cac cca tgc cat cca aac tgc acc caa ggg tgt aac ggt ccc act agt 1988
His Pro Cys His Pro Asn Cys Thr Gln Gly Cys Asn Gly Pro Thr Ser
615                 620                 625                 630

cat gac tgc att tac tac cca tgg acg ggc cat tcc act tta cca caa 2036
His Asp Cys Ile Tyr Tyr Pro Trp Thr Gly His Ser Thr Leu Pro Gln
                635                 640                 645

cat gct aga act ccc ctg att gca gct gga gta att ggt ggg ctc ttc 2084
His Ala Arg Thr Pro Leu Ile Ala Ala Gly Val Ile Gly Gly Leu Phe
            650                 655                 660

att ctg gtc att gtg ggt ctg aca ttt gct gtt tat gtt aga agg aag 2132
Ile Leu Val Ile Val Gly Leu Thr Phe Ala Val Tyr Val Arg Arg Lys
        665                 670                 675

agc atc aaa aag aaa aga gcc ttg aga aga ttc ttg gaa aca gag ttg 2180
Ser Ile Lys Lys Lys Arg Ala Leu Arg Arg Phe Leu Glu Thr Glu Leu
    680                 685                 690

gtg gaa cca tta act ccc agt ggc aca gca ccc aat caa gct caa ctt 2228
Val Glu Pro Leu Thr Pro Ser Gly Thr Ala Pro Asn Gln Ala Gln Leu
695                 700                 705                 710

cgt att ttg aaa gaa act gag ctg aag agg gta aaa gtc ctt ggc tca 2276
Arg Ile Leu Lys Glu Thr Glu Leu Lys Arg Val Lys Val Leu Gly Ser
                715                 720                 725

ggt gct ttt gga acg gtt tat aaa ggt att tgg gta cct gaa gga gaa 2324
Gly Ala Phe Gly Thr Val Tyr Lys Gly Ile Trp Val Pro Glu Gly Glu
            730                 735                 740

act gtg aag att cct gtg gct att aag att ctt aat gag aca act ggt 2372
Thr Val Lys Ile Pro Val Ala Ile Lys Ile Leu Asn Glu Thr Thr Gly
        745                 750                 755

ccc aag gca aat gtg gag ttc atg gat gaa gct ctg atc atg gca agt 2420
Pro Lys Ala Asn Val Glu Phe Met Asp Glu Ala Leu Ile Met Ala Ser
    760                 765                 770

atg gat cat cca cac cta gtc cgg ttg ctg ggt gtg tgt ctg agc cca 2468
Met Asp His Pro His Leu Val Arg Leu Leu Gly Val Cys Leu Ser Pro
775                 780                 785                 790

acc atc cag ctg gtt act caa ctt atg ccc cat ggc tgc ctg ttg gag 2516
Thr Ile Gln Leu Val Thr Gln Leu Met Pro His Gly Cys Leu Leu Glu
                795                 800                 805

tat gtc cac gag cac aag gat aac att gga tca caa ctg ctg ctt aac 2564
Tyr Val His Glu His Lys Asp Asn Ile Gly Ser Gln Leu Leu Leu Asn
            810                 815                 820

tgg tgt gtc cag ata gct aag gga atg atg tac ctg gaa gaa aga cga 2612
Trp Cys Val Gln Ile Ala Lys Gly Met Met Tyr Leu Glu Glu Arg Arg
        825                 830                 835

ctc gtt cat cgg gat ttg gca gcc cgt aat gtc tta gtg aaa tct cca 2660
Leu Val His Arg Asp Leu Ala Ala Arg Asn Val Leu Val Lys Ser Pro
    840                 845                 850

aac cat gtg aaa atc aca gat ttt ggg cta gcc aga ctc ttg gaa gga 2708
Asn His Val Lys Ile Thr Asp Phe Gly Leu Ala Arg Leu Leu Glu Gly
855                 860                 865                 870

gat gaa aaa gag tac aat gct gat gga gga aag atg cca att aaa tgg 2756
Asp Glu Lys Glu Tyr Asn Ala Asp Gly Gly Lys Met Pro Ile Lys Trp

                875                 880                 885

atg gct ctg gag tgt ata cat tac agg aaa ttc acc cat cag agt gac 2804
Met Ala Leu Glu Cys Ile His Tyr Arg Lys Phe Thr His Gln Ser Asp
            890                 895                 900

gtt tgg agc tat gga gtt act ata tgg gaa ctg atg acc ttt gga gga 2852
Val Trp Ser Tyr Gly Val Thr Ile Trp Glu Leu Met Thr Phe Gly Gly
        905                 910                 915

aaa ccc tat gat gga att cca acg cga gaa atc cct gat tta tta gag 2900
Lys Pro Tyr Asp Gly Ile Pro Thr Arg Glu Ile Pro Asp Leu Leu Glu
    920                 925                 930

aaa gga gaa cgt ttg cct cag cct ccc atc tgc act att gac gtt tac 2948
Lys Gly Glu Arg Leu Pro Gln Pro Pro Ile Cys Thr Ile Asp Val Tyr
935                 940                 945                 950

atg gtc atg gtc aaa tgt tgg atg att gat gct gac agt aga cct aaa 2996
Met Val Met Val Lys Cys Trp Met Ile Asp Ala Asp Ser Arg Pro Lys
                955                 960                 965

ttt aag gaa ctg gct gct gag ttt tca agg atg gct cga gac cct caa 3044
Phe Lys Glu Leu Ala Ala Glu Phe Ser Arg Met Ala Arg Asp Pro Gln
            970                 975                 980

aga tac cta gtt att cag ggt gat gat cgt atg aag ctt ccc agt cca 3092
Arg Tyr Leu Val Ile Gln Gly Asp Asp Arg Met Lys Leu Pro Ser Pro
        985                 990                 995

aat gac agc aag ttc ttt cag aat ctc ttg gat gaa gag gat ttg 3137
Asn Asp Ser Lys Phe Phe Gln Asn Leu Leu Asp Glu Glu Asp Leu
    1000                1005                1010

gaa gat atg atg gat gct gag gag tac ttg gtc cct cag gct ttc 3182
Glu Asp Met Met Asp Ala Glu Glu Tyr Leu Val Pro Gln Ala Phe
    1015                1020                1025

aac atc cca cct ccc atc tat act tcc aga gca aga att gac tcg 3227
Asn Ile Pro Pro Pro Ile Tyr Thr Ser Arg Ala Arg Ile Asp Ser
    1030                1035                1040

aat agg aac cag ttt gta tac cga gat gga ggt ttt gct gct gaa 3272
Asn Arg Asn Gln Phe Val Tyr Arg Asp Gly Gly Phe Ala Ala Glu
    1045                1050                1055

caa gga gtg tct gtg ccc tac aga gcc cca act agc aca att cca 3317
Gln Gly Val Ser Val Pro Tyr Arg Ala Pro Thr Ser Thr Ile Pro
    1060                1065                1070

gaa gct cct gtg gca cag ggt gct act gct gag att ttt gat gac 3362
Glu Ala Pro Val Ala Gln Gly Ala Thr Ala Glu Ile Phe Asp Asp
    1075                1080                1085

tcc tgc tgt aat ggc acc cta cgc aag cca gtg gca ccc cat gtc 3407
Ser Cys Cys Asn Gly Thr Leu Arg Lys Pro Val Ala Pro His Val
    1090                1095                1100

caa gag gac agt agc acc cag agg tac agt gct gac ccc acc gtg 3452
Gln Glu Asp Ser Ser Thr Gln Arg Tyr Ser Ala Asp Pro Thr Val
    1105                1110                1115

ttt gcc cca gaa cgg agc cca cga gga gag ctg gat gag gaa ggt 3497

Phe Ala Pro Glu Arg Ser Pro Arg Gly Glu Leu Asp Glu Glu Gly
    1120                1125                1130

tac atg act cct atg cga gac aaa ccc aaa caa gaa tac ctg aat 3542
Tyr Met Thr Pro Met Arg Asp Lys Pro Lys Gln Glu Tyr Leu Asn
    1135                1140                1145

cca gtg gag gag aac cct ttt gtt tct cgg aga aaa aat gga gac 3587
Pro Val Glu Glu Asn Pro Phe Val Ser Arg Arg Lys Asn Gly Asp
    1150                1155                1160

ctt caa gca ttg gat aat ccc gaa tat cac aat gca tcc aat ggt 3632
Leu Gln Ala Leu Asp Asn Pro Glu Tyr His Asn Ala Ser Asn Gly
    1165                1170                1175

cca ccc aag gcc gag gat gag tat gtg aat gag cca ctg tac ctc 3677
Pro Pro Lys Ala Glu Asp Glu Tyr Val Asn Glu Pro Leu Tyr Leu
    1180                1185                1190

aac acc ttt gcc aac acc ttg gga aaa gct gag tac ctg aag aac 3722
Asn Thr Phe Ala Asn Thr Leu Gly Lys Ala Glu Tyr Leu Lys Asn
    1195                1200                1205

aac ata ctg tca atg cca gag aag gcc aag aaa gcg ttt gac aac 3767
Asn Ile Leu Ser Met Pro Glu Lys Ala Lys Lys Ala Phe Asp Asn
    1210                1215                1220

cct gac tac tgg aac cac agc ctg cca cct cgg agc acc ctt cag 3812
Pro Asp Tyr Trp Asn His Ser Leu Pro Pro Arg Ser Thr Leu Gln
    1225                1230                1235

cac cca gac tac ctg cag gag tac agc aca aaa tat ttt tat aaa 3857
His Pro Asp Tyr Leu Gln Glu Tyr Ser Thr Lys Tyr Phe Tyr Lys
    1240                1245                1250

cag aat ggg cgg atc cgg cct att gtg gca gag aat cct gaa tac 3902
Gln Asn Gly Arg Ile Arg Pro Ile Val Ala Glu Asn Pro Glu Tyr
    1255                1260                1265

ctc tct gag ttc tcc ctg aag cca ggc act gtg ctg ccg cct cca 3947
Leu Ser Glu Phe Ser Leu Lys Pro Gly Thr Val Leu Pro Pro Pro
    1270                1275                1280

cct tac aga cac cgg aat act gtg gtg taa gctcagttgt ggttttttag 3997
Pro Tyr Arg His Arg Asn Thr Val Val
    1285                1290

gtggagagac acacctgctc caatttcccc acccccctct ctttctctgg tggtcttcct 4057

tctaccccaa ggccagtagt tttgacactt cccagtggaa gatacagaga tgcaatgata 4117

gttatgtgct tacctaactt gaacattaga gggaaagact gaaagagaaa gataggagga 4177

accacaatgt ttcttcattt ctctgcatgg gttggtcagg agaatgaaac agctagagaa 4237

ggaccagaaa atgtaaggca atgctgccta ctatcaaact agctgtcact ttttttcttt 4297

ttctttttct ttctttgttt ctttcttcct cttctttttt tttttttttt ttaaagcaga 4357

tggttgaaac acccatgcta tctgttccta tctgcaggaa ctgatgtgtg catatttagc 4417

atccctggaa atcataataa agtttccatt agaacaaaag aataacattt tctataacat 4477

atgatggtgt ctgaaattga gaatccagtt tctttcccca gcagtttctg tcctagcaag 4537

taagaatggc caactcaact ttcataattt aaaaatctcc attaaagtta taactagtaa 4597

ttatgttttc aacacttttt ggtttttttc attttgtttt gctctgaccg attcctttat 4657

atttgctccc ctatttttgg ctttaatttc taattgcaaa gatgtttaca tcaaagcttc 4717

ttcacagaat ttaagcaaga aatattttaa tatagtgaaa tggccactac tttaagtata 4777

caatctttaa aataagaaag ggaggctaat atttttcatg ctatcaaatt atcttcaccc 4837

tcatccttta catttttcaa catttttttt tctccataaa tgacactact tgataggccg 4897

ttggttgtct gaagagtaga agggaaacta agagacagtt ctctgtggtt caggaaaact 4957

actgatactt tcaggggtgg cccaatgagg gaatccattg aactggaaga aacacactgg 5017

attgggtatg tctacctggc agatactcag aaatgtagtt tgcacttaag ctgtaatttt 5077

atttgttctt tttctgaact ccattttgga ttttgaatca agcaatatgg aagcaaccag 5137

caaattaact aatttaagta catttttaaa aaaagagcta agataaagac tgtggaaatg 5197

ccaaaccaag caaattagga accttgcaac ggtatccagg gactatgatg agaggccagc 5257

acattatctt catatgtcac ctttgctacg caaggaaatt tgttcagttc gtatacttcg 5317

taagaaggaa tgcgagtaag gattggcttg aattccatgg aatttctagt atgagactat 5377

ttatatgaag tagaaggtaa ctctttgcac ataaattggt ataataaaaa gaaaaacaca 5437

aacattcaaa gcttagggat aggtccttgg gtcaaaagtt gtaaataaat gtgaaacatc 5497

ttctcatgca attattttat tatccaacac actaatcttt tgatacttta tataattccc 5557

tttcttcata tactgcatcc agtactagaa ccatcattat tatgtatcat tttgaaagaa 5617

tacctgatga gatgaaggat gagaacaaat gacagagatg agtctccaag taaagggggc 5677

ctcacatcaa taattaggaa acttagatat aagtcgccct tttctgaaaa ttctacccca 5737

agtcatttag atttttaaaa aatatttcta atgttaaaat attgggacca aattagaatc 5797

aatagtataa gattaattaa ttagagtaaa aatatctatt aaggcagaga aagtttagag 5857

aaaaaaatcc aaagaaattt gtgtttcttc ctattctgaa caagtaaatc catccatcca 5917

tccatccaaa cctcctttat ctaactgtgt ctactaaaag caccatgttt tgtggggaac 5977

actcagataa atggaatatc atcctcaact tcaaaattct atgatctagg agatttaatt 6037

aaaatgacat tttaattttt ctatgcgttc caacaatcag attgcatagt ctcttttgtg 6097

aatagctgtc atataatcag ttgtactgta agatatctcc tttaaactca tttgggatat 6157

aagttaaaca tccttcaaat tgttgatgtt gacaaacagg ataatttcaa taatattatt 6217

caaacataaa ctggtctagg agaatattgc atcactgact aattagccta tctagagtct 6277

aacttcacca ttaaaccaaa agcagatggt ggtccttggc caagaatatt ggagacattg 6337

gagttggttt ttttctaagc tataagaagt gaggcgagct gaaaaagtat ggtagagcag 6397

gagaagggtt tgtgagattc cttctagtga agttcaccct caaacttttc aggggtaaag 6457

acacagagtg attcaggggc cacaatctaa tagctcaggg ctctcctatc cattcagaga 6517

agtctctagg aaaagggatc tcatatcagt acttatgaaa aattgaatat aagcctccct 6577

ttctaaataa atctgcatcg agtcatcaca gccctctttt tggatactat accttgattt 6637

tttttttctg atttacaata tgcatatggt ttctactggg ctatagaaag cagaatcact 6697

cattttggag aaggaaaaaa tgaatagtta aaacaaactt ttaactgtta aggtaacaga 6757

aatgtattta gtgaatgtct ctttcctcct aagaacacaa gacttctaca tgttgggtaa 6817

tacctagaga tgcatgtagg aataatccaa aatgacccaa atgctttata atagcaccac 6877

tttataattc ttttgaatga tttctgtagt atataattga cttcagttgt ttgagtgttt 6937

tttgttttat ttttgtcccc cctgggaaaa catatttcag catgtataag agggagaaaa 6997

aaagtttcat tccttccaga gaataactta tttagtccag tagggtagaa ttttaaaatg 7057

tcagttaaag tcttcaaagt gcttgggggg atatcagatt ccagaggcca attgtagcaa 7117

ttgaaatttg cagaatcaat tatgtaaatc tgagacaaat tagtattaaa attacacgga 7177

gtatattttt taaatcaccc aactttgtag attataccta ttttgggcag gtatggaaaa 7237

attttgcagt taaatgattg cctaaagaaa gtggtaaaca ggtgaggaaa gatggcctct 7297

gatctaggat agatccagaa ccacaaagca tctgcaccac aaaaggtgtt agactaccaa 7357

gcagctcctg gttttctgca tagtattagt agcacagctt aggatgagaa tcctttctcc 7417

agtaacattc ttaaaatagc atgaaaaaca acgcaaaact caaatttcta ttaaaacaca 7477

caaactaaaa tcaagtgatt cttttttgta gattagggag aaggactgaa tatctaattt 7537

aagagaagga atagtgttta agtgttatag tgtgtgagct aataccttct aaaggaaaga 7597

catggcatga agattgtgca tacttacaat gctaaggaaa aatcaagaaa aggactgtgt 7657

gaggctctgc tactagatga agttggaagg actattaatg tgcttcttga agtatcaaaa 7717

atgaaaagaa aattaaaatt gtttaagcct gacagggaag gatgtaaata caagtttttc 7777

tagagctctc taacctttat ttcaaaactg gaattattca tccatctgta attgttgata 7837

atttaactag tatatgtagt tcataaggta atagaaaagg tgatcatgaa agcatgtata 7897

taactggaca gaaccacgat aatgctataa gatgtagatt tagttaggtt atcagatgtt 7957

aaatgatttt aatattatta aataaatcaa actagaaaac taaccacaag tataatgtaa 8017

caaagttaaa tgcaggatat aaaaatgtag gatggatttt gcatagtaaa aagataagtt 8077

tgccatttaa aattgttgtt tgttgggttt agctgaaagt aggcatatat ggttccactt 8137

gggaaaactt gctttaaagc attacaatga acaatttttt ctcattctct tattccttta 8197

tcacttttta aatgtaaaga aaattgtatt tatttatttt tttaaataaa caccaccttg 8257

cagaatttaa taggcaaaca tgttacatat gactaagtaa gggtcttcaa gatgaagtaa 8317

agaaaatgta aatgttctat taccttatgc agagacaaaa aaaaaaagga gtggtgtcat 8377

ttagctagca aacaaacaaa atacagttaa ttggtgatat gtcctttctt ttctcactat 8437

gccctcttgc ctccaaaaat gacaacaaag aatcacaatt tttctgataa ataaatgcta 8497

aaccaagcgt ttcaaactat tgcattgcca ttcttttgga ctttagttat tagaatgatg 8557

attgttatag ggcaaatgag aaatccatgt gcatcagctt ctagttgtta aaaaaaccag 8617

ataaattaac ttctactgta tactgtgggc agaggatcct agagctgatc ctacaacatc 8677

agcttctagt tgttaaaaaa aaaaaaagaa acagataaat taacttctac tgtatatact 8737

gtgggcagag gatcttactg tgcctctgtt tgtgtacatg gacttcggtg tgtatcagtt 8797

tgaaggacag ccttgcccca tgtaaacata taaatgcaga ttggtatcgc ctggttgcta 8857

tttgcttaag aacaaatatt atacagatga gatcaggcat aattttaaaa gatcattatc 8917

agtggagacc tcattattac tgatattaca atggggccag tttttatact tctgggtaga 8977

attaataaaa tttttctgat cccagagatc tgagttctct ctgcagttgg aaacaagaag 9037

ctgttgtggg cattgtgtcg ggccaggggc ccttgtgttt gtgtgggcaa atatctttta 9097

gcagtgtgag ctgctttttt cttttcatta aaagtctctc taaaataata gaaatttcag 9157

atactcggtt caagtctcac tgattttgta gaggtccaaa aatgtaggat ctgtcacttt 9217

tgcaggcccc tgcctcacct aattcctggc caggtgacat tttgggcaga agtaaatgct 9277

tctatagtca caagctaaaa tgactctaag ccccaatttc acggggggta ttcacatgct 9337

tcctctggaa aatactcttt gacagtcagc tttgcaagta agtgattacc ttgttaggaa 9397

tcaaagaaaa atgtatttct ctctgacctt tagaggaaaa tagaatcctt cccttttttg 9457

cccattgaca caactggcac tgctctcttc cctttctacc accctggttc aaagtagtcc 9517

cccgatgctg tcctgttcct ttcttaagcc atagtggatc tctgagatcc tacaccccac 9577

tttgtgaaac actgacttca tctttgccct cgaatgcctg attttttcat aagagattct 9637

agcaatttgg acactgttta agtgaactat caaactaccg catagagaat atttaagcta 9697

ttaaaattat ggtttcccat gaagatcaat tctctgtgtc cttccctata ggaatttgag 9757

acgagttagc cctgtgatga atcttgaaac tcacatatgt ccacatacac ttggtagaac 9817

ttcgatttaa tctttacata aaagctgtac atataaccaa gaagttattt ttgccagtaa 9877

attaacttat ttgctttatt catcttattt ggttcctaat cgtaaatatt ttgtagctgc 9937

tgtaaatttt tttctcccaa atgaggagtc ttattatcat aaaggtaaag gctattcagc 9997

tttgataacc acctgcaatt cttttttgga tcattcatcc atctaacaaa tacataatga 10057

ggacagttca tgttaatgaa aatccatgtt gtttaataga atgccatcct ttacctactt 10117

ttgctcttta tggacgtttt tcttttcatg ctctagtgag ctttccctat atcatgagaa 10177

gtggttatat ttgtgcaaat atacaaatat aggaaaacaa agattcatac ctgtaggcaa 10237

tagtctaact tgtccaaacc actttgcctt tactgctatt tttatcccca atgcgtagat 10297

atttccccca ggcctatagc ctttgtgaag gaaagcaaat catacctcct gtatattgac 10357

acgaatctgg ttttcaaatg tcatttccag attttttagt taattggggg ttgtcctttt 10417

cccttaatgt gagagtcatt ttcctgtata tttctggatc tctcaggggc tgggaggggg 10477

gagtgagggg actacaacca tagcactcca agaacccttt tgggattact ccagtaatca 10537

actacgaaag ttattttcta aatgtagata tgtaaggtgt tcttttaaag taaggtactt 10597

tgaaatatgt agcataaact ggtactgctg ttaaatgggt cgattattaa acggagcagc 10657

tgtgtgaggg cagctaactt tgaatgcctg tctccctggc tggtgtgtct ccttctcatg 10717

ttgagagcac cagggattgc gtggctgcat gctgaaaccg cattttccca tggtgtatga 10777

ctagttcatc tctttcttga gcaccattac aagaagatca aatgaaaatg agatcaatgt 10837

ggaagacaat tcatagcaca aaaaaagtca tcttaaatct actctcaaac attcatctta 10897

tacatgcatc aaagtaattt actgacatca gtttgggtga gagagggagt cactttactg 10957

aaaaggcaga ggcttaaggt gtatacattt gtactcactt ccttattttc ttaacttgta 11017

agcagaaaac aagccctctc tcttgtgaag tatcttcaaa ggattggggt gcaaaaatac 11077

cttgctggta agccatcaat gttttattta aatccctgca ttcaaagtta gctgcctttt 11137

tgaaataaac aaacaaaaaa tactactgta tgtttgaaaa tgtgaatagt atttttatag 11197

cttgttaaag acatggctag ttgcatttgt aaataagtat aatgttgctt tgattttctt 11257

ttgtggacat ctttatttgg aacataattg tctttagggt tgatttgtat ataagtaatt 11317

ggcctgtgat tgtttctttt ttggttggaa gttatcattt tgacattact tgtgattctg 11377

tgttcagcac tattgtgatg tgttcaacct ctgcactcgc ttacacaata ggatatgcca 11437

attgtgtgtg gtgtaatgtt attttgattt ttttccatgt tattgatgaa ggatcatgca 11497

cctaacacat actaactttt ttaatgttag gcatattttt agtatacttt ctcttattct 11557

ttcttctcct ccaacctttt acccatcctc cttcctttcc ctcattcctg ttgttatttg 11617

agaatgaggg agaaacagta ttttacattt atgtaattag gcttttccgt tagttctcaa 11677

ggatcctctt ttggctcttg ggaaagaatt gtacctgtac aaggcaatta tagaatgcga 11737

actgctttgc ctcattccat actgatcatc ccagctgaac aatttgaaaa ctgttctgcc 11797

tttttgttac atgaatctgt cagaaatata tttttaattt aatataaatg aaattcaata 11857

aaatatgaaa caaacgttaa aaaaaaaaaa aaaaaa 11893

<210> 22
<211> 1292
<212> PRT

<213>  Homo sapiens

<400>  22

Met Lys Pro Ala Thr Gly Leu Trp Val Trp Val Ser Leu Leu Val Ala
1               5                   10                  15

Ala Gly Thr Val Gln Pro Ser Asp Ser Gln Ser Val Cys Ala Gly Thr
            20                  25                  30

Glu Asn Lys Leu Ser Ser Leu Ser Asp Leu Glu Gln Gln Tyr Arg Ala
        35                  40                  45

Leu Arg Lys Tyr Tyr Glu Asn Cys Glu Val Val Met Gly Asn Leu Glu
    50                  55                  60

Ile Thr Ser Ile Glu His Asn Arg Asp Leu Ser Phe Leu Arg Ser Val
65                  70                  75                  80

Arg Glu Val Thr Gly Tyr Val Leu Val Ala Leu Asn Gln Phe Arg Tyr
                85                  90                  95

Leu Pro Leu Glu Asn Leu Arg Ile Ile Arg Gly Thr Lys Leu Tyr Glu
            100                 105                 110

Asp Arg Tyr Ala Leu Ala Ile Phe Leu Asn Tyr Arg Lys Asp Gly Asn
        115                 120                 125

Phe Gly Leu Gln Glu Leu Gly Leu Lys Asn Leu Thr Glu Ile Leu Asn
    130                 135                 140

Gly Gly Val Tyr Val Asp Gln Asn Lys Phe Leu Cys Tyr Ala Asp Thr
145                 150                 155                 160

Ile His Trp Gln Asp Ile Val Arg Asn Pro Trp Pro Ser Asn Leu Thr
                165                 170                 175

Leu Val Ser Thr Asn Gly Ser Ser Gly Cys Gly Arg Cys His Lys Ser
            180                 185                 190

Cys Thr Gly Arg Cys Trp Gly Pro Thr Glu Asn His Cys Gln Thr Leu
        195                 200                 205

Thr Arg Thr Val Cys Ala Glu Gln Cys Asp Gly Arg Cys Tyr Gly Pro
    210                 215                 220

Tyr Val Ser Asp Cys Cys His Arg Glu Cys Ala Gly Gly Cys Ser Gly
225                 230                 235                 240

Pro Lys Asp Thr Asp Cys Phe Ala Cys Met Asn Phe Asn Asp Ser Gly
                245                 250                 255

Ala Cys Val Thr Gln Cys Pro Gln Thr Phe Val Tyr Asn Pro Thr Thr
            260                 265                 270

Phe Gln Leu Glu His Asn Phe Asn Ala Lys Tyr Thr Tyr Gly Ala Phe
        275                 280                 285

Cys Val Lys Lys Cys Pro His Asn Phe Val Val Asp Ser Ser Ser Cys
    290                 295                 300

Val Arg Ala Cys Pro Ser Ser Lys Met Glu Val Glu Glu Asn Gly Ile
305                 310                 315                 320

Lys Met Cys Lys Pro Cys Thr Asp Ile Cys Pro Lys Ala Cys Asp Gly
                325                 330                 335

Ile Gly Thr Gly Ser Leu Met Ser Ala Gln Thr Val Asp Ser Ser Asn
            340                 345                 350

Ile Asp Lys Phe Ile Asn Cys Thr Lys Ile Asn Gly Asn Leu Ile Phe
        355                 360                 365

Leu Val Thr Gly Ile His Gly Asp Pro Tyr Asn Ala Ile Glu Ala Ile
    370                 375                 380

Asp Pro Glu Lys Leu Asn Val Phe Arg Thr Val Arg Glu Ile Thr Gly
385                 390                 395                 400

Phe Leu Asn Ile Gln Ser Trp Pro Pro Asn Met Thr Asp Phe Ser Val
                405                 410                 415

Phe Ser Asn Leu Val Thr Ile Gly Gly Arg Val Leu Tyr Ser Gly Leu
            420                 425                 430

Ser Leu Leu Ile Leu Lys Gln Gln Gly Ile Thr Ser Leu Gln Phe Gln
        435                 440                 445

Ser Leu Lys Glu Ile Ser Ala Gly Asn Ile Tyr Ile Thr Asp Asn Ser
    450                 455                 460

Asn Leu Cys Tyr Tyr His Thr Ile Asn Trp Thr Thr Leu Phe Ser Thr
465                 470                 475                 480

Ile Asn Gln Arg Ile Val Ile Arg Asp Asn Arg Lys Ala Glu Asn Cys
                485                 490                 495

Thr Ala Glu Gly Met Val Cys Asn His Leu Cys Ser Ser Asp Gly Cys
            500                 505                 510

Trp Gly Pro Gly Pro Asp Gln Cys Leu Ser Cys Arg Arg Phe Ser Arg
        515                 520                 525

Gly Arg Ile Cys Ile Glu Ser Cys Asn Leu Tyr Asp Gly Glu Phe Arg
    530                 535                 540

Glu Phe Glu Asn Gly Ser Ile Cys Val Glu Cys Asp Pro Gln Cys Glu
545                 550                 555                 560

Lys Met Glu Asp Gly Leu Leu Thr Cys His Gly Pro Gly Pro Asp Asn
                565                 570                 575

Cys Thr Lys Cys Ser His Phe Lys Asp Gly Pro Asn Cys Val Glu Lys
            580                 585                 590

Cys Pro Asp Gly Leu Gln Gly Ala Asn Ser Phe Ile Phe Lys Tyr Ala
        595                 600                 605

Asp Pro Asp Arg Glu Cys His Pro Cys His Pro Asn Cys Thr Gln Gly
    610                 615                 620

Cys Asn Gly Pro Thr Ser His Asp Cys Ile Tyr Tyr Pro Trp Thr Gly
625                 630                 635                 640

His Ser Thr Leu Pro Gln His Ala Arg Thr Pro Leu Ile Ala Ala Gly
                645                 650                 655

Val Ile Gly Gly Leu Phe Ile Leu Val Ile Val Gly Leu Thr Phe Ala
            660                 665                 670

Val Tyr Val Arg Arg Lys Ser Ile Lys Lys Lys Arg Ala Leu Arg Arg
        675                 680                 685

Phe Leu Glu Thr Glu Leu Val Glu Pro Leu Thr Pro Ser Gly Thr Ala
    690                 695                 700

Pro Asn Gln Ala Gln Leu Arg Ile Leu Lys Glu Thr Glu Leu Lys Arg
705                 710                 715                 720

Val Lys Val Leu Gly Ser Gly Ala Phe Gly Thr Val Tyr Lys Gly Ile
                725                 730                 735

Trp Val Pro Glu Gly Glu Thr Val Lys Ile Pro Val Ala Ile Lys Ile

740                 745                 750

Leu Asn Glu Thr Thr Gly Pro Lys Ala Asn Val Glu Phe Met Asp Glu
        755                 760                 765

Ala Leu Ile Met Ala Ser Met Asp His Pro His Leu Val Arg Leu Leu
    770                 775                 780

Gly Val Cys Leu Ser Pro Thr Ile Gln Leu Val Thr Gln Leu Met Pro
785                 790                 795                 800

His Gly Cys Leu Leu Glu Tyr Val His Glu His Lys Asp Asn Ile Gly
                805                 810                 815

Ser Gln Leu Leu Leu Asn Trp Cys Val Gln Ile Ala Lys Gly Met Met
            820                 825                 830

Tyr Leu Glu Glu Arg Arg Leu Val His Arg Asp Leu Ala Ala Arg Asn
        835                 840                 845

Val Leu Val Lys Ser Pro Asn His Val Lys Ile Thr Asp Phe Gly Leu
    850                 855                 860

Ala Arg Leu Leu Glu Gly Asp Glu Lys Glu Tyr Asn Ala Asp Gly Gly
865                 870                 875                 880

Lys Met Pro Ile Lys Trp Met Ala Leu Glu Cys Ile His Tyr Arg Lys
                885                 890                 895

Phe Thr His Gln Ser Asp Val Trp Ser Tyr Gly Val Thr Ile Trp Glu
            900                 905                 910

Leu Met Thr Phe Gly Gly Lys Pro Tyr Asp Gly Ile Pro Thr Arg Glu
        915                 920                 925

Ile Pro Asp Leu Leu Glu Lys Gly Glu Arg Leu Pro Gln Pro Pro Ile
    930                 935                 940

Cys Thr Ile Asp Val Tyr Met Val Met Val Lys Cys Trp Met Ile Asp
945                 950                 955                 960

Ala Asp Ser Arg Pro Lys Phe Lys Glu Leu Ala Ala Glu Phe Ser Arg
                965                 970                 975

Met Ala Arg Asp Pro Gln Arg Tyr Leu Val Ile Gln Gly Asp Asp Arg
            980                 985                 990

Met Lys Leu Pro Ser Pro Asn Asp Ser Lys Phe Phe Gln Asn Leu Leu
        995                 1000                1005

Asp Glu Glu Asp Leu Glu Asp Met Met Asp Ala Glu Glu Tyr Leu
    1010                1015                1020

Val Pro Gln Ala Phe Asn Ile Pro Pro Pro Ile Tyr Thr Ser Arg
    1025                1030                1035

Ala Arg Ile Asp Ser Asn Arg Asn Gln Phe Val Tyr Arg Asp Gly
    1040                1045                1050

Gly Phe Ala Ala Glu Gln Gly Val Ser Val Pro Tyr Arg Ala Pro
    1055                1060                1065

Thr Ser Thr Ile Pro Glu Ala Pro Val Ala Gln Gly Ala Thr Ala
    1070                1075                1080

Glu Ile Phe Asp Asp Ser Cys Cys Asn Gly Thr Leu Arg Lys Pro
    1085                1090                1095

Val Ala Pro His Val Gln Glu Asp Ser Ser Thr Gln Arg Tyr Ser
    1100                1105                1110

Ala Asp Pro Thr Val Phe Ala Pro Glu Arg Ser Pro Arg Gly Glu
    1115                1120                1125

Leu Asp Glu Glu Gly Tyr Met Thr Pro Met Arg Asp Lys Pro Lys
    1130                1135                1140

Gln Glu Tyr Leu Asn Pro Val Glu Glu Asn Pro Phe Val Ser Arg
    1145                1150                1155

Arg Lys Asn Gly Asp Leu Gln Ala Leu Asp Asn Pro Glu Tyr His
    1160                1165                1170

Asn Ala Ser Asn Gly Pro Pro Lys Ala Glu Asp Glu Tyr Val Asn
    1175                1180                1185

Glu Pro Leu Tyr Leu Asn Thr Phe Ala Asn Thr Leu Gly Lys Ala
    1190                1195                1200

Glu Tyr Leu Lys Asn Asn Ile Leu Ser Met Pro Glu Lys Ala Lys
    1205                1210                1215

Lys Ala Phe Asp Asn Pro Asp Tyr Trp Asn His Ser Leu Pro Pro
    1220                1225                1230

Arg Ser Thr Leu Gln His Pro Asp Tyr Leu Gln Glu Tyr Ser Thr
    1235                1240                1245

Lys Tyr Phe Tyr Lys Gln Asn Gly Arg Ile Arg Pro Ile Val Ala
    1250                1255                1260

Glu Asn Pro Glu Tyr Leu Ser Glu Phe Ser Leu Lys Pro Gly Thr
    1265                1270                1275

Val Leu Pro Pro Pro Pro Tyr Arg His Arg Asn Thr Val Val
    1280                1285                1290

<210> 23
<211> 5459
<212> DNA
<213> Homo sapiens

<220>
<221> CDS
<222> (216)..(3866)

<400> 23
agagccgggc tgctggtgca gcagaggctg aggcatcagg tgcagctgca tccggatctc      60

ctgccttgga gcgtactcct tgtctctaag tcgggaggca ggacgtggtc aggccggggc     120

tgtggaggtg cgctgtgtcc cctgaggcct agaggattcg ggctgcggcc cgtcggaacc     180

agtcagggag gcgcccacac tcctgacagg ataag atg gcg gcg atg gcg cct        233
                                       Met Ala Ala Met Ala Pro
                                       1               5

gga ggt agt ggc agt ggt ggc ggc gtg aat cca ttt ctc agt gat tcg       281
Gly Gly Ser Gly Ser Gly Gly Gly Val Asn Pro Phe Leu Ser Asp Ser
            10                  15                  20

gat gag gac gat gac gag gta gct gca aca gag gaa cgg cgg gca gta       329
Asp Glu Asp Asp Asp Glu Val Ala Ala Thr Glu Glu Arg Arg Ala Val
        25                  30                  35

ctt cgg ctg ggc gcc gga agt ggc cta gat cct ggc tct gcg ggc tcg       377
Leu Arg Leu Gly Ala Gly Ser Gly Leu Asp Pro Gly Ser Ala Gly Ser
    40                  45                  50

ctg tcg cca cag gat ccc gtg gcc tta gga agc agt gcg cgg cca ggg       425
Leu Ser Pro Gln Asp Pro Val Ala Leu Gly Ser Ser Ala Arg Pro Gly
55                  60                  65                  70

ctc cct ggg gag gcg tcg gcg gct gca gtg gcc ctg ggg ggc acc ggg       473
Leu Pro Gly Glu Ala Ser Ala Ala Ala Val Ala Leu Gly Gly Thr Gly
                75                  80                  85

gag acc ccg gcc cga tta tca att gat gcg atc gct gct cag ctg ttg       521
Glu Thr Pro Ala Arg Leu Ser Ile Asp Ala Ile Ala Ala Gln Leu Leu
            90                  95                  100

cgc gat caa tac ttg ctg acc gcc ctg gag ctg cat acc gag ctg tta       569

Arg Asp Gln Tyr Leu Leu Thr Ala Leu Glu Leu His Thr Glu Leu Leu
        105                 110                 115

gag agt ggc cgg gag ctg cct cgg ctg cgc gac tac ttc tcc aat cca        617
Glu Ser Gly Arg Glu Leu Pro Arg Leu Arg Asp Tyr Phe Ser Asn Pro
    120                 125                 130

ggc aac ttc gag agg caa agt gga acc ccg ccg ggg atg ggg gcg cca        665
Gly Asn Phe Glu Arg Gln Ser Gly Thr Pro Pro Gly Met Gly Ala Pro
135                 140                 145                 150

ggg gtc cct gga gca gcc ggc gtt ggg ggc gct gga ggt cgg gaa ccg        713
Gly Val Pro Gly Ala Ala Gly Val Gly Gly Ala Gly Gly Arg Glu Pro
                155                 160                 165

agt aca gcg tcg ggc ggg gga cag ctc aat cga gct ggg agc att agt        761
Ser Thr Ala Ser Gly Gly Gly Gln Leu Asn Arg Ala Gly Ser Ile Ser
            170                 175                 180

acc ctt gat tct tta gac ttt gca aga tat tca gat gat ggt aac agg        809
Thr Leu Asp Ser Leu Asp Phe Ala Arg Tyr Ser Asp Asp Gly Asn Arg
        185                 190                 195

gaa aca gat gaa aaa gtg gca gtc ctg gag ttt gaa cta cgg aaa gcc        857
Glu Thr Asp Glu Lys Val Ala Val Leu Glu Phe Glu Leu Arg Lys Ala
    200                 205                 210

aag gag acc att cag gcc ctc cga gcc aac ctg aca aag gcc gca gaa        905
Lys Glu Thr Ile Gln Ala Leu Arg Ala Asn Leu Thr Lys Ala Ala Glu
215                 220                 225                 230

cat gaa gtt cct tta cag gaa cga aaa aat tac aaa tca agt cct gaa        953
His Glu Val Pro Leu Gln Glu Arg Lys Asn Tyr Lys Ser Ser Pro Glu
                235                 240                 245

att cag gag cca atc aaa cct ctt gaa aag aga gct cta aac ttc tta       1001
Ile Gln Glu Pro Ile Lys Pro Leu Glu Lys Arg Ala Leu Asn Phe Leu
            250                 255                 260

gtc aat gaa ttt tta ttg aag aat aac tat aag ctt aca tca ata acc       1049
Val Asn Glu Phe Leu Leu Lys Asn Asn Tyr Lys Leu Thr Ser Ile Thr
        265                 270                 275

ttt tca gat gaa aac gat gat cag gat ttt gaa tta tgg gat gat gta       1097
Phe Ser Asp Glu Asn Asp Asp Gln Asp Phe Glu Leu Trp Asp Asp Val
    280                 285                 290

gga tta aac att cca aaa cct cca gac tta ttg caa ctc tac cgg gat       1145
Gly Leu Asn Ile Pro Lys Pro Pro Asp Leu Leu Gln Leu Tyr Arg Asp
295                 300                 305                 310

ttt gga aat cat caa gta act gga aaa gat ctt gta gat gtg gcc agt       1193
Phe Gly Asn His Gln Val Thr Gly Lys Asp Leu Val Asp Val Ala Ser
                315                 320                 325

gga gta gaa gaa gat gaa tta gag gcc ctt aca cca att ata agc aac       1241
Gly Val Glu Glu Asp Glu Leu Glu Ala Leu Thr Pro Ile Ile Ser Asn
            330                 335                 340

ctt cct cca act ctt gaa act ccc cag cct gca gag aac tcc atg tta       1289
Leu Pro Pro Thr Leu Glu Thr Pro Gln Pro Ala Glu Asn Ser Met Leu
        345                 350                 355

gta cag aaa tta gaa gat aaa att agt ttg tta aat agt gag aaa tgg 1337
Val Gln Lys Leu Glu Asp Lys Ile Ser Leu Leu Asn Ser Glu Lys Trp
    360                 365                 370

tca ttg atg gag caa atc aga aga ctt aaa agt gaa atg gac ttc ctc 1385
Ser Leu Met Glu Gln Ile Arg Arg Leu Lys Ser Glu Met Asp Phe Leu
375                 380                 385                 390

aaa aat gaa cac ttt gcc atc cca gca gtt tgt gac tct gtt cag cct 1433
Lys Asn Glu His Phe Ala Ile Pro Ala Val Cys Asp Ser Val Gln Pro
                395                 400                 405

cct ttg gat cag ttg ccc cac aaa gac tct gag gac agt gga cag cat 1481
Pro Leu Asp Gln Leu Pro His Lys Asp Ser Glu Asp Ser Gly Gln His
            410                 415                 420

cca gat gta aat agt tca gac aag gga aaa aac aca gac atc cat ctt 1529
Pro Asp Val Asn Ser Ser Asp Lys Gly Lys Asn Thr Asp Ile His Leu
        425                 430                 435

tca ata tca gat gaa gct gat tcc act att cct aaa gag aat tcc cca 1577
Ser Ile Ser Asp Glu Ala Asp Ser Thr Ile Pro Lys Glu Asn Ser Pro
    440                 445                 450

aat tca ttc ccc agg aga gaa aga gaa gga atg cca cct tct tct cta 1625
Asn Ser Phe Pro Arg Arg Glu Arg Glu Gly Met Pro Pro Ser Ser Leu
455                 460                 465                 470

tca agt aaa aag aca gtt cat ttt gat aaa cct aat agg aaa ttg tct 1673
Ser Ser Lys Lys Thr Val His Phe Asp Lys Pro Asn Arg Lys Leu Ser
                475                 480                 485

cct gca ttc cat caa gca cta ctc tct ttt tgt cga atg tca gca gat 1721
Pro Ala Phe His Gln Ala Leu Leu Ser Phe Cys Arg Met Ser Ala Asp
            490                 495                 500

agt cgt tta gga tac gag gtg tct cgt att gca gac agt gaa aaa agc 1769
Ser Arg Leu Gly Tyr Glu Val Ser Arg Ile Ala Asp Ser Glu Lys Ser
        505                 510                 515

gtt atg tta atg ctg gga cgc tgc ctg cca cac att gtt ccc aat gtg 1817
Val Met Leu Met Leu Gly Arg Cys Leu Pro His Ile Val Pro Asn Val
    520                 525                 530

cta ttg gca aag aga gag gag ttg atc ccc ctc ata ttg tgt aca gca 1865
Leu Leu Ala Lys Arg Glu Glu Leu Ile Pro Leu Ile Leu Cys Thr Ala
535                 540                 545                 550

tgt cta cat cct gag cct aaa gag cga gat cag ctt ctc cac ata ctt 1913
Cys Leu His Pro Glu Pro Lys Glu Arg Asp Gln Leu Leu His Ile Leu
                555                 560                 565

ttc aat ttg atc aag agg cca gat gat gag caa agg caa atg ata ctg 1961
Phe Asn Leu Ile Lys Arg Pro Asp Asp Glu Gln Arg Gln Met Ile Leu
            570                 575                 580

aca ggt tgt gtg gca ttt gcg cgt cat gtt gga cca aca cgt gta gaa 2009
Thr Gly Cys Val Ala Phe Ala Arg His Val Gly Pro Thr Arg Val Glu
        585                 590                 595

gct gaa ctt tta cca cag tgt tgg gaa cag att aat cac aaa tac cca 2057
Ala Glu Leu Leu Pro Gln Cys Trp Glu Gln Ile Asn His Lys Tyr Pro
    600                 605                 610

gaa aga cga ctg ctt gtg gca gaa tcc tgt gga gca ctg gca cct tac 2105
Glu Arg Arg Leu Leu Val Ala Glu Ser Cys Gly Ala Leu Ala Pro Tyr
615                 620                 625                 630

ctt cct aaa gaa atc cgt agc tcc ttg gtt ctt tca atg ttg caa caa 2153
Leu Pro Lys Glu Ile Arg Ser Ser Leu Val Leu Ser Met Leu Gln Gln
                635                 640                 645

atg tta atg gaa gat aag gca gat ttg gta aga gaa gct gtt atc aaa 2201
Met Leu Met Glu Asp Lys Ala Asp Leu Val Arg Glu Ala Val Ile Lys
            650                 655                 660

agc ctt ggt atc att atg gga tac att gat gat cca gac aaa tat cat 2249
Ser Leu Gly Ile Ile Met Gly Tyr Ile Asp Asp Pro Asp Lys Tyr His
        665                 670                 675

cag ggt ttt gaa ttg ttg ctg tca gcc ttg ggt gat ccc tca gaa aga 2297
Gln Gly Phe Glu Leu Leu Leu Ser Ala Leu Gly Asp Pro Ser Glu Arg
    680                 685                 690

gta gtt agt gct aca cat caa gta ttt tta cca gct tac gct gcg tgg 2345
Val Val Ser Ala Thr His Gln Val Phe Leu Pro Ala Tyr Ala Ala Trp
695                 700                 705                 710

act aca gaa ctt gga aat tta cag tct cat ctt ata ctt aca cta ctg 2393
Thr Thr Glu Leu Gly Asn Leu Gln Ser His Leu Ile Leu Thr Leu Leu
                715                 720                 725

aac aag att gaa aaa ctt ctc agg gaa gga gaa cat gga ctg gat gaa 2441
Asn Lys Ile Glu Lys Leu Leu Arg Glu Gly Glu His Gly Leu Asp Glu
            730                 735                 740

cac aaa ctc cac atg tat ctt tct gcc ttg cag tcc ttg atc cca tct 2489
His Lys Leu His Met Tyr Leu Ser Ala Leu Gln Ser Leu Ile Pro Ser
        745                 750                 755

ctc ttt gca tta gtg cta cag aat gca cct ttc tcc agc aaa gcc aag 2537
Leu Phe Ala Leu Val Leu Gln Asn Ala Pro Phe Ser Ser Lys Ala Lys
    760                 765                 770

ctt cat ggt gaa gtg cca cag ata gaa gtg act agg ttt cct cgg cct 2585
Leu His Gly Glu Val Pro Gln Ile Glu Val Thr Arg Phe Pro Arg Pro
775                 780                 785                 790

atg tcg cct ctt caa gat gtg tcc act att atc gga agt cgt gag caa 2633
Met Ser Pro Leu Gln Asp Val Ser Thr Ile Ile Gly Ser Arg Glu Gln
                795                 800                 805

ttg gca gtg ctg ctg caa ctt tat gac tac cag cta gaa caa gag ggt 2681
Leu Ala Val Leu Leu Gln Leu Tyr Asp Tyr Gln Leu Glu Gln Glu Gly
            810                 815                 820

aca aca ggc tgg gag agt tta ctg tgg gtt gtc aat caa ttg ttg cca 2729
Thr Thr Gly Trp Glu Ser Leu Leu Trp Val Val Asn Gln Leu Leu Pro
        825                 830                 835

caa ctt ata gaa ata gtt ggc aaa att aat gtt act tca act gcc tgt 2777
Gln Leu Ile Glu Ile Val Gly Lys Ile Asn Val Thr Ser Thr Ala Cys
    840                 845                 850

gtc cat gaa ttc tcc aga ttt ttc tgg cgc ctt tgc cgg aca ttt ggc 2825
Val His Glu Phe Ser Arg Phe Phe Trp Arg Leu Cys Arg Thr Phe Gly

855                 860                 865                 870

aaa att ttt aca aac act aag gta aaa cct cag ttc cag gag att tta      2873
Lys Ile Phe Thr Asn Thr Lys Val Lys Pro Gln Phe Gln Glu Ile Leu
                875                 880                 885

aga cta tct gaa gaa aac att gat tcc tca gca gga aat ggg gtc ctc      2921
Arg Leu Ser Glu Glu Asn Ile Asp Ser Ser Ala Gly Asn Gly Val Leu
            890                 895                 900

act aaa gct aca gtc ccc att tat gca aca gga gtc ctt acg tgt tat      2969
Thr Lys Ala Thr Val Pro Ile Tyr Ala Thr Gly Val Leu Thr Cys Tyr
        905                 910                 915

att cag gaa gaa gac cga aaa ctg tta gtt gga ttc tta gaa gat gta      3017
Ile Gln Glu Glu Asp Arg Lys Leu Leu Val Gly Phe Leu Glu Asp Val
    920                 925                 930

atg acg ctg ctt tca tta tct cat gct cct ctt gat agc ctg aag gct      3065
Met Thr Leu Leu Ser Leu Ser His Ala Pro Leu Asp Ser Leu Lys Ala
935                 940                 945                 950

tct ttt gtg gaa ttg ggt gca aac cca gcc tac cat gag tta cta tta      3113
Ser Phe Val Glu Leu Gly Ala Asn Pro Ala Tyr His Glu Leu Leu Leu
                955                 960                 965

act gtt ttg tgg tat ggt gtt gtc cat act tca gca ctc gtg agg tgt      3161
Thr Val Leu Trp Tyr Gly Val Val His Thr Ser Ala Leu Val Arg Cys
            970                 975                 980

act gct gct aga atg ttt gag ctg act ctt cga ggc atg agt gaa gcg      3209
Thr Ala Ala Arg Met Phe Glu Leu Thr Leu Arg Gly Met Ser Glu Ala
        985                 990                 995

tta gtt  gac aag cgg gtt gct  ccg gcc ctt gtt acc  ttg tcc agt      3254
Leu Val  Asp Lys Arg Val Ala  Pro Ala Leu Val Thr  Leu Ser Ser
    1000                 1005                 1010

gat cct  gaa ttc tct gtc agg  att gcc aca att cca  gcc ttt ggc      3299
Asp Pro  Glu Phe Ser Val Arg  Ile Ala Thr Ile Pro  Ala Phe Gly
    1015                 1020                 1025

act att  atg gaa aca gta att  caa aga gag ttg ctg  gaa aga gtg      3344
Thr Ile  Met Glu Thr Val Ile  Gln Arg Glu Leu Leu  Glu Arg Val
    1030                 1035                 1040

aaa atg  cag ttg gct tct ttc  ctg gaa gat cct cag  tat caa gac      3389
Lys Met  Gln Leu Ala Ser Phe  Leu Glu Asp Pro Gln  Tyr Gln Asp
    1045                 1050                 1055

caa cat  tct ttg cat aca gag  atc ata aaa aca ttt  ggt aga gtt      3434
Gln His  Ser Leu His Thr Glu  Ile Ile Lys Thr Phe  Gly Arg Val
    1060                 1065                 1070

ggc cct  aac gca gaa ccc agg  ttc cga gat gag ttt  gtt ata cca      3479
Gly Pro  Asn Ala Glu Pro Arg  Phe Arg Asp Glu Phe  Val Ile Pro
    1075                 1080                 1085

cat ttg  cat aag tta gcc ttg  gtg aac aac tta cag  att gtg gat      3524
His Leu  His Lys Leu Ala Leu  Val Asn Asn Leu Gln  Ile Val Asp
    1090                 1095                 1100

tct aaa  aga ctg gac att gct  acg cat ctt ttt gaa  gcc tac agt      3569

Ser Lys Arg Leu Asp Ile Ala Thr His Leu Phe Glu Ala Tyr Ser
    1105                1110                1115

gca ctt tcc tgt tgt ttc att tca gag gat tta atg gtt aat cac    3614
Ala Leu Ser Cys Cys Phe Ile Ser Glu Asp Leu Met Val Asn His
    1120                1125                1130

ttt tta cct ggt ctc aga tgt tta cgg act gac atg gaa cat ctc    3659
Phe Leu Pro Gly Leu Arg Cys Leu Arg Thr Asp Met Glu His Leu
    1135                1140                1145

tct cca gag cat gag gtt att tta agt tcc atg ata aaa gaa tgt    3704
Ser Pro Glu His Glu Val Ile Leu Ser Ser Met Ile Lys Glu Cys
    1150                1155                1160

gaa caa aaa gtt gaa aac aag acc gtc caa gag cct caa ggc tca    3749
Glu Gln Lys Val Glu Asn Lys Thr Val Gln Glu Pro Gln Gly Ser
    1165                1170                1175

atg tca att gct gca agc tta gtg agt gaa gat aca aag acc aag    3794
Met Ser Ile Ala Ala Ser Leu Val Ser Glu Asp Thr Lys Thr Lys
    1180                1185                1190

ttt ttg aac aaa atg ggc cag ttg aca aca tca ggt gcc atg ttg    3839
Phe Leu Asn Lys Met Gly Gln Leu Thr Thr Ser Gly Ala Met Leu
    1195                1200                1205

gcc aat gta ttt cag aga aag aag tag aagcaggaaa gaagccccca    3886
Ala Asn Val Phe Gln Arg Lys Lys
    1210                1215

gtaaacacta agatggacct caagccgact ggttccttgt acttgaagta cttgcctttt    3946

ttgtttcctc agttttatgt tcttgcatta taattttatc ctaacctcca aagatatttg    4006

cactgctttt aattactgct gtatatttgt tgattttgga gttacaactg tggtgataga    4066

aaattgagtt gatggtctgt accaagtccc ttgtctatgt tcttgtcttt cagaataatt    4126

tttatataaa tatatatata gtgaagaagt tttttttaat ttttggatgg gatattcgca    4186

aatatctgta ttatacacta agctattaca atggtactta aaataatgta aatttgaagt    4246

cattgttata aaataataaa gtggagatta cttaagtatt taaattatga aagaataatg    4306

cagacttttt attgtttctt aactgactag aaagagccac cagcattact ctgtgccttt    4366

tggacatcag tttgtgtgtt ctgtaggaat tgtgtgcatt ccattcacac agtatttctt    4426

taggatgctg tgatgatttg aattacaaat cctacagtca atagctaaag acgaaacctt    4486

catttcagaa ctctcatgaa tattctttaa gtgctattta aacctcccca gcacttagat    4546

gcatataatg gacttacctg aggaaagaca gcacataggc atggggagag gtaaccaagg    4606

tgaattttac aaactggtgt atagtagatt taaatgctca aaaataaatg taactgagaa    4666

gagttaataa ttgtgagatt tttcccaaat tgagatacag aagaaaatat agtttgaatc    4726

tgaaatttaa cacttattta tgtaaaacac tttatttaag atattttcta atgattttaa    4786

ttttagagag taccttttca ttctgtgtgt tacagagaag tactgaaaag ttaaggacac    4846

ttgggggcta cttttttccc tctaaactaa aaaagacatt ggctgaatta taactagtta 4906

gttatcacct cgtcccttaa agtcagtgac ctcctgtgtt tgatgtatat tacatagagt 4966

cttaagtcag tgtacagttc cactggaatt tgacagttgt ctctacagtc atgcaactcg 5026

aagtagaaaa gagtgctgga cataggaagg gggtgcttgg tttgaggggt taatgtgagg 5086

cctttttgaa aaatgaatat tttgataaaa agaattcttg ttttagcaca gttgatgcac 5146

ataagtgatt ctcatatttg ttgtataaac tggtttaata catttggaac atagttggat 5206

tacattcatt tcctgggaaa gctagcttac catacattca agtttataaa acaatttgcc 5266

ataggcaaag ccatttaaaa agttcattct gaaattattt catttaccta cagtgaaata 5326

attgtgaact aagtagtctt tctgaaaact gttgggttct aggcattcct gagaaattga 5386

aagtggctac ctttcatgtc aaaaatgttg atctattata aataaaatgt ttttgcatat 5446

gtttttgaaa aaa 5459

<210> 24
<211> 1216
<212> PRT
<213> Homo sapiens

<400> 24

Met Ala Ala Met Ala Pro Gly Gly Ser Gly Ser Gly Gly Gly Val Asn
1               5                   10                  15

Pro Phe Leu Ser Asp Ser Asp Glu Asp Asp Asp Glu Val Ala Ala Thr
            20                  25                  30

Glu Glu Arg Arg Ala Val Leu Arg Leu Gly Ala Gly Ser Gly Leu Asp
        35                  40                  45

Pro Gly Ser Ala Gly Ser Leu Ser Pro Gln Asp Pro Val Ala Leu Gly
    50                  55                  60

Ser Ser Ala Arg Pro Gly Leu Pro Gly Glu Ala Ser Ala Ala Ala Val
65                  70                  75                  80

Ala Leu Gly Gly Thr Gly Glu Thr Pro Ala Arg Leu Ser Ile Asp Ala
                85                  90                  95

Ile Ala Ala Gln Leu Leu Arg Asp Gln Tyr Leu Leu Thr Ala Leu Glu
            100                 105                 110

Leu His Thr Glu Leu Leu Glu Ser Gly Arg Glu Leu Pro Arg Leu Arg
        115                 120                 125

Asp Tyr Phe Ser Asn Pro Gly Asn Phe Glu Arg Gln Ser Gly Thr Pro

130                 135                 140

Pro Gly Met Gly Ala Pro Gly Val Pro Gly Ala Ala Gly Val Gly Gly
145                 150                 155                 160

Ala Gly Gly Arg Glu Pro Ser Thr Ala Ser Gly Gly Gly Gln Leu Asn
                165                 170                 175

Arg Ala Gly Ser Ile Ser Thr Leu Asp Ser Leu Asp Phe Ala Arg Tyr
            180                 185                 190

Ser Asp Asp Gly Asn Arg Glu Thr Asp Glu Lys Val Ala Val Leu Glu
        195                 200                 205

Phe Glu Leu Arg Lys Ala Lys Glu Thr Ile Gln Ala Leu Arg Ala Asn
    210                 215                 220

Leu Thr Lys Ala Ala Glu His Glu Val Pro Leu Gln Glu Arg Lys Asn
225                 230                 235                 240

Tyr Lys Ser Ser Pro Glu Ile Gln Glu Pro Ile Lys Pro Leu Glu Lys
                245                 250                 255

Arg Ala Leu Asn Phe Leu Val Asn Glu Phe Leu Leu Lys Asn Asn Tyr
            260                 265                 270

Lys Leu Thr Ser Ile Thr Phe Ser Asp Glu Asn Asp Asp Gln Asp Phe
        275                 280                 285

Glu Leu Trp Asp Asp Val Gly Leu Asn Ile Pro Lys Pro Pro Asp Leu
    290                 295                 300

Leu Gln Leu Tyr Arg Asp Phe Gly Asn His Gln Val Thr Gly Lys Asp
305                 310                 315                 320

Leu Val Asp Val Ala Ser Gly Val Glu Glu Asp Glu Leu Glu Ala Leu
                325                 330                 335

Thr Pro Ile Ile Ser Asn Leu Pro Pro Thr Leu Glu Thr Pro Gln Pro
            340                 345                 350

Ala Glu Asn Ser Met Leu Val Gln Lys Leu Glu Asp Lys Ile Ser Leu
        355                 360                 365

Leu Asn Ser Glu Lys Trp Ser Leu Met Glu Gln Ile Arg Arg Leu Lys
    370                 375                 380

Ser Glu Met Asp Phe Leu Lys Asn Glu His Phe Ala Ile Pro Ala Val
385                 390                 395                 400

Cys Asp Ser Val Gln Pro Pro Leu Asp Gln Leu Pro His Lys Asp Ser
                405                 410                 415

Glu Asp Ser Gly Gln His Pro Asp Val Asn Ser Ser Asp Lys Gly Lys
            420                 425                 430

Asn Thr Asp Ile His Leu Ser Ile Ser Asp Glu Ala Asp Ser Thr Ile
        435                 440                 445

Pro Lys Glu Asn Ser Pro Asn Ser Phe Pro Arg Arg Glu Arg Glu Gly
    450                 455                 460

Met Pro Pro Ser Ser Leu Ser Ser Lys Lys Thr Val His Phe Asp Lys
465                 470                 475                 480

Pro Asn Arg Lys Leu Ser Pro Ala Phe His Gln Ala Leu Leu Ser Phe
                485                 490                 495

Cys Arg Met Ser Ala Asp Ser Arg Leu Gly Tyr Glu Val Ser Arg Ile
            500                 505                 510

Ala Asp Ser Glu Lys Ser Val Met Leu Met Leu Gly Arg Cys Leu Pro
        515                 520                 525

His Ile Val Pro Asn Val Leu Leu Ala Lys Arg Glu Glu Leu Ile Pro
    530                 535                 540

Leu Ile Leu Cys Thr Ala Cys Leu His Pro Glu Pro Lys Glu Arg Asp
545                 550                 555                 560

Gln Leu Leu His Ile Leu Phe Asn Leu Ile Lys Arg Pro Asp Asp Glu
                565                 570                 575

Gln Arg Gln Met Ile Leu Thr Gly Cys Val Ala Phe Ala Arg His Val
            580                 585                 590

Gly Pro Thr Arg Val Glu Ala Glu Leu Leu Pro Gln Cys Trp Glu Gln
        595                 600                 605

Ile Asn His Lys Tyr Pro Glu Arg Arg Leu Leu Val Ala Glu Ser Cys
    610                 615                 620

Gly Ala Leu Ala Pro Tyr Leu Pro Lys Glu Ile Arg Ser Ser Leu Val
625                 630                 635                 640

Leu Ser Met Leu Gln Gln Met Leu Met Glu Asp Lys Ala Asp Leu Val
                645                 650                 655

Arg Glu Ala Val Ile Lys Ser Leu Gly Ile Ile Met Gly Tyr Ile Asp
            660                 665                 670

Asp Pro Asp Lys Tyr His Gln Gly Phe Glu Leu Leu Leu Ser Ala Leu
        675                 680                 685

Gly Asp Pro Ser Glu Arg Val Val Ser Ala Thr His Gln Val Phe Leu
    690                 695                 700

Pro Ala Tyr Ala Ala Trp Thr Thr Glu Leu Gly Asn Leu Gln Ser His
705                 710                 715                 720

Leu Ile Leu Thr Leu Leu Asn Lys Ile Glu Lys Leu Leu Arg Glu Gly
                725                 730                 735

Glu His Gly Leu Asp Glu His Lys Leu His Met Tyr Leu Ser Ala Leu
            740                 745                 750

Gln Ser Leu Ile Pro Ser Leu Phe Ala Leu Val Leu Gln Asn Ala Pro
        755                 760                 765

Phe Ser Ser Lys Ala Lys Leu His Gly Glu Val Pro Gln Ile Glu Val
    770                 775                 780

Thr Arg Phe Pro Arg Pro Met Ser Pro Leu Gln Asp Val Ser Thr Ile
785                 790                 795                 800

Ile Gly Ser Arg Glu Gln Leu Ala Val Leu Leu Gln Leu Tyr Asp Tyr
                805                 810                 815

Gln Leu Glu Gln Glu Gly Thr Thr Gly Trp Glu Ser Leu Leu Trp Val
            820                 825                 830

Val Asn Gln Leu Leu Pro Gln Leu Ile Glu Ile Val Gly Lys Ile Asn
        835                 840                 845

Val Thr Ser Thr Ala Cys Val His Glu Phe Ser Arg Phe Phe Trp Arg
    850                 855                 860

Leu Cys Arg Thr Phe Gly Lys Ile Phe Thr Asn Thr Lys Val Lys Pro
865                 870                 875                 880

Gln Phe Gln Glu Ile Leu Arg Leu Ser Glu Glu Asn Ile Asp Ser Ser
                885                 890                 895

Ala Gly Asn Gly Val Leu Thr Lys Ala Thr Val Pro Ile Tyr Ala Thr
            900                 905                 910

Gly Val Leu Thr Cys Tyr Ile Gln Glu Glu Asp Arg Lys Leu Leu Val
        915                 920                 925

Gly Phe Leu Glu Asp Val Met Thr Leu Leu Ser Leu Ser His Ala Pro
    930                 935                 940

Leu Asp Ser Leu Lys Ala Ser Phe Val Glu Leu Gly Ala Asn Pro Ala
945                 950                 955                 960

Tyr His Glu Leu Leu Leu Thr Val Leu Trp Tyr Gly Val Val His Thr
                965                 970                 975

Ser Ala Leu Val Arg Cys Thr Ala Ala Arg Met Phe Glu Leu Thr Leu
            980                 985                 990

Arg Gly Met Ser Glu Ala Leu Val Asp Lys Arg Val Ala Pro Ala Leu
        995                 1000                1005

Val Thr Leu Ser Ser Asp Pro Glu Phe Ser Val Arg Ile Ala Thr
    1010                1015                1020

Ile Pro Ala Phe Gly Thr Ile Met Glu Thr Val Ile Gln Arg Glu
    1025                1030                1035

Leu Leu Glu Arg Val Lys Met Gln Leu Ala Ser Phe Leu Glu Asp
    1040                1045                1050

Pro Gln Tyr Gln Asp Gln His Ser Leu His Thr Glu Ile Ile Lys
    1055                1060                1065

Thr Phe Gly Arg Val Gly Pro Asn Ala Glu Pro Arg Phe Arg Asp
    1070                1075                1080

Glu Phe Val Ile Pro His Leu His Lys Leu Ala Leu Val Asn Asn
    1085                1090                1095

Leu Gln Ile Val Asp Ser Lys Arg Leu Asp Ile Ala Thr His Leu
    1100                1105                1110

Phe Glu Ala Tyr Ser Ala Leu Ser Cys Cys Phe Ile Ser Glu Asp
    1115                1120                1125

Leu Met Val Asn His Phe Leu Pro Gly Leu Arg Cys Leu Arg Thr

1130                 1135                 1140

Asp Met Glu His Leu Ser Pro Glu His Glu Val Ile Leu Ser Ser
    1145                 1150                 1155

Met Ile Lys Glu Cys Glu Gln Lys Val Glu Asn Lys Thr Val Gln
    1160                 1165                 1170

Glu Pro Gln Gly Ser Met Ser Ile Ala Ala Ser Leu Val Ser Glu
    1175                 1180                 1185

Asp Thr Lys Thr Lys Phe Leu Asn Lys Met Gly Gln Leu Thr Thr
    1190                 1195                 1200

Ser Gly Ala Met Leu Ala Asn Val Phe Gln Arg Lys Lys
    1205                 1210                 1215

<210>  25
<211>  5629
<212>  DNA
<213>  Homo sapiens

<220>
<221>  CDS
<222>  (191)..(3535)

<400>  25
agtcccgcga ccgaagcagg gcgcgcagca gcgctgagtg ccccggaacg tgcgtcgcgc      60
ccccagtgtc cgtcgcgtcc gccgcgcccc gggcggggat ggggcggcca gactgagcgc     120
cgcacccgcc atccagaccc gccggcccta gccgcagtcc ctccagccgt ggccccagcg     180
cgcacgggcg atg gcg aag gcg acg tcc ggt gcc gcg ggg ctg cgt ctg        229
ctg ttg ctg ctg ctg ctg ccg ctg cta ggc aaa gtg gca ttg ggc ctc       277
tac ttc tcg agg gat gct tac tgg gag aag ctg tat gtg gac cag gcg       325
gcc ggc acg ccc ttg ctg tac gtc cat gcc ctg cgg gac gcc cct gag       373
gag gtg ccc agc ttc cgc ctg ggc cag cat ctc tac ggc acg tac cgc       421
aca cgg ctg cat gag aac aac tgg atc tgc atc cag gag gac acc ggc       469
ctc ctc tac ctt aac cgg agc ctg gac cat agc tcc tgg gag aag ctc 517
agt gtc cgc aac cgc ggc ttt ccc ctg ctc acc gtc tac ctc aag gtc 565
ttc ctg tca ccc aca tcc ctt cgt gag ggc gag tgc cag tgg cca ggc 613
tgt gcc cgc gta tac ttc tcc ttc ttc aac acc tcc ttt cca gcc tgc 661
agc tcc ctc aag ccc cgg gag ctc tgc ttc cca gag aca agg ccc tcc 709
ttc cgc att cgg gag aac cga ccc cca ggc acc ttc cac cag ttc cgc 757
ctg ctg cct gtg cag ttc ttg tgc ccc aac atc agc gtg gcc tac agg 805
ctc ctg gag ggt gag ggt ctg ccc ttc cgc tgc gcc ccg gac agc ctg 853
gag gtg agc acg cgc tgg gcc ctg gac cgc gag cag cgg gag aag tac 901
gag ctg gtg gcc gtg tgc acc gtg cac gcc ggc gcg cgc gag gag gtg 949
gtg atg gtg ccc ttc ccg gtg acc gtg tac gac gag gac gac tcg gcg 997
ccc acc ttc ccc gcg ggc gtc gac acc gcc agc gcc gtg gtg gag ttc 1045
aag cgg aag gag gac acc gtg gtg gcc acg ctg cgt gtc ttc gat gca 1093
gac gtg gta cct gca tca ggg gag ctg gtg agg cgg tac aca agc acg 1141
ctg ctc ccc ggg gac acc tgg gcc cag cag acc ttc cgg gtg gaa cac 1189
tgg ccc aac gag acc tcg gtc cag gcc aac ggc agc ttc gtg cgg gcg 1237
acc gta cat gac tat agg ctg gtt ctc aac cgg aac ctc tcc atc tcg       1285
gag aac cgc acc atg cag ctg gcg gtg ctg gtc aat gac tca gac ttc       1333
cag ggc cca gga gcg ggc gtc ctc ttg ctc cac ttc aac gtg tcg gtg       1381
ctg ccg gtc agc ctg cac ctg ccc agt acc tac tcc ctc tcc gtg agc       1429
agg agg gct cgc cga ttt gcc cag atc ggg aaa gtc tgt gtg gaa aac       1477
tgc cag gca ttc agt ggc atc aac gtc cag tac aag ctg cat tcc tct       1525
ggt gcc aac tgc agc acg cta ggg gtg gtc acc tca gcc gag gac acc       1573
tcg ggg atc ctg ttt gtg aat gac acc aag gcc ctg cgg cgg ccc aag       1621
tgt gcc gaa ctt cac tac atg gtg gtg gcc acc gac cag cag acc tct       1669
agg cag gcc cag gcc cag ctg ctt gta aca gtg gag ggg tca tat gtg       1717
gcc gag gag gcg ggc tgc ccc ctg tcc tgt gca gtc agc aag aga cgg       1765
ctg gag tgt gag gag tgt ggc ggc ctg ggc tcc cca aca ggc agg tgt       1813
gag tgg agg caa gga gat ggc aaa ggg atc acc agg aac ttc tcc acc       1861
tgc tct ccc agc acc aag acc tgc ccc gac ggc cac tgc gat gtt gtg       1909
gag acc caa gac atc aac att tgc cct cag gac tgc ctc cgg ggc agc       1957
att gtt ggg gga cac gag cct ggg gag ccc cgg ggg att aaa gct ggc       2005
tat ggc acc tgc aac tgc ttc cct gag gag gag aag tgc ttc tgc gag      2053
ccc gaa gac atc cag gat cca ctg tgc gac gag ctg tgc cgc acg gtg      2101
atc gca gcc gct gtc ctc ttc tcc ttc atc gtc tcg gtg ctg ctg tct      2149
gcc ttc tgc atc cac tgc tac cac aag ttt gcc cac aag cca ccc atc      2197
tcc tca gct gag atg acc ttc cgg agg ccc gcc cag gcc ttc ccg gtc      2245
agc tac tcc tct tcc ggt gcc cgc cgg ccc tcg ctg gac tcc atg gag      2293
aac cag gtc tcc gtg gat gcc ttc aag atc ctg gag gat cca aag tgg      2341
gaa ttc cct cgg aag aac ttg gtt ctt gga aaa act cta gga gaa ggc      2389
gaa ttt gga aaa gtg gtc aag gca acg gcc ttc cat ctg aaa ggc aga      2437
gca ggg tac acc acg gtg gcc gtg aag atg ctg aaa gag aac gcc tcc      2485
ccg agt gag ctt cga gac ctg ctg tca gag ttc aac gtc ctg aag cag      2533
gtc aac cac cca cat gtc atc aaa ttg tat ggg gcc tgc agc cag gat      2581
ggc ccg ctc ctc ctc atc gtg gag tac gcc aaa tac ggc tcc ctg cgg      2629
ggc ttc ctc cgc gag agc cgc aaa gtg ggg cct ggc tac ctg ggc agt      2677
gga ggc agc cgc aac tcc agc tcc ctg gac cac ccg gat gag cgg gcc      2725
ctc acc atg ggc gac ctc atc tca ttt gcc tgg cag atc tca cag ggg 2773
atg cag tat ctg gcc gag atg aag ctc gtt cat cgg gac ttg gca gcc 2821
aga aac atc ctg gta gct gag ggg cgg aag atg aag att tcg gat ttc 2869
ggc ttg tcc cga gat gtt tat gaa gag gat tcc tac gtg aag agg agc 2917
cag ggt cgg att cca gtt aaa tgg atg gca att gaa tcc ctt ttt gat 2965
cat atc tac acc acg caa agt gat gta tgg tct ttt ggt gtc ctg ctg 3013
tgg gag atc gtg acc cta ggg gga aac ccc tat cct ggg att cct cct 3061
gag cgg ctc ttc aac ctt ctg aag acc ggc cac cgg atg gag agg cca 3109
gac aac tgc agc gag gag atg tac cgc ctg atg ctg caa tgc tgg aag 3157
cag gag ccg gac aaa agg ccg gtg ttt gcg gac atc agc aaa gac ctg 3205
gag aag atg atg gtt aag agg aga gac tac ttg gac ctt gcg gcg 3250
tcc act cca tct gac tcc ctg att tat gac gac ggc ctc tca gag 3295
gag gag aca ccg ctg gtg gac tgt aat aat gcc ccc ctc cct cga 3340
gcc ctc cct tcc aca tgg att gaa aac aaa ctc tat ggc atg tca 3385
gac ccg aac tgg cct gga gag agt cct gta cca ctc acg aga gct 3430
gat ggc act aac act ggg ttt cca aga tat cca aat gat agt gta 3475
tat gct aac tgg atg  ctt tca ccc tca gcg  gca aaa tta atg gac      3520
acg ttt gat agt taa catttctttg tgaaaggtaa tggactcaca aggggaagaa      3575
acatgctgag aatggaaagt ctaccggccc tttctttgtg aacgtcacat tggccgagcc      3635
gtgttcagtt cccaggtggc agactcgttt ttggtagttt gttttaactt ccaaggtggt      3695
tttacttctg atagccggtg attttccctc ctagcagaca tgccacaccg ggtaagagct      3755
ctgagtctta gtggttaagc attcctttct cttcagtgcc cagcagcacc cagtgttggt      3815
ctgtgtccat cagtgaccac caacattctg tgttcacatg tgtgggtcca acacttacta      3875
cctggtgtat gaaattggac ctgaactgtt ggatttttct agttgccgcc aaacaaggca      3935
aaaaaattta aacatgaagc acacacacaa aaaaggcagt aggaaaaatg ctggccctga      3995
tgacctgtcc ttattcagaa tgagagactg cggggggggc ctgggggtag tgtcaatgcc      4055
cctccagggc tggaggggaa gaggggcccc gaggatgggc ctgggctcag cattcgagat      4115
cttgagaatg attttttttt aatcatgcaa cctttcctta ggaagacatt tggttttcat      4175
catgattaag atgattccta gatttagcac aatggagaga ttccatgcca tctttactat      4235
gtggatggtg gtatcaggga agagggctca caagacacat ttgtcccccg ggcccaccac      4295
atcatcctca cgtgttcggt actgagcagc cactacccct gatgagaaca gtatgaagaa      4355
agggggctgt tggagtccca gaattgctga cagcagaggc tttgctgctg tgaatcccac      4415
ctgccaccag cctgcagcac accccacagc caagtagagg cgaaagcagt ggctcatcct      4475
acctgttagg agcaggtagg gcttgtactc actttaattt gaatcttatc aacttactca      4535
taaagggaca ggctagctag ctgtgttaga agtagcaatg acaatgacca aggactgcta      4595
cacctctgat tacaattctg atgtgaaaaa gatggtgttt ggctcttata gagcctgtgt      4655
gaaaggccca tggatcagct cttcctgtgt ttgtaattta atgctgctac aagatgtttc      4715
tgtttcttag attctgacca tgactcataa gcttcttgtc attcttcatt gcttgtttgt      4775
ggtcacagat gcacaacact cctccagtct tgtgggggca gcttttggga agtctcagca      4835
gctcttctgg ctgtgttgtc agcactgtaa cttcgcagaa aagagtcgga ttaccaaaac      4895
actgcctgct cttcagactt aaagcactga taggacttaa aatagtctca ttcaaatact      4955
gtattttata taggcatttc acaaaaacag caaaattgtg gcattttgtg aggccaaggc      5015
ttggatgcgt gtgtaataga gccttgtggt gtgtgcgcac acacccagag ggagagtttg      5075
aaaaatgctt attggacacg taacctggct ctaatttggg ctgtttttca gatacactgt      5135
gataagttct tttacaaata tctatagaca tggtaaactt ttggttttca gatatgctta      5195
atgatagtct tactaaatgc agaaataaga ataaactttc tcaaattatt aaaaatgcct 5255
acacagtaag tgtgaattgc tgcaacaggt ttgttctcag gagggtaaga actccaggtc 5315
taaacagctg acccagtgat ggggaattta tccttgacca atttatcctt gaccaataac 5375
ctaattgtct attcctgagt tataaaagtc cccatcctta ttagctctac tggaattttc 5435
atacacgtaa atgcagaagt tactaagtat taagtattac tgagtattaa gtagtaatct 5495
gtcagttatt aaaatttgta aaatctattt atgaaaggtc attaaaccag atcatgttcc 5555
tttttttgta atcaaggtga ctaagaaaat cagttgtgta aataaaatca tgtatcataa 5615
aaaaaaaaaa aaaa 5629

<210> 26
<211> 1114
<212> PRT
<213> Homo sapiens

<400> 26

<210>  27
<211>  3905
<212>  DNA
<213>  Homo sapiens

<220>

<221>  CDS
<222>  (216)..(3266)

<400>  27
gacagatacc ctccttccgg ccgcgccact cgggaggcgg atcccgtggg cctgaggagg      60

cttcccccgc ccggtttgct ttccctccct cgctggcgct gccgcgagtc caccgagcgg     120

cctctgagga gcagccgcag gaggaggagg aggtcgtcgg gggcggcggg cggagaccgc     180

gctctcgctt ccccggcggc ggcaagggca ggaca atg gag gtg gcg gtg gag       233
                                       Met Glu Val Ala Val Glu
                                       1               5

aag gcg gtg gcg gcg gcg gca gcg gcc tcg gct gcg gcc tcc ggg ggg      281
Lys Ala Val Ala Ala Ala Ala Ala Ala Ser Ala Ala Ala Ser Gly Gly
            10                  15                  20

ccc tcg gcg gcg ccg agc ggg gag aac gag gcc gag agt cgg cag ggc      329
Pro Ser Ala Ala Pro Ser Gly Glu Asn Glu Ala Glu Ser Arg Gln Gly
        25                  30                  35

ccg gac tcg gag cgc ggc ggc gag gcg gcc cgg ctc aac ctg ttg gac      377
Pro Asp Ser Glu Arg Gly Gly Glu Ala Ala Arg Leu Asn Leu Leu Asp
    40                  45                  50

act tgc gcc gtg tgc cac cag aac atc cag agc cgg gcg ccc aag ctg      425
Thr Cys Ala Val Cys His Gln Asn Ile Gln Ser Arg Ala Pro Lys Leu
55                  60                  65                  70

ctg ccc tgc ctg cac tct ttc tgc cag cgc tgc ctg ccc gcg ccc cag      473
Leu Pro Cys Leu His Ser Phe Cys Gln Arg Cys Leu Pro Ala Pro Gln
                75                  80                  85

cgc tac ctc atg ctg ccc gcg ccc atg ctg ggc tcg gcc gag acc ccg      521
Arg Tyr Leu Met Leu Pro Ala Pro Met Leu Gly Ser Ala Glu Thr Pro
            90                  95                  100

cca ccc gtc cct gcc ccc ggc tcg ccg gtc agc ggc tcg tcg ccg ttc      569
Pro Pro Val Pro Ala Pro Gly Ser Pro Val Ser Gly Ser Ser Pro Phe
        105                 110                 115

gcc acc caa gtt gga gtc att cgt tgc cca gtt tgc agc caa gaa tgt      617
Ala Thr Gln Val Gly Val Ile Arg Cys Pro Val Cys Ser Gln Glu Cys
    120                 125                 130

gca gag aga cac atc ata gat aac ttt ttt gtg aag gac act act gag      665
Ala Glu Arg His Ile Ile Asp Asn Phe Phe Val Lys Asp Thr Thr Glu
135                 140                 145                 150

gtt ccc agc agt aca gta gaa aag tca aat cag gta tgt aca agc tgt      713
Val Pro Ser Ser Thr Val Glu Lys Ser Asn Gln Val Cys Thr Ser Cys
                155                 160                 165

gag gac aac gca gaa gcc aat ggg ttt tgt gta gag tgt gtt gaa tgg      761
Glu Asp Asn Ala Glu Ala Asn Gly Phe Cys Val Glu Cys Val Glu Trp
            170                 175                 180

ctc tgc aag acg tgt atc aga gct cat cag agg gta aag ttc aca aaa      809
Leu Cys Lys Thr Cys Ile Arg Ala His Gln Arg Val Lys Phe Thr Lys
        185                 190                 195

gac cac act gtc aga cag aaa gag gaa gta tct cca gag gca gtt ggt      857

Asp His Thr Val Arg Gln Lys Glu Glu Val Ser Pro Glu Ala Val Gly
    200                 205                 210

gtc acc agc cag cga cca gtg ttt tgt cct ttt cat aaa aag gag cag     905
Val Thr Ser Gln Arg Pro Val Phe Cys Pro Phe His Lys Lys Glu Gln
215                 220                 225                 230

ctg aag ctg tac tgt gag aca tgt gac aaa ctg aca tgt cga gac tgt     953
Leu Lys Leu Tyr Cys Glu Thr Cys Asp Lys Leu Thr Cys Arg Asp Cys
                235                 240                 245

cag ttg tta gaa cat aaa gag cat aga tac caa ttt ata gaa gaa gct     1001
Gln Leu Leu Glu His Lys Glu His Arg Tyr Gln Phe Ile Glu Glu Ala
            250                 255                 260

ttt cag aat cag aaa gtg atc ata gat aca cta atc acc aaa ctg atg     1049
Phe Gln Asn Gln Lys Val Ile Ile Asp Thr Leu Ile Thr Lys Leu Met
        265                 270                 275

gaa aaa aca aaa tac ata aaa ttc aca gga aat cag atc caa aac aga     1097
Glu Lys Thr Lys Tyr Ile Lys Phe Thr Gly Asn Gln Ile Gln Asn Arg
    280                 285                 290

att att gaa gta aat caa aat caa aag cag gtg gaa cag gat att aaa     1145
Ile Ile Glu Val Asn Gln Asn Gln Lys Gln Val Glu Gln Asp Ile Lys
295                 300                 305                 310

gtt gct ata ttt aca ctg atg gta gaa ata aat aaa aaa gga aaa gct     1193
Val Ala Ile Phe Thr Leu Met Val Glu Ile Asn Lys Lys Gly Lys Ala
                315                 320                 325

cta ctg cat cag tta gag agc ctt gca aag gac cat cgc atg aaa ctt     1241
Leu Leu His Gln Leu Glu Ser Leu Ala Lys Asp His Arg Met Lys Leu
            330                 335                 340

atg caa caa caa cag gaa gtg gct gga ctc tct aaa caa ttg gag cat     1289
Met Gln Gln Gln Gln Glu Val Ala Gly Leu Ser Lys Gln Leu Glu His
        345                 350                 355

gtc atg cat ttt tct aaa tgg gca gtt tcc agt ggc agc agt aca gca     1337
Val Met His Phe Ser Lys Trp Ala Val Ser Ser Gly Ser Ser Thr Ala
    360                 365                 370

tta ctt tat agc aaa cga ctg att aca tac cgg tta cgg cac ctc ctt     1385
Leu Leu Tyr Ser Lys Arg Leu Ile Thr Tyr Arg Leu Arg His Leu Leu
375                 380                 385                 390

cgt gca agg tgt gat gca tcc cca gtg acc aac aac acc atc caa ttt     1433
Arg Ala Arg Cys Asp Ala Ser Pro Val Thr Asn Asn Thr Ile Gln Phe
                395                 400                 405

cac tgt gat cct agt ttc tgg gct caa aat atc atc aac tta ggt tct     1481
His Cys Asp Pro Ser Phe Trp Ala Gln Asn Ile Ile Asn Leu Gly Ser
            410                 415                 420

tta gta atc gag gat aaa gag agc cag cca caa atg cct aag cag aat     1529
Leu Val Ile Glu Asp Lys Glu Ser Gln Pro Gln Met Pro Lys Gln Asn
        425                 430                 435

cct gtc gtg gaa cag aat tca cag cca cca agt ggt tta tca tca aac     1577
Pro Val Val Glu Gln Asn Ser Gln Pro Pro Ser Gly Leu Ser Ser Asn
    440                 445                 450

cag tta tcc aag ttc cca aca cag atc agc cta gct caa tta cgg ctc 1625
Gln Leu Ser Lys Phe Pro Thr Gln Ile Ser Leu Ala Gln Leu Arg Leu
455                 460                 465                 470

cag cat atg cag caa cag caa ccg cct cca cgt ttg ata aac ttt cag 1673
Gln His Met Gln Gln Gln Gln Pro Pro Pro Arg Leu Ile Asn Phe Gln
                475                 480                 485

aat cac agc ccc aaa ccc aat gga cca gtt ctt cct cct cat cct caa 1721
Asn His Ser Pro Lys Pro Asn Gly Pro Val Leu Pro Pro His Pro Gln
            490                 495                 500

caa ctg aga tat cca cca aac cag aac ata cca cga caa gca ata aag 1769
Gln Leu Arg Tyr Pro Pro Asn Gln Asn Ile Pro Arg Gln Ala Ile Lys
        505                 510                 515

cca aac ccc cta cag atg gct ttc ttg gct caa caa gcc ata aaa cag 1817
Pro Asn Pro Leu Gln Met Ala Phe Leu Ala Gln Gln Ala Ile Lys Gln
    520                 525                 530

tgg cag atc agc agt gga cag gga acc cca tca act acc aac agc aca 1865
Trp Gln Ile Ser Ser Gly Gln Gly Thr Pro Ser Thr Thr Asn Ser Thr
535                 540                 545                 550

tcc tct act cct tcc agc ccc acg att act agt gca gca gga tat gat 1913
Ser Ser Thr Pro Ser Ser Pro Thr Ile Thr Ser Ala Ala Gly Tyr Asp
                555                 560                 565

gga aag gct ttt ggt tca cct atg atc gat ttg agc tca cca gtg gga 1961
Gly Lys Ala Phe Gly Ser Pro Met Ile Asp Leu Ser Ser Pro Val Gly
            570                 575                 580

ggg tct tat aat ctt ccc tct ctt ccg gat att gac tgt tca agt act 2009
Gly Ser Tyr Asn Leu Pro Ser Leu Pro Asp Ile Asp Cys Ser Ser Thr
        585                 590                 595

att atg ctg gac aat att gtg agg aaa gat act aat ata gat cat ggc 2057
Ile Met Leu Asp Asn Ile Val Arg Lys Asp Thr Asn Ile Asp His Gly
    600                 605                 610

cag cca aga cca ccc tca aac aga acg gtc cag tca cca aat tca tca 2105
Gln Pro Arg Pro Pro Ser Asn Arg Thr Val Gln Ser Pro Asn Ser Ser
615                 620                 625                 630

gtg cca tct cca ggc ctt gca gga cct gtt act atg act agt gta cac 2153
Val Pro Ser Pro Gly Leu Ala Gly Pro Val Thr Met Thr Ser Val His
                635                 640                 645

ccc cca ata cgt tca cct agt gcc tcc agc gtt gga agc cga gga agc 2201
Pro Pro Ile Arg Ser Pro Ser Ala Ser Ser Val Gly Ser Arg Gly Ser
            650                 655                 660

tct ggc tct tcc agc aaa cca gca gga gct gac tct aca cac aaa gtc 2249
Ser Gly Ser Ser Ser Lys Pro Ala Gly Ala Asp Ser Thr His Lys Val
        665                 670                 675

cca gtg gtc atg ctg gag cca att cga ata aaa caa gaa aac agt gga 2297
Pro Val Val Met Leu Glu Pro Ile Arg Ile Lys Gln Glu Asn Ser Gly
    680                 685                 690

cca ccg gaa aat tat gat ttc cct gtt gtt ata gtg aag caa gaa tca 2345
Pro Pro Glu Asn Tyr Asp Phe Pro Val Val Ile Val Lys Gln Glu Ser
695                 700                 705                 710

gat gaa gaa tct agg cct caa aat gcc aat tat cca aga agc ata ctc 2393
Asp Glu Glu Ser Arg Pro Gln Asn Ala Asn Tyr Pro Arg Ser Ile Leu
                715                 720                 725

acc tcc ctg ctc tta aat agc agt cag agc tct act tct gag gag act 2441
Thr Ser Leu Leu Leu Asn Ser Ser Gln Ser Ser Thr Ser Glu Glu Thr
            730                 735                 740

gtg cta aga tca gat gcc cct gat agt aca gga gat caa cct gga ctt 2489
Val Leu Arg Ser Asp Ala Pro Asp Ser Thr Gly Asp Gln Pro Gly Leu
        745                 750                 755

cac cag gac aat tcc tca aat gga aag tct gaa tgg ttg gat cct tcc 2537
His Gln Asp Asn Ser Ser Asn Gly Lys Ser Glu Trp Leu Asp Pro Ser
    760                 765                 770

cag aag tca cct ctt cat gtt gga gag aca agg aaa gag gat gac ccc 2585
Gln Lys Ser Pro Leu His Val Gly Glu Thr Arg Lys Glu Asp Asp Pro
775                 780                 785                 790

aat gag gac tgg tgt gca gtt tgt caa aac gga ggg gaa ctc ctc tgc 2633
Asn Glu Asp Trp Cys Ala Val Cys Gln Asn Gly Gly Glu Leu Leu Cys
                795                 800                 805

tgt gaa aag tgc ccc aaa gta ttc cat ctt tct tgt cat gtg ccc aca 2681
Cys Glu Lys Cys Pro Lys Val Phe His Leu Ser Cys His Val Pro Thr
            810                 815                 820

ttg aca aat ttt cca agt gga gag tgg att tgc act ttc tgc cga gac 2729
Leu Thr Asn Phe Pro Ser Gly Glu Trp Ile Cys Thr Phe Cys Arg Asp
        825                 830                 835

tta tct aaa cca gaa gtt gaa tat gat tgt gat gct ccc agt cac aac 2777
Leu Ser Lys Pro Glu Val Glu Tyr Asp Cys Asp Ala Pro Ser His Asn
    840                 845                 850

tca gaa aaa aag aaa act gaa ggc ctt gtt aag tta aca cct ata gat 2825
Ser Glu Lys Lys Lys Thr Glu Gly Leu Val Lys Leu Thr Pro Ile Asp
855                 860                 865                 870

aaa agg aag tgt gag cgc cta ctt tta ttt ctt tac tgc cat gaa atg 2873
Lys Arg Lys Cys Glu Arg Leu Leu Leu Phe Leu Tyr Cys His Glu Met
                875                 880                 885

agc ctg gct ttt caa gac cct gtt cct cta act gtg cct gat tat tac 2921
Ser Leu Ala Phe Gln Asp Pro Val Pro Leu Thr Val Pro Asp Tyr Tyr
            890                 895                 900

aaa ata att aaa aat cca atg gat ttg tca acc atc aag aaa aga cta 2969
Lys Ile Ile Lys Asn Pro Met Asp Leu Ser Thr Ile Lys Lys Arg Leu
        905                 910                 915

caa gaa gat tat tcc atg tac tca aaa cct gaa gat ttt gta gct gat 3017
Gln Glu Asp Tyr Ser Met Tyr Ser Lys Pro Glu Asp Phe Val Ala Asp
    920                 925                 930

ttt aga ttg atc ttt caa aac tgt gct gaa ttc aat gag cct gat tca 3065
Phe Arg Leu Ile Phe Gln Asn Cys Ala Glu Phe Asn Glu Pro Asp Ser
935                 940                 945                 950

gaa gta gcc aat gct ggt ata aaa ctt gaa aat tat ttt gaa gaa ctt 3113
Glu Val Ala Asn Ala Gly Ile Lys Leu Glu Asn Tyr Phe Glu Glu Leu

955                 960                 965

cta aag aac ctc tat cca gaa aaa agg ttt ccc aaa cca gaa ttc agg      3161
Leu Lys Asn Leu Tyr Pro Glu Lys Arg Phe Pro Lys Pro Glu Phe Arg
            970                 975                 980

aat gaa tca gaa gat aat aaa ttt agt gat gat tca gat gat gac ttt      3209
Asn Glu Ser Glu Asp Asn Lys Phe Ser Asp Asp Ser Asp Asp Asp Phe
        985                 990                 995

gta cag  ccc cgg aag aaa cgc  ctc aaa agc att gaa  gaa cgc cag      3254
Val Gln  Pro Arg Lys Lys Arg  Leu Lys Ser Ile Glu  Glu Arg Gln
    1000                 1005                 1010

ttg ctt  aaa taa tatgcagcac cactagcttg tgctggtttt tagatttttt      3306
Leu Leu  Lys
    1015

tgttttcaaa aaaacatttg tcagtaattt aacatcacta caaaaagaag agtttgtgac      3366

tattctcatc tctgttttgg acgtttacta gactttgatt tccttaatag cccatttctg      3426

ttaacctctt atcactaaga aagaaaggaa agaaggagat gaatagaaga aagaaaatgg      3486

aaagaaggaa aaaaggagga tagaaaaagg atggaagaaa gaagcattga aaacaaagac      3546

attcttccca cttcttggat ttttaaacca cagtctggag tgatagctac tgtagaaagg      3606

aaatagactt tgtatgaact ctttaagttg aaaagtaaaa aatatatgtg gtttggatgt      3666

gtgctttaat tcagctttag aaattaatac cactacccgt gaattatatg gcctgacaat      3726

atgaattagg tgtactgtac tgaagaacag tactccacaa acatgggtgg taacaagagt      3786

tccatcccag gaggccaaac ggtgcaacag aagggtaggt tagatgctat taagaaggca      3846

cttaatagta catcatgtaa gatggcaact gtattaaaga aaaatccgga aaacaaaaa      3905

<210>  28
<211>  1016
<212>  PRT
<213>  Homo sapiens

<400>  28

Met Glu Val Ala Val Glu Lys Ala Val Ala Ala Ala Ala Ala Ala Ser
1               5                   10                  15

Ala Ala Ala Ser Gly Gly Pro Ser Ala Ala Pro Ser Gly Glu Asn Glu
            20                  25                  30

Ala Glu Ser Arg Gln Gly Pro Asp Ser Glu Arg Gly Gly Glu Ala Ala
        35                  40                  45

Arg Leu Asn Leu Leu Asp Thr Cys Ala Val Cys His Gln Asn Ile Gln
    50                  55                  60

Ser Arg Ala Pro Lys Leu Leu Pro Cys Leu His Ser Phe Cys Gln Arg

65                  70                  75                  80

Cys Leu Pro Ala Pro Gln Arg Tyr Leu Met Leu Pro Ala Pro Met Leu
                85                  90                  95

Gly Ser Ala Glu Thr Pro Pro Pro Val Pro Ala Pro Gly Ser Pro Val
            100                 105                 110

Ser Gly Ser Ser Pro Phe Ala Thr Gln Val Gly Val Ile Arg Cys Pro
        115                 120                 125

Val Cys Ser Gln Glu Cys Ala Glu Arg His Ile Ile Asp Asn Phe Phe
    130                 135                 140

Val Lys Asp Thr Thr Glu Val Pro Ser Ser Thr Val Glu Lys Ser Asn
145                 150                 155                 160

Gln Val Cys Thr Ser Cys Glu Asp Asn Ala Glu Ala Asn Gly Phe Cys
                165                 170                 175

Val Glu Cys Val Glu Trp Leu Cys Lys Thr Cys Ile Arg Ala His Gln
            180                 185                 190

Arg Val Lys Phe Thr Lys Asp His Thr Val Arg Gln Lys Glu Glu Val
        195                 200                 205

Ser Pro Glu Ala Val Gly Val Thr Ser Gln Arg Pro Val Phe Cys Pro
    210                 215                 220

Phe His Lys Lys Glu Gln Leu Lys Leu Tyr Cys Glu Thr Cys Asp Lys
225                 230                 235                 240

Leu Thr Cys Arg Asp Cys Gln Leu Leu Glu His Lys Glu His Arg Tyr
                245                 250                 255

Gln Phe Ile Glu Glu Ala Phe Gln Asn Gln Lys Val Ile Ile Asp Thr
            260                 265                 270

Leu Ile Thr Lys Leu Met Glu Lys Thr Lys Tyr Ile Lys Phe Thr Gly
        275                 280                 285

Asn Gln Ile Gln Asn Arg Ile Ile Glu Val Asn Gln Asn Gln Lys Gln
    290                 295                 300

Val Glu Gln Asp Ile Lys Val Ala Ile Phe Thr Leu Met Val Glu Ile
305                 310                 315                 320

Asn Lys Lys Gly Lys Ala Leu Leu His Gln Leu Glu Ser Leu Ala Lys
                325                 330                 335

Asp His Arg Met Lys Leu Met Gln Gln Gln Glu Val Ala Gly Leu
            340                 345                 350

Ser Lys Gln Leu Glu His Val Met His Phe Ser Lys Trp Ala Val Ser
        355                 360                 365

Ser Gly Ser Ser Thr Ala Leu Leu Tyr Ser Lys Arg Leu Ile Thr Tyr
    370                 375                 380

Arg Leu Arg His Leu Leu Arg Ala Arg Cys Asp Ala Ser Pro Val Thr
385                 390                 395                 400

Asn Asn Thr Ile Gln Phe His Cys Asp Pro Ser Phe Trp Ala Gln Asn
                405                 410                 415

Ile Ile Asn Leu Gly Ser Leu Val Ile Glu Asp Lys Glu Ser Gln Pro
            420                 425                 430

Gln Met Pro Lys Gln Asn Pro Val Val Glu Gln Asn Ser Gln Pro Pro
        435                 440                 445

Ser Gly Leu Ser Ser Asn Gln Leu Ser Lys Phe Pro Thr Gln Ile Ser
    450                 455                 460

Leu Ala Gln Leu Arg Leu Gln His Met Gln Gln Gln Pro Pro Pro
465                 470                 475                 480

Arg Leu Ile Asn Phe Gln Asn His Ser Pro Lys Pro Asn Gly Pro Val
                485                 490                 495

Leu Pro Pro His Pro Gln Gln Leu Arg Tyr Pro Pro Asn Gln Asn Ile
            500                 505                 510

Pro Arg Gln Ala Ile Lys Pro Asn Pro Leu Gln Met Ala Phe Leu Ala
        515                 520                 525

Gln Gln Ala Ile Lys Gln Trp Gln Ile Ser Ser Gly Gln Gly Thr Pro
    530                 535                 540

Ser Thr Thr Asn Ser Thr Ser Ser Thr Pro Ser Ser Pro Thr Ile Thr
545                 550                 555                 560

Ser Ala Ala Gly Tyr Asp Gly Lys Ala Phe Gly Ser Pro Met Ile Asp
                565                 570                 575

Leu Ser Ser Pro Val Gly Gly Ser Tyr Asn Leu Pro Ser Leu Pro Asp
580 585 590

Ile Asp Cys Ser Ser Thr Ile Met Leu Asp Asn Ile Val Arg Lys Asp
                595                 600                 605

Thr Asn Ile Asp His Gly Gln Pro Arg Pro Pro Ser Asn Arg Thr Val
            610                 615                 620

Gln Ser Pro Asn Ser Ser Val Pro Ser Pro Gly Leu Ala Gly Pro Val
625 630 635 640

Thr Met Thr Ser Val His Pro Pro Ile Arg Ser Pro Ser Ala Ser Ser
645 650 655

Val Gly Ser Arg Gly Ser Ser Gly Ser Ser Ser Lys Pro Ala Gly Ala
660 665 670

Asp Ser Thr His Lys Val Pro Val Val Met Leu Glu Pro Ile Arg Ile
                675                 680                 685

Lys Gln Glu Asn Ser Gly Pro Pro Glu Asn Tyr Asp Phe Pro Val Val
            690                 695                 700

Ile Val Lys Gln Glu Ser Asp Glu Glu Ser Arg Pro Gln Asn Ala Asn
705 710 715 720

Tyr Pro Arg Ser Ile Leu Thr Ser Leu Leu Leu Asn Ser Ser Gln Ser
725 730 735

Ser Thr Ser Glu Glu Thr Val Leu Arg Ser Asp Ala Pro Asp Ser Thr
740 745 750

Gly Asp Gln Pro Gly Leu His Gln Asp Asn Ser Ser Asn Gly Lys Ser
                755                 760                 765

Glu Trp Leu Asp Pro Ser Gln Lys Ser Pro Leu His Val Gly Glu Thr
            770                 775                 780

Arg Lys Glu Asp Asp Pro Asn Glu Asp Trp Cys Ala Val Cys Gln Asn
785 790 795 800

Gly Gly Glu Leu Leu Cys Cys Glu Lys Cys Pro Lys Val Phe His Leu
805 810 815

Ser Cys His Val Pro Thr Leu Thr Asn Phe Pro Ser Gly Glu Trp Ile
820 825 830

Cys Thr Phe Cys Arg Asp Leu Ser Lys Pro Glu Val Glu Tyr Asp Cys
                835                 840                 845

Asp Ala Pro Ser His Asn Ser Glu Lys Lys Lys Thr Glu Gly Leu Val
            850                 855                 860

Lys Leu Thr Pro Ile Asp Lys Arg Lys Cys Glu Arg Leu Leu Leu Phe
865 870 875 880

Leu Tyr Cys His Glu Met Ser Leu Ala Phe Gln Asp Pro Val Pro Leu
885 890 895

Thr Val Pro Asp Tyr Tyr Lys Ile Ile Lys Asn Pro Met Asp Leu Ser
900 905 910

Thr Ile Lys Lys Arg Leu Gln Glu Asp Tyr Ser Met Tyr Ser Lys Pro
                915                 920                 925

Glu Asp Phe Val Ala Asp Phe Arg Leu Ile Phe Gln Asn Cys Ala Glu
            930                 935                 940

Phe Asn Glu Pro Asp Ser Glu Val Ala Asn Ala Gly Ile Lys Leu Glu
945 950 955 960

Asn Tyr Phe Glu Glu Leu Leu Lys Asn Leu Tyr Pro Glu Lys Arg Phe
965 970 975

Pro Lys Pro Glu Phe Arg Asn Glu Ser Glu Asp Asn Lys Phe Ser Asp
980 985 990

Asp Ser Asp Asp Asp Phe Val Gln Pro Arg Lys Lys Arg Leu Lys Ser
                995                 1000                1005

Ile Glu Glu Arg Gln Leu Leu Lys
1010 1015

<210> 29
<211> 2949
<212> DNA
<213> Homo sapiens

<220>
<221> CDS
<222> (62)..(2362)

<400> 29
cgcctccctt ccccctcccc gcccgacagc ggccgctcgg gccccggctc tcggttataa 60
g atg gcg gcg ctg agc ggt ggc ggt ggt ggc ggc gcg gag ccg ggc cag 109
gct ctg ttc aac ggg gac atg gag ccc gag gcc ggc gcc ggc gcc ggc 157
gcc gcg gcc tct tcg gct gcg gac cct gcc att ccg gag gag gtg tgg 205
aat atc aaa caa atg att aag ttg aca cag gaa cat ata gag gcc cta 253
ttg gac aaa ttt ggt ggg gag cat aat cca cca tca ata tat ctg gag 301
gcc tat gaa gaa tac acc agc aag cta gat gca ctc caa caa aga gaa 349
caa cag tta ttg gaa tct ctg ggg aac gga act gat ttt tct gtt tct 397
agc tct gca tca atg gat acc gtt aca tct tct tcc tct tct agc ctt 445
tca gtg cta cct tca tct ctt tca gtt ttt caa aat ccc aca gat gtg 493
gca cgg agc aac ccc aag tca cca caa aaa cct atc gtt aga gtc ttc 541
ctg ccc aac aaa cag agg aca gtg gta cct gca agg tgt gga gtt aca 589
gtc cga gac agt cta aag aaa gca ctg atg atg aga ggt cta atc cca 637
gag tgc tgt gct gtt tac aga att cag gat gga gag aag aaa cca att 685
ggt tgg gac act gat att tcc tgg ctt act gga gaa gaa ttg cat gtg 733
gaa gtg ttg gag aat gtt cca ctt aca aca cac aac ttt gta cga aaa 781
acg ttt ttc acc tta gca ttt tgt gac ttt tgt cga aag ctg ctt ttc 829
cag ggt ttc cgc tgt caa aca tgt ggt tat aaa ttt cac cag cgt tgt 877
agt aca gaa gtt cca ctg atg tgt gtt aat tat gac caa ctt gat ttg 925
ctg ttt gtc tcc aag ttc ttt gaa cac cac cca ata cca cag gaa gag 973
gcg tcc tta gca gag act gcc cta aca tct gga tca tcc cct tcc gca 1021
ccc gcc tcg gac tct att ggg ccc caa att ctc acc agt ccg tct cct 1069
tca aaa tcc att cca att cca cag ccc ttc cga cca gca gat gaa gat 1117
cat cga aat caa ttt ggg caa cga gac cga tcc tca tca gct ccc aat 1165
gtg cat ata aac aca ata gaa cct gtc aat att gat gac ttg att aga 1213
gac caa gga ttt cgt ggt gat gga gga tca acc aca ggt ttg tct gct 1261
acc ccc cct gcc tca tta cct ggc tca cta act aac gtg aaa gcc tta 1309
cag aaa tct cca gga cct cag cga gaa agg aag tca tct tca tcc tca 1357
gaa gac agg aat cga atg aaa aca ctt ggt aga cgg gac tcg agt gat 1405
gat tgg gag att cct gat ggg cag att aca gtg gga caa aga att gga 1453
tct gga tca ttt gga aca gtc tac aag gga aag tgg cat ggt gat gtg 1501
gca gtg aaa atg ttg aat gtg aca gca cct aca cct cag cag tta caa 1549
gcc ttc aaa aat gaa gta gga gta ctc agg aaa aca cga cat gtg aat 1597
atc cta ctc ttc atg ggc tat tcc aca aag cca caa ctg gct att gtt      1645
acc cag tgg tgt gag ggc tcc agc ttg tat cac cat ctc cat atc att      1693
gag acc aaa ttt gag atg atc aaa ctt ata gat att gca cga cag act      1741
gca cag ggc atg gat tac tta cac gcc aag tca atc atc cac aga gac      1789
ctc aag agt aat aat ata ttt ctt cat gaa gac ctc aca gta aaa ata      1837
ggt gat ttt ggt cta gct aca gtg aaa tct cga tgg agt ggg tcc cat      1885
cag ttt gaa cag ttg tct gga tcc att ttg tgg atg gca cca gaa gtc      1933
atc aga atg caa gat aaa aat cca tac agc ttt cag tca gat gta tat      1981
gca ttt gga att gtt ctg tat gaa ttg atg act gga cag tta cct tat      2029
tca aac atc aac aac agg gac cag ata att ttt atg gtg gga cga gga      2077
tac ctg tct cca gat ctc agt aag gta cgg agt aac tgt cca aaa gcc      2125
atg aag aga tta atg gca gag tgc ctc aaa aag aaa aga gat gag aga      2173
cca ctc ttt ccc caa att ctc gcc tct att gag ctg ctg gcc cgc tca      2221
ttg cca aaa att cac cgc agt gca tca gaa ccc tcc ttg aat cgg gct      2269
ggt ttc caa aca gag gat ttt agt cta tat gct tgt gct tct cca aaa      2317
aca ccc atc cag gca ggg gga tat ggt gcg ttt cct gtc cac tga          2362
aacaaatgag tgagagagtt caggagagta gcaacaaaag gaaaataaat gaacatatgt    2422
ttgcttatat gttaaattga ataaaatact ctcttttttt ttaaggtgaa ccaaagaaca    2482
cttgtgtggt taaagactag atataatttt tccccaaact aaaatttata cttaacattg    2542
gatttttaac atccaagggt taaaatacat agacattgct aaaaattggc agagcctctt    2602
ctagaggctt tactttctgt tccgggtttg tatcattcac ttggttattt taagtagtaa    2662
acttcagttt ctcatgcaac ttttgttgcc agctatcaca tgtccactag ggactccaga    2722
agaagaccct acctatgcct gtgtttgcag gtgagaagtt ggcagtcggt tagcctgggt    2782
tagataaggc aaactgaaca gatctaattt aggaagtcag tagaatttaa taattctatt    2842
attattctta ataatttttc tataactatt tctttttata acaatttgga aaatgtggat    2902
gtcttttatt tccttgaagc aataaactaa gtttcttttt ataaaaa                  2949

<210>  30
<211>  766
<212>  PRT
<213>  Homo sapiens

<400>  30

<210>  31
<211>  1506
<212>  DNA
<213>  Homo sapiens

<220>
<221>  CDS
<222>  (188)..(886)

<400>  31
ctgcctgggg agcccccccg ccccacatcc tgccccgcaa aaggcagctt caccaaagtg      60

gggtatttcc agcctttgta gctttcactt ccacatctac caagtgggcg gagtggcctt     120

ctgtggacga atcagattcc tctccagcac cgactttaag aggcgagccg gggggtcagg     180

gtcccag atg cac agg agg aga agc agg agc tgt cgg gaa gat cag aag       229
        Met His Arg Arg Arg Ser Arg Ser Cys Arg Glu Asp Gln Lys
        1               5                   10

cca gtc atg gat gac cag cgc gac ctt atc tcc aac aat gag caa ctg       277
Pro Val Met Asp Asp Gln Arg Asp Leu Ile Ser Asn Asn Glu Gln Leu
15                  20                  25                  30

ccc atg ctg ggc cgg cgc cct ggg gcc ccg gag agc aag tgc agc cgc       325
Pro Met Leu Gly Arg Arg Pro Gly Ala Pro Glu Ser Lys Cys Ser Arg
                35                  40                  45

gga gcc ctg tac aca ggc ttt tcc atc ctg gtg act ctg ctc ctc gct 373
Gly Ala Leu Tyr Thr Gly Phe Ser Ile Leu Val Thr Leu Leu Leu Ala
            50                  55                  60

ggc cag gcc acc acc gcc tac ttc ctg tac cag cag cag ggc cgg ctg 421
Gly Gln Ala Thr Thr Ala Tyr Phe Leu Tyr Gln Gln Gln Gly Arg Leu
        65                  70                  75

gac aaa ctg aca gtc acc tcc cag aac ctg cag ctg gag aac ctg cgc 469
Asp Lys Leu Thr Val Thr Ser Gln Asn Leu Gln Leu Glu Asn Leu Arg
    80                  85                  90

atg aag ctt ccc aag cct ccc aag cct gtg agc aag atg cgc atg gcc 517
Met Lys Leu Pro Lys Pro Pro Lys Pro Val Ser Lys Met Arg Met Ala
95                  100                 105                 110

acc ccg ctg ctg atg cag gcg ctg ccc atg gga gcc ctg ccc cag ggg 565
Thr Pro Leu Leu Met Gln Ala Leu Pro Met Gly Ala Leu Pro Gln Gly
                115                 120                 125

ccc atg cag aat gcc acc aag tat ggc aac atg aca gag gac cat gtg 613
Pro Met Gln Asn Ala Thr Lys Tyr Gly Asn Met Thr Glu Asp His Val
            130                 135                 140

atg cac ctg ctc cag aat gct gac ccc ctg aag gtg tac ccg cca ctg 661
Met His Leu Leu Gln Asn Ala Asp Pro Leu Lys Val Tyr Pro Pro Leu
        145                 150                 155

aag ggg agc ttc ccg gag aac ctg aga cac ctt aag aac acc atg gag 709
Lys Gly Ser Phe Pro Glu Asn Leu Arg His Leu Lys Asn Thr Met Glu
    160                 165                 170

acc ata gac tgg aag gtc ttt gag agc tgg atg cac cat tgg ctc ctg 757
Thr Ile Asp Trp Lys Val Phe Glu Ser Trp Met His His Trp Leu Leu
175                 180                 185                 190

ttt gaa atg agc agg cac tcc ttg gag caa aag ccc act gac gct cca 805
Phe Glu Met Ser Arg His Ser Leu Glu Gln Lys Pro Thr Asp Ala Pro
                195                 200                 205

ccg aaa gag tca ctg gaa ctg gag gac ccg tct tct ggg ctg ggt gtg 853
Pro Lys Glu Ser Leu Glu Leu Glu Asp Pro Ser Ser Gly Leu Gly Val
            210                 215                 220

acc aag cag gat ctg ggc cca gtc ccc atg tga gagcagcaga ggcggtcttc 906
Thr Lys Gln Asp Leu Gly Pro Val Pro Met
        225                 230

aacatcctgc cagccccaca cagctacagc tttcttgctc ccttcagccc ccagcccctc 966

ccccatctcc caccctgtac ctcatcccat gagaccctgg tgcctggctc tttcgtcacc 1026

cttggacaag acaaaccaag tcggaacagc agataacaat gcagcaaggc cctgctgccc 1086

aatctccatc tgtcaacagg ggcgtgaggt cccaggaagt ggccaaaagc tagacagatc 1146

cccgttcctg acatcacagc agcctccaac acaaggctcc aagacctagg ctcatggacg 1206

agatgggaag gcacagggag aagggataac cctacaccca gaccccaggc tggacatgct 1266

gactgtcctc tcccctccag cctttggcct tggcttttct agcctattta cctgcaggct 1326

gagccactct cttccctttc cccagcatca ctccccaagg aagagccaat gttttccacc 1386

cataatcctt tctgccgacc cctagttccc tctgctcagc caagcttgtt atcagctttc    1446

agggccatgg ttcacattag aataaaaggt agtaattaga acaaaaaaaa aaaaaaaaaa    1506

<210>  32
<211>  232
<212>  PRT
<213>  Homo sapiens

<400>  32

Met His Arg Arg Arg Ser Arg Ser Cys Arg Glu Asp Gln Lys Pro Val
1               5                   10                  15

Met Asp Asp Gln Arg Asp Leu Ile Ser Asn Asn Glu Gln Leu Pro Met
            20                  25                  30

Leu Gly Arg Arg Pro Gly Ala Pro Glu Ser Lys Cys Ser Arg Gly Ala
        35                  40                  45

Leu Tyr Thr Gly Phe Ser Ile Leu Val Thr Leu Leu Leu Ala Gly Gln
    50                  55                  60

Ala Thr Thr Ala Tyr Phe Leu Tyr Gln Gln Gln Gly Arg Leu Asp Lys
65                  70                  75                  80

Leu Thr Val Thr Ser Gln Asn Leu Gln Leu Glu Asn Leu Arg Met Lys
                85                  90                  95

Leu Pro Lys Pro Pro Lys Pro Val Ser Lys Met Arg Met Ala Thr Pro
            100                 105                 110

Leu Leu Met Gln Ala Leu Pro Met Gly Ala Leu Pro Gln Gly Pro Met
        115                 120                 125

Gln Asn Ala Thr Lys Tyr Gly Asn Met Thr Glu Asp His Val Met His
    130                 135                 140

Leu Leu Gln Asn Ala Asp Pro Leu Lys Val Tyr Pro Pro Leu Lys Gly
145                 150                 155                 160

Ser Phe Pro Glu Asn Leu Arg His Leu Lys Asn Thr Met Glu Thr Ile
                165                 170                 175

Asp Trp Lys Val Phe Glu Ser Trp Met His His Trp Leu Leu Phe Glu
            180                 185                 190

Met Ser Arg His Ser Leu Glu Gln Lys Pro Thr Asp Ala Pro Pro Lys
        195                 200                 205

Glu Ser Leu Glu Leu Glu Asp Pro Ser Ser Gly Leu Gly Val Thr Lys
    210                 215                 220

Gln Asp Leu Gly Pro Val Pro Met
225                 230

<210>  33
<211>  1638
<212>  DNA
<213>  Homo sapiens

<220>
<221>  CDS
<222>  (518)..(1141)

<400>  33
gaggccaggg gagggtgcga aggaggcgcc tgcctccaac ctgcgggcgg gaggtgggtg      60

gctgcggggc aattgaaaaa gagccggcga ggagttcccc gaaacttgtt ggaactccgg     120

gctcgcgcgg aggccaggag ctgagcggcg gcggctgccg gacgatggga gcgtgagcag     180

gacggtgata acctctcccc gatcgggttg cgagggcgcc gggcagaggc caggacgcga     240

gccgccagcg gtgggaccca tcgacgactt cccggggcga caggagcagc cccgagagcc     300

agggcgagcg cccgttccag gtggccggac cgcccgccgc gtccgcgccg cgctccctgc     360

aggcaacggg agacgccccc gcgcagcgcg agcgcctcag cgcggccgct cgctctcccc     420

ctcgagggac aaactttccc caaacccgat ccgagccctt ggaccaaact cgcctgcgcc     480

gagagccgtc cgcgtagagc gctccgtctc cggcgag atg tcc gag cgc aaa gaa      535
                                        Met Ser Glu Arg Lys Glu
                                        1               5

ggc aga ggc aaa ggg aag ggc aag aag aag gag cga ggc tcc ggc aag      583
Gly Arg Gly Lys Gly Lys Gly Lys Lys Lys Glu Arg Gly Ser Gly Lys
            10                  15                  20

aag ccg gag tcc gcg gcg ggc agc cag agc cca gcc ttg cct ccc cga      631
Lys Pro Glu Ser Ala Ala Gly Ser Gln Ser Pro Ala Leu Pro Pro Arg
        25                  30                  35

ttg aaa gag atg aaa agc cag gaa tcg gct gca ggt tcc aaa cta gtc      679
Leu Lys Glu Met Lys Ser Gln Glu Ser Ala Ala Gly Ser Lys Leu Val
    40                  45                  50

ctt cgg tgt gaa acc agt tct gaa tac tcc tct ctc aga ttc aag tgg      727
Leu Arg Cys Glu Thr Ser Ser Glu Tyr Ser Ser Leu Arg Phe Lys Trp
55                  60                  65                  70

ttc aag aat ggg aat gaa ttg aat cga aaa aac aaa cca caa aat atc      775
Phe Lys Asn Gly Asn Glu Leu Asn Arg Lys Asn Lys Pro Gln Asn Ile
                75                  80                  85

aag ata caa aaa aag cca ggg aag tca gaa ctt cgc att aac aaa gca      823
Lys Ile Gln Lys Lys Pro Gly Lys Ser Glu Leu Arg Ile Asn Lys Ala

                    90                  95                  100

tca ctg gct gat tct gga gag tat atg tgc aaa gtg atc agc aaa tta       871
Ser Leu Ala Asp Ser Gly Glu Tyr Met Cys Lys Val Ile Ser Lys Leu
        105                 110                 115

gga aat gac agt gcc tct gcc aat atc acc atc gtg gaa tca aac gct       919
Gly Asn Asp Ser Ala Ser Ala Asn Ile Thr Ile Val Glu Ser Asn Ala
    120                 125                 130

aca tct aca tcc acc act ggg aca agc cat ctt gta aaa tgt gcg gag       967
Thr Ser Thr Ser Thr Thr Gly Thr Ser His Leu Val Lys Cys Ala Glu
135                 140                 145                 150

aag gag aaa act ttc tgt gtg aat gga ggg gag tgc ttc atg gtg aaa      1015
Lys Glu Lys Thr Phe Cys Val Asn Gly Gly Glu Cys Phe Met Val Lys
                155                 160                 165

gac ctt tca aac ccc tcg aga tac ttg tgc aag tgc cca aat gag ttt      1063
Asp Leu Ser Asn Pro Ser Arg Tyr Leu Cys Lys Cys Pro Asn Glu Phe
            170                 175                 180

act ggt gat cgc tgc caa aac tac gta atg gcc agc ttc tac agt acg      1111
Thr Gly Asp Arg Cys Gln Asn Tyr Val Met Ala Ser Phe Tyr Ser Thr
        185                 190                 195

tcc act ccc ttt ctg tct ctg cct gaa tag gagcatgctc agttggtgct        1161
Ser Thr Pro Phe Leu Ser Leu Pro Glu
    200                 205

gctttcttgt tgctgcatct cccctcagat tccacctaga gctagatgtg tcttaccaga    1221

tctaatattg actgcctctg cctgtcgcat gagaacatta acaaaagcaa ttgtattact    1281

tcctctgttc gcgactagtt ggctctgaga tactaatagg tgtgtgaggc tccggatgtt    1341

tctggaattg atattgaatg atgtgataca aattgatagt caatatcaag cagtgaaata    1401

tgataataaa ggcatttcaa agtctcactt ttattgataa aataaaaatc attctactga    1461

acagtccatc ttctttatac aatgaccaca tcctgaaaag ggtgttgcta agctgtaacc    1521

gatatgcact tgaaatgatg gtaagttaat tttgattcag aatgtgttat ttgtcacaaa    1581

taaacataat aaaaggagtt cagatgtttt tcttcattaa ccaaaaaaaa aaaaaaa       1638


<210>  34
<211>  207
<212>  PRT
<213>  Homo sapiens

<400>  34

Met Ser Glu Arg Lys Glu Gly Arg Gly Lys Gly Lys Gly Lys Lys Lys
1               5                   10                  15


Glu Arg Gly Ser Gly Lys Lys Pro Glu Ser Ala Ala Gly Ser Gln Ser
            20                  25                  30


Pro Ala Leu Pro Pro Arg Leu Lys Glu Met Lys Ser Gln Glu Ser Ala

35                  40                  45

Ala Gly Ser Lys Leu Val Leu Arg Cys Glu Thr Ser Ser Glu Tyr Ser
    50                  55                  60

Ser Leu Arg Phe Lys Trp Phe Lys Asn Gly Asn Glu Leu Asn Arg Lys
65                  70                  75                  80

Asn Lys Pro Gln Asn Ile Lys Ile Gln Lys Lys Pro Gly Lys Ser Glu
                85                  90                  95

Leu Arg Ile Asn Lys Ala Ser Leu Ala Asp Ser Gly Glu Tyr Met Cys
            100                 105                 110

Lys Val Ile Ser Lys Leu Gly Asn Asp Ser Ala Ser Ala Asn Ile Thr
        115                 120                 125

Ile Val Glu Ser Asn Ala Thr Ser Thr Ser Thr Thr Gly Thr Ser His
    130                 135                 140

Leu Val Lys Cys Ala Glu Lys Glu Lys Thr Phe Cys Val Asn Gly Gly
145                 150                 155                 160

Glu Cys Phe Met Val Lys Asp Leu Ser Asn Pro Ser Arg Tyr Leu Cys
                165                 170                 175

Lys Cys Pro Asn Glu Phe Thr Gly Asp Arg Cys Gln Asn Tyr Val Met
            180                 185                 190

Ala Ser Phe Tyr Ser Thr Ser Thr Pro Phe Leu Ser Leu Pro Glu
        195                 200                 205

<210>  35
<211>  1625
<212>  DNA
<213>  Homo sapiens

<220>
<221>  CDS
<222>  (1)..(1125)

<400>  35
atg gag cta cag cct cct gaa gcc tcg atc gcc gtc gtg tcg att ccg      48
Met Glu Leu Gln Pro Pro Glu Ala Ser Ile Ala Val Val Ser Ile Pro
1               5                   10                  15

cgc cag ttg cct ggc tca cat tcg gag gct ggt gtc cag ggt ctc agc      96
Arg Gln Leu Pro Gly Ser His Ser Glu Ala Gly Val Gln Gly Leu Ser
            20                  25                  30

gcg ggg gac gac tca gag acg ggg tct gac tgt gtt acc cag gct ggt      144

Ala Gly Asp Asp Ser Glu Thr Gly Ser Asp Cys Val Thr Gln Ala Gly
        35                  40                  45

ctt caa ctc ttg gcc tca agt gat cct cct gcc tta gct tcc aag aat 192
Leu Gln Leu Leu Ala Ser Ser Asp Pro Pro Ala Leu Ala Ser Lys Asn
    50                  55                  60

gct gag gtt aca gta gaa acg ggg ttt cac cat gtt agc cag gct gat 240
Ala Glu Val Thr Val Glu Thr Gly Phe His His Val Ser Gln Ala Asp
65                  70                  75                  80

att gaa ttc ctg acc tca att gat ccg act gcc tcg gcc tcc gga agt 288
Ile Glu Phe Leu Thr Ser Ile Asp Pro Thr Ala Ser Ala Ser Gly Ser
                85                  90                  95

gct ggg att aca ggc acc atg agc cag gac acc gag gtg gat atg aag 336
Ala Gly Ile Thr Gly Thr Met Ser Gln Asp Thr Glu Val Asp Met Lys
            100                 105                 110

gag gtg gag ctg aat gag tta gag ccc gag aag cag ccg atg aac gcg 384
Glu Val Glu Leu Asn Glu Leu Glu Pro Glu Lys Gln Pro Met Asn Ala
        115                 120                 125

gcg tct ggg gcg gcc atg tcc ctg gcg gga gcc gag aag aat ggt ctg 432
Ala Ser Gly Ala Ala Met Ser Leu Ala Gly Ala Glu Lys Asn Gly Leu
    130                 135                 140

gtg aag atc aag gtg gcg gaa gac gag gcg gag gcg gca gcc gcg gct 480
Val Lys Ile Lys Val Ala Glu Asp Glu Ala Glu Ala Ala Ala Ala Ala
145                 150                 155                 160

aag ttc acg ggc ctg tcc aag gag gag ctg ctg aag gtg gca ggc agc 528
Lys Phe Thr Gly Leu Ser Lys Glu Glu Leu Leu Lys Val Ala Gly Ser
                165                 170                 175

ccc ggc tgg gta cgc acc cgc tgg gca ctg ctg ctg ctc ttc tgg ctc 576
Pro Gly Trp Val Arg Thr Arg Trp Ala Leu Leu Leu Leu Phe Trp Leu
            180                 185                 190

ggc tgg ctc ggc atg ctt gct ggt gcc gtg gtc ata atc gtg cga gcg 624
Gly Trp Leu Gly Met Leu Ala Gly Ala Val Val Ile Ile Val Arg Ala
        195                 200                 205

ccg cgt tgt cgc gag cta ccg gcg cag aag tgg tgg cac acg ggc gcc 672
Pro Arg Cys Arg Glu Leu Pro Ala Gln Lys Trp Trp His Thr Gly Ala
    210                 215                 220

ctc tac cgc atc ggc gac ctt cag gcc ttc cag ggc cac ggc gcg ggc 720
Leu Tyr Arg Ile Gly Asp Leu Gln Ala Phe Gln Gly His Gly Ala Gly
225                 230                 235                 240

aac ctg gcg ggt ctg aag ggg cgt ctc gat tac ctg agc tct ctg aag 768
Asn Leu Ala Gly Leu Lys Gly Arg Leu Asp Tyr Leu Ser Ser Leu Lys
                245                 250                 255

gtg aag ggc ctt gtg ctg ggt cca att cac aag aac cag aag gat gat 816
Val Lys Gly Leu Val Leu Gly Pro Ile His Lys Asn Gln Lys Asp Asp
            260                 265                 270

gtc gct cag act gac ttg ctg cag atc gac ccc aat ttt ggc tcc aag 864
Val Ala Gln Thr Asp Leu Leu Gln Ile Asp Pro Asn Phe Gly Ser Lys
        275                 280                 285

gaa gat ttt gac agt ctc ttg caa tcg gct aaa aaa aag act aca tct      912
Glu Asp Phe Asp Ser Leu Leu Gln Ser Ala Lys Lys Lys Thr Thr Ser
    290                 295                 300

aca tcc acc act ggg aca agc cat ctt gta aaa tgt gcg gag aag gag      960
Thr Ser Thr Thr Gly Thr Ser His Leu Val Lys Cys Ala Glu Lys Glu
305                 310                 315                 320

aaa act ttc tgt gtg aat gga ggg gag tgc ttc atg gtg aaa gac ctt      1008
Lys Thr Phe Cys Val Asn Gly Gly Glu Cys Phe Met Val Lys Asp Leu
                325                 330                 335

tca aac ccc tcg aga tac ttg tgc aag tgc cca aat gag ttt act ggt      1056
Ser Asn Pro Ser Arg Tyr Leu Cys Lys Cys Pro Asn Glu Phe Thr Gly
            340                 345                 350

gat cgc tgc caa aac tac gta atg gcc agc ttc tac agt acg tcc act      1104
Asp Arg Cys Gln Asn Tyr Val Met Ala Ser Phe Tyr Ser Thr Ser Thr
        355                 360                 365

ccc ttt ctg tct ctg cct gaa taggagcatg ctcagttggt gctgctttct      1155
Pro Phe Leu Ser Leu Pro Glu
    370                 375

tgttgctgca tctcccctca gattccacct agagctagat gtgtcttacc agatctaata      1215

ttgactgcct ctgcctgtcg catgagaaca ttaacaaaag caattgtatt acttcctctg      1275

ttcgcgacta gttggctctg agatactaat aggtgtgtga ggctccggat gtttctggaa      1335

ttgatattga atgatgtgat acaaattgat agtcaatatc aagcagtgaa atatgataat      1395

aaaggcattt caaagtctca cttttattga taaaataaaa atcattctac tgaacagtcc      1455

atcttcttta tacaatgacc acatcctgaa aagggtgttg ctaagctgta accgatatgc      1515

acttgaaatg atggtaagtt aattttgatt cagaatgtgt tatttgtcac aaataaacat      1575

aataaaagga gttcagatgt ttttcttcat taaccaaaaa aaaaaaaaaa      1625

<210>  36
<211>  375
<212>  PRT
<213>  Homo sapiens

<400>  36

Met Glu Leu Gln Pro Pro Glu Ala Ser Ile Ala Val Val Ser Ile Pro
1               5                   10                  15

Arg Gln Leu Pro Gly Ser His Ser Glu Ala Gly Val Gln Gly Leu Ser
            20                  25                  30

Ala Gly Asp Asp Ser Glu Thr Gly Ser Asp Cys Val Thr Gln Ala Gly
        35                  40                  45

Leu Gln Leu Leu Ala Ser Ser Asp Pro Pro Ala Leu Ala Ser Lys Asn
    50                  55                  60

Ala Glu Val Thr Val Glu Thr Gly Phe His His Val Ser Gln Ala Asp
65                  70                  75                  80

Ile Glu Phe Leu Thr Ser Ile Asp Pro Thr Ala Ser Ala Ser Gly Ser
                85                  90                  95

Ala Gly Ile Thr Gly Thr Met Ser Gln Asp Thr Glu Val Asp Met Lys
            100                 105                 110

Glu Val Glu Leu Asn Glu Leu Glu Pro Glu Lys Gln Pro Met Asn Ala
        115                 120                 125

Ala Ser Gly Ala Ala Met Ser Leu Ala Gly Ala Glu Lys Asn Gly Leu
    130                 135                 140

Val Lys Ile Lys Val Ala Glu Asp Glu Ala Glu Ala Ala Ala Ala Ala
145                 150                 155                 160

Lys Phe Thr Gly Leu Ser Lys Glu Glu Leu Leu Lys Val Ala Gly Ser
                165                 170                 175

Pro Gly Trp Val Arg Thr Arg Trp Ala Leu Leu Leu Leu Phe Trp Leu
            180                 185                 190

Gly Trp Leu Gly Met Leu Ala Gly Ala Val Val Ile Ile Val Arg Ala
        195                 200                 205

Pro Arg Cys Arg Glu Leu Pro Ala Gln Lys Trp Trp His Thr Gly Ala
    210                 215                 220

Leu Tyr Arg Ile Gly Asp Leu Gln Ala Phe Gln Gly His Gly Ala Gly
225                 230                 235                 240

Asn Leu Ala Gly Leu Lys Gly Arg Leu Asp Tyr Leu Ser Ser Leu Lys
                245                 250                 255

Val Lys Gly Leu Val Leu Gly Pro Ile His Lys Asn Gln Lys Asp Asp
            260                 265                 270

Val Ala Gln Thr Asp Leu Leu Gln Ile Asp Pro Asn Phe Gly Ser Lys
        275                 280                 285

Glu Asp Phe Asp Ser Leu Leu Gln Ser Ala Lys Lys Lys Thr Thr Ser
    290                 295                 300

Thr Ser Thr Thr Gly Thr Ser His Leu Val Lys Cys Ala Glu Lys Glu
305                 310                 315                 320

Lys Thr Phe Cys Val Asn Gly Gly Glu Cys Phe Met Val Lys Asp Leu
                325                 330                 335

Ser Asn Pro Ser Arg Tyr Leu Cys Lys Cys Pro Asn Glu Phe Thr Gly
            340                 345                 350

Asp Arg Cys Gln Asn Tyr Val Met Ala Ser Phe Tyr Ser Thr Ser Thr
        355                 360                 365

Pro Phe Leu Ser Leu Pro Glu
    370                 375

<210>  37
<211>  20
<212>  DNA
<213>  Artificial Sequence

<220>
<223>  Primer sequence

<400>  37
cagaaggatg atgtcgctca                                                  20

<210>  38
<211>  1896
<212>  DNA
<213>  Homo sapiens

<220>
<221>  CDS
<222>  (1)..(1893)

<400>  38
atg gag cta cag cct cct gaa gcc tcg atc gcc gtc gtg tcg att ccg       48
Met Glu Leu Gln Pro Pro Glu Ala Ser Ile Ala Val Val Ser Ile Pro
1               5                   10                  15

cgc cag ttg cct ggc tca cat tcg gag gct ggt gtc cag ggt ctc agc       96
Arg Gln Leu Pro Gly Ser His Ser Glu Ala Gly Val Gln Gly Leu Ser
            20                  25                  30

gcg ggg gac gac tca gag acg ggg tct gac tgt gtt acc cag gct ggt      144
Ala Gly Asp Asp Ser Glu Thr Gly Ser Asp Cys Val Thr Gln Ala Gly
        35                  40                  45

ctt caa ctc ttg gcc tca agt gat cct cct gcc tta gct tcc aag aat      192
Leu Gln Leu Leu Ala Ser Ser Asp Pro Pro Ala Leu Ala Ser Lys Asn
    50                  55                  60

gct gag gtt aca gta gaa acg ggg ttt cac cat gtt agc cag gct gat      240
Ala Glu Val Thr Val Glu Thr Gly Phe His His Val Ser Gln Ala Asp
65                  70                  75                  80

att gaa ttc ctg acc tca att gat ccg act gcc tcg gcc tcc gga agt      288
Ile Glu Phe Leu Thr Ser Ile Asp Pro Thr Ala Ser Ala Ser Gly Ser

85                  90                  95

gct ggg att aca ggc acc atg agc cag gac acc gag gtg gat atg aag          336
Ala Gly Ile Thr Gly Thr Met Ser Gln Asp Thr Glu Val Asp Met Lys
            100                 105                 110

gag gtg gag ctg aat gag tta gag ccc gag aag cag ccg atg aac gcg          384
Glu Val Glu Leu Asn Glu Leu Glu Pro Glu Lys Gln Pro Met Asn Ala
        115                 120                 125

gcg tct ggg gcg gcc atg tcc ctg gcg gga gcc gag aag aat ggt ctg          432
Ala Ser Gly Ala Ala Met Ser Leu Ala Gly Ala Glu Lys Asn Gly Leu
    130                 135                 140

gtg aag atc aag gtg gcg gaa gac gag gcg gag gcg gca gcc gcg gct          480
Val Lys Ile Lys Val Ala Glu Asp Glu Ala Glu Ala Ala Ala Ala Ala
145                 150                 155                 160

aag ttc acg ggc ctg tcc aag gag gag ctg ctg aag gtg gca ggc agc          528
Lys Phe Thr Gly Leu Ser Lys Glu Glu Leu Leu Lys Val Ala Gly Ser
                165                 170                 175

ccc ggc tgg gta cgc acc cgc tgg gca ctg ctg ctg ctc ttc tgg ctc          576
Pro Gly Trp Val Arg Thr Arg Trp Ala Leu Leu Leu Leu Phe Trp Leu
            180                 185                 190

ggc tgg ctc ggc atg ctt gct ggt gcc gtg gtc ata atc gtg cga gcg          624
Gly Trp Leu Gly Met Leu Ala Gly Ala Val Val Ile Ile Val Arg Ala
        195                 200                 205

ccg cgt tgt cgc gag cta ccg gcg cag aag tgg tgg cac acg ggc gcc          672
Pro Arg Cys Arg Glu Leu Pro Ala Gln Lys Trp Trp His Thr Gly Ala
    210                 215                 220

ctc tac cgc atc ggc gac ctt cag gcc ttc cag ggc cac ggc gcg ggc          720
Leu Tyr Arg Ile Gly Asp Leu Gln Ala Phe Gln Gly His Gly Ala Gly
225                 230                 235                 240

aac ctg gcg ggt ctg aag ggg cgt ctc gat tac ctg agc tct ctg aag          768
Asn Leu Ala Gly Leu Lys Gly Arg Leu Asp Tyr Leu Ser Ser Leu Lys
                245                 250                 255

gtg aag ggc ctt gtg ctg ggt cca att cac aag aac cag aag gat gat          816
Val Lys Gly Leu Val Leu Gly Pro Ile His Lys Asn Gln Lys Asp Asp
            260                 265                 270

gtc gct cag act gac ttg ctg cag atc gac ccc aat ttt ggc tcc aag          864
Val Ala Gln Thr Asp Leu Leu Gln Ile Asp Pro Asn Phe Gly Ser Lys
        275                 280                 285

gaa gat ttt gac agt ctc ttg caa tcg gct aaa aaa aag agc atc cgt          912
Glu Asp Phe Asp Ser Leu Leu Gln Ser Ala Lys Lys Lys Ser Ile Arg
    290                 295                 300

gtc att ctg gac ctt act ccc aac tac cgg ggt gag aac tcg tgg ttc          960
Val Ile Leu Asp Leu Thr Pro Asn Tyr Arg Gly Glu Asn Ser Trp Phe
305                 310                 315                 320

tcc act cag gtt gac act gtg gcc acc aag gtg aag gat gct ctg gag          1008
Ser Thr Gln Val Asp Thr Val Ala Thr Lys Val Lys Asp Ala Leu Glu
                325                 330                 335

ttt tgg ctg caa gct ggc gtg gat ggg ttc cag gtt cgg gac ata gag          1056

Phe Trp Leu Gln Ala Gly Val Asp Gly Phe Gln Val Arg Asp Ile Glu
            340                 345                 350

aat ctg aag gat gca tcc tca ttc ttg gct gag tgg caa aat atc acc      1104
Asn Leu Lys Asp Ala Ser Ser Phe Leu Ala Glu Trp Gln Asn Ile Thr
        355                 360                 365

aag ggc ttc agt gaa gac agg ctc ttg att gcg ggg act aac tcc tcc      1152
Lys Gly Phe Ser Glu Asp Arg Leu Leu Ile Ala Gly Thr Asn Ser Ser
    370                 375                 380

gac ctt cag cag atc ctg agc cta ctc gaa tcc aac aaa gac ttg ctg      1200
Asp Leu Gln Gln Ile Leu Ser Leu Leu Glu Ser Asn Lys Asp Leu Leu
385                 390                 395                 400

ttg act agc tca tac ctg tct gat tct ggt tct act ggg gag cat aca      1248
Leu Thr Ser Ser Tyr Leu Ser Asp Ser Gly Ser Thr Gly Glu His Thr
                405                 410                 415

aaa tcc cta gtc aca cag tat ttg aat gcc act ggc aat cgc tgg tgc      1296
Lys Ser Leu Val Thr Gln Tyr Leu Asn Ala Thr Gly Asn Arg Trp Cys
            420                 425                 430

agc tgg agt ttg tct cag gca agg ctc ctg act tcc ttc ttg ccg gct      1344
Ser Trp Ser Leu Ser Gln Ala Arg Leu Leu Thr Ser Phe Leu Pro Ala
        435                 440                 445

caa ctt ctc cga ctc tac cag ctg atg ctc ttc acc ctg cca ggg acc      1392
Gln Leu Leu Arg Leu Tyr Gln Leu Met Leu Phe Thr Leu Pro Gly Thr
    450                 455                 460

cct gtt ttc agc tac ggg gat gag att ggc ctg gat gca gct gcc ctt      1440
Pro Val Phe Ser Tyr Gly Asp Glu Ile Gly Leu Asp Ala Ala Ala Leu
465                 470                 475                 480

cct gga cag cct atg gag gct cca gtc atg ctg tgg gat gag tcc agc      1488
Pro Gly Gln Pro Met Glu Ala Pro Val Met Leu Trp Asp Glu Ser Ser
                485                 490                 495

ttc cct gac atc cca ggg gct gta agt gcc aac atg act gtg aag ggc      1536
Phe Pro Asp Ile Pro Gly Ala Val Ser Ala Asn Met Thr Val Lys Gly
            500                 505                 510

cag agt gaa gac cct ggc tcc ctc ctt tcc ttg ttc cgg cgg ctg agt      1584
Gln Ser Glu Asp Pro Gly Ser Leu Leu Ser Leu Phe Arg Arg Leu Ser
        515                 520                 525

gac cag cgg agt aag gag cgc tcc cta ctg cat ggg gac ttc cac gcg      1632
Asp Gln Arg Ser Lys Glu Arg Ser Leu Leu His Gly Asp Phe His Ala
    530                 535                 540

ttc tcc gct ggg cct gga ctc ttc tcc tat atc cgc cac tgg gac cag      1680
Phe Ser Ala Gly Pro Gly Leu Phe Ser Tyr Ile Arg His Trp Asp Gln
545                 550                 555                 560

aat gag cgt ttt ctg gta gtg ctt aac ttt ggg gat gtg ggc ctc tcg      1728
Asn Glu Arg Phe Leu Val Val Leu Asn Phe Gly Asp Val Gly Leu Ser
                565                 570                 575

gct gga ctg cag gcc tcc gac ctg cct gcc agc gcc agc ctg cca gcc      1776
Ala Gly Leu Gln Ala Ser Asp Leu Pro Ala Ser Ala Ser Leu Pro Ala
            580                 585                 590

aag gct gac ctc ctg ctc agc acc cag cca ggc cgt gag gag ggc tcc 1824
Lys Ala Asp Leu Leu Leu Ser Thr Gln Pro Gly Arg Glu Glu Gly Ser
        595                 600                 605

cct ctt gag ctg gaa cgc ctg aaa ctg gag cct cac gaa ggg ctg ctg 1872
Pro Leu Glu Leu Glu Arg Leu Lys Leu Glu Pro His Glu Gly Leu Leu
    610                 615                 620

ctc cgc ttc ccc tac gcg gcc tga 1896
Leu Arg Phe Pro Tyr Ala Ala
625                 630

<210> 39
<211> 631
<212> PRT
<213> Homo sapiens

<400> 39

Met Glu Leu Gln Pro Pro Glu Ala Ser Ile Ala Val Val Ser Ile Pro
1               5                   10                  15

Arg Gln Leu Pro Gly Ser His Ser Glu Ala Gly Val Gln Gly Leu Ser
            20                  25                  30

Ala Gly Asp Asp Ser Glu Thr Gly Ser Asp Cys Val Thr Gln Ala Gly
        35                  40                  45

Leu Gln Leu Leu Ala Ser Ser Asp Pro Pro Ala Leu Ala Ser Lys Asn
    50                  55                  60

Ala Glu Val Thr Val Glu Thr Gly Phe His His Val Ser Gln Ala Asp
65                  70                  75                  80

Ile Glu Phe Leu Thr Ser Ile Asp Pro Thr Ala Ser Ala Ser Gly Ser
                85                  90                  95

Ala Gly Ile Thr Gly Thr Met Ser Gln Asp Thr Glu Val Asp Met Lys
            100                 105                 110

Glu Val Glu Leu Asn Glu Leu Glu Pro Glu Lys Gln Pro Met Asn Ala
        115                 120                 125

Ala Ser Gly Ala Ala Met Ser Leu Ala Gly Ala Glu Lys Asn Gly Leu
    130                 135                 140

Val Lys Ile Lys Val Ala Glu Asp Glu Ala Glu Ala Ala Ala Ala Ala
145                 150                 155                 160

Lys Phe Thr Gly Leu Ser Lys Glu Glu Leu Leu Lys Val Ala Gly Ser
                165                 170                 175

Pro Gly Trp Val Arg Thr Arg Trp Ala Leu Leu Leu Leu Phe Trp Leu
        180                 185                 190

Gly Trp Leu Gly Met Leu Ala Gly Ala Val Val Ile Ile Val Arg Ala
    195                 200                 205

Pro Arg Cys Arg Glu Leu Pro Ala Gln Lys Trp Trp His Thr Gly Ala
    210                 215                 220

Leu Tyr Arg Ile Gly Asp Leu Gln Ala Phe Gln Gly His Gly Ala Gly
225                 230                 235                 240

Asn Leu Ala Gly Leu Lys Gly Arg Leu Asp Tyr Leu Ser Ser Leu Lys
                245                 250                 255

Val Lys Gly Leu Val Leu Gly Pro Ile His Lys Asn Gln Lys Asp Asp
            260                 265                 270

Val Ala Gln Thr Asp Leu Leu Gln Ile Asp Pro Asn Phe Gly Ser Lys
        275                 280                 285

Glu Asp Phe Asp Ser Leu Leu Gln Ser Ala Lys Lys Lys Ser Ile Arg
    290                 295                 300

Val Ile Leu Asp Leu Thr Pro Asn Tyr Arg Gly Glu Asn Ser Trp Phe
305                 310                 315                 320

Ser Thr Gln Val Asp Thr Val Ala Thr Lys Val Lys Asp Ala Leu Glu
                325                 330                 335

Phe Trp Leu Gln Ala Gly Val Asp Gly Phe Gln Val Arg Asp Ile Glu
            340                 345                 350

Asn Leu Lys Asp Ala Ser Ser Phe Leu Ala Glu Trp Gln Asn Ile Thr
        355                 360                 365

Lys Gly Phe Ser Glu Asp Arg Leu Leu Ile Ala Gly Thr Asn Ser Ser
    370                 375                 380

Asp Leu Gln Gln Ile Leu Ser Leu Leu Glu Ser Asn Lys Asp Leu Leu
385                 390                 395                 400

Leu Thr Ser Ser Tyr Leu Ser Asp Ser Gly Ser Thr Gly Glu His Thr
                405                 410                 415

Lys Ser Leu Val Thr Gln Tyr Leu Asn Ala Thr Gly Asn Arg Trp Cys
            420                 425                 430

Ser Trp Ser Leu Ser Gln Ala Arg Leu Leu Thr Ser Phe Leu Pro Ala
        435                 440                 445

Gln Leu Leu Arg Leu Tyr Gln Leu Met Leu Phe Thr Leu Pro Gly Thr
    450                 455                 460

Pro Val Phe Ser Tyr Gly Asp Glu Ile Gly Leu Asp Ala Ala Ala Leu
465                 470                 475                 480

Pro Gly Gln Pro Met Glu Ala Pro Val Met Leu Trp Asp Glu Ser Ser
                485                 490                 495

Phe Pro Asp Ile Pro Gly Ala Val Ser Ala Asn Met Thr Val Lys Gly
            500                 505                 510

Gln Ser Glu Asp Pro Gly Ser Leu Leu Ser Leu Phe Arg Arg Leu Ser
        515                 520                 525

Asp Gln Arg Ser Lys Glu Arg Ser Leu Leu His Gly Asp Phe His Ala
    530                 535                 540

Phe Ser Ala Gly Pro Gly Leu Phe Ser Tyr Ile Arg His Trp Asp Gln
545                 550                 555                 560

Asn Glu Arg Phe Leu Val Val Leu Asn Phe Gly Asp Val Gly Leu Ser
                565                 570                 575

Ala Gly Leu Gln Ala Ser Asp Leu Pro Ala Ser Ala Ser Leu Pro Ala
            580                 585                 590

Lys Ala Asp Leu Leu Leu Ser Thr Gln Pro Gly Arg Glu Glu Gly Ser
        595                 600                 605

Pro Leu Glu Leu Glu Arg Leu Lys Leu Glu Pro His Glu Gly Leu Leu
    610                 615                 620

Leu Arg Phe Pro Tyr Ala Ala
625                 630